# EUROPEAN PATENT APPLICATION

(11) **EP 4 098 747 A1**
(43) Date of publication of application: **07.12.2022**
(21) Application number: 21747552.4
(22) Date of filing: 29.01.2021
(51) Int. Cl.: C12N 15/113, A61K 31/7088, A61K 31/711, A61K 31/7115, A61K 31/712, A61K 31/7125, A61K 48/00, A61P 25/00, A61P 25/08, A61P 25/14, A61P 25/18, A61P 25/28, A61P 43/00, C12N 5/10, C12N 15/11, C12N 15/12

(54) **ANTISENSE OLIGONUCLEOTIDE OF ATN1**

(30) Priority: 31.01.2020 JP 2020015521; 02.11.2020 JP 2020183718
(71) Applicant: SANWA KAGAKU KENKYUSHO CO., LTD., Aichi 461-8631 (JP); Nissan Chemical Corporation, Tokyo 103-6119 (JP)
(72) Inventor: HASHIMOTO Shinji, Inabe-shi, Mie 511-0406 (JP); GOTO Izumi, Inabe-shi, Mie 511-0406 (JP); IRIYAMA Yusuke, Funabashi-shi, Chiba 274-8507 (JP)
(74) Representative: dompatent von Kreisler Selting Werner - Partnerschaft von Patent- und Rechtsanwälten mbB
(86) International application number: PCT/JP2021/003293
(87) International publication number: WO 2021/153747

(57) **Abstract**

Provided is an antisense oligonucleotide capable of controlling ATN1 gene expression and treating dentatorubral-pallidoluysian atrophy.

A compound comprising a modified oligonucleotide consisting of 8 to 80 linked nucleosides and having a nucleobase sequence including at least 8 contiguous nucleobases that are complementary to a transcript of ATN1, or a pharmacologically acceptable salt thereof.

## Description

### Technical Field

The present invention relates to an antisense oligonucleotide of ATN1.

### Background Art

Dentatorubral-pallidoluysian atrophy (DRPLA) is an autosomal dominant spinocerebellar degeneration with lesions in a cerebellar dentate nucleus, a red nucleus, a pallidus, and a Luys body, caused by abnormal elongation of CAG repeats of an ATN1 gene. DRPLA is a progressive disease that causes ataxia, myoclonus, epilepsy, and progressive decline in intelligence in children, and ataxia, choreoathetosis, dementia, and personality change in adults. Onset ages widely range from less than one year to the seventies, with an average onset age of 31.5 years. It is known that the severity of clinical symptoms correlates with the number of CAG repeats (see, for example, Non Patent Literature 1).

As a substance that inhibits expression of an ATN1 gene, an RNaseH-independent antisense oligonucleotide against CAG repeats (see, for example, Non Patent Literature 2), ss-siRNA (see, for example, Patent Literature 1 and Non Patent Literature 3), and the like have been reported, and it is indicated that the substances selectively inhibit an allele in which repeats are abnormally elongated. In addition, it has been reported that a morpholino nucleic acid or an RNaseH-dependent LNA gapmer inhibits expression of an ATN1 gene, but the sequence thereof and the degree of inhibition are not described (see, for example, Non Patent Literature 4).

### Citation List

### Patent Literature

Patent Literature 1: WO 2015/017675 A

### Non Patent Literature

Non Patent Literature 1: Movement Disorders, 2010 Aug 15, 25(11), pp 1694-700
Non Patent Literature 2: PLoS One, 2011, 6(9), e24308.
Non Patent Literature 3: Biochemistry, 2014 Jul 22, 53(28), pp 4510-8
Non Patent Literature 4: The 38th Annual Meeting of the Japan Neuroscience Society 2015 2P107

### Summary of Invention

### Technical Problem

An object of the present invention is to provide a novel antisense oligonucleotide that inhibits expression of an ATN1 gene.

### Solution to Problem

The present inventors intensively conducted studies in order to find a compound having an antisense effect on ATN1, and as a result, have found that a compound of the present invention has an excellent ATN1 gene expression inhibitory action, thereby completing the present invention.

That is, the present invention has the following features.
1. A compound comprising a modified oligonucleotide which consists of 8 to 80 nucleosides and has a nucleobase sequence including at least 8 contiguous nucleobases of any one of nucleobase sequences of SEQ ID NOs: 3 to 716 and 718 to 770 (3 to 487, 488 to 716, and 718 to 770), or a pharmacologically acceptable salt thereof.
2. The compound or pharmacologically acceptable salt thereof according to 1, comprising a modified oligonucleotide which has a nucleobase sequence including any one of nucleobase sequences of SEQ ID NOs: 3 to 716 and 718 to 770 (3 to 487, 488 to 716, and 718 to 770).
3. The compound or pharmacologically acceptable salt thereof according to 1 or 2, comprising a modified oligonucleotide which has any one of nucleobase sequences of SEQ ID NOs: 3 to 716 and 718 to 770 (3 to 487, 488 to 716, and 718 to 770).
4. A compound comprising a modified oligonucleotide which consists of 8 to 80 nucleosides complementary to a part in a nucleobase sequence selected from the group consisting of nucleobase sequences represented by position numbers 59 to 104, 109 to 133, 173 to 192, 207 to 272, 300 to 319, 353 to 372, 419 to 434, 458 to 509, 523 to 559, 561 to 618, 626 to 685, 766 to 785, 787 to 819, 838 to 855, 880 to 922, 924 to 943, 970 to 989, 994 to 1039, 1055 to 1074, 1079 to 1098, 1157 to 1176, 1196 to 1223, 1310 to 1329, 1339 to 1399, 1423 to 1442, 1614 to 1633, 1650 to 1670, 1806 to 1833, 1844 to 1865, 1896 to 1915, 1924 to 1992, 2023 to 2042, 2058 to 2081, 2103 to 2124, 2398 to 2417, 2480 to 2520, 2526 to 2562, 2568 to 2587, 2853 to 2872, 2876 to 2923, 2931 to 2950, 2972 to 3016, 3072 to 3099, 3109 to 3128, 3192 to 3211, 3267 to 3290, 3333 to 3379, 3419 to 3557, 3765 to 3784, 3789 to 3853, 3888 to 3926, 4022 to 4062, 4118 to 4139, 4141 to 4156, 4218 to 4236, and 4266 to 4281 of nucleobases of SEQ ID NO: 1, wherein the modified oligonucleotide is at least 80% complementary to the part in the selected nucleobase sequence of SEQ ID NO: 1, or a pharmacologically acceptable salt thereof.
5. The compound or pharmacologically acceptable salt thereof according to 4, comprising a modified oligonucleotide which consists of 8 to 80 nucleosides complementary to a part in a nucleobase sequence represented by position numbers 3419 to 3557 of nucleobases of SEQ ID NO: 1, wherein the modified oligonucleotide is at least 80% complementary to the part in the nucleobase sequence represented by position numbers 3419 to 3557 of the nucleobases of SEQ ID NO: 1.
6. A compound comprising a modified oligonucleotide which consists of 8 to 80 nucleosides complementary to a part in a nucleobase sequence selected from the group consisting of nucleobase sequences represented by position numbers 383 to 398, 986 to 1001, 1004 to 1019, 1378 to 1393, 1398 to 1413, 1853 to 1868, 1971 to 1986, 2189 to 2204, 2522 to 2537, 2562 to 2577, 3662 to 3677, 3987 to 4002, 4217 to 4232, 4266 to 4281, 4335 to 4350, 4360 to 4375, 4477 to 4492, 4562 to 4577, 4636 to 4651, 4687 to 4702, 4734 to 4749, 4840 to 4855, 5536 to 5563, 5568 to 5592, 5632 to 5651, 5666 to 5715, 5906 to 5925, 5959 to 5978, 6176 to 6191, 6215 to 6266, 6963 to 6978, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9336 to 9351, 9534 to 9570, 9576 to 9595, 9923 to 9938, 10001 to 10016, 10056 to 10071, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12508 to 12680, 12723 to 12738, 13454 to 13518, 13553 to 13591, 13687 to 13727, 13783 to 13804, 13806 to 13821, 13883 to 13901, and 13931 to 13946 of nucleobases of SEQ ID NO: 2, wherein the modified oligonucleotide is at least 80% complementary to the part in the selected nucleobase sequence of SEQ ID NO: 2, or a pharmacologically acceptable salt thereof.
7. The compound or pharmacologically acceptable salt thereof according to 6, comprising a modified oligonucleotide which consists of 8 to 80 nucleosides complementary to a part in a nucleobase sequence selected from the group consisting of nucleobase sequences represented by position numbers 5536 to 5563, 5568 to 5592, 5632 to 5651, 5666 to 5715, 5906 to 5925, 5959 to 5978, 6176 to 6191, 6215 to 6266, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9534 to 9570, 9576 to 9595, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12572 to 12680, 13454 to 13518, 13553 to 13591, 13687 to 13727, 13783 to 13804, 13806 to 13821, 13883 to 13901, and 13931 to 13946 of nucleobases of SEQ ID NO: 2, wherein the modified oligonucleotide is at least 80% complementary to the part in the selected nucleobase sequence of SEQ ID NO: 2.
8. A compound comprising a modified oligonucleotide which consists of 8 to 80 nucleosides complementary to a part in a nucleobase sequence selected from the group consisting of nucleobase sequences represented by position numbers 4320 to 4390 and 12508 to 12680 of nucleobases of SEQ ID NO: 2, wherein the modified oligonucleotide is at least 80% complementary to the part in the selected nucleobase sequence of SEQ ID NO: 2, or a pharmacologically acceptable salt thereof.
9. The compound or pharmacologically acceptable salt thereof according to 6 or 7, comprising a modified oligonucleotide which consists of 8 to 80 nucleosides complementary to a part in a nucleobase sequence selected from the group consisting of nucleobase sequences represented by position numbers 10840 to 10865 and 12572 to 12680 of nucleobases of SEQ ID NO: 2, wherein the modified oligonucleotide is at least 80% complementary to the part in the selected nucleobase sequence of SEQ ID NO: 2.
10. The compound or pharmacologically acceptable salt thereof according to any one of 1 to 9, comprising a modified oligonucleotide which consists of 8 to 80 nucleosides and has a nucleobase sequence including at least 8 contiguous nucleobases of any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 494, 513, 515, 533, 534, 544, 551, 552, 554, 566, 574, 582, 585, 586, 589, 592, 593, 595, 596, 638, 639, 642, 644, 661, 662, 663, 664, 665, 666, 667, 668, 669,670, 671, 672, 673, 674, 675, 676, 677, 696, 697, 699, 706, 713, 715, 716, 722, 723, 724, 727, 728, 729, 730, 731, 732, 733, 734, 735, 736, 737, 738, 739, 740, 741, 742, 743, 744, 745, 746, 748, 750, 751, 752, 753, 754, 755, 756, 757, 758, 759, 760, 761, 762, 763, 764, 765, 766, 767, 768, 769, and 770.
11. The compound or pharmacologically acceptable salt thereof according to any one of 1 to 10, comprising a modified oligonucleotide which consists of 8 to 80 nucleosides and has a nucleobase sequence including at least 8 contiguous nucleobases of any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.
12. The compound or pharmacologically acceptable salt thereof according to any one of 1 to 11, comprising a modified oligonucleotide which consists of 12 to 80 nucleosides and has a nucleobase sequence including any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 488, 489, 490, 491, 492, 494, 513, 515, 533, 534, 544, 551, 552, 554, 566, 574, 582, 585, 586, 589, 592, 593, 595, 596, 638, 639, 642, 644, 661, 662, 663, 664, 665, 666, 667, 668, 669,670, 671, 672, 673, 674, 675, 676, 677, 696, 697, 699, 706, 713, 715, 716, 722, 723, 724, 727, 728, 729, 730, 731, 732, 733, 734, 735, 736, 737, 738, 739, 740, 741, 742, 743, 744, 745, 746, 748, 750, 751, 752, 753, 754, 755, 756, 757, 758, 759, 760, 761, 762, 763, 764, 765, 766, 767, 768, 769, and 770.
13. The compound or pharmacologically acceptable salt thereof according to any one of 1 to 12, comprising a modified oligonucleotide which consists of 12 to 80 nucleosides and has a nucleobase sequence including any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, and 241.
14. The compound or pharmacologically acceptable salt thereof according to any one of 1 to 11, comprising a modified oligonucleotide which consists of 16 to 80 nucleosides and has a nucleobase sequence including any one of nucleobase sequences selected from the group consisting of SEQ ID NOs: 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.
15. The compound or pharmacologically acceptable salt thereof according to any one of 1 to 14, wherein the modified oligonucleotide includes a phosphorothioate bond.
16. The compound or pharmacologically acceptable salt thereof according to any one of 1 to 15, wherein the modified oligonucleotide includes at least one nucleoside selected from the group consisting of a 2'-modified nucleoside and a 2'-4'-bridged nucleoside.
17. The compound or pharmacologically acceptable salt thereof according to 16, wherein the 2'-4'-bridged nucleoside is at least one selected from the group consisting of LNA, ENA, cEt, AmNA, scpBNA, and GuNA.
18. The compound or pharmacologically acceptable salt thereof according to 17, wherein the 2'-4'-bridged nucleoside is LNA.
19. The compound or pharmacologically acceptable salt thereof according to any one of 16 to 18, wherein the 2'-modified nucleoside is at least one selected from the group consisting of a 2'-O-MCE nucleoside, a 2'-O-MOE nucleoside, a 2'-O-NMA nucleoside, and a 2'-O-Me nucleoside.
20. The compound or pharmacologically acceptable salt thereof according to 19, wherein the 2'-modified nucleoside is at least one selected from the group consisting of a 2'-O-MCE nucleoside and a 2'-O-MOE nucleoside.
21. The compound or pharmacologically acceptable salt thereof according to any one of 1 to 20, wherein the modified oligonucleotide includes 5-methylcytosine.
22. The compound or pharmacologically acceptable salt thereof according to any one of 1 to 21, wherein
   the modified oligonucleotide includes a gap segment, a 5' wing segment, and a 3' wing segment,
   the gap segment includes at least two deoxyribonucleosides, and a 5'-terminal and a 3'-terminal of the gap segment are deoxyribonucleosides,
   the nucleoside at the 3'-terminal of the 5' wing segment is a sugar-modified nucleoside and is linked to the 5'-terminal of the gap segment, and
   the nucleoside at the 5'-terminal of the 3' wing segment is a sugar-modified nucleoside and is linked to the 3'-terminal of the gap segment.
23. The compound or pharmacologically acceptable salt thereof according to 22, wherein
   the gap segment consists of 5 to 30 deoxyribonucleosides,
   the 5' wing segment and the 3' wing segment each independently consist of 1 to 10 sugar-modified nucleosides independently selected from the group consisting of LNA, a 2'-O-MCE nucleoside, and a 2'-O-MOE nucleoside,
   each of the wing segments includes at least one phosphorothioate bond, and
   each of cytosines of the gap segment and each wing segment is replaced with 5-methylcytosine.
24. The compound or pharmacologically acceptable salt thereof according to 23, wherein
   the gap segment consists of 8 to 12 deoxyribonucleosides,
   the 5' wing segment and the 3' wing segment each independently consist of 2 to 5 sugar-modified nucleosides independently selected from the group consisting of LNA and a 2'-O-MCE nucleoside, and include at least one 2'-O-MCE nucleoside, and
   the gap segment includes at least one phosphorothioate bond.
25. The compound or pharmacologically acceptable salt thereof according to 23, wherein
   the gap segment consists of 8 to 12 deoxyribonucleosides,
   the 5' wing segment and the 3' wing segment each independently consist of 3 to 6 sugar-modified nucleosides selected from the group consisting of a 2'-O-MOE nucleoside and a 2'-O-MCE nucleoside, and
   the gap segment includes at least one phosphorothioate bond.
26. The compound or pharmacologically acceptable salt thereof according to 25, wherein the 5' wing segment and the 3' wing segment each independently consist of five 2'-O-MOE nucleosides.
27. The compound or pharmacologically acceptable salt thereof according to 25, wherein the 5' wing segment and the 3' wing segment each independently consist of five 2'-O-MCE nucleosides.
28. The compound or pharmacologically acceptable salt thereof according to 24, wherein the 5' wing segment and the 3' wing segment are each independently selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, and VVVL, in which L represents LNA, and V represents a 2'-O-MCE nucleoside.
29. The compound or pharmacologically acceptable salt thereof according to 24, wherein the 5' wing segment and the 3' wing segment are each independently selected from the group consisting of VLL, LVL, LLV, LVV, VLV, VVL, VVLL, VLVL, LVLV, LLVV, VLLL, LVLL, LLVL, LLLV, LVVV, VLVV, VVLV, and VVVL, in which L represents LNA, and V represents a 2'-O-MCE nucleoside.
30. The compound or pharmacologically acceptable salt thereof according to any one of 1 to 29, wherein the modified oligonucleotide consists of 15 to 25 nucleosides.
31. The compound or pharmacologically acceptable salt thereof according to any one of 1 to 30, wherein the modified oligonucleotide is an antisense oligonucleotide.
32. The compound or pharmacologically acceptable salt thereof according to any one of 1 to 31, wherein the compound including the modified oligonucleotide includes a prodrug moiety.
33. The compound or pharmacologically acceptable salt thereof according to any one of 1 to 32, wherein the compound including the modified oligonucleotide includes a functional molecule.
34. The compound or pharmacologically acceptable salt thereof according to any one of 1 to 33, consisting of the modified oligonucleotide.
35. The salt according to any one of 1 to 34, wherein the pharmacologically acceptable salt is a sodium salt.
36. A medicinal drug containing, as an active ingredient, the compound or pharmacologically acceptable salt according to any one of 1 to 35.
37. A drug for treating, preventing, and/or ameliorating a disease or a condition to which an ATN1 gene expression inhibitory action is effective, the drug comprising, as an active ingredient, the compound or pharmacologically acceptable salt according to any one of t 1 to 35.
38. An ATN1 gene expression inhibitor comprising, as an active ingredient, the compound or pharmacologically acceptable salt according to any one of 1 to 35.
39. A drug for treating, preventing, and/or ameliorating dentatorubral-pallidoluysian atrophy, the drug containing, as an active ingredient, the compound or pharmacologically acceptable salt according to any one of 1 to 35.

### Advantageous Effects of Invention

The present invention can provide a novel antisense oligonucleotide which has an excellent ATN1 gene expression inhibitory action and is useful particularly for treating, ameliorating, and/or preventing dentatorubral-pallidoluysian atrophy.

### Description of Embodiments

It is understood that both the above summary and the following detailed description are exemplary and explanatory only and do not limit the present invention as claimed. Herein, a singular form includes a plural form unless otherwise specified.

In the present description, an antisense oligonucleotide may be referred to as "ASO".

A nucleic acid in nature is most basically composed of adenosine (A), thymidine (T) (or uridine (U)), cytidine (C), and guanosine (G). These basic nucleic acids are often referred to as AT(U)GC and the like. Therefore, herein, for example, regarding a sequence of an ATN1 gene and the like, when the sequence is indicated by "nucleobase sequence" or "SEQ ID NO", the sequence is basically a sequence of A, T, G, C, and U.

On the other hand, a nucleic acid constituting an ASO of the present invention also includes those in which the basic nucleic acids (AT(U)CG) are structurally modified in addition to the basic nucleic acids (AT(U)CG). Although details of the modification will be described later, the modification includes modification of a sugar moiety, an internucleoside bond, a nucleobase, and/or the like.

Therefore, herein, for example, for the ASO or the like of the present invention, when a nucleobase sequence is described with A, T, G, C, and U in description of "compound" or "compound to which a compound number (M-number and L-number) is imparted", A, T, G, C, and U also include those in which A, T, G, C, and U are structurally modified.

Hereinafter, the present invention will be described in more detail.

The following terms have the following meanings unless otherwise specified.

"May be substituted" means having no substituent or having a substituent.

"Nucleoside" is a term well known to a person skilled in the art, and is generally understood as a molecule to which a sugar and a nucleobase are bonded and which can be a unit constituting a nucleic acid. Herein, the nucleoside is a broader concept, and includes a deoxyribonucleoside, a ribonucleoside, and a sugar-modified nucleoside described later. The nucleobase may be modified.

"Deoxyribonucleoside" means a molecule having a nucleobase on a carbon atom at a 1'-position of 2'-deoxyribose. The deoxyribonucleoside in the present invention may be a naturally occurring deoxyribonucleoside or a deoxyribonucleoside in which a nucleobase moiety of a naturally occurring deoxyribonucleoside is modified. A plurality of modifications may be made to one deoxyribonucleoside in combination. The modified deoxyribonucleoside is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A and the like.

"Ribonucleoside" means a molecule having a nucleobase on a carbon atom at a 1'-position of ribose. The ribonucleoside in the present invention may be a naturally occurring ribonucleoside or a ribonucleoside in which a nucleobase moiety of a naturally occurring ribonucleoside is modified. A plurality of modifications may be made to one ribonucleoside in combination. The modified ribonucleoside is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A and the like.

"Modified sugar" means
(Z1) a molecule in which ribose or 2'-deoxyribose is partially substituted with one or more substituents,
(Z2) a pentasaccharide or a hexasaccharide different from ribose and 2'-deoxyribose (for example, hexitol, threose, or the like),
(Z3) a molecule in which the entire ribose or 2'-deoxyribose or a tetrahydrofuran ring thereof is replaced with a 5- to 7-membered saturated or unsaturated ring (for example, cyclohexane, cyclohexene, morpholine, or the like) or a partial structure in which a 5- to 7-membered ring can be formed by a hydrogen bond (for example, a peptide structure),
   or
(Z4) a molecule in which ribose or 2'-deoxyribose is replaced with an alkylene glycol having 2 to 6 carbon atoms (for example, ethylene glycol, propylene glycol, or the like).

The modified sugar includes a "2'-modified sugar" and a "2'-4'-bridged sugar" described later.

Examples of the modified sugar and the sugar-modified nucleoside described later include sugars, sugar-modified nucleosides, and the like disclosed as being preferably used in an antisense method in JP H10-304889 A, WO 2005/021570 A, JP H10-195098 A, JP 2002-521310 A, WO 2007/143315 A, WO 2008/043753 A, WO 2008/029619 A, WO 2008/049085 A, WO 2017/142054 A and the like(hereinafter, these literatures are referred to as "literatures related to an antisense method"). The modified sugar and the sugar-modified nucleoside are also disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

Examples of a modified sugar that is partially substituted with one substituent include ribose or 2'-deoxyribose in which any position of a sugar moiety is substituted with the following (i) or (ii).
(i) C₁₋₆ alkyl group.
(ii) A C₁₋₆ alkyl group substituted with at least one selected from the group consisting of a halogen atom, a C₁₋₆ alkoxy group, a halo C₁₋₆ alkoxy group, a mono- or di-C₁₋₆ alkylamino group, a 5- to 10-membered heterocyclic group, a carboxy group, a carbamoyl group, and an N-substituted carbamoyl group.

Here, examples of the N-substituted carbamoyl group include an N-methyl-carbamoyl group and an N-ethyl-carbamoyl group, and the methyl group and the ethyl group of the N-methyl-carbamoyl group and the N-ethyl-carbamoyl group may be substituted with a 5- to 10-membered heterocyclic group or a mono- or di-C₁₋₆ alkylamino group. Specific examples of the N-substituted carbamoyl group include a N-methylcarbamoyl group, a N-ethylcarbamoyl group, a N-dimethylaminoethyl-carbamoyl group, a N-morpholinoethylcarbamoyl group, a N-(2-pyridylethyl) carbamoyl group, a N-((benzimidazol-1-yl) ethyl) carbamoyl group, and the like.

"Sugar-modified nucleoside" means a molecule having the "modified sugar" instead of a sugar moiety of a deoxyribonucleoside or a ribonucleoside. For example, the sugar-modified nucleoside includes "2'-modified nucleoside" and "2'-4'-bridged nucleoside" described later. When the modified sugar is (Z3) as defined above, the sugar-modified nucleoside also includes a molecule in which a modified sugar and a nucleobase are bonded to each other via a methylene chain or the like.

"2'-Modified sugar" means a non-bridged sugar in which an oxygen atom or a carbon atom at a 2'-position of ribose is modified, and includes "2'-O-Me", "2'-O-MOE", "2'-O-MCE", "2'-O-NMA", "2'-DMAECE", "2'-MorECE", "2'-PyECE", and "BimECE".

"2'-Modified nucleoside" means a molecule having a nucleobase at a 1'-position of the 2'-modified sugar, and examples thereof include "2'-O-Me nucleoside", "2'-O-MOE nucleoside", "2'-O-MCE nucleoside", "2'-O-NMA nucleoside", "2'-DMAECE nucleoside", "2'-MorECE nucleoside", "2'-PyECE nucleoside", and "BimECE nucleoside".

"2'-O-Me" (also referred to as 2'-O-methyl) means a sugar in which a hydroxy group at a 2'-position of ribose is replaced with a methoxy group.

"2'-O-Me nucleoside" (also referred to as 2'-O-methyl nucleoside) means a molecule having a nucleobase at a 1'-position of "2'-O-Me".

"2'-O-MOE" (also referred to as 2'-O-methoxyethyl) means a sugar in which a hydroxy group at a 2'-position of ribose is replaced with a 2-methoxyethyloxy group.

"2'-O-MOE nucleoside" (also referred to as 2'-O-methoxyethyl nucleoside) means a molecule having a nucleobase at a 1'-position of "2'-O-MOE".

"2'-O-MCE" (also referred to as 2'-O-methylcarbamoylethyl) means a sugar in which a hydroxy group at a 2'-position of ribose is replaced with a methylcarbamoylethyloxy group.

"2'-O-MCE nucleoside" (also referred to as 2'-O-methylcarbamoylethyl nucleoside) means a molecule having a nucleobase at a 1'-position of "2'-O-MCE".

"2'-O-NMA" means a sugar in which a hydroxy group at a 2'-position of ribose is replaced with a [2-(methylamino)-2-oxoethyl] oxy group.

"2'-O-NMA nucleoside" means a molecule having a nucleobase at a 1'-position of "2'-O-NMA".

"2'-AP" ("2'-O-AP") means a sugar in which a hydroxy group at a 2'-position of ribose is replaced with a 3-aminopropyloxy group.

"2'-AP nucleoside" ("2'-O-AP nucleoside") means a molecule having a nucleobase at a 1'-position of "2'-AP".

"2'-F" means a sugar in which a hydroxy group at a 2'-position of ribose is replaced with a fluorine atom.

"2'-F nucleoside" means a molecule having a nucleobase at a 1'-position of "2'-F".

"2'-DMAECE" is a modified sugar having the following structure.

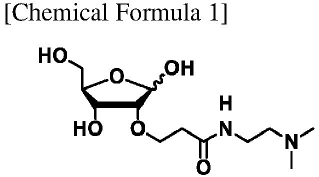

"2'-MorECE" is a modified sugar having the following structure.

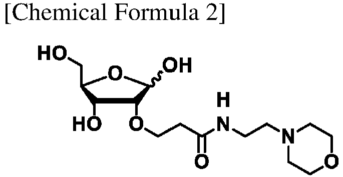

"2'-PyECE" is a modified sugar having the following structure.

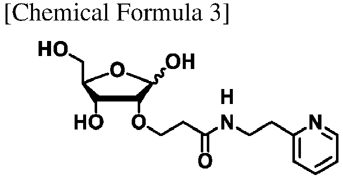

"BimECE" is a modified sugar having the following structure.

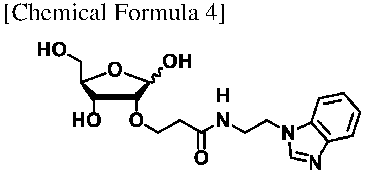

"2'-DMAECE nucleoside", "2'-MorECE nucleoside", "2'-PyECE nucleoside", and "BimECE nucleoside" mean molecules having a nucleobase at a 1'-position of "2'-DMAECE", "2'-MorECE", "2'-PyECE", and "BimECE", respectively.

"2'-4'-Bridged sugar" means a sugar substituted with a bridging unit at two positions of a 2'-position and a 4'-position in ribose. Examples of the bridging unit include a C₂₋₆ alkylene group (the alkylene group has no substituent or has one or more substituents selected from the group consisting of a halogen atom, an oxo group, and a thioxo group, and one or two methylene groups of the alkylene group are not replaced or independently replaced with a group selected from the group consisting of -O-, -S-, and -NR¹- (R¹ represents a hydrogen atom, a C₁₋₆ alkyl group, or a halo C₁₋₆ alkyl group)).

"2'-4'-bridged nucleoside" (2',4'-BNA) means a molecule having a nucleobase at a 1'-position of the 2'-4'-bridged sugar. Examples thereof include α-L-methyleneoxy (4'-CH₂-O-2') BNA or β-D-methyleneoxy (4'-CH₂-O-2') BNA also referred to as LNA (locked nucleic acid (registered trademark)) described later, ethyleneoxy (4'-(CH₂)₂-O-2') BNA also referred to as ENA, β-D-thio (4'-CH₂-S-2') BNA, aminooxy (4'-CH₂-O-N(R¹¹)-2') BNA (R¹¹ is H or CH₃), oxyamino (4'-CH₂-N(R¹²)-O-2') BNA (R¹² is H or CH₃) also referred to as 2',4'-BNA^{NC}, 2',4'-BNA^{COC}, 3'-amino-2',4'-BNA, 5'-methyl BNA, (4'-CH(CH₃)-O-2') BNA also referred to as cEt, (4'-CH(CH₂OCH₃)-O-2') BNA also referred to as cMOE-BNA, amide-based BNA (4'-C(=O)-N(R¹³)-2') BNA (R¹³ is H or CH₃) also referred to as AmNA, (4'-C (spiro-cyclopropyl)-O-2') BNA also referred to as scpBNA, (4'-CH₂-N(R¹⁴)-2') BNA (R¹⁴ is C(=NH₂⁺)NHR¹⁵ and R¹⁵ is H or CH₃) also referred to as GuNA, other BNAs known to a person skilled in the art, and the like.

In the "deoxyribonucleoside", "ribonucleoside", "2'-modified nucleoside", "2'-4'-bridged nucleoside", and the like, examples of a bond between a carbon atom at a 1'-position and a nucleobase include an α-glycosidic bond and a β-glycosidic bond, but the bond is usually a β-glycosidic bond.

"n-" means normal, "s-" means secondary, and "t-" means tertiary.

"Halogen atom" means a fluorine atom, a chlorine atom, a bromine atom, or an iodine atom.

The "C₁₋₆ alkyl group" means a linear or branched saturated hydrocarbon group having 1 to 6 carbon atoms, and examples thereof include a methyl group, an ethyl group, a n-propyl group, an isopropyl group, a n-butyl group, an isobutyl group, a s-butyl group, a t-butyl group, a n-pentyl group, an isopentyl group, a neopentyl group, a n-hexyl group, an isohexyl group, and the like.

"Halo C₁₋₆ alkyl group" means a group in which a hydrogen atom at any position of the "C₁₋₆ alkyl group" is substituted with one or more of the "halogen atoms".

"C₁₋₆ alkylene group" means a divalent group obtained by removing one hydrogen atom at any position from a linear or branched saturated hydrocarbon group having 1 to 6 carbon atoms, and examples thereof include a methylene group, an ethylene (ethanediyl) group, a propane-1,3-diyl (trimethylene) group, a propane-2,2-diyl group, a 2,2-dimethyl-propane-1,3-diyl group, a hexane-1,6-diyl (hexamethylene) group, a 3-methylbutane-1,2-diyl group, and the like.

"C₂₋₆ alkylene group" means a linear or branched divalent group having 2 to 6 carbon atoms in the "C₁₋₆ alkylene group", and examples thereof are similar to those of the "C₁₋₆ alkylene group" except for a methylene group.

"C₂₋₂₀ alkylene group" means a divalent group obtained by removing one hydrogen atom at any position from a linear or branched saturated hydrocarbon group having 2 to 20 carbon atoms. Similarly, "C₈₋₁₂ alkylene group" means a divalent group obtained by removing one hydrogen atom at any position from a linear or branched saturated hydrocarbon group having 8 to 12 carbon atoms.

"C₂₋₂₀ alkenylene group" means a divalent group obtained by removing one hydrogen atom at any position from a linear or branched unsaturated hydrocarbon group having 2 to 20 carbon atoms and containing at least one double bond.

"C₁₋₆ alkoxy group" means a group in which the "C₁₋₆ alkyl group" is bonded to an oxy group.

"Halo C₁₋₆ alkoxy group" means a group in which a hydrogen atom at any position of the "C₁₋₆ alkoxy group" is substituted with one or more of the "halogen atoms".

"Mono- or di-C₁₋₆ alkylamino group" means a group in which one hydrogen atom of an amino group is replaced with one "C₁₋₆ alkyl group" or a group in which two hydrogen atoms of an amino group are replaced with two identical or different "C₁₋₆ alkyl groups", and examples thereof include methylamino, ethylamino, propylamino, isopropylamino, butylamino, dimethylamino, diethylamino, dipropylamino, dibutylamino, a N-ethyl-N-methylamino group and the like.

"5- to 10-Membered heterocyclic group" means a 5- to 10-membered monocyclic or fused polycyclic aromatic or non-aromatic heterocyclic group containing 1 to 4 heteroatoms selected from a nitrogen atom, a sulfur atom, and an oxygen atom as ring-constituting atoms in addition to a carbon atom.

Preferable examples of the "5- to 10-membered heterocyclic group" include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, triazolyl, tetrazolyl, triazinyl, benzothiophenyl, benzofuranyl, benzimidazolyl, benzoxazolyl, benzisoxazolyl, benzothiazolyl, benzoisothiazolyl, benzotriazolyl, imidazolopyridinyl, thienopyridinyl, pyrrolopyridinyl, pyrazolopyridinyl, oxazolopyridinyl, thiazolopyridinyl, imidazopyradinyl, imidazolopyrimidinyl, aziridinyl, oxiranyl, azetidinyl, oxetanyl, thietanyl, tetrahydrothienyl, tetrahydrofuranyl, pyrrolinyl, pyrrolidinyl, oxopyrrolidinyl, imidazolinyl, oxoimidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, tetrahydropyridazinyl, dihydropyranyl, tetrahydropyranyl, tetrahydrothiopyranyl, morpholinyl, thiomorpholinyl and the like.

"Oxo group" refers to a group (= O) obtained by substitution with an oxygen atom via a double bond. When an oxo group is substituted with a carbon atom, the oxo group forms a carbonyl group together with the carbon atom.

"Thioxo group" refers to a group (= S) obtained by substitution with a sulfur atom via a double bond. When a thioxo group is substituted with a carbon atom, the thioxo group forms thiocarbonyl together with the carbon atom.

"Nucleobase" is a purine base or a pyrimidine base, and may be a naturally occurring nucleobase or one obtained by modifying a naturally occurring nucleobase. Examples of the naturally occurring nucleobase include adenine (A), guanine (G), thymine (T), cytosine (C), and uracil (U). The "nucleobase" includes the naturally occurring nucleobase and the "modified nucleobase" described later.

Examples of modification of a nucleobase in the "modified nucleobase" include halogenation, methylation, ethylation, n-propylation, isopropylation, cyclopropylation, n-butylation, isobutylation, s-butylation, t-butylation, cyclobutylation, hydroxylation, amination, thiation, demethylation, and the like. More specific examples thereof include: 5-methylation, 5-fluorination, 5-bromination, 5-iodination, and N4-methylation of cytosine; 2-thiation, 5-demethylation, 5-fluoration, 5-bromination, and 5-iodoation of thymine; 2-thiation, 5-fluorination, 5-bromination, and 5-iodination of uracil; N6-Methylation and 8-bromination of adenine; N2-methylation and 8-bromination of guanine; and the like. Examples of modification of a nucleobase moiety in a nucleoside are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

The nucleobase in a nucleoside is preferably at least one selected from the group consisting of adenine, guanine, thymine, cytosine, uracil, and 5-methylcytosine.

"5-Methylcytosine" means a cytosine having a methyl group at a 5-position.

"Nucleobase sequence" means a sequence from a 5' side to a 3' side of nucleobases of each nucleoside contained in an oligonucleotide.

"Contiguous nucleobases" means a sequence from a 5' side to a 3' side of nucleobases in a contiguous part of the "nucleobase sequence".

"Internucleoside bond" means a group or a bond that forms a covalent bond between adjacent nucleosides in an oligonucleotide. "Internucleoside bond" includes a phosphodiester bond and "modified internucleoside bond" described below.

"Modified internucleoside bond" means a modified phosphodiester bond, and examples thereof include a phosphorothioate bond, a methyl phosphonate bond (including a chiral-methyl phosphonate bond), a methyl thiophosphonate bond, a phosphorodithioate bond, a phosphoramidate bond, a phosphorodiamidate bond, a phosphoramidothioate bond, a boranophosphate bond, and the like. Examples of modification of a phosphodiester bond are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, and the like, and can be used for a modified phosphodiester bond.

"Modified nucleoside" means a nucleoside having a modified sugar moiety and/or a modified nucleobase.

"Oligonucleotide" means a molecule having a structure in which two or more identical or different "nucleosides" are independently linked to each other by the "internucleoside bond" (for example, a phosphodiester bond or a modified phosphodiester bond).

"Modified oligonucleotide" means an oligonucleotide including at least one selected from the group consisting of a modified internucleoside bond, a modified sugar, and a modified nucleobase.

"Compound comprising a modified oligonucleotide" means a compound comprising a modified oligonucleotide in a chemical structure thereof, and may be the modified oligonucleotide itself. Examples of the compound comprising a modified oligonucleotide include a compound in which a functional molecule is directly or indirectly bonded to the modified oligonucleotide as described later, a compound including a prodrug moiety described later, and the modified oligonucleotide itself.

"DNA" means a polynucleotide or an oligonucleotide in which the two or more identical or different "deoxyribonucleosides" are linked to each other by the "internucleoside bond".

"RNA" means a polynucleotide or an oligonucleotide in which the two or more identical or different "ribonucleosides" are linked to each other by the "internucleoside bond".

"Antisense effect" means that a target RNA selected corresponding to a target gene hybridizes with, for example, an oligonucleotide having a sequence complementary to a partial sequence of the target RNA to control a function of the target RNA. For example, when the target RNA is mRNA, the antisense effect means inhibition of translation of the target RNA by hybridization, a splicing function converting effect such as exon skipping, degradation of the target RNA by recognition of a portion that has hybridized, and the like.

In the present invention, the target RNA is "ATN1 mRNA" and/or "ATN1 pre-mRNA".

"Antisense oligonucleotide" (ASO) is an oligonucleotide having the antisense effect. Examples thereof include DNA, a gapmer, a mixmer, and the like, but are not limited thereto, and may be RNA, an oligonucleotide designed to usually have the antisense effect, or the like.

"Hybridize" means an action of forming a double strand between oligonucleotides including sequences complementary to each other or portions thereof, and a phenomenon that oligonucleotides including sequences complementary to each other or portions thereof form a double strand therebetween.

"Complementary" means that two nucleobases can form a Watson-Crick base pair (natural base pair) or a non-Watson-Crick base pair (Hoogsteen base pair, and the like) via a hydrogen bond. Two oligonucleotides or portions thereof can "hybridize" with each other when their sequences are complementary to each other. In order for two oligonucleotides or portions thereof to hybridize with each other, they need not to be completely complementary to each other, but complementarity for two oligonucleotides or portions thereof to hybridize with each other is preferably 70% or more, more preferably 80% or more, and still more preferably 90% or more (for example, 95%, 96%, 97%, 98%, or 99% or more). Complementarity between sequences is determined by using a computer program that automatically identifies a partial sequence of an oligonucleotide. For example, OligoAnalyzer is one type of such software and is provided by Integrated DNA Technologies. This program can also be used in a website.

"Gapmer" means an oligonucleotide including a "gap segment", a "5' wing segment", and a "3' wing segment" described later.

"Gap segment" is a region including "at least four contiguous nucleosides recognized by RNaseH", and is not particularly limited as long as it includes four or more contiguous nucleosides and is recognized by RNaseH. However, the contiguous nucleosides are preferably independently selected from a deoxyribonucleoside and a sugar-modified nucleoside. Nucleosides at a 5'-terminal and a 3'-terminal of the gap segment are preferably deoxyribonucleosides.

"5' Wing segment" is a region linked to a 5' side of the gap segment and including "at least one nucleoside" without including the "at least four contiguous nucleosides recognized by RNaseH". Here, a sugar moiety of a nucleoside at a 3'-terminal of the 5' wing segment is different from a sugar moiety of a nucleoside at a 5'-terminal of the gap segment. Due to the difference in sugar moiety, a boundary between the 5' wing segment and the gap segment is confirmed. (For example, the nucleoside at the 5'-terminal of the gap segment is a deoxyribonucleoside, and the nucleoside at the 3'-terminal of the 5' wing segment is a sugar-modified nucleoside.) The nucleoside at the 3'-terminal of the 5' wing segment is generally a sugar-modified nucleoside. The 5' wing segment is not particularly limited as long as it satisfies the above definition, but the at least one nucleoside is preferably independently selected from a deoxyribonucleoside and a sugar-modified nucleoside, and includes at least one sugar-modified nucleoside.

"3' Wing segment" is a region linked to a 3' side of the gap segment and including "at least one nucleoside" without including the "at least four contiguous nucleosides recognized by RNaseH". Here, a sugar moiety of a nucleoside at a 5'-terminal of the 3' wing segment is different from a sugar moiety of a nucleoside at a 3'-terminal of the gap segment. Due to the difference in sugar moiety, a boundary between the 3' wing segment and the gap segment is confirmed. (For example, the nucleoside at the 3'-terminal of the gap segment is a deoxyribonucleoside, and the nucleoside at the 5'-terminal of the 3' wing segment is a sugar-modified nucleoside.) The nucleoside at the 5'-terminal of the 3' wing segment is generally a sugar-modified nucleoside. The 3' wing segment is not particularly limited as long as it satisfies the above definition, but the at least one nucleoside is preferably independently selected from a deoxyribonucleoside and a sugar-modified nucleoside, and includes at least one sugar-modified nucleoside.

"RNaseH" is generally known as ribonuclease that recognizes a double strand in which DNA and RNA in a living body hybridize with each other, and cleaves the RNA to generate single-stranded DNA. RNaseH can recognize not only a double strand in which DNA and RNA hybridize with each other but also a double strand in which at least one of a nucleobase moiety, a phosphodiester bond moiety, and a sugar moiety of at least one of DNA and RNA is modified. For example, RNaseH can also recognize a double strand in which DNA modified with a phosphorothioate bond and

### RNA hybridize with each other.

Thus, DNA can be recognized by RNaseH when DNA hybridizes with RNA. In addition, RNA can be cleaved by RNaseH when RNA hybridizes with DNA. The same applies to a case where at least one of a nucleobase moiety, a phosphodiester bond moiety, and a sugar moiety is modified in at least one of DNA and RNA. For example, a typical example is an oligonucleotide in which a phosphodiester bond moiety of DNA is modified with phosphorothioate, and the like.

Examples of modification of DNA and/or RNA that can be recognized by RNaseH are described, for example, in Nucleic Acids Research, 2014, 42, pp 5378-5389, Bioorganic & Medicinal Chemistry Letters, 2008, 18, pp 2296-2300, Molecular BioSystems, 2009, 5, pp 838-843, Nucleic Acid Therapeutics, 2015, 25, pp 266-274, The Journal of Biological Chemistry, 2004, 279, pp 36317-36326, and the like.

RNaseH used in the present invention is preferably mammalian RNaseH, more preferably human RNaseH, and particularly preferably human RNaseH1.

"At least four contiguous nucleosides recognized by RNaseH" are not particularly limited as long as they include four or more contiguous nucleosides and are recognized by RNaseH, but examples thereof include "at least four contiguous deoxyribonucleosides", and the like. The number of nucleosides constituting "at least four contiguous nucleosides recognized by RNaseH" is, for example, 5 to 30, preferably 5 to 15, more preferably 8 to 12, and particularly preferably 10.

A person skilled in the art can determine whether or not certain at least four contiguous nucleosides are "at least four contiguous nucleosides recognized by RNaseH" by the structure of a sugar moiety of the contiguous nucleosides.

"ATN1" (Atrophin1) means any protein of Atrophin1. "ATN1 mRNA" means mRNA encoding ATN1 protein, and "ATN1 pre-mRNA" means pre-mRNA encoding ATN1 protein. "ATN1 nucleic acid" means any nucleic acid encoding ATN1, and includes, for example, "ATN1 mRNA" and "ATN1 pre-mRNA".

"ATN1 mRNA" is represented by, for example, SEQ ID NO: 1, and "ATN1 pre-mRNA" is represented by, for example, SEQ ID NO: 2 or 717. "ATN1 mRNA" and "ATN1 pre-mRNA" each have nucleosides linked to each other by a phosphodiester bond, and usually thymine is replaced with uracil. "ATN1 mRNA" and "ATN1 pre-mRNA" do not have a modified sugar, a modified nucleobase, or a modified internucleoside bond in addition to the nucleosides.

"Expression of a gene" means that coding information of a gene is converted into a structure in a cell or a function thereof. Such a structure is not limited, but examples thereof include products of transcription and translation (mRNA, pre-mRNA, protein, and the like).

Dentatorubral-pallidoluysian atrophy (DRPLA) is an autosomal dominant spinocerebellar degeneration with lesions in a cerebellar dentate nucleus, a red nucleus, a pallidus, and/or a Luys body. It has been clarified that a cause of DRPLA is abnormal elongation of CAG repeats of an ATN1 gene. A healthy person has a sequence including 7 to 23 CAG repeats, whereas a DRPLA patient has, for example, a sequence including 49 or more CAG repeats.

Since the length of the CAG repeats in an ATN1 gene of a DRPLA patient varies from patient to patient, an antisense oligonucleotide and ss-siRNA targeting a CAG repeat sequence may have a large variation in efficacy thereof. In addition, CAG repeats are present in many genes other than ATN1, and therefore may have an off-target effect. The antisense oligonucleotide of the present invention has complementarity with ATN1 mRNA and/or ATN1 pre-mRNA, and does not target CAG repeats of an ATN1 gene, that is, has complementarity with a sequence other than a CAG repeat sequence. Therefore, it is expected that the antisense oligonucleotide of the present invention has a small variation in efficacy and has a small off-target effect.

"CAG repeat sequence" is a sequence in which three base units of cytosine (C), adenine (A), and guanine (G) are repeated in this order. A person skilled in the art can determine from a base sequence of an ASO that the CAG repeat sequence is not a target.

Next, preferable forms of the modified oligonucleotide of the present invention or a compound comprising the modified oligonucleotide will be described. Note that the following description will be made by using an antisense oligonucleotide (ASO) or a compound comprising the ASO, which is a preferable example of a modified oligonucleotide or a compound comprising the modified oligonucleotide, but the description is appropriately applied to a modified oligonucleotide or a compound comprising the modified oligonucleotide.

The ASO of the present invention targets mRNA or pre-mRNA of ATN1. The ASO of the present invention does not need to hybridize with the entire mRNA or pre-mRNA of ATN1, and usually hybridizes with at least a part thereof. For example, a part of mRNA or pre-mRNA of ATN1 hybridizes with an oligonucleotide having a sequence complementary to a partial sequence of mRNA or pre-mRNA of ATN1 (such as a gapmer, an oligonucleotide designed to usually have an antisense effect, or the like) to control expression of the ATN1 gene. In addition, the entire ASO does not need to hybridize, and a part of the ASO does not need to hybridize.

Complementarity between a nucleobase sequence of the ASO of the present invention and a partial sequence of mRNA or pre-mRNA of ATN1 is preferably 70% or more, more preferably 80% or more, and still more preferably 90% or more (for example, 95%, 96%, 97%, 98%, or 99% or more). In order for the ASO of the present invention to hybridize with at least a part of a partial sequence of mRNA or pre-mRNA of ATN1, the sequences do not need to be completely complementary to each other, but are more preferably completely complementary to each other.

The ASO of the present invention includes a gap segment, a 5' wing segment, and a 3' wing segment.

The gap segment consists of at least five nucleosides independently selected from the group consisting of a deoxyribonucleoside, a ribonucleoside, and a sugar-modified nucleoside, and 3'-terminals and 5'-terminals of the nucleosides are each independently a deoxyribonucleoside. The gap segment preferably includes "at least four contiguous deoxyribonucleosides".

The number of nucleosides included in the gap segment is preferably 5 to 30, more preferably 5 to 15, still more preferably 8 to 12, further still more preferably 9 to 11, and particularly preferably 10.

In a certain embodiment, the gap segment consists of deoxyribonucleosides.

An internucleoside bond included in the gap segment is preferably at least one independently selected from a phosphodiester bond and a modified phosphodiester bond, and more preferably at least one independently selected from a phosphodiester bond and a phosphorothioate bond. The internucleoside bond included in the gap segment preferably includes at least one phosphorothioate bond, more preferably 50% or more of the internucleoside bonds are phosphorothioate bonds, still more preferably 75% or more of the internucleoside bonds are phosphorothioate bonds, further still more preferably 80% or more of the internucleoside bonds are phosphorothioate bonds, further still more preferably 90% or more of the internucleoside bonds are phosphorothioate bonds, and particularly preferably all of the internucleoside bonds are phosphorothioate bonds.

The 5' wing segment consists of at least one nucleoside independently selected from the group consisting of a deoxyribonucleoside, a ribonucleoside, and a sugar-modified nucleoside. However, a 3'-terminal of the 5' wing segment bonded to the gap segment is a sugar-modified nucleoside and does not include "at least four contiguous nucleosides recognized by RNaseH."

The 3' wing segment consists of at least one nucleoside independently selected from the group consisting of a deoxyribonucleoside, a ribonucleoside, and a sugar-modified nucleoside. However, a 5'-terminal of the 3' wing segment bonded to the gap segment is a sugar-modified nucleoside and does not include "at least four contiguous nucleosides recognized by RNaseH."

The 5' wing segment and the 3' wing segment each preferably do not include "at least four contiguous deoxyribonucleosides".

Hereinafter, a property common to the 5' wing segment and the 3' wing segment will be described as a wing segment.

The number of nucleosides included in each of the wing segments is 1 to 15, preferably 1 to 10, more preferably 1 to 7, still more preferably 2 to 6, further still more preferably 3 to 5, and particularly preferably 3 or 5.

A sugar-modified nucleoside included in the wing segment is preferably a nucleoside having increased affinity for a partial sequence of ATN1 mRNA and/or ATN1 pre-mRNA or a nucleoside having increased resistance to a nucleolytic enzyme due to substitution or the like. More preferably, the sugar-modified nucleoside included in the wing segment is independently selected from a 2'-modified nucleoside and 2',4'-BNA.

The 2'-modified nucleoside included in the wing segment is preferably at least one independently selected from the group consisting of a 2'-O-Me nucleoside, a 2'-O-MOE nucleoside, a 2'-AP nucleoside, a 2'-F nucleoside, a 2'-O-NMA nucleoside, a 2'-O-MCE nucleoside, a 2'-DMAECE nucleoside, a 2'-MorECE nucleoside, a 2'-PyECE nucleoside, and a BimECE nucleoside, more preferably at least one independently selected from the group consisting of a 2'-O-Me nucleoside, a 2'-O-MOE nucleoside, a 2'-O-NMA nucleoside, and a 2'-O-MCE nucleoside, and still more preferably at least one independently selected from a 2'-O-MOE nucleoside and a 2'-O-MCE nucleoside.

The 2',4'-BNA included in the wing segment is preferably at least one independently selected from the group consisting of LNA, cEt, ENA, BNA^{NC}, AmNA, GuNA, and scpBNA, and more preferably LNA.

The sugar-modified nucleoside included in the wing segment is more preferably at least one independently selected from a 2'-O-MOE nucleoside, LNA, and a 2'-O-MCE nucleoside. Still more preferably the sugar-modified nucleoside included in the wing segment is a 2'-O-MOE nucleoside, or is a 2'-O-MCE nucleoside, or is LNA and a 2'-O-MCE nucleoside. Particularly preferably the sugar-modified nucleoside included in the wing segment is a 2'-O-MOE nucleoside, or is LNA and a 2'-O-MCE nucleoside.

Preferably, each of the wing segments consists of 1 to 10 nucleosides independently selected from the group consisting of a sugar-modified nucleoside and a deoxyribonucleoside, and includes at least one sugar-modified nucleoside. More preferably, each of the wing segments consists of 2 to 6 nucleosides independently selected from the group consisting of a sugar-modified nucleoside and a deoxyribonucleoside, and includes at least two sugar-modified nucleoside. Still more preferably, each of the wing segments consists of 2 to 6 nucleosides independently selected from the group consisting of a 2'-modified nucleoside and 2',4'-BNA. Further still more preferably, each of the wing segments consists of 3 to 5 nucleosides independently selected from the group consisting of a 2'-modified nucleoside and 2',4'-BNA.

More specifically, each of the wing segments consists of 1 to 10, preferably 2 to 6, more preferably 3 to 5 nucleosides independently selected from LNA, a 2'-O-MCE nucleoside, and a 2'-O-MOE nucleoside. More preferably, each of the wing segments consists of 2 to 5, more preferably 3 to 5 nucleosides independently selected from LNA and a 2'-O-MCE nucleoside. Among these, still more preferably, each of the wing segments includes at least one 2'-O-MCE nucleoside, and further still more preferably, each of the wing segments includes at least one LNA and at least one 2'-O-MCE nucleoside. Further still more preferably, each of the wing segments consists of two LNAs and one or two 2'-O-MCE nucleosides. Particularly preferably, each of the wing segments consists of two LNAs and one 2'-O-MCE nucleoside. As another aspect, each of the wing segments consists of preferably three to six, particularly preferably five 2'-O-MOE nucleosides. As still another aspect, each of the wing segments consists of preferably three to six, particularly preferably five 2'-O-MCE nucleosides.

As another preferable aspect, each of the wing segments is an oligonucleotide consisting of 2 to 5 nucleosides independently selected from the group consisting of 2',4'-BNA and a deoxyribonucleoside and including at least two 2',4'-BNAs. For such an oligonucleotide, WO 2016/127002 A and the like can be referred to.

In some embodiments of the present invention, the 5' wing segment and the 3' wing segment each independently include 3, 4, 5, or 6 sugar-modified nucleosides, and the gap segment consists of 8, 9, 10, 11, 12, 13, or 14 deoxyribonucleosides. The number of nucleosides in such a gapmer can be represented by ((5' wing segment)-(gap segment)-(3' wing segment)), and the gapmer includes 5-10-5, 5-11-4, 4-11-5, 4-12-4, 3-14-3, 6-8-6, 3-12-3, 3-10-3, 4-10-4, 3-10-4, 4-10-3, 3-9-3, 4-9-4, 3-9-4, 4-9-3, 3-8-3, 3-8-4, 4-8-3, and 4-8-4 nucleosides.

In some embodiments, the ASO includes at least 11, 12, 13, 14, or 15 contiguous nucleosides, the gap segment includes at least 5, 6, 7, 8, or 9 contiguous nucleosides, and the 5' wing segment and the 3' wing segment are any of the following (i) to (iv):
(i) The 5' wing segment and the 3' wing segment each independently include three, four, five, or six 2'-O-MOE nucleosides.
(ii) The 5' wing segment and the 3' wing segment each independently include three, four, five, or six 2'-O-MCE nucleosides.
(iii) The 5' wing segment and the 3' wing segment each independently include two, three, four, five, or six LNAs.
(iv) The 5' wing segment and the 3' wing segment each independently include three or four selected from a 2'-O-MCE nucleoside and LNA, and include at least one 2'-O-MCE nucleoside and at least one LNA.

The 5' wing segment and the 3' wing segment in the ASO of the present invention are preferably selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, and VVVL.

More preferably, the 5' wing segment and the 3' wing segment are selected from the group consisting of VLL, LVL, LLV, LVV, VLV, VVL, VVLL, VLVL, VLLV, LVLV, LLVV, LVVL, VLLL, LVLL, LLVL, LLLV, LVVV, VLVV, VVLV, and VVVL.

Still more preferably, the 5' wing segment and the 3' wing segment are selected from the group consisting of VLL, LVL, LLV, LVV, VLV, VVL, VVLL, VLVL, LVLV, LLVV, VLLL, LVLL, LLVL, LLLV, LVVV, VLVV, VVLV, and VVVL.

Further still more preferably, the 5' wing segment is selected from the group consisting of VLL, VLV, VVL, VVLL, VLVL, VLLL, VLVV, VVLV, and VVVL, and the 3' wing segment is selected from the group consisting of LLV, VLV, LVV, LLVV, LVLV, VLLV, VVLV, VLVV, and LVVV.

Further still more preferably, the 5' wing segment is selected from the group consisting of VLL, VLV, VVL, and VLVL, and the 3' wing segment is selected from the group consisting of LLV, VLV, LVV, and LVLV.

Particularly preferably, the 5' wing segment is VLL, and the 3' wing segment is LLV.

As another aspect, preferably, the 5' wing segment is selected from the group consisting of LLL, VLL, LVL, VVL, LLV, VLVL, LVLV, LLVV, and VLLV, and the 3' wing segment is selected from the group consisting of LLL, LLV, LVL, LVV, VLL, LVLV, and VLLV.

More preferably, the 5' wing segment is selected from the group consisting of VLL, LVL, VVL, LLV, VLVL, and VLLV, and the 3' wing segment is selected from the group consisting of LLV, LVL, LVV, VLL, LVLV, and VLLV.

Here, L and V in the 5' wing segment and the 3' wing segment are sugar-modified nucleosides having different modified sugars, and a left side represents a 5' side and a right side represents a 3' side. Preferably, L represents a 2'-4'-bridged nucleoside and V represents a 2'-modified nucleoside. Particularly preferably, L represents LNA and V represents a 2'-O-MCE nucleoside.

An internucleoside bond included in the 5' wing segment is preferably at least one independently selected from a phosphodiester bond and a modified phosphodiester bond, and more preferably at least one independently selected from a phosphodiester bond and a phosphorothioate bond. The internucleoside bond included in the 5' wing segment preferably includes at least one phosphorothioate bond, more preferably 50% or more of the internucleoside bonds are phosphorothioate bonds, still more preferably 60% or more of the internucleoside bonds are phosphorothioate bonds, further still more preferably 75% or more of the internucleoside bonds are phosphorothioate bonds, further still more preferably 80% or more of the internucleoside bonds are phosphorothioate bonds, further still more preferably 90% or more of the internucleoside bonds are phosphorothioate bonds, and particularly preferably all of the internucleoside bonds are phosphorothioate bonds.

The 3' wing segment is similar to the 5' wing segment.

As another aspect, all of the internucleoside bonds included in the wing segment may be phosphodiester bonds from a viewpoint that toxicity can be reduced. However, preferably, some of the internucleoside bonds are phosphodiester bonds.

An internucleoside bond at a 5'-terminal included in the 5' wing segment is preferably a phosphorothioate bond. An internucleoside bond at a 3'-terminal included in the 3' wing segment is preferably a phosphorothioate bond. Other internucleoside bonds of the 5' wing segment other than the internucleoside bond at the 5'-terminal of the 5' wing segment may be each a phosphodiester bond or a phosphorothioate bond. However, preferably, 50% or more of the internucleoside bonds are phosphodiester bonds, more preferably, 60% or more of the internucleoside bonds are phosphodiester bonds, still more preferably, 75% or more of the internucleoside bonds are phosphodiester bonds, further still more preferably, 80% or more of the internucleoside bonds are phosphodiester bonds, further still more preferably, 90% or more of the internucleoside bonds are phosphodiester bonds, and particularly preferably, all of the internucleoside bonds are phosphodiester bonds. Other internucleoside bonds of the 3' wing segment other than the internucleoside bond at the 3'-terminal of the 3' wing segment may be each a phosphodiester bond or a phosphorothioate bond. However, preferably, 50% or more of the internucleoside bonds are phosphodiester bonds, more preferably, 60% or more of the internucleoside bonds are phosphodiester bonds, still more preferably, 75% or more of the internucleoside bonds are phosphodiester bonds, further still more preferably, 80% or more of the internucleoside bonds are phosphodiester bonds, further still more preferably, 90% or more of the internucleoside bonds are phosphodiester bonds, and particularly preferably, all of the internucleoside bonds are phosphodiester bonds. Among the internucleoside bonds included in each of the wing segments, an internucleoside bond located closest to the gap segment is preferably a phosphodiester bond.

For example, when the 5' wing segment includes two internucleoside bonds, the internucleoside bond at the 5'-terminal is preferably a phosphorothioate bond, and the internucleoside bond at a side of the gap segment is preferably a phosphodiester bond.

When the 5' wing segment includes three internucleoside bonds, the internucleoside bond at the 5'-terminal is preferably a phosphorothioate bond, and the other two internucleoside bonds included in the 5' wing segment are each independently preferably a phosphodiester bond or a phosphorothioate bond, and particularly preferably a phosphodiester bond.

When the 5' wing segment includes four internucleoside bonds, the internucleoside bond at the 5'-terminal is preferably a phosphorothioate bond, and the other three internucleoside bonds included in the 5' wing segment are each independently preferably a phosphodiester bond or a phosphorothioate bond, and particularly preferably a phosphodiester bond.

When the 3' wing segment includes two internucleoside bonds, the internucleoside bond at the 3'-terminal is preferably a phosphorothioate bond, and the internucleoside bond at a side of the gap segment is preferably a phosphodiester bond.

When the 3' wing segment includes three internucleoside bonds, the internucleoside bond at the 3'-terminal is preferably a phosphorothioate bond, and the other two internucleoside bonds included in the 3' wing segment are each independently preferably a phosphodiester bond or a phosphorothioate bond, and particularly preferably a phosphodiester bond.

When the 3' wing segment includes four internucleoside bonds, the internucleoside bond at the 3'-terminal is preferably a phosphorothioate bond, and the other three internucleoside bonds included in the 3' wing segment are each independently preferably a phosphodiester bond or a phosphorothioate bond, and particularly preferably a phosphodiester bond.

In the ASO of the present invention, preferably, the 3'-terminal of the 5' wing segment and the 5'-terminal of the gap segment are linked to each other by forming a phosphodiester bond or a modified phosphodiester bond, and the 5'-terminal of the 3' wing segment and the 3'-terminal of the gap segment are linked to each other by forming a phosphodiester bond or a modified phosphodiester bond. More preferably, the 3'-terminal of the 5' wing segment and the 5'-terminal of the gap segment are linked to each other by forming a modified phosphodiester bond, and the 5'-terminal of the 3' wing segment and the 3'-terminal of the gap segment are linked to each other by forming a modified phosphodiester bond. Still more preferably, the 3'-terminal of the 5' wing segment and the 5'-terminal of the gap segment are linked to each other by forming a phosphorothioate bond, and the 5'-terminal of the 3' wing segment and the 3'-terminal of the gap segment are linked to each other by forming a phosphorothioate bond.

In another preferable embodiment of the ASO of the present invention, the 3'-terminal of the 5' wing segment and the 5'-terminal of the gap segment are linked to each other by forming a phosphodiester bond, and the 5'-terminal of the 3' wing segment and the 3'-terminal of the gap segment are linked to each other by forming a phosphorothioate bond.

The present invention includes an ASO in which the 3'-terminal of the 5' wing segment and the 5'-terminal of the gap segment are linked to each other by forming a phosphorothioate bond, and the 5'-terminal of the 3' wing segment and the 3'-terminal of the gap segment are linked to each other by forming a phosphodiester bond. The present invention includes an ASO in which the 3'-terminal of the 5' wing segment and the 5'-terminal of the gap segment are linked to each other by forming a phosphodiester bond, and the 5'-terminal of the 3' wing segment and the 3'-terminal of the gap segment are linked to each other by forming a phosphodiester bond.

The ASO of the present invention is preferably complementary to a part in a nucleobase sequence selected from the group consisting of nucleobase sequences represented by position numbers 59 to 104, 109 to 133, 173 to 192, 207 to 272, 300 to 319, 353 to 372, 419 to 434, 458 to 509, 523 to 559, 561 to 618, 626 to 685, 766 to 785, 787 to 819, 838 to 855, 880 to 922, 924 to 943, 970 to 989, 994 to 1039, 1055 to 1074, 1079 to 1098, 1157 to 1176, 1196 to 1223, 1310 to 1329, 1339 to 1399, 1423 to 1442, 1614 to 1633, 1650 to 1670, 1806 to 1833, 1844 to 1865, 1896 to 1915, 1924 to 1992, 2023 to 2042, 2058 to 2081, 2103 to 2124, 2398 to 2417, 2480 to 2520, 2526 to 2562, 2568 to 2587, 2853 to 2872, 2876 to 2923, 2931 to 2950, 2972 to 3016, 3072 to 3099, 3109 to 3128, 3192 to 3211, 3267 to 3290, 3333 to 3379, 3419 to 3557, 3765 to 3784, 3789 to 3853, 3888 to 3926, 4022 to 4062, 4118 to 4139, 4141 to 4156, 4218 to 4236, and 4266 to 4281 of nucleobases of ATN1 mRNA represented by SEQ ID NO: 1.

More preferably, the ASO of the present invention is complementary to a part in a nucleobase sequence selected from the group consisting of nucleobase sequences represented by position numbers 238 to 272, 537 to 559, 585 to 618, 664 to 683, 787 to 819, 838 to 855, 924 to 943, 972 to 987, 994 to 1029, 1055 to 1074, 1080 to 1095, 1204 to 1219, 1310 to 1329, 1362 to 1397, 1650 to 1670, 1806 to 1833, 1844 to 1865, 1896 to 1915, 1955 to 1992, 2058 to 2081, 2103 to 2124, 2480 to 2503, 2876 to 2923, 2931 to 2946, 2979 to 3016, 3109 to 3128, 3192 to 3211, 3267 to 3290, 3333 to 3379, 3419 to 3557, 3812 to 3833, 3888 to 3926, 4022 to 4062, 4118 to 4137, 4218 to 4236, and 4266 to 4281 of nucleobases of ATN1 mRNA represented by SEQ ID NO: 1.

Still more preferably, the ASO of the present invention is complementary to a part of a nucleobase sequence represented by position numbers 3419 to 3557 of nucleobases of ATN1 mRNA represented by SEQ ID NO: 1.

Particularly preferable positions of ATN1 mRNA complementary to the ASO of the present invention are listed below.

The ASO of the present invention is complementary to a part of a nucleobase sequence represented by position numbers 3452 to 3467 of nucleobases of ATN1 mRNA represented by SEQ ID NO: 1.

The ASO of the present invention is complementary to a part of a nucleobase sequence represented by position numbers 3459 to 3474 of nucleobases of ATN1 mRNA represented by SEQ ID NO: 1.

The ASO of the present invention is complementary to a part of a nucleobase sequence represented by position numbers 3460 to 3475 of nucleobases of ATN1 mRNA represented by SEQ ID NO: 1.

The ASO of the present invention is complementary to a part of a nucleobase sequence represented by position numbers 3461 to 3476 of nucleobases of ATN1 mRNA represented by SEQ ID NO: 1.

The ASO of the present invention is complementary to a part of a nucleobase sequence represented by position numbers 3488 to 3503 of nucleobases of ATN1 mRNA represented by SEQ ID NO: 1.

Complementarity between the ASO of the present invention and a corresponding part of ATN1 mRNA represented by SEQ ID NO: 1 (for example, each of the above-described target regions) is at least 80%, preferably 85% or more, more preferably 90% or more, and still more preferably 95%, 96%, 97%, 98%, or 99% or more.

The ASO of the present invention preferably consists of 8 to 80 nucleosides complementary to a part of each of the above-described target regions of ATN1 mRNA represented by SEQ ID NO: 1. The ASO of the present invention consists of more preferably 10 to 50 nucleosides, still more preferably 16 to 20 nucleosides, particularly preferably 16 or 20 nucleosides.

The ASO of the present invention is preferably complementary to a part in a nucleobase sequence selected from the group consisting of nucleobase sequences represented by position numbers 383 to 398, 986 to 1001, 1004 to 1019, 1378 to 1393, 1398 to 1413, 1853 to 1868, 1971 to 1986, 2189 to 2204, 2522 to 2537, 2562 to 2577, 3662 to 3677, 3987 to 4002, 4217 to 4232, 4266 to 4281, 4335 to 4350, 4360 to 4375, 4477 to 4492, 4562 to 4577, 4636 to 4651, 4687 to 4702, 4734 to 4749, 4840 to 4855, 5536 to 5563, 5568 to 5592, 5632 to 5651, 5666 to 5715, 5906 to 5925, 5959 to 5978, 6176 to 6191, 6215 to 6266, 6963 to 6978, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9336 to 9351, 9534 to 9570, 9576 to 9595, 9923 to 9938, 10001 to 10016, 10056 to 10071, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12508 to 12680, 12723 to 12738, 13454 to 13518, 13553 to 13591, 13687 to 13727, 13783 to 13804, 13806 to 13821, 13883 to 13901, and 13931 to 13946 of nucleobases of ATN1 pre-mRNA represented by SEQ ID NO: 2.

The ASO of the present invention is preferably complementary to a part in a nucleobase sequence selected from the group consisting of nucleobase sequences represented by position numbers 383 to 398, 986 to 1001, 1004 to 1019, 1378 to 1393, 1398 to 1413, 1853 to 1868, 1971 to 1986, 2189 to 2204, 2522 to 2537, 2562 to 2577, 3662 to 3677, 3987 to 4002, 4217 to 4232, 4266 to 4281, 4335 to 4350, 4360 to 4375, 4477 to 4492, 4562 to 4577, 4636 to 4651, 4687 to 4702, 4734 to 4749, 4840 to 4855, 5536 to 5563, 5568 to 5592, 5632 to 5651, 5666 to 5715, 5906 to 5925, 5959 to 5978, 6176 to 6191, 6215 to 6266, 6963 to 6978, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9336 to 9351, 9534 to 9570, 9576 to 9595, 9923 to 9938, 10001 to 10016, 10056 to 10071, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12508 to 12523, 12572 to 12680, 12723 to 12738, 13454 to 13518, 13553 to 13591, 13687 to 13727, 13783 to 13804, 13806 to 13821, 13883 to 13901, and 13931 to 13946 of nucleobases of ATN1 pre-mRNA represented by SEQ ID NO: 2.

The ASO of the present invention is preferably complementary to a part in a nucleobase sequence selected from the group consisting of nucleobase sequences represented by position numbers 5536 to 5563, 5568 to 5592, 5632 to 5651, 5666 to 5715, 5906 to 5925, 5959 to 5978, 6176 to 6191, 6215 to 6266, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9534 to 9570, 9576 to 9595, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12572 to 12680, 13454 to 13518, 13553 to 13591, 13687 to 13727, 13783 to 13804, 13806 to 13821, 13883 to 13901, and 13931 to 13946 of nucleobases of ATN1 pre-mRNA represented by SEQ ID NO: 2.

More preferably, the ASO of the present invention is complementary to a part in a nucleobase sequence selected from the group consisting of nucleobase sequences represented by position numbers 986 to 1001, 1004 to 1019, 1378 to 1393, 1398 to 1413, 4217 to 4232, 4335 to 4350, 4360 to 4375, 4477 to 4492, 4562 to 4577, 4636 to 4651, 4687 to 4702, 4734 to 4749, 4840 to 4855, 5697 to 5715, 6963 to 6978, 7262 to 7284, 7310 to 7343, 7389 to 7408, 7512 to 7544, 7563 to 7580, 7649 to 7668, 7697 to 7712, 7719 to 7754, 7780 to 7799, 7805 to 7820, 7929 to 7944, 8035 to 8054, 8087 to 8122, 8375 to 8395, 8531 to 8558, 8569 to 8590, 8621 to 8640, 8680 to 8717, 8783 to 8806, 8828 to 8849, 9205 to 9228, 10297 to 10344, 10352 to 10367, 10400 to 10437, 10530 to 10549, 10613 to 10632, 10688 to 10711, 10754 to 10800, 10840 to 10865, 12508 to 12523, 12572 to 12680, 12723 to 12738, 13477 to 13498, 13553 to 13591, 13687 to 13727, 13783 to 13802, 13883 to 13901, and 13931 to 13946 of nucleobases of ATN1 pre-mRNA represented by SEQ ID NO: 2.

Still more preferably, the ASO of the present invention is complementary to a part in a nucleobase sequence selected from the group consisting of nucleobase sequences represented by position numbers 5697 to 5715, 7262 to 7284, 7310 to 7343, 7389 to 7408, 7512 to 7544, 7563 to 7580, 7649 to 7668, 7697 to 7712, 7719 to 7754, 7780 to 7799, 7805 to 7820, 7929 to 7944, 8035 to 8054, 8087 to 8122, 8375 to 8395, 8531 to 8558, 8569 to 8590, 8621 to 8640, 8680 to 8717, 8783 to 8806, 8828 to 8849, 9205 to 9228, 10297 to 10344, 10352 to 10367, 10400 to 10437, 10530 to 10549, 10613 to 10632, 10688 to 10711, 10754 to 10800, 10840 to 10865, 12572 to 12680, 13477 to 13498, 13553 to 13591, 13687 to 13727, 13783 to 13802, 13883 to 13901, and 13931 to 13946 of nucleobases of ATN1 pre-mRNA represented by SEQ ID NO: 2.

Further still more preferably, the ASO of the present invention is complementary to a part in a nucleobase sequence selected from the group consisting of nucleobase sequences represented by position numbers 10840 to 10865 and 12572 to 12680 of nucleobases.

Particularly preferably, the ASO of the present invention is complementary to a part in a nucleobase sequence represented by position numbers 12572 to 12680 of nucleobases.

As another aspect, the ASO of the present invention is preferably complementary to a part in a nucleobase sequence selected from the group consisting of nucleobase sequences represented by position numbers 4320 to 4390 and 12508 to 12680 of nucleobases of ATN1 pre-mRNA represented by SEQ ID NO: 2.

As another aspect, the ASO of the present invention is preferably complementary to a part in a nucleobase sequence represented by position numbers 4320 to 4390 of nucleobases of ATN1 pre-mRNA represented by SEQ ID NO: 2.

As another aspect, the ASO of the present invention is more preferably complementary to a part in a nucleobase sequence represented by position numbers 12508 to 12680 of nucleobases of ATN1 pre-mRNA represented by SEQ ID NO: 2.

Particularly preferable positions of ATN1 pre-mRNA complementary to the ASO of the present invention are listed below.

The ASO of the present invention is complementary to a part in a nucleobase sequence represented by position numbers 12575 to 12590 of nucleobases of ATN1 pre-mRNA represented by SEQ ID NO: 2.

The ASO of the present invention is complementary to a part in a nucleobase sequence represented by position numbers 12582 to 12597 of nucleobases of ATN1 pre-mRNA represented by SEQ ID NO: 2.

The ASO of the present invention is complementary to a part in a nucleobase sequence represented by position numbers 12583 to 12598 of nucleobases of ATN1 pre-mRNA represented by SEQ ID NO: 2.

The ASO of the present invention is complementary to a part in a nucleobase sequence represented by position numbers 12584 to 12599 of nucleobases of ATN1 pre-mRNA represented by SEQ ID NO: 2.

The ASO of the present invention is complementary to a part in a nucleobase sequence represented by position numbers 12611 to 12626 of nucleobases of ATN1 pre-mRNA represented by SEQ ID NO: 2.

Complementarity between the ASO of the present invention and a corresponding part of ATN1 pre-mRNA represented by SEQ ID NO: 2 (for example, each of the above-described target regions) is at least 80%, preferably 85% or more, more preferably 90% or more, and still more preferably 95%, 96%, 97%, 98%, or 99% or more.

The ASO of the present invention preferably consists of 8 to 80 nucleosides complementary to a part of each of the above-described target regions of ATN1 pre-mRNA represented by SEQ ID NO: 2. The ASO of the present invention consists of more preferably 10 to 50 nucleosides, still more preferably 16 to 20 nucleosides, particularly preferably 16 or 20 nucleosides.

The nucleobase sequence in the ASO of the present invention includes preferably at least 8, more preferably 8, 9, 10, 11, 12, 13, 14, or 15 contiguous nucleobases of any of nucleobase sequences of SEQ ID NOs: 3 to 716 and 717 to 770 (3 to 487, 488 to 716, and 717 to 770 in one aspect).

The nucleobase sequence in the ASO of the present invention more preferably includes any of nucleobase sequences of SEQ ID NOs: 3 to 716 and 717 to 770 (3 to 487, 488 to 716, and 717 to 770 in one aspect).

The nucleobase sequence in the ASO of the present invention preferably has a nucleobase sequence including at least 8, preferably 8, 9, 10, 11, 12, 13, 14, or 15 contiguous nucleobases of any one of nucleobase sequences of nucleobase sequence group A (A1, A2, and A3), and more preferably has any one of nucleobase sequences of nucleobase sequence group A (A1, A2, and A3).

As another aspect, the nucleobase sequence in the ASO of the present invention more preferably has a nucleobase sequence including at least 8, preferably 8, 9, 10, 11, 12, 13, 14, or 15 contiguous nucleobases of any one of nucleobase sequences of nucleobase sequence group B (B1, B2, and B3), and still more preferably has any one of nucleobase sequences of nucleobase sequence group B (B1, B2, and B3).

As another aspect, the nucleobase sequence in the ASO of the present invention further still more preferably has a nucleobase sequence including at least 8, preferably 8, 9, 10, 11, 12, 13, 14, or 15 contiguous nucleobases of any one of nucleobase sequences of nucleobase sequence group C (C1 and C2), and further still more preferably has any one of nucleobase sequences of nucleobase sequence group C (C1 and C2).

Nucleobase sequence group A1:
A group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.

Nucleobase sequence group A2:
A group consisting of SEQ ID NOs: 488, 489, 490, 491, 492, 494, 513, 515, 533, 534, 544, 551, 552, 554, 566, 574, 582, 585, 586, 589, 592, 593, 595, 596, 638, 639, 642, 644, 661, 662, 663, 664, 665, 666, 667, 668, 669,670, 671, 672, 673, 674, 675, 676, 677, 696, 697, 699, 706, 713, 715, and 716.

Nucleobase sequence group A3:
A group consisting of SEQ ID NOs: 722, 723, 724, 727, 728, 729, 730, 731, 732, 733, 734, 735, 736, 737, 738, 739, 740, 741, 742, 743, 744, 745, 746,748, 750, 751, 752, 753, 754, 755, 756, 757, 758, 759, 760, 761, 762, 763, 764, 765, 766, 767, 768, 769, and 770.

Nucleobase sequence group B1:
A group consisting of SEQ ID NOs: 19, 37, 38, 47, 55, 59, 61, 62, 67, 68, 70, 72, 75, 78, 82, 83, 84, 85, 86, 88, 89, 91, 101, 102, 103, 104, 105, 106, 107, 109, 111, 112, 113, 114, 117, 120, 121, 122, 124, 128, 138, 151, 152, 153, 154, 156, 157, 159, 160, 161, 163, 168, 169, 171, 174, 180, 181, 184, 185, 186, 187, 188, 189, 190, 192, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 298, 299, 302, 310, 316, 320, 326, 327, 328, 332, 333, 337, 345, 346, 350, 355, 357, 358, 361, 362, 363, 364, 365, 366, 367, 372, 375, 376, 377, 378, 379, 382, 394, 395, 397, 398, 400, 401, 403, 404, 407, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 477, 478, 479, 480, 482, 483, 484, 485, and 486.

Nucleobase sequence group B2:
A group consisting of SEQ ID NOs: 488, 489, 490, 491, 492, 513, 515, 533, 534, 585, 586, 589, 592, 593, 595, 596, 661, 662, 663, 664, 665, 666, 667, 668, 669,670, 671, 672, 673, 674, 675, 676, 677, 697, 699, 706, 713, 715, and 716.

Nucleobase sequence group B3:
A group consisting of SEQ ID NOs: 722, 723, 724, 727, 728, 729, 730, 731, 732, 733, 734, 735, 736, 737, 738, 739, 740, 741, 742, 743, 744, 745, 746,748, 750, 751, 752, 753, 754, 755, 756, 757, 758, 759, 760, 761, 762, 763, 764, 765, 766, 767, 768, 769, and 770.

Nucleobase sequence group C1:
A group consisting of SEQ ID NOs: 128, 203, 204, 205, 206, 207, 208, 209, 215, 216, 218, 219, 220, 221, 222, 223, 224, 225, 226, 227, 228, 229, 230, 231, 232, 233, 234, 235, 236, 238, 239, 240, 363, 364, 365, 417, 418, 419, 420, 421, 422, 423, 424, 425, 426, 427, 428, 429, 430, 431, 432, 433, 434, 435, 436, 437, 438, 439, 440, 441, 442 444, 445, 446, 447, 448, 449, 450, 451, 452, 443, 454, 455, 456, 457, 458, 459, 460, 461, 462, 453 463, 464, 465, 466, and 467.

Nucleobase sequence group C2:
A group consisting of SEQ ID NOs: 488, 489, 490, 491, 492, 664, 665, 666, 667, 668, 669, 670, 671, 672, 673, 674, 675, 676, 677, 715, and 716.

In another aspect, the nucleobase sequence in the ASO of the present invention preferably has a nucleobase sequence including at least 8, preferably 8, 9, 10, 11, 12, 13, 14, or 15 contiguous nucleobases of any one of nucleobase sequences of nucleobase sequence groups D1, D2, and D4, and more preferably has any one of nucleobase sequences of nucleobase sequence groups D1, D2, and D4. In such an aspect, the ASO of the present invention consists of preferably 8 to 80, more preferably 12 to 80, still more preferably 12 to 50, further still more preferably 16 to 20, further still more preferably 16 to 18, and particularly preferably 16 nucleosides.

In another aspect, the nucleobase sequence in the ASO of the present invention preferably has a nucleobase sequence including at least 8, preferably 8, 9, 10, 11, 12, 13, 14, or 15 contiguous nucleobases of any one of nucleobase sequences of nucleobase sequence group D3, and more preferably has any one of nucleobase sequences of nucleobase sequence groups D3. In such an aspect, the ASO of the present invention consists of preferably 8 to 80, more preferably 12 to 80, still more preferably 12 to 50, further still more preferably 16 to 20, and particularly preferably 18 nucleosides.

As still another aspect, the nucleobase sequence in the ASO of the present invention more preferably has a nucleobase sequence including at least 8, preferably 8, 9, 10, 11, 12, 13, 14, or 15 contiguous nucleobases of any one of nucleobase sequences of nucleobase sequence group E, and still more preferably has any one of nucleobase sequences of nucleobase sequence group E. In such an aspect, the ASO of the present invention consists of preferably 8 to 80, more preferably 16 to 80, still more preferably 16 to 50, further still more preferably 16 to 20, and particularly preferably 20 nucleosides.

Nucleobase sequence group D1:
A group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, and 241.

Among the SEQ ID NOs listed in nucleobase sequence group D1, particularly preferable SEQ ID NOs are listed below.

SEQ ID NOs: 208, 215, 220, 221, and 222.

Nucleobase sequence group D2:
A group consisting of SEQ ID NOs: 494, 513, 515, 533, 534, 544, 551, 552, 554, 566, 574, 582, 585, 586, 589, 592, 593, 595, 596, 638, 639, 642, 644, 661, 696, 697, 699, 706, and 713.

Nucleobase sequence group D3:
A group consisting of SEQ ID NOs: 488, 489, 490, 491, 492, 662, 663, 664, 665, 666, 667, 668, 669, 670, 671, 672, 673, 674, 675, 676, 677, 715, and 716.

Nucleobase sequence group D4:
A group consisting of SEQ ID NOs: 722, 723, 724, 727, 728, 729, 730, 731, 732, 733, 734, 735, 736, 737, 738, 739, 740, 741, 742, 743, 744, 745, 746,748, 750, 751, 752, 753, 754, 755, 756, 757, 758, 759, 760, 761, 762, 763, 764, 765, 766, 767, 768, 769, and 770.

Nucleobase sequence group E:
A group consisting of SEQ ID NOs: 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.

An ASO having a nucleobase sequence including at least 8 contiguous nucleobases of any one of nucleobase sequences of nucleobase sequence groups D1 and D2 preferably includes at least one LNA and at least one 2'-O-MCE nucleoside in each of the wing segments. Above all, the ASO preferably includes one or two LNAs and one or two 2'-O-MCE nucleosides, and more preferably includes two LNAs and one 2'-O-MCE nucleoside, or one LNA and two 2'-O-MCE nucleosides. The ASO may include two LNAs and two 2'-O-MCE nucleosides. Particularly preferably, the ASO includes two LNAs and one 2'-O-MCE nucleoside.

An ASO having a nucleobase sequence including at least 8 contiguous nucleobases of any one of nucleobase sequences of nucleobase sequence group E includes particularly preferably at least one 2'-O-MOE nucleoside or at least one 2'-O-MCE nucleoside in each of the wing segments. Above all, the ASO preferably includes five 2'-O-MOE nucleosides or five 2'-O-MCE nucleosides, and particularly preferably includes five 2'-O-MOE nucleosides.

An ASO having a nucleobase sequence including at least 8 contiguous nucleobases of any one of nucleobase sequences of nucleobase sequence group D3 preferably includes at least one LNA and at least one 2'-O-MCE nucleoside in each of the wing segments. Above all, the ASO preferably includes one or two LNAs and one or two 2'-O-MCE nucleosides, and particularly preferably includes two LNAs and two 2'-O-MCE nucleosides.

An ASO having a nucleobase sequence including at least 8 contiguous nucleobases of any one of nucleobase sequences of nucleobase sequence group D4 preferably includes at least one LNA in each of the wing segments. Above all, the ASO preferably has one to three LNAs, and particularly preferably has three LNAs.

A functional molecule may be directly or indirectly bonded to the ASO of the present invention. The bond between the functional molecule and the ASO may be direct or indirect via another substance. However, the oligonucleotide and the functional molecule are preferably bonded to each other by a covalent bond, an ionic bond, or a hydrogen bond. More preferably, the oligonucleotide and the functional molecule are directly bonded to each other by a covalent bond or bonded to each other via a linker (linking group) by a covalent bond from a viewpoint of high bonding stability.

When the functional molecule is bonded to the ASO by a covalent bond, the functional molecule is preferably directly or indirectly bonded to a 3'-terminal or a 5'-terminal of the ASO molecule. The bonding between the linker or the functional molecule and a terminal nucleoside of the ASO molecule is selected depending on the functional molecule.

The linker or the functional molecule and the terminal nucleoside of the ASO molecule are preferably linked to each other by a phosphodiester bond or a modified phosphodiester bond, and more preferably linked to each other by a phosphodiester bond.

The linker or the functional molecule may be directly linked to an oxygen atom at a 3' position of a nucleoside at a 3'-terminal or an oxygen atom at a 5'-position of a nucleoside at a 5'-terminal of the ASO molecule.

The structure of "functional molecule" (conjugate) is not particularly limited, and bonding of the functional molecule to the ASO imparts a desired function to the ASO. Examples of the desired function include a labeling function, a purification function, and a function of delivery to a target site. Examples of a molecule that imparts a labeling function include compounds such as a fluorescent protein, luciferase, and the like. Examples of a molecule that impart a purification function include compounds such as biotin, avidin, His tag peptide, GST tag peptide, FLAG tag peptide, and the like.

In addition, a molecule having a function of delivering the ASO to a target site is preferably bonded as the functional molecule from a viewpoint of highly specifically and efficiently delivering the ASO to a target site (for example, brain or spinal cord) with very effectively controlling expression of an ATN1 gene by the ASO. For the molecule having the function of delivery, for example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to.

Examples of the functional molecule capable of efficiently delivering the ASO of the present invention to the brain, the spinal cord, and the like with high specificity include a lipid and a sugar (for example, glucose, sucrose, or the like). Examples of the lipid include: cholesterol; a fat-soluble vitamin such as vitamin E (a tocopherol or a tocotrienol), vitamin A, vitamin D, vitamin K, or the like; a fatty acid; an intermediate metabolite such as acylcarnitine, acyl-CoA or the like; a glycolipid; a glyceride; derivatives thereof; and the like.

As a functional molecule capable of delivering the ASO of the present invention to the liver with high specificity, a sugar derivative having an interaction with an asialoglycoprotein receptor may be used.

"Asialoglycoprotein receptor" is present on a surface of a liver cell, and has an action of recognizing a galactose residue of asialoglycoprotein and taking in and decomposing a molecule of the asialoglycoprotein into the cell. "Sugar derivative having an interaction with an asialoglycoprotein receptor" is preferably a compound that has a structure similar to that of a galactose residue and is taken into a cell by an interaction with an asialoglycoprotein receptor, and examples thereof include a GalNAc (N-acetylgalactosamine) derivative, a galactose derivative, and a lactose derivative.

Examples of a functional molecule capable of efficiently delivering the ASO to an organ with high specificity by interacting with various proteins on a surface of a cell of the organ include a ligand of a receptor, an antibody, and a peptide or a protein of fragments thereof.

The linker via which the functional molecule and the ASO are bonded to each other only needs to exhibit the function of the functional molecule as the ASO molecule, and therefore is not particularly limited as long as the linker stably bonds the functional molecule and the oligonucleotide to each other. Examples of the linker include a group derived from an oligonucleotide consisting of 1 to 20 nucleosides, a group derived from a polypeptide consisting of 1 to 20 amino acids, an alkylene consisting of 1 to 20 carbon atoms, and an alkenylene consisting of 2 to 20 carbon atoms. The group derived from an oligonucleotide consisting of 1 to 20 nucleosides is a divalent group obtained by removing a hydrogen atom or the like from each of a 3'-terminal and a 5'-terminal of an oligonucleotide consisting of 1 to 20 nucleosides (a nucleoside when the number of nucleosides is 1). For the group derived from an oligonucleotide consisting of 1 to 20 nucleosides, for example, WO 2017/053995 A can be referred to. WO 2017/053995 A describes, for example, a 3-base linker having a TCA motif, a 1- to 5-base linker having no TCA motif, and the like. The group derived from a polypeptide consisting of 1 to 20 amino acids is a divalent group obtained by removing two groups selected from hydroxy, a hydrogen atom, amino, and the like from a polypeptide consisting of 1 to 20 amino acids (an amino acid when the number of amino acids is 1).

A compound comprising the ASO of the present invention may include a prodrug moiety.

The prodrug is a derivative of a pharmaceutical compound having a group capable of being chemically or metabolically decomposed, and is a compound guided to a pharmacologically active pharmaceutical compound by solvolysis or decomposition in vivo under physiological conditions. A method for selecting and manufacturing an appropriate prodrug derivative is described, for example, in Design of Prodrugs (Elsevier, Amsterdam, 1985). Examples of a prodrug moiety such as an acyloxy derivative manufactured by reacting a hydroxy group of an ASO (or a compound containing an ASO) with a suitable acyl halide, a suitable acid anhydride, or a suitable halogenated alkyloxycarbonyl compound include -O-C(=O)C₂H₅, -O-C(=O)(t-Bu), -O-C(=O)C₁₅H₃₁, -O-C(=O)-(m-CO₂Na-Ph), -O-C(=O)CH₂CH₂CO₂Na, - OC(=O)CH(NH₂)CH₃, -O-C(=O)CH₂N(CH₃)₂, -O-CH₂OC(=O)CH₃, and the like.

The prodrug of the compound comprising the ASO of the present invention also includes, for example, a complex obtained by hybridization of two or more oligonucleotides. Examples of such a preferable structure (prodrug) include a doublestranded oligonucleotide (for example, WO 2013/089283 A, WO 2017/068791 A, WO 2017/068790 A, or WO 2018/003739 A), which includes an oligonucleotide complementary to an ASO (or a compound comprising an ASO), in which the complementary oligonucleotide includes an oligonucleotide including a ribonucleoside (for example, RNA), an oligonucleotide including a peptide nucleic acid (PNA), or an oligonucleotide including a deoxyribonucleoside (for example, DNA). Examples of the preferable structure (prodrug) include a single-stranded oligonucleotide in which an RNA complementary to an ASO is bonded by a linker (for example, WO 2017/131124 A or WO 2018/143475 A), and the like. The linker may be an oligonucleotide linker or a linker including a non-nucleoside structure. Examples of the preferable structure (prodrug) also include a single-stranded oligonucleotide in which an RNA complementary to ASO is directly linked (WO 2019/022196 A).

The compound of the present invention includes a compound in which two identical or different ASOs are linked to each other. For a structure in which two ASOs are linked to each other, for example, WO 2017/131124 A and WO 2018/143475 A can be referred to.

The compound comprising an ASO includes those present via tautomerism thereof or geometric isomerism thereof, and also includes those present as a mixture thereof or a mixture of isomers thereof. When an asymmetric center is present, or when an asymmetric center is generated as a result of isomerization, the compound comprising an ASO also includes those present as each optical isomer and those present as a mixture thereof at any ratio. In a case of a compound having two or more asymmetric centers, diastereomers due to optical isomerism of each of the asymmetric centers are also present. The present invention also includes those including all these types at any ratio. An optically active form can be obtained by a well-known method for this purpose.

For example, when the compound comprising the ASO of the present invention includes a modified phosphodiester bond (for example, a phosphorothioate bond) and the phosphorus atom is an asymmetric atom, both forms of an oligonucleotide in which a stereoscopic effect of the phosphorus atom is controlled and an oligonucleotide in which the stereoscopic effect of the phosphorus atom is not controlled are included in the scope of the present invention.

The compound comprising the ASO of the present invention, a prodrug thereof, or a pharmacologically acceptable salt thereof can be present in any crystalline form depending on manufacturing conditions, and can be present in any hydrate, but these crystalline forms, hydrates, and mixtures thereof are also included in the scope of the present invention. In addition, the compound comprising the ASO of the present invention, a prodrug thereof, or a pharmacologically acceptable salt thereof may be present as a solvate containing an organic solvent such as acetone, ethanol, 1-propanol, or 2-propanol, but all of these forms are included in the scope of the present invention.

The compound comprising the ASO of the present invention can be converted into a pharmacologically acceptable salt or can be liberated from a generated salt, as necessary. Examples of the pharmacologically acceptable salt of the compound comprising the ASO include salts with an alkali metal (lithium, sodium, potassium, or the like), an alkaline earth metal (calcium or the like), magnesium, ammonium, an organic base (triethylamine, trimethylamine, or the like), an amino acid (glycine, lysine, glutamic acid, or the like), an inorganic acid (hydrochloric acid, hydrobromic acid, phosphoric acid, sulfuric acid, or the like), or an organic acid (acetic acid, citric acid, maleic acid, fumaric acid, tartaric acid, benzenesulfonic acid, methanesulfonic acid, p-toluenesulfonic acid, or the like).

In particular, a partial structure represented by -P(=O)(OH)- may be converted into an anionic partial structure represented by -P(=O)(O⁻)- to form a salt with an alkali metal (lithium, sodium, potassium, or the like), an alkaline earth metal (calcium or the like), magnesium, ammonium, or the like. A partial structure represented by - P(=O)(SH)- that forms a phosphorothioate bond may be converted into an anionic partial structure represented by -P(=O)(S⁻)- to form a salt with an alkali metal, an alkaline earth metal, ammonium, or the like similarly. The same applies to other modified phosphodiester bonds.

The pharmacologically acceptable salt is particularly preferably a sodium salt.

The compound comprising the ASO of the present invention can be prepared by appropriately selecting a method known to a person skilled in the art. For example, a person skilled in the art can synthesize the compound comprising the ASO of the present invention by designing a nucleoside sequence of the ASO based on information of a nucleoside sequence of a target RNA, and using a commercially available automatic nucleic acid synthesizer (manufactured by Applied Biosystems, manufactured by Beckman, manufactured by GeneDesign Inc., or the like). The compound comprising the ASO of the present invention can also be synthesized by a reaction using an enzyme. Examples of the enzyme include polymerase, ligase, restriction enzyme, and the like, but are not limited thereto. That is, the method for manufacturing the compound comprising the ASO according to the present embodiment can include a step of extending a nucleoside chain at a 3'-terminal or a 5'-terminal.

Many methods for bonding a functional molecule and an oligonucleotide to each other are well known in the present field. For example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to. For example, a functional molecule and a linker are bonded to each other by a known method, then the bonded product is guided to an amidite form with an amiditization reagent or an H-phosphonate form with an H-phosphonate reagent, and can be bonded to an oligonucleotide.

By purifying the resulting oligonucleotide by reverse phase column chromatography or the like, a compound comprising an ASO can be prepared.

The compound comprising the ASO of the present invention or a pharmacologically acceptable salt thereof can effectively inhibit expression of an ATN1 gene. A disease that can be treated, prevented, and ameliorated by a nucleic acid pharmaceutical using the compound comprising the ASO of the present invention or a pharmacologically acceptable salt thereof is not particularly limited as long as it is a disease to which an ATN1 gene expression inhibitory action is effective, but examples thereof include dentatorubral-pallidoluysian atrophy.

The present invention can provide a composition containing the compound comprising the ASO as an active ingredient, for example, for inhibiting expression of an ATN1 gene by an antisense effect. In particular, the compound comprising the ASO of the present invention can also provide a pharmaceutical composition for treating, preventing, and/or ameliorating a disease to which inhibition of expression of an ATN1 gene is effective, such as dentatorubral-pallidoluysian atrophy.

A pharmaceutical composition containing the compound comprising the ASO of the present invention or a pharmacologically acceptable salt thereof can be formulated by a known pharmaceutical method. For example, the pharmaceutical composition can be used transintestinally (orally or the like) or non-transintestinally as an injection, a capsule, a tablet, a pill, a liquid, a powder, a granule, a fine granule, a film coating agent, a pellet, a troche, a sublingual agent, a chewing agent, a buccal agent, a paste, a syrup, a suspension agent, an elixir agent, an emulsion, a coating agent, an ointment, a plaster, a cataplasm, a transdermal absorption formulation, a lotion, an inhalation agent, an aerosol agent, a suppository, or the like.

In the formulation, the pharmaceutical composition can be appropriately combined with a pharmacologically acceptable carrier or a carrier acceptable as food/drink, specifically, sterile water, physiological saline, vegetable oil, a solvent, a base, an emulsifier, a suspension agent, a surfactant, a pH adjuster, a stabilizer, a flavoring agent, a fragrance, an excipient, a vehicle, a preservative, a binder, a diluent, an isotonizing agent, a soothing agent, an extender, a disintegrant, a buffer, a coating agent, a lubricant, a coloring agent, a sweetening agent, a thickening agent, a corrective, a solubilizing agent, other additives, or the like.

An administration form of the composition containing the compound comprising the ASO of the present invention or a pharmacologically acceptable salt thereof is not particularly limited, and examples thereof include transintestinal (oral or the like) and non-transintestinal administration. More preferable examples thereof include intravenous administration, intraarterial administration, intraperitoneal administration, subcutaneous administration, intradermal administration, airway administration, rectal administration, intramuscular administration, intrathecal administration, intracerebroventricular administration, nasal administration, intravitreal administration, and administration by infusion. Still more preferable examples thereof include intrathecal administration, intracerebroventricular administration, and nasal administration. Particularly preferable examples thereof include intrathecal administration.

A formulation containing the compound comprising the ASO of the present invention or a pharmacologically acceptable salt thereof and making intrathecal administration possible can be manufactured by a known conventional method. For example, a preparation obtained by adding an additive such as a buffer or an isotonizing agent to the compound comprising the ASO of the present invention or a pharmacologically acceptable salt thereof is completely dissolved in water for injection, and then the resulting preparation solution is sterilized by filtration. A prefilled syringe formulation can be manufactured by filling the preparation solution into a sterilized syringe, a formulation for injection can be manufactured by filling the preparation solution into a sterilized vial, and a formulation for preparation at the time of use can be manufactured by filling the preparation solution into a sterilized vial and then freeze-drying the preparation solution. Instead of the filtration sterilization, terminal sterilization such as autoclaving or gamma irradiation may be performed.

Various mammalian diseases can be treated, prevented and/or ameliorated by the composition containing the compound comprising the ASO of the present invention or a pharmacologically acceptable salt thereof. For example, diseases in mammals including but not limited to a human, a cow, a sheep, a goat, a horse, a dog, a cat, a gerbil, rat, a rabbit, a chimpanzee, a bovine, an ovine, an equine, a canine, a feline, and a rodent species such as a mouse can be treated, prevented and/or ameliorated. The mammal is particularly preferably a human.

When the composition containing the compound comprising the ASO of the present invention or a pharmacologically acceptable salt thereof is administered to or ingested by a mammal including a human, a dose thereof or an intake thereof is preferably an effective amount. The effective amount means the sufficient amount of the compound to exhibit a desired pharmacological effect in an individual who needs a drug, changes depending on the age, weight, symptom, and health condition of an individual whose disease is treated, prevented, and/or ameliorated, blending in the composition, and the like, and is appropriately selected. The dose or the intake is preferably 0.0001 mg/kg/day to 100 mg/kg/day in terms of ASO.

Examples of a preferable method using the ASO of the present invention include those described below.

A method for controlling expression of an ATN1 gene, the method including a step of bringing a cell into contact with the compound comprising the ASO of the present invention or a pharmacologically acceptable salt thereof.

A method for controlling expression of an ATN1 gene in a mammal, the method including a step of administering a pharmaceutical composition containing the compound comprising the ASO of the present invention or a pharmacologically acceptable salt thereof to the mammal.

Use of the compound comprising the ASO of the present invention or a pharmacologically acceptable salt thereof for controlling expression of an ATN1 gene in a mammal.

Use of the compound comprising the ASO of the present invention or a pharmacologically acceptable salt thereof for manufacturing a drug for controlling expression of an ATN1 gene in a mammal.

### Examples

Hereinafter, the present invention will be described more specifically based on Examples, but the embodiments of the present invention are not limited to the following Examples.

In Tables (1 to 53) in Examples, "Cmpd No" means a compound number.

In the notation of a chemical structure of a sequence in Examples (chemical structures of Tables 1 to 10, 26 to 33, and 48 and 51), unless otherwise specified, "(L)" means LNA, "(V)" means 2'-O-MCE nucleoside, "(m)" means 2'-O-MOE nucleoside, a lower case alphabet (excluding "m" in the (m)) means deoxyribonucleoside, "^" means a phosphorothioate bond, "5(x)" means that a nucleobase of the deoxyribonucleoside is 5-methylcytosine, and "5" in" 5(m)", "5(V)", and,"5(L)" means that a nucleobase of the nucleoside is 5-methylcytosine.

Note that in the notation of a chemical structure (Tables 1 to 10, 26 to 33, 48, and 51) in Examples, when "^" is not described between two adjacent nucleobases, the internucleoside bond between the two nucleobases is a phosphodiester bond. For example, in the notation "A(L)G(L)", the internucleoside bond between A and G is a phosphodiester bond, and in the notation "G(L)a", the internucleoside bond between G and a is a phosphodiester bond.

### (Manufacturing Example 1)

Antisense oligonucleotides (compounds represented by chemical structures corresponding to compound numbers) described in Tables 1 to 10 were prepared using an automatic nucleic acid synthesizer nS-8II (manufactured by GeneDesign Inc.) or 3900 DNA Synthesizer (manufactured by Applied Biosystems Inc.). Note that the title rows in Tables 2 to 10 are the same as that in Table 1, and thus are omitted.

**[Table 1]**

| Cmpd No | Chemical structure |
|---|---|
| M-0001 | T(m)^5(m)^A(m)^A(m)^T(m)^g^a^a^a^5(x)^5(x)^t^g^5(x)^t^G(m)^G(m)^T(m)^A(m)^G(m) |
| M-0002 | 5(m)^T(m)^G(m)^T(m)^T(m)^t^t^5(x)^a^a^t^g^a^a^a^5(m)^5(m)^T(m)^G(m)^5(m) |
| M-0003 | G(m)^A(m)^T(m)^5(m)^T(m)^g^t^t^t^t^5(x)^a^a^t^g^A(m)^A(m)^A(m)^5(m)^5(m) |
| M-0004 | A(m)^G(m)^G(m)^A(m)^T(m)^5(x)^t^g^t^t^t^t^5(x)^a^a^T(m)^G(m)^A(m)^A(m)^A(m) |
| M-0005 | 5(m)^T(m)^T(m)^T(m)^T(m)^g^5(x)^a^g^g^a^t^5(x)^t^g^T(m)^T(m)^T(m)^T(m)^5(m) |
| M-0006 | G(m)^A(m)^T(m)^5(m)^T(m)^5(x)^5(x)^a^a^g^t^t^t^5(x)^5(x)^A(m)^G(m)^T(m)^G(m)^T(m) |
| M-0007 | A(m)^G(m)^5(m)^A(m)^G(m)^g^a^t^5(x)^t^5(x)^5(x)^a^a^g^T(m)^T(m)^T(m)^5(m)^5(m) |
| M-0008 | G(m)^G(m)^G(m)^A(m)^A(m)^g^5(x)^a^g^g^a^t^5(x)^t^5(x)^5(m)^A(m)^A(m)^G(m)^T(m) |
| M-0009 | A(m)^5(m)^A(m)^G(m)^A(m)^a^a^5(x)^t^t^5(x)^t^5(x)^t^g^5(m)^T(m)^5(m)^5(m)^A(m) |
| M-0010 | A(m)^T(m)^A(m)^5(m)^A(m)^g^a^a^a^5(x)^t^t^5(x)^t^t^5(x)^T(m)^G(m)^5(m)^T(m)^5(m) |
| M-0011 | T(m)^G(m)^A(m)^A(m)^T(m)^a^5(x)^a^g^a^a^a^5(x)^t^t^5(m)^T(m)^5(m)^T(m)^G(m) |
| M-0012 | 5(m)^A(m)^G(m)^5(m)^T(m)^g^a^a^t^a^5(x)^a^g^a^a^A(m)^5(m)^T(m)^T(m)^5(m) |
| M-0013 | 5(m)^A(m)^G(m)^G(m)^5(m)^t^g^t^g^g^a^g^a^5(x)^5(x)^5(m)^5(m)^A(m)^T(m)^T(m) |
| M-0014 | G(m)^T(m)^5(m)^T(m)^T(m)^5(x)^a^t^t^5(x)^t^t^5(x)^a^g^G(m)^5(m)^T(m)^G(m)^T(m) |
| M-0015 | 5(m)^G(m)^T(m)^G(m)^T(m)^5(x)^t^t^5(x)^a^t^t^5(x)^t^t^5(m)^A(m)^G(m)^G(m)^5(m) |
| M-0016 | 5(m)^T(m)^G(m)^T(m)^5(m)^g^t^g^t^5(x)^t^t^5(x)^a^t^T(m)^5(m)^T(m)^T(m)^5(m) |
| M-0017 | 5(m)^A(m)^T(m)^5(m)^G(m)^a^g^t^5(x)^t^t^t^a^t^t^5(m)^T(m)^G(m)^T(m)^5(m) |
| M-0018 | A(m)^5(m)^T(m)^5(m)^5(m)^t^5(x)^a^t^t^g^a^5(x)^a^t^5(m)^G(m)^A(m)^G(m)^T(m) |
| M-0019 | 5(m)^A(m)^5(m)^T(m)^5(m)^5(x)^t^5(x)^a^t^t^g^a^5(x)^a^T(m)^5(m)^G(m)^A(m)^G(m) |
| M-0020 | T(m)^5(m)^5(m)^A(m)^5(m)^t^5(x)^5(x)^t^5(x)^a^t^t^g^a^5(m)^A(m)^T(m)^5(m)^G(m) |
| M-0021 | 5(m)^T(m)^T(m)^5(m)^5(m)^g^t^5(x)^5(x)^a^5(x)^t^5(x)^5(x)^t^5(m)^A(m)^T(m)^T(m)^G(m) |
| M-0022 | 5(m)^T(m)^5(m)^G(m)^A(m)^t^5(x)^t^5(x)^a^g^t^t^5(x)^t^T(m)^5(m)^5(m)^5(m)^G(m) |
| M-0023 | 5(m)^A(m)^G(m)^5(m)^T(m)^t^t^g^5(x)^5(x)^a^t^5(x)^a^5(x)^T(m)^G(m)^5(m)^T(m)^G(m) |
| M-0024 | T(m)^5(m)^T(m)^5(m)^A(m)^g^5(x)^t^t^t^g^5(x)^5(x)^a^t^5(m)^A(m)^5(m)^T(m)^G(m) |
| M-0025 | T(m)^T(m)^5(m)^T(m)^5(m)^a^g^5(x)^t^t^t^g^5(x)^5(x)^a^T(m)^5(m)^A(m)^5(m)^T(m) |
| M-0026 | T(m)^G(m)^5(m)^5(m)^T(m)^g^g^a^5(x)^t^t^5(x)^t^5(x)^a^G(m)^5(m)^T(m)^T(m)^T(m) |
| M-0027 | 5(m)^T(m)^G(m)^5(m)^5(m)^t^g^g^a^5(x)^t^t^5(x)^t^5(x)^A(m)^G(m)^5(m)^T(m)^T(m) |
| M-0028 | T(m)^T(m)^G(m)^T(m)^T(m)^g^a^5(x)^5(x)^t^t^t^g^g^g^G(m)^T(m)^G(m)^G(m)^A(m) |
| M-0029 | G(m)^A(m)^5(m)^5(m)^5(m)^t^g^5(x)^t^t^g^t^t^g^a^5(m)^5(m)^T(m)^T(m)^T(m) |
| M-0030 | T(m)^5(m)^T(m)^5(m)^T(m)^g^a^g^a^t^5(x)^t^5(x)^5(x)^t^5(m)^A(m)^5(m)T(m)^5(m) |
| M-0031 | T(m)^5(m)^A(m)^5(m)^T(m)^5(x)^t^5(x)^t^g^a^g^a^t^5(m)^T(m)^5(m)^5(m)^T(m)^5(m) |
| M-0032 | A(m)^5(m)^T(m)^T(m)^T(m)^5(x)^a^5(x)^t^5(x)^t^5(x)^t^g^a^G(m)^A(m)^T(m)^5(m)^T(m) |
| M-0033 | 5(m)^5(m)^T(m)^5(m)^A(m)^5(x)^t^t^t^5(x)^a^5(x)^t^5(x)^t^5(m)^T(m)^G(m)^A(m)^G(m) |
| M-0034 | 5(m)^T(m)^5(m)^5(m)^T(m)^5(x)^a^5(x)^t^t^t^5(x)^a^5(x)^t^5(m)^T(m)^5(m)^T(m)^G(m) |
| M-0035 | G(m)^G(m)^T(m)^5(m)^T(m)^5(x)^5(x)^t^5(x)^a^5(x)^t^t^t^5(x)^A(m)^5(m)^T(m)^5(m)^T(m) |
| M-0036 | T(m)^T(m)^G(m)^G(m)^T(m)^5(x)^t^t^t^t^t^t^g^g^t^G(m)^5(m)^A(m)^T(m)^T(m) |
| M-0037 | 5(m)^T(m)^5(m)^A(m)^G(m)^t^t^t^g^g^t^5(x)^t^t^T(m)^T(m)^T(m)^T(m)^G(m) |
| M-0038 | T(m)^5(m)^5(m)^T(m)^G(m)^5(x)^t^5(m)^a^g^t^t^t^t^g^G(m)^T(m)^5(m)^T(m)^T(m) |
| M-0039 | A(m)^T(m)^5(m)^G(m)^G(m)^a^g^g^g^a^g^a^5(x)^t^g^T(m)^G(m)^G(m)^5(m)^5(m) |
| M-0040 | 5(m)^5(m)^A(m)^A(m)^G(m)^5(x)^t^a^t^5(x)^5(x)^a^g^a^t^5(m)^G(m)^G(m)^A(m)^G(m) |
| M-0041 | A(m)^T(m)^5(m)^A(m)^T(m)^5(x)^a^t^t^a^a^g^g^5(x)^t^5(m)^5(m)^G(m)^5(m)^5(m) |
| M-0042 | 5(m)^5(m)^A(m)^T(m)^5(m)^a^t^5(x)^a^t^t^a^a^g^g^5(m)^T(m)^5(m)^5(m)^G(m) |
| M-0043 | T(m)^G(m)^5(m)^5(m)^A(m)^t^5(x)^a^t^5(x)^a^t^t^a^a^G(m)^G(m)^5(m)^T(m)^5(m) |
| M-0044 | 5(m)^T(m)^G(m)^5(m)^5(m)^a^t^5(x)^a^t^5(x)^a^t^t^a^A(m)^G(m)^G(m)^5(m)^T(m) |
| M-0045 | T(m)^G(m)^5(m)^T(m)^G(m)^5(x)^5(x)^a^t^5(x)^a^t^5(x)^a^t^T(m)^A(m)^A(m)^G(m)^G(m) |
| M-0046 | G(m)^5(m)^T(m)^G(m)^5(m)^t^g^5(x)^5(x)^a^t^5(x)^a^t^5(x)^A(m)^T(m)^T(m)^A(m)^A(m) |
| M-0047 | 5(m)^5(m)^A(m)^G(m)^G(m)^g^5(x)^t^g^t^a^g^a^t^a^5(m)^T(m)^G(m)^G(m)^G(m) |
| M-0048 | T(m)^5(m)^A(m)^T(m)^T(m)^5(x)^t^5(x)^5(x)^a^5(x)^a^5(x)^t^t^5(m)^5(m)^A(m)^G(m)^G(m) |
| M-0049 | G(m)^T(m)^5(m)^A(m)^G(m)^a^g^t^5(x)^a^t^t'5(x)^t^5(x)^5(m)^A(m)^5(m)^A(m)^5(m) |
| M-0050 | G(m)^A(m)^G(m)^T(m)^5(m)^a^g^a^g^t^5(x)^a^t^t^5(x)^T(m)^5(m)^5(m)^A(m)^5(m) |

**[Table 2]**

| | |
|---|---|
| M-0051 | A(m)^T(m)^G(m)^A(m)^G(m)^t^5(x)^a^g^a^g^t^5(x)^a^t^T(m)^5(m)^T(m)^5(m)^5(m) |
| M-0052 | A(m)^A(m)^G(m)^A(m)^T(m)^g^a^g^t^5(x)^a^g^a^g^t^5(m)^A(m)^T(m)^T(m)^5(m) |
| M-0053 | G(m)^A(m)^5(m)^A(m)^G(m)^g^5(x)5(x)^a^g^a^a^g^a^t^G(m)^A(m)^G(m)^T(m)^5(m) |
| M-0054 | T(m)^T(m)^5(m)^A(m)^A(m)^a^g^5(x)^t^a^g^5(x)^5(x)^t^5(x)^T(m)^G(m)^G(m)^5(m)^T(m) |
| M-0055 | G(m)^G(m)^G(m)^T(m)^T(m)^5(x)^a^a^a^g^5(x)^t^a^g^5(x)^5(m)^T(m)^5(m)^T(m)^G(m) |
| M-0056 | A(m)^G(m)^T(m)^G(m)^G(m)^g^t^g^t^5(x)^a^5(x)^a^g^a^A(m)^G(m)^G(m)^A(m)^T(m) |
| M-0057 | G(m)^A(m)^G(m)^5(m)^A(m)^t^g^a^t^a^t^5(x)^5(x)^a^g^T(m)^G(m)^G(m)^G(m)^T(m) |
| M-0058 | G(m)^5(m)^5(m)^5(m)^5(m)^5(x)^a^g^g^a^t^a^g^a^g^5(m)^T(m)^G(m)^T(m)^G(m) |
| M-0059 | 5(m)^A(m)^G(m)^A(m)^5(m)^a^a^a^a^5(x)^t^5(x)^5(x)^a^5(x)^5(m)^A(m)^G(m)^T(m)^G(m) |
| M-0060 | T(m)^5(m)^5(m)^A(m)^G(m)^a^5(x)^a^a^a^a^5(x)^t^5(x)^5(x)^A(m)^5(m)^5(m)^A(m)^G(m) |
| M-0061 | 5(m)^5(m)^5(m)^5(m)^5(m)^5(x)^t^t^g^g^g^a^5(x)^5(x)^5(x)^A(m)^T(m)^T(m)^G(m)^G(m) |
| M-0062 | G(m)^T(m)^G(m)^5(m)^T(m)^g^5(x)^t^t^a^5(x)^5(x)^5(x)^5(x)^5(x)^A(m)^(m)^T(m)^A(m)^G(m) |
| M-0063 | 5(m)^T(m)^G(m)^A(m)^A(m)^a^t^g^g^g^a^g^t^a^g^T(m)^G(m)^G(m)^G(m)^T(m) |
| M-0064 | A(m)^T(m)^A(m)^5(m)^T(m)^g^a^a^a^t^g^g^g^a^g^T(m)^A(m)^G(m)^T(m)^G(m) |
| M-0065 | 5(m)^A(m)^G(m)^A(m)^G(m)^5(x)^t^t^g^a^t^a^5(x)^t^g^A(m)^A(m)^A(m)^T(m)^G(m) |
| M-0066 | 5(m)^5(m)^5(m)^5(m)^A(m)^g^a^g%5(x)^t^t^g^a^t^a^5(m)^T(m)^G(m)^A(m)^A(m) |
| M-0067 | T(m)^A(m)^G(m)^5(m)^5(m)^5(x)^5(x)^a^g^a^g^5(x)^t^t^g^A(m)^T(m)^A(m)^5(m)^T(m) |
| M-0068 | G(m)^5(m)^A(m)^5(m)^5(m)^a^5(x)^t^a^g^5(x)^5(x)^5(x)^5(x)^a^G(m)^A(m)^G(m)^5(m)^T(m) |
| M-0069 | 5(m)^A(m)^5(m)^5(m)^5(m)^a^5(x)^t^g^g^a^g^t^g^g^T(m)^A(m)^G(m)^G(m)^5(m) |
| M-0070 | G(m)^T(m)^G(m)^G(m)^A(m)^g^5(x)^a^g^a^a^g^g^t^a^G(m)^G(m)^T(m)^T(m)^5(m) |
| M-0071 | A(m)^5(m)^A(m)^T(m)^G(m)^g^g^g^g^a^a^g^t^t^g^G(m)^5(m)^T(m)^G(m)^G(m) |
| M-0072 | A(m)^G(m)^G(m)^5(m)^T(m)^g^a^t^g^5(x)^a^t^t^g^t^T(m)^G(m)^A(m)^G(m)^G(m) |
| M-0073 | G(m)^A(m)^T(m)^G(m)^A(m)^5(x)^5(x)^a^g^g^t^a^g^t^g^G(m)^T(m)^T(m)^G(m)^G(m) |
| M-0074 | A(m)^G(m)^A(m)^G(m)^G(m)^g^5(x)^a^g^a^t^g^a^5(x)^5(x)^A(m)^G(m)^G(m)^T(m)^A(m) |
| M-0075 | G(m)^A(m)^G(m)^5(m)^A(m)^g^a^a^g^a^g^g^a^a^g^5(m)^A(m)^G(m)^G(m)^A(m) |
| M-0076 | T(m)^G(m)^A(m)^A(m)^T(m)^a^a^g^g^a^a^a^5(x)^5(x)^t^5(m)^A(m)^T(m)^G(m)^G(m) |
| M-0077 | A(m)^5(m)^T(m)^A(m)^5(m)^t^a^g^a^g^g^a^t^g^a^A(m)^T(m)^A(m)^A(m)^G(m) |
| M-0078 | G(m)^5(m)^T(m)^A(m)^5(m)^t^a^5(x)^t^a^g^a^g^g^a^T(m)^G(m)^A(m)^A(m)^T(m) |
| M-0079 | 5(m)^T(m)^G(m)^5(m)^T(m)^g^5(x)^a^g^a^g^5(x)^t^a^5(x)^T(m)^A(m)^5(m)^T(m)^A(m) |
| M-0080 | G(m)^A(m)^A(m)^5(m)^T(m)^g^g^a^a^g^a^g^a^G(m)^5(m)^T(m)^G(m)^5(m) |
| M-0081 | G(m)^A(m)^A(m)^G(m)^A(m)^g^g^a^g^g^a^a^g^a^a^5(m)^T(m)^G(m)^G(m)^A(m) |
| M-0082 | T(m)^G(m)^5(m)^5(m)^T(m)^g^g^g^a^a^g^5(x)^t^g^g^G(m)^A(m)^A(m)^G(m)^G(m) |
| M-0083 | G(m)^5(m)^5(m)^5(m)^5(m)^a^g^t^a^g^a^g^g^g^a^G(m)^G(m)^G(m)^A(m)^A(m) |
| M-0084 | G(m)^T(m)^G(m)^A(m)^T(m)^g^t^g^t^t^g^a^g^a^5(x)^T(m)^G(m)^G(m)^T(m)^G(m) |
| M-0085 | A(m)^T(m)^G(m)^G(m)^5(m)^g^t^a^a^g^g^g^t^g^t^G(m)^5(m)^G(m)^T(m)^G(m) |
| M-0086 | 5(m)^5(m)^5(m)^T(m)^G(m)^g^t^g^g^g^t^a^g^g^g^5(m)^5(m)^T(m)^5(m)^A(m) |
| M-0087 | 5(m)^5(m)^T(m)^G(m)^5(m)^t^t^g^g^5(x)^t^g^t^a^g^G(m)^A(m)^5(m)^A(m)^5(m) |
| M-0088 | A(m)^G(m)^A(m)^G(m)^G(m)^a^a^g^a^g^a^5(x)^t^g^g^A(m)^G(m)^G(m)^G(m)^5(m) |
| M-0089 | G(m)^A(m)^5(m)^5(m)^5(m)^t^t^g^a^g^a^a^g^t^g^G(m)^A(m)^A(m)^G(m)^A(m) |
| M-0090 | 5(m)^A(m)^5(m)^5(m)^G(m)^g^t^g^g^a^a^a^g^G(m)^T(m)^A(m)^G(m)^G(m) |
| M-0091 | 5(m)^5(m)^G(m)^T(m)^G(m)^g^a^a^a^g^g^g^t^a^g^5(m)^5(m)^G(m)^A(m)^A(m) |
| M-0092 | A(m)^G(m)^G(m)^5(m)^5(m)^g^t^t^t^t^g^t^a^g^5(x)^5(m)^T(m)^G(m)^5(m)^T(m) |
| M-0093 | G(m)^G(m)^T(m)^G(m)^G(m)^5(x)^t^g^t^5(x)^t^t^g^t^a^G(m)^G(m)^5(m)^5(m)^5(m) |
| M-0094 | 5(m)^T(m)^5(m)^5(m)^T(m)^g^t^t^t^g^a^t^5(x)^t^g^5(m)^G(m)^T(m)^G(m)^G(m) |
| M-0095 | 5(m)^T(m)^5(m)^T(m)^5(m)^g^g^g^g^g^t^5(x)^t^5(x)^a^T(m)^A(m)^5(m)^T(m)^5(m) |
| M-0096 | G(m)^T(m)^A(m)^5(m)^A(m)^t^5(x)^t^a^5(x)^5(x)^a^5(x)^5(x)^t^T(m)^G(m)^G(m)^G(m)^A(m) |
| M-0097 | 5(m)^T(m)^G(m)^G(m)^G(m)^t^a^5(x)^a^t^5(x)^t^a^5(x)^5(x)^A(m)^5(m)^5(m)^T(m)^T(m) |
| M-0098 | 5(m)^A(m)^G(m)^A(m)^5(m)^t^g^a^5(x)t^g^g^5(x)^a^t^G(m)^G(m)^5(m)^T(m)^G(m) |
| M-0099 | 5(m)^G(m)^A(m)^T(m)^5(m)^5(x)^a^g^g^t^g^t^t^t^g^T(m)^T(m)^G(m)^A(m)^A(m) |
| M-0100 | 5(m)^G(m)^5(m)^A(m)^5(m)^g^a^g^t^t^g^a^a^g^5(x)^5(m)^G(m)^5(m)^G(m)^A(m) |

**[Table 3]**

| | |
|---|---|
| M-0101 | G(m)^G(m)^5(m)^A(m)^5(m)^g^a^a^g^t^a^5(x)^a^g^g^T(m)^5(m)^G(m)^5(m)^T(m) |
| M-0102 | T(m)^5(m)^5(m)^T(m)^G(m)^a^g^5(x)^5(x)^a^a^5(x)^t^t^5(x)^A(m)^5(m)^G(m)^5(m)^T(m) |
| M-0103 | G(m)^G(m)^A(m)^G(m)^T(m)^g^t^5(x)^a^g^g^a^5(x)^5(x)^5(x)^A(m)^G(m)^G(m)^T(m)^A(m) |
| M-0104 | T(m)^A(m)^T(m)^T(m)^5(m)^a^5(x)^t^g^a^g^a^g^t^g^5(m)^G(m)^5(m)^A(m)^A(m) |
| M-0105 | 5(m)^A(m)^G(m)^G(m)^A(m)^g^a^5(x)^a^t^g^a^5(x)^a^t^G(m)^A(m)^G(m)^G(m)^5(m) |
| M-0106 | 5(m)^5(m)^A(m)^G(m)^G(m)^a^g^a^5(x)^a^t^g^a^5(x)^a^T(m)^G(m)^A(m)^G(m)^G(m) |
| M-0107 | G(m)^G(m)^5(m)^A(m)^5(m)^g^t^a^g^a^a^t^g^g^a^T(m)^G(m)^G(m)^T(m)^T(m) |
| M-0108 | G(m)^G(m)^A(m)^5(m)^A(m)^t^t^g^t^a^a^5(x)^5(x)^5(x)^a^G(m)^G(m)^A(m)^G(m)^5(m) |
| M-0109 | A(m)^T(m)^5(m)^A(m)^5(m)^t^g^5(x)^t^g^t^a^5(x)^a^g^G(m)^G(m)^5(m)^5(m)^G(m) |
| M-0110 | T(m)^T(m)^5(m)^A(m)^5(m)^5(x)^t^5(x)^a^a^a^g^5(x)^5(x)^a^G(m)^G(m)^5(m)^T(m)^T(m) |
| M-0111 | T(m)^5(m)^A(m)^5(m)^T(m)^a^g^g^5(x)^t^t^5(x)^a^5(x)^5(x)^T(m)^5(m)^A(m)^A(m)^A(m) |
| M-0112 | T(m)^T(m)^5(m)^5(m)^A(m)^g^5(x)^t^5(x)^a^5(x)^t^a^g^g^5(m)^T(m)^T(m)^5(m)^A(m) |
| M-0113 | A(m)^T(m)^G(m)^T(m)^A(m)^g^g^g^g^t^t^5(x)^5(x)^a^g^5(m)^T(m)^5(m)^A(m)^5(m) |
| M-0114 | 5(m)^G(m)^G(m)^5(m)^5(m)^5(x)^a^g^g^g^a^5(x)^5(x)^5(x)^5(x)^A(m)^T(m)^G(m)^T(m)^A(m) |
| M-0115 | G(m)^5(m)^T(m)^5(m)^G(m)^g^a^t^g^a^a^a^g^g^g^G(m)^A(m)^A(m)^A(m)^G(m) |
| M-0116 | G(m)^5(m)^T(m)^5(m)^A(m)^g^5(x)^a^t^a^g^g^a^5(x)^a^T(m)^G(m)^T(m)^5(m)^A(m) |
| M-0117 | 5(m)^G(m)^G(m)^G(m)^5(m)^5(x)^a^g^t^g^g^g^t^5(x)^a^T(m)^T(m)^G(m)^5(m)^5(m) |
| M-0118 | A(m)^G(m)^T(m)^5(m)^A(m)^5(x)^a^t^t^g^a^g^5(x)^a^t^5(m)^T(m)^G(m)^5(m)^A(m) |
| M-0119 | G(m)^G(m)^G(m)^A(m)^G(m)^t^5(x)^a^5(x)^a^t^t^g^a^a^5(m)^A(m)^T(m)^5(m)^T(m) |
| M-0120 | A(m)^T(m)^G(m)^G(m)^A(m)^t^a^g^5(x)^a^t^5(x)^t^t^g^5(m)^T(m)^G(m)^G(m)^T(m) |
| M-0121 | T(m)^G(m)^5(m)^A(m)^T(m)^g^g^a^t^a^g^5(x)^a^t^5(x)^T(m)^T(m)^G(m)^5(m)^T(m) |
| M-0122 | 5(m)^A(m)^A(m)^T(m)^G(m)^a^g^a^g^g^g^t^g^5(x)^a^5(m)^5(m)^G(m)^A(m)^G(m) |
| M-0123 | T(m)^A(m)^A(m)^G(m)^G(m)^t^g^a^g^a^5(x)^5(x)^5(x)^t^g^A(m)^G(m)^G(m)^5(m)^5(m) |
| M-0124 | A(m)^T(m)^5(m)^5(m)^G(m)^g^g^t^a^a^g^g^t^g^a^G(m)^A(m)^5(m)^5(m)^5(m) |
| M-0125 | G(m)^G(m)^G(m)^T(m)^T(m)^a^g^g^g^a^g^a^g^t^t^5(m)^5(m)^A(m)^G(m)^5(m) |
| M-0126 | G(m)^T(m)^G(m)^5(m)^A(m)^g^a^g^g^g^t^g^a^g^g^A(m)6A(m)^G(m)^5(m)^A(m) |
| M-0127 | G(m)^T(m)^G(m)^A(m)^5(m)^t^g^t^a^g^t^a^g^t^5(x)^5(m)^T(m)^5(m)^5(m)^T(m) |
| M-0128 | T(m)^5(m)^5(m)^T(m)^T(m)^5(x)^t^t^5(x)^a^g^g^t^g^a^5(m)^T(m)^G(m)^T(m)^A(m) |
| M-0129 | 5(m)^T(m)^T(m)^T(m)^5(m)^5(x)^t^t^5(x)^t^t^5(x)^a^g^g^T(m)^G(m)^A(m)^5(m)^T(m) |
| M-0130 | A(m)^G(m)^G(m)^T(m)^T(m)^5(x)^t^a^5(x)^a^g^t^g^g^5(x)^T(m)^T(m)^G(m)^T(m)^5(m) |
| M-0131 | ^Am)^G(m)^5(m)^5(m)^A(m)^t^g^g^t^g^5(x)^t^5(x)^t^5(x)^T(m)^T(m)^G(m)^A(m)^T(m) |
| M-0132 | A(m)^G(m)^G(m)^T(m)^5(m)^5(x)^a^a^t^g^t^a^g^g^a^G(m)^5(m)^5(m)^A(m)^T(m) |
| M-0133 | G(m)^T(m)^G(m)^5(m)^T(m)^5(x)^5(x)^a^a^g^g^t^5(x)^5(x)^a^A(m)^T(m)^G(m)^T(m)^A(m) |
| M-0134 | G(m)^5(m)^A(m)^5(m)^5(m)^5(x)^t^5(x)^t^t^g^g^5(x)^t^5(x)^T(m)^G(m)^G(m)^G(m)^T(m) |
| M-0135 | A(m)^G(m)^G(m)^5(m)^5(m)^5(x)^t^g^5(x)^a^a^5(x)^t^g^a^G(m)^5(m)^A(m)^G(m)^5(m) |
| M-0136 | T(m)^5(m)^5(m)^A(m)^A(m)^g^a^t^t^5(x)^t^g^t^g^5(x)^G(m)^5(m)^T(m)^T(m)^T(m) |
| M-0137 | A(m)^G(m)^A(m)^G(m)^A(m)^5(x)^5(x)^t^g^g^t^5(x)^5(x)^a^a^G(m)^A(m)^T(m)^T(m)^5(m) |
| M-0138 | 5(m)^A(m)^A(m)^G(m)^G(m)^a^a^g^a^g^a^g^a^5(x)^5(x)^T(m)^G(m)^G(m)^T(m)^5(m) |
| M-0139 | G(m)^G(m)^A(m)^5(m)^A(m)^a^g^g^a^a^g^a^g^a^g^A(m)^5(m)^5(m)^T(m)^G(m) |
| M-0140 | A(m)^A(m)^T(m)^A(m)^A(m)^t^5(x)^a^5(x)^a^5(x)^5(x)^a^a^5(x)^G(m)^G(m)^G(m)^G(m)^5(m) |
| M-0141 | A(m)^A(m)^A(m)^T(m)^A(m)^a^t^5(x)^a^5(x)^a^5(x)^5(x)^a^a^5(m)^G(m)^G(m)^G(m)^G(m) |
| M-0142 | G(m)^A(m)^T(m)^G(m)^A(m)^a^a^t^a^a^t^5(x)^a^5(x)^a^5(m)^5(m)^A(m)^A(m)^5(m) |
| M-0143 | A(m)^5(m)^A(m)^G(m)^A(m)^t^g^a^a^a^t^a^a^t^5(x)^A(m)^5(m)^A(m)^5(m)^5(m) |
| M-0144 | 5(m)^T(m)^A(m)^A(m)^5(m)^a^g^a^t^g^a^a^a^t^a^A(m)^T(m)^5(m)^A(m)^5(m) |
| M-0145 | T(m)^5(m)^(m)^A(m)^A(m)^5(x)^a^g^a^t^g^a^a^a^t^A(m)^A(m)^T(m)^5(m)^A(m) |
| M-0146 | 5(m)^A(m)^T(m)^5(m)^T(m)^a^a^5(x)^a^g^a^t^g^a^a^A(m)^T(m)^A(m)^A(m)^T(m) |
| M-0147 | 5(m)^A(m)^5(m)^A(m)^T(m)^5(x)^t^a^a^5(x)^a^g^a^t^g^A(m)^A(m)^A(m)^T(m)^A(m) |
| M-0148 | A(m)^A(m)^5(m)^A(m)^G(m)^5(x)^5(x)^a^5(x)^a^t^5(x)^t^a^a^5(m)^A(m)^G(m)^A(m)^T(m) |
| M-0149 | T(m)^A(m)^5(m)^G(m)^5(m)^a^a^a^a^5(x)^a^g^5(x)^5(x)^a^5(m)^A(m)^T(m)^5(m)^T(m) |
| M-0150 | 5(m)^A(m)^A(m)^G(m)^G(m)^g^g^5(x)^a^t^a^5(x)^a^t^t^G(m)^5(m)^A(m)^G(m)^A(m) |

**[Table 4]**

| | |
|---|---|
| M-0151 | G(m)^5(m)^T(m)^G(m)^5(m)^a^g^a^g^5(x)^t^a^5(x)^t^a^5(m)^T(m)^A(m)^G(m)^A(m) |
| M-0152 | G(m)^5(m)^T(m)^G(m)^5(m)^t^g^5(x)^a^g^a^g^5(x)^t^a^5(m)^T(m)^A(m)^5(m)^T(m) |
| M-0153 | 5(m)^T(m)^G(m)^G(m)^T(m)^g^g^g^t^a^g^g^g^5(x)^5(x)^T(m)^5(m)^A(m)^G(m)^A(m) |
| M-0154 | 5(m)^5(m)^T(m)^G(m)^G(m)^t^g^g^g^t^a^g^g^g^5(x)^5(m)^T(m)^5(m)^A(m)^G(m) |
| M-0155 | G(m)^T(m)^G(m)^G(m)^A(m)^a^a^g^g^g^t^a^g^5(x)^5(x)^G(m)^A(m)^A(m)^G(m)^A(m) |
| M-0156 | 5(m)^G(m)^T(m)^G(m)^G(m)^a^a^a^g^g^g^t^a^g^5(x)^5(m)^G(m)^A(m)^A(m)^G(m) |
| M-0157 | A(m)^5(m)^5(m)^G(m)^T(m)^g^g^a^a^a^g^g^g^t^a^G(m)^5(m)^5(m)^G(m)^A(m) |
| M-0158 | G(m)^A(m)^5(m)^5(m)^G(m)^t^g^g^a^a^a^g^g^g^t^A(m)^G(m)^5(m)^5(m)^G(m) |
| M-0159 | G(m)^5(m)^5(m)^G(m)^T(m)^t^t^t^g^t^a^g^5(x)^5(x)^t^G(m)^5(m)^T(m)^G(m)^G(m) |
| M-0160 | G(m)^G(m)^5(m)^5(m)^G(m)^t^t^t^t^g^t^a^g^5(x)^5(x)^T(m)^G(m)^5(m)^T(m)^G(m) |
| M-0161 | G(m)^G(m)^T(m)^A(m)^5(m)^a^t^5(x)^t^a^5(x)^5(x)^a^5(x)^5(x)^T(m)^T(m)^G(m)^G(m)^G(m) |
| M-0162 | G(m)^G(m)^G(m)^T(m)^A(m)^5(x)^a^t^5(x)^t^a^5(x)^5(x)^a^5(x)^5(m)^T(m)^T(m)^G(m)^G(m) |
| M-0163 | A(m)^T(m)^T(m)^5(m)^A(m)^5(x)^t^g^a^g^a^g^t^g^5(x)^G(m)^5(m)^A(m)^A(m)^G(m) |
| M-0164 | 5(m)^A(m)^T(m)^A(m)^T(m)^t^5(x)^a^5(x)^t^g^a^g^a^g^(m)^G(m)^5(m)^G(m)^5(m) |
| M-0165 | G(m)^G(m)^A(m)^G(m)^A(m)^5(x)^a^t^g^a^5(x)^a^t^g^a^G(m)^G(m)^5(m)^5(m)^G(m) |
| M-0166 | 5(m)^A(m)^5(m)^T(m)^G(m)^5(x)^t^g^t^a^5(x)^a^g^g^g^5(m)^5(m)^G(m)^G(m)^G(m) |
| M-0167 | G(m)^G(m)^A(m)^T(m)^5(m)^a^5(x)^t^g^5(x)^t^g^t^a^5(x)^A(m)^G(m)^G(m)^G(m)^5(m) |
| M-0168 | T(m)^5(m)^A(m)^G(m)^5(m)^a^t^a^g^g^a^5(x)^a^t^g^T(m)^5(m)^A(m)^G(m)^G(m) |
| M-0169 | 5(m)^5(m)^G(m)^5(m)^T(m)^5(x)^a^g^5(x)^a^t^a^g^g^a^5(m)^A(m)^T(m)^G(m)^T(m) |
| M-0170 | G(m)^5(m)^5(m)^G(m)^G(m)^g^5(x)^5(x)^a^g^t^g^g^g^t^5(m)^A(m)^T(m)^T(m)^G(m) |
| M-0171 | G(m)^G(m)^A(m)^T(m)^A(m)^g^5(x)^a^t^5(x)^t^t^g^5(x)^t^G(m)^G(m)^T(m)^G(m)^5(m) |
| M-0172 | T(m)^G(m)^G(m)^A(m)^T(m)^a^g^5(x)^a^t^5(x)^t^t^g^5(x)^T(m)^G(m)^G(m)^T(m)^G(m) |
| M-0173 | 5(m)^A(m)^T(m)^G(m)^G(m)^a^t^a^g^5(x)^a^t^5(x)^t^t^G(m)^5(m)^T(m)^G(m)^G(m) |
| M-0174 | G(m)^5(m)^A(m)^T(m)^G(m)^g^a^t^a^g^5(x)^a^t^5(x)^t^T(m)^G(m)^5(m)^T(m)^G(m) |
| M-0175 | G(m)^5(m)^T(m)^G(m)^5(m)^a^t^g^g^a^t^a^g^5(x)^a^(m)^5(m)^T(m)^T(m)^G(m) |
| M-0176 | T(m)^G(m)^A(m)^G(m)^A(m)^g^g^g^t^g^5(x)^a^5(x)^5(x)^g^A(m)^G(m)^G(m)^5(m)^A(m) |
| M-0177 | A(m)^T(m)^G(m)^A(m)^G(m)^a^g^g^g^t^g^5(x)^a^5(x)^5(x)^G(m)^A(m)^G(m)^G(m)^5(m) |
| M-0178 | A(m)^A(m)^T(m)^G(m)^A(m)^g^a^g^g^g^t^g^5(x)^a^5(x)^5(m)^G(m)^A(m)^G(m)^G(m) |
| M-0179 | T(m)^5(m)^A(m)^A(m)^T(m)^g^a^g^a^g^g^g^t^g^5(x)^A(m)^5(m)^5(m)^G(m)^A(m) |
| M-0180 | G(m)^T(m)^5(m)^A(m)^A(m)^t^g^a^g^a^g^g^g^t^g^5(m)^A(m)^5(m)^5(m)^G(m) |
| M-0181 | G(m)^G(m)^T(m)^5(m)^A(m)^a^t^g^a^g^a^g^g^g^t^G(m)^5(m)^A(m)^5(m)^5(m) |
| M-0182 | G(m)^G(m)^G(m)^T(m)^5(m)^a^a^t^g^a^g^a^g^g^g^T(m)^G(m)^5(m)^A(m)^5(m) |
| M-0183 | G(m)^G(m)^G(m)^G(m)^T(m)^5(x)^a^a^t^g^a^g^a^g^g^G(m)^T(m)^G(m)^5(m)^A(m) |
| M-0184 | G(m)^G(m)^G(m)^G(m)^G(m)^t^5(x)^a^a^t^g^a^g^a^g^G(m)^G(m)^T(m)^G(m)^5(m) |
| M-0185 | ^(m)^G(m)^G(m)^G(m)^G(m)^g^t^5(x)^a^a^t^g^g^a^G(m)^G(m)^G(m)^T(m)^G(m) |
| M-0186 | 5(m)^A(m)^G(m)^G(m)^G(m)^g^g^t^5(x)^a^a^t^g^a^g^A(m)^G(m)^G(m)^G(m)^T(m) |
| M-0187 | 5(m)^5(m)^A(m)^G(m)^G(m)^g^g^g^t^5(x)^a^a^t^g^a^G(m)^A(m)^G(m)^G(m)^G(m) |
| M-0188 | G(m)^5(m)^5(m)^A(m)^G(m)^g^g^g^g^t^5(x)^a^a^t^g^A(m)^G(m)^A(m)^G(m)^G(m) |
| M-0189 | G(m)^G(m)^5(m)^5(m)^A(m)^g^g^g^g^t^5(x)^a^a^t^G(m)^A(m)^G(m)^A(m)^G(m) |
| M-0190 | A(m)^G(m)^G(m)^5(m)^5(m)^a^g^g^g^g^g^t^5(x)^a^a^T(m)^G(m)^A(m)^G(m)^A(m) |
| M-0191 | G(m)^A(m)^G(m)^G(m)^5(m)^5(x)^a^g^g^g^g^g^t^5(x)^a^A(m)^T(m)^G(m)^A(m)^G(m) |
| M-0192 | T(m)^G(m)^A(m)^G(m)^G(m)^5(x)^5(x)^a^g^g^g^g^g^t^5(x)^A(m)^A(m)^T(m)^G(m)^A(m) |
| M-0193 | 5(m)^T(m)^G(m)^A(m)^G(m)^g^5(x)^5(x)^a^g^g^g^g^g^t^5(m)^A(m)^A(m)^T(m)^G(m) |
| M-0194 | 5(m)^5(m)^T(m)^G(m)^A(m)^g^g^5(x)^5(x)^a^g^g^g^g^g^T(m)^5(m)^A(m)^A(m)^T(m) |
| M-0195 | 5(m)^5(m)^5(m)^T(m)^G(m)^a^g^g^5(x)^5(x)^a^g^g^g^g^G(m)^T(m)^5(m)^A(m)^A(m) |
| M-0196 | A(m)^5(m)^5(m)^5(m)^T(m)^g^a^g^g^5(x)^5(x)^a^g^g^g^G(m)^G(m)^T(m)^5(m)^A(m) |
| M-0197 | A(m)^G(m)^A(m)^5(m)^5(m)^5(x)^t^g^a^g^g^5(x)^5(x)^a^g^G(m)^G(m)^G(m)^G(m)^T(m) |
| M-0198 | T(m)^G(m)^A(m)^G(m)^A(m)^5(x)^5(x)^5(x)^t^g^a^g^g^5(x)^5(x)^A(m)^G(m)^G(m)^G(m)^G(m) |
| M-0199 | G(m)^T(m)^G(m)^A(m)^G(m)^a^5(x)^5(x)^5(x)^t^g^a^g^g^5(x)^5(m)^A(m)^G(m)^G(m)^G(m) |
| M-0200 | G(m)^G(m)^T(m)^G(m)^A(m)^g^a^5(x)^5(x)^5(x)^t^g^a^g^g^5(m)^5(m)^A(m)^G(m)^G(m) |

**[Table 5]**

| | |
|---|---|
| M-0201 | A(m)^G(m)^G(m)^T(m)^G(m)^a^g^a^5(x)^5(x)^5(x)^t^g^a^g^G(m)^5(m)^5(m)^A(m)^G(m) |
| M-0202 | A(m)^A(m)^G(m)^G(m)^T(m)^g^a^g^a^5(x)^5(x)^5(x)^t^g^a^G(m)^G(m)^5(m)^5(m)^A(m) |
| M-0203 | G(m)^T(m)^A(m)^A(m)^G(m)^g^t^g^a^g^a^5(x)^5(x)^5(x)^t^G(m)^A(m)^G(m)^G(m)^5(m) |
| M-0204 | G(m)^G(m)^T(m)^A(m)^A(m)^g^g^t^g^a^g^a^5(x)^5(x)^5(x)^T(m)^G(m)^A(m)^G(m)^G(m) |
| M-0205 | G(m)^G(m)^G(m)^T(m)^A(m)^a^g^g^t^g^a^g^a^5(x)^5(x)^5(m)^T(m)^G(m)^A(m)^G(m) |
| M-0206 | 5(m)^G(m)^G(m)^G(m)^T(m)^a^a^g^g^t^g^a^g^a^5(x)^5(m)^5(m)^T(m)^G(m)^A(m) |
| M-0207 | 5(m)^5(m)^G(m)^G(m)^G(m)^t^a^a^g^g^t^g^a^g^a^5(m)^5(m)^5(m)^T(m)^G(m) |
| M-0208 | T(m)^5(m)^5(m)^G(m)^G(m)^g^t^a^a^g^g^t^g^a^g^A(m)^5(m)^5(m)^5(m)^T(m) |
| M-0209 | G(m)^A(m)^T(m)^5(m)^5(m)^g^g^g^t^a^a^g^g^t^g^A(m)^G(m)^A(m)^5(m)^5(m) |
| M-0210 | G(m)^G(m)^A(m)^T(m)^5(m)^5(x)^g^g^g^t^a^a^g^g^t^G(m)^A(m)^G(m)^A(m)^5(m) |
| M-0211 | G(m)^G(m)^G(m)^A(m)^T(m)^5(x)^5(x)^g^g^g^t^a^a^g^g^T(m)^G(m)^A(m)^G(m)^A(m) |
| M-0212 | G(m)^G(m)^G(m)^G(m)^A(m)^t^5(x)^5(x)^g^g^g^t^a^a^g^G(m)^T(m)^G(m)^A(m)^G(m) |
| M-0213 | A(m)^G(m)^G(m)^G(m)^G(m)^a^t^5(x)^5(x)^g^g^g^t^a^a^G(m)^G(m)^T(m)^G(m)^A(m) |
| M-0214 | T(m)^A(m)^G(m)^G(m)^G(m)^g^a^t^5(x)^5(x)^g^g^g^t^a^A(m)^G(m)^G(m)^T(m)^G(m) |
| M-0215 | G(m)^T(m)^A(m)^G(m)^G(m)^g^g^a^t^5(x)^5(x)^g^g^g^t^A(m)^A(m)^G(m)^G(m)^T(m) |
| M-0216 | G(m)^G(m)^T(m)^A(m)^G(m)^g^g^g^a^t^5(x)^5(x)^g^g^g^T(m)^A(m)^A(m)^G(m)^G(m) |
| M-0217 | G(m)^G(m)^G(m)^T(m)^A(m)^g^g^g^g^a^t^5(x)^5(x)^g^g^G(m)^T(m)^A(m)^A(m)^G(m) |
| M-0218 | T(m)^G(m)^G(m)^G(m)^T(m)^a^g^g^g^g^a^t^5(x)^5(x)^g^G(m)^G(m)^T(m)^A(m)^A(m) |
| M-0219 | 5(m)^T(m)^G(m)^G(m)^G(m)^t^a^g^g^g^g^a^t^5(x)^5(x)^G(m)^G(m)^G(m)^T(m)^A(m) |
| M-0220 | G(m)^5(m)^T(m)^G(m)^G(m)^g^t^a^g^g^g^g^a^t^5(x)^5(m)^G(m)^G(m)^G(m)^T(m) |
| M-0221 | A(m)^G(m)^5(m)^T(m)^G(m)^g^g^t^a^g^g^g^g^a^t^5(m)^5(m)^G(m)^G(m)^G(m) |
| M-0222 | 5(m)^A(m)^G(m)^5(m)^T(m)^g^g^g^t^a^g^g^g^g^a^T(m)^5(m)^5(m)^G(m)^G(m) |
| M-0223 | 5(m)^5(m)^A(m)^G(m)^5(m)^t^g^g^g^t^a^g6g^g^g^A(m)^T(m)^5(m)^5(m)^G(m) |
| M-0224 | T(m)^5(m)^5(m)^A(m)^G(m)^5(x)^t^g^g^g^t^a^g^g^g^G(m)^A(m)^T(m)^5(m)^5(m) |
| M-0225 | T(m)^T(m)^5(m)^5(m)^A(m)^g^5(x)^t^g^g^g^t^a^g^g^G(m)^G(m)^A(m)^T(m)^5(m) |
| M-0226 | G(m)^T(m)^(m)^5(m)^5(m)^a^g^5(x)^t^g^g^g^t^a^g^G(m)^G(m)^G(m)^A(m)^T(m) |
| M-0227 | A(m)^G(m)^T(m)^T(m)^5(m)^5(x)^a^g^5(x)^t^g^g^g^t^a^G(m)^G(m)^G(m)^G(m)^A(m) |
| M-0228 | T(m)^A(m)^G(m)^G(m)^G(m)^a^g^a^g^t^t^5(x)^5(x)^a^g^5(m)^T(m)^G(m)^G(m)^G(m) |
| M-0229 | T(m)^T(m)^A(m)^G(m)^G(m)^g^a^g^a^g^t^t^5(x)^5(x)^a^G(m)^5(m)^T(m)^G(m)^G(m) |
| M-0230 | G(m)^T(m)^T(m)^A(m)^G(m)^g^g^a^g^a^g^t^t^5(x)^5(x)^A(m)^G(m)^5(m)^T(m)^G(m) |
| M-0231 | G(m)^G(m)^T(m)^T(m)^A(m)^g^g^g^a^g^a^g^t^t^5(x)^5(m)^A(m)^G(m)^5(m)^T(m) |
| M-0232 | G(m)^G(m)^G(m)^G(m)^T(m)^t^a^g^g^g^a^g^t^T(m)^5(m)^5(m)^A(m)^G(m) |
| M-0233 | G(m)^G(m)^G(m)^G(m)^G(m)^t^t^a^g^g^g^a^g^a^g^T(m)^T(m)^5(m)^5(m)^A(m) |
| M-0234 | A(m)^G(m)^G(m)^G(m)^G(m)^g^t^t^a^g^g^g^a^g^a^G(m)^T(m)^T(m)^5(m)^5(m) |
| M-0235 | 5(m)^A(m)^G(m)^G(m)^G(m)^g^g^t^t^a^g^g^g^a^g^A(m)^G(m)^T(m)^T(m)^5(m) |
| M-0236 | A(m)^G(m)^5(m)^A(m)^G(m)^g^g^g^g^t^t^a^g^g^g^A(m)^G(m)^A(m)^G(m)^T(m) |
| M-0237 | G(m)^A(m)^A(m)^G(m)^5(m)^a^g^g^g^g^g^t^t^a^g^G(m)^G(m)^A(m)^G(m)^A(m) |
| M-0238 | G(m)^G(m)^A(m)^G(m)^5(m)^5(x)^a^t^g^g^t^g^5(x)^t^5(x)^T(m)^5(m)^T(m)^T(m)^G(m) |
| M-0239 | A(m)^G(m)^5(m)^A(m)^5(m)^5(x)^5(x)^t^5(x)^t^t^g^g^5(x)^t^5(m)^T(m)^G(m)^G(m)^G(m) |
| M-0240 | G(m)^5(m)^5(m)^5(m)^T(m)^g^5(x)^a^a^5(x)^t^g^a^g^5(x)^A(m)^G(m)^5(m)^A(m)^5(m) |
| M-0241 | G(m)^A(m)^G(m)^G(m)^5(m)^5(x)^5(x)^t^g^5(x)^a^a^5(x)^t^g^A(m)^G(m)^5(m)^A(m)^G(m) |
| M-0242 | A(m)^G(m)^G(m)^A(m)^A(m)^g^a^g^a^g^a^5(x)^5(x)^t^g^G(m)^T(m)^5(m)^5(m)^A(m) |
| M-0243 | A(m)^A(m)^G(m)^G(m)^A(m)^a^g^a^g^a^g^a^5(x)^5(x)^t^G(m)^G(m)^T(m)^5(m)^5(m) |
| M-0244 | A(m)^5(m)^A(m)^A(m)^G(m)^g^a^a^g^a^g^a^g^a^5(x)^5(m)^T(m)^G(m)^G(m)^T(m) |
| M-0245 | G(m)^A(m)^5(m)^A(m)^A(m)^g^g^a^a^g^a^g^a^g^a^5(m)^5(m)^T(m)^G(m)^G(m) |
| M-0246 | T(m)^G(m)^A(m)^A(m)^A(m)^t^a^a^t^5(x)^a^5(x)^a^5(x)^5(x)^A(m)^A(m)^5(m)^G(m)^G(m) |

**[Table 6]**

| | |
|---|---|
| L-0001 | T(V)^5(L)^A(L)^a^t^g^a^a^a^5(x)^5(x)^t^g^5(L)^T(L)^G(V) |
| L-0002 | T(V)^T(L)^5(L)^a^a^t^g^a^a^a^5(x)^5(x)^t^G(L)^5(L)^T(V) |
| L-0003 | G(V)^T(L)^T(L)^t^t^5(x)^a^a^t^g^a^a^a^5(L)^5(L)^T(V) |
| L-0004 | 5(V)^T(L)^G(L)^t^t^t^t^5(x)^a^a^t^g^a^A(L)^A(L)^5(V) |
| L-0005 | A(V)^T(L)^5(L)^t^g^t^t^t^t^5(x)^a^a^t^G(L)^A(L)^A(V) |
| L-0006 | 5(V)^A(L)^G(L)^g^a^t^5(x)^t^g^t^t^t^t^5(L)^A(L)^A(V) |
| L-0007 | T(V)^G(L)^5(L)^a^g^g^a^t^5(x)^t^g^t^t^T(L)^T(L)^5(V) |
| L-0008 | A(V)^5(L)^5(L)^t^g^g^a^a^5(x)^t^t^t^t^G(L)^5(L)^A(V) |
| L-0009 | G(V)^5(L)^A(L)^5(x)^5(x)^t^g^g^a^a^5(x)^t^t^T(L)^T(L)^G(V) |
| L-0010 | 5(V)^T(L)^5(L)^5(x)^a^a^g^t^t^t^5(x)^5(x)^a^G(L)^T(L)^G(V) |
| L-0011 | A(V)^G(L)^5(L)^a^g^g^a^t^5(x)^t^5(x)^5(x)^a^A(L)^G(L)^T(V) |
| L-0012 | G(V)^A(L)^A(L)^g^5(x)^a^g^g^a^t^5(x)^t^5(x)^5(L)^A(L)^A(V) |
| L-0013 | A(V)^T(L)^A(L)^5(x)^a^g^a^a^a^5(x)^t^t^5(x)^T(L)^5(L)^T(V) |
| L-0014 | 5(V)^T(L)^T(L)^5(x)^a^t^t^5(x)^t^t^5(x)^a^g^G(L)^5(L)^T(V) |
| L-0015 | 5(V)^G(L)^T(L)^g^t^5(x)^t^t^5(x)^a^t^t^5(x)^T(L)^T(L)^5(V) |
| L-0016 | T(V)^5(L)^T(L)^t^t^a^t^t^5(x)^t^g^t^5(x)^G(L)^T(L)^G(V) |
| L-0017 | G(V)^T(L)^5(L)^t^t^t^a^t^t^5(x)^t^g^t^5(L)^G(L)^T(V) |
| L-0018 | G(V)^A(L)^G(L)^t^5(x)^t^t^t^a^t^t^5(x)^t^G(L)^T(L)^5(V) |
| L-0019 | A(V)^T(L)^5(L)^g^a^g^t^5(x)^t^t^t^a^t^T(L)^5(L)^T(V) |
| L-0020 | A(V)^5(L)^T(L)^5(x)^5(x)^t^5(x)^a^t^t^g^a^5(x)^A(L)^T(L)^5(V) |
| L-0021 | A(V)^T(L)^5(L)^t^5(x)^a^g^t^t^5(x)^t^r5(x)^5(L)^5(L)^G(V) |
| L -0022 | 5(V)^G(L)^A(L)^t^5(x)^t^5(x)^a^g^t^t^5(x)^t^T(L)^5(L)^5(V) |
| L -0023 | 5(V)^T(L)^5(L)^g^a^t^5(x)^t^5(x)^a^g^t^t^5(L)^T(L)^T(V) |
| L -0024 | T(V)^T(L)^T(L)^g^5(x)^5(x)^a^t^5(x)^a^5(x)^t^g^5(L)^T(L)^G(V) |
| L -0025 | 5(V)^T(L)^T(L)^t^g^5(x)^5(x)^a^t^5(x)^a^5(x)^t^G(L)^5(L)^T(V) |
| L -0026 | 5(V)^T(L)^G(L)^5(x)^5(x)^t^g^g^a^5(x)^t^t^5(x)^T(L)^5(L)^A(V) |
| L-0027 | G(V)^T(L)^T(L)^g^a^5(x)^5(x)^t^t^t^g^g^g^G(L)^T(L)^G(V) |
| L-0028 | A(V)^5(L)^5(L)^5(x)^t^g^5(x)^t^t^g^t^t^g^A(L)^5(L)^5(V) |
| L -0029 | A(V)^5(L)^T(L)^t^t^5(x)^a^5(x)^t^5(x)^t^5(x)^t^G(L)^A(L)^G(V) |
| L -0030 | 5(V)^T(L)^5(L)^5(x)^t^5(x)^a^5(x)^t^t^t^5(x)^a^5(L)^T(L)^5(V) |
| L-0031 | T(V)^5(L)^T(L)^5(x)^5(x)^t^5(x)^a^5(x)^t^t^t^5(x)^A(L)^5(L)^T(V) |
| L-0032 | T(V)^G(L)^5(L)^a^t^t^g^g^t^5(x)^t^5(x)^5(x)^T(L)^5(L)^A(V) |
| L-0033 | T(V)^5(L)^T(L)^t^t^t^t^t^g^g^t^g^5(x)^A(L)^T(L)^T(V) |
| L -0034 | 5(V)^T(L)^G(L)^5(x)^t^5(x)^a^g^t^t^t^t^g^G(L)^T(L)^5(V) |
| L-0035 | A(V)^A(L)^G(L)^5(x)^t^a^t^5(x)^5(x)^a^g^a^t^5(L)^G(L)^G(V) |
| L-0036 | 5(V)^A(L)^A(L)^g^5(x)^t^a^t^5(x)^5(x)^a^g^a^T(L)^5(L)^G(V) |
| L-0037 | 5(V)^5(L)^A(L)^a^g^5(x)^t^a^t^5(x)^5(x)^a^g^A(L)^T(L)^5(V) |
| L -0038 | 5(V)^5(L)^G(L)^t^5(x)^5(x)^a^a^g^5(x)^t^a^t^5(L)^5(L)^A(V) |
| L-0039 | T(V)^5(L)^A(L)^t^t^a^a^g^g^5(x)^t^5(x)^5(x)^G(L)^5(L)^5(V) |
| L-0040 | T(V)^5(L)^A(L)^t^5(x)^a^t^t^a^a^g^g^5(x)^T(L)^5(L)^5(V) |
| L -0041 | 5(V)^T(L)^G(L)^5(x)^5(x)^a^t^5(x)^a^t^5(x)^a^t^T(L)^A(L)^A(V) |
| L-0042 | G(V)^5(L)^T(L)^g^5(x)^5(x)^a^t^5(x)^a^t^5(x)^a^T(L)^T(L)^A(V) |
| L-0043 | T(V)^G(L)^5(L)^t^g^5(x)^5(x)^a^t^5(x)^a^t^5(x)^A(L)^T(L)^T(V) |
| L -0044 | T(V)^A(L)^T(L)^5(x)^5(x)^5(x)^t^a^g^g^g^t^5(x)^G(L)^5(L)^T(V) |
| L-0045 | G(V)^A(L)^T(L)^a^t^5(x)^5(x)^5(x)^t^a^g^g^g^T(L)^5(L)^G(V) |
| L-0046 | T(V)^T(L)^5(L)^g^g^t^t^g^t^5(x)^5(x)^t^g^G(L)^T(L)^5(V) |
| L-0047 | G(V)^5(L)^T(L)^g^t^a^g^a^t^a^5(x)^t^g^G(L)^G(L)^G(V) |
| L-0048 | 5(V)^A(L)^G(L)^g^g^5(x)^t^g^t^a^g^a^t^A(L)^5(L)^T(V) |
| L -0049 | T(V)^5(L)^T(L)^5(x)^5(x)^a^5(x)^a^5(x)^t^t^5(x)^5(x)^A(L)^G(L)^G(V) |
| L -0050 | T(V)^T(L)^5(L)^t^5(x)^5(x)^a^5(x)^a^5(x)^t^t^5(x)^5(L)^A(L)^G(V) |

**[Table 7]**

| | |
|---|---|
| L-0051 | 5(V)^A(L)^G(L)^a^g^t^5(x)^a^t^t^5(x)^t^5(x)^5(L)^A(L)^5(V) |
| L -0052 | A(V)^G(L)^T(L)^5(x)^a^g^a^g^t^5(x)^a^t^t^5(L)^T(L)^5(V) |
| L-0053 | G(V)^G(L)^5(L)^5(x)^a^g^a^a^g^a^t^g^a^G(L)^T(L)^5(V) |
| L-0054 | T(V)^T(L)^5(L)^a^a^a^g^5(x)^t^a^g^5(x)^5(x)^T(L)^5(L)^T(V) |
| L-0055 | G(V)^T(L)^T(L)^5(x)^a^a^a^g^5(x)^t^a^g^5(x)^5(L)^T(L)^5(V) |
| L -0056 | G(V)^G(L)^G(L)^g^t^t^5(x)^a^a^a^g^5(x)^t^A(L)^G(L)^5(V) |
| L-0057 | T(V)^G(L)^G(L)^g^t^g^t-5(x)^a^5(x)^a^g^a^A(L)^G(L)^G(V) |
| L -0058 | G(V)^A(L)^G(L)^5(x)^a^t^g^a^t^a^t^5(x)^5(x)^A(L)^G(L)^T(V) |
| L-0059 | G(V)^5(L)^5(L)^t^g^g^a^a^5(x)^a^t^t^5(x)^G(L)^A(L)^G(V) |
| L -0060 | A(V)^G(L)^5(L)^5(x)^t^g^g^a^a^5(x)^a^t^t^5(L)^G(L)^A(V) |
| L-0061 | 5(V)^5(L)^A(L)^g^g^a^t^a^g^a^g^5(x)^t^G(L)^T(L)^G(V) |
| L-0062 | G(V)^A(L)^5(L)^a^a^a^a^5(x)^t^5(x)^5(x)^a^5(x)^5(L)^A(L)^G(V) |
| L-0063 | 5(V)^5(L)^A(L)^g^a^5(x)^a^a^a^a^5(x)^t^5(x)^5(L)^A(L)^5(V) |
| L-0064 | G(V)^G(L)^T(L)^5(x)^5(x)^a^g^a^5(x)^a^a^a^a^5(L)^T(L)^5(V) |
| L -0065 | G(V)^5(L)^T(L)^g^5(x)^t^t^a^5(x)^5(x)^5(x)^5(x)^5(x)^A(L)^T(L)^T(V) |
| L -0066 | A(V)^A(L)^T(L)^g^g^g^a^g^t^a^g^t^g^G(L)^G(L)^T(V) |
| L-0067 | 5(V)^T(L)^G(L)^a^a^a^t^g^g^g^a^g^t^A(L)^G(L)^T(V) |
| L-0068 | G(V)^A(L)^G(L)^5(x)^t^t^g^a^t^a^5(x)^t^g^A(L)^A(L)^A(V) |
| L-0069 | 5(V)^5(L)^5(L)^5(x)^a^g^a^g^5(x)^t^t^g^a^T(L)^A(L)^5(V) |
| L-0070 | G(V)^A(L)^G(L)^5(x)^a^g^a^a^g^g^t^a^g^G(L)^T(L)^T(V) |
| L-0071 | A(V)^T(L)^G(L)^g^g^g^g^a^a^g^t^t^g^G(L)^5(L)^T(V) |
| L-0072 | 5(V)^T(L)^G(L)^a^t^g^5(x)^a^t^t^g^t^t^G(L)^A(L)^G(V) |
| L-0073 | G(V)^G(L)^5(L)^a^g^a^t^g^a^5(x)^5(x)^a^g^G(L)^T(L)^A(V) |
| L-0074 | T(V)^5(L)^5(L)^a^5(x)^5(x)^5(x)^a^t^a^5(x)^5(x)^5(x)^T(L)^G(L)^T(V) |
| L-0075 | G(V)^A(L)^G(L)^g^a^a^g^t^5(x)^5(x)^a^5(x)^5(x)^5(L)^A(L)^T(V) |
| L-0076 | A(V)^G(L)^5(L)^a^g^a^a^g^a^g^g^a^a^G(L)^5(L)^A(V) |
| L-0077 | A(V)^T(L)^A(L)^a^g^g^a^a^a^5(x)^5(x)^t^5(x)^A(L)^T(L)^G(V) |
| L-0078 | G(V)^A(L)^A(L)^t^a^a^g^g^a^a^a^5(x)^5(x)^T(L)^5(L)^A(V) |
| L-0079 | 5(V)^T(L)^A(L)^5(x)^t^a^g^a^g^g^a^t^g^A(L)^A(L)^T(V) |
| L-0080 | 5(V)^A(L)^G(L)^a^g^5(x)^t^a^5(x)^t^a^5(x)^t^A(L)^G(L)^A(V) |
| L-0081 | 5(V)^T(L)^G(L)^5(x)^a^g^a^g^5(x)^t^a^5(x)^t^A(L)^5(L)^T(V) |
| L-0082 | A(V)^5(L)^T(L)^g^g^a^a^g^a^g^g^a^g^G(L)^5(L)^T(V) |
| L-0083 | A(V)^T(L)^G(L)^t^g^t^t^g^a^g^a^5(x)^t^G(L)^G(L)^T(V) |
| L-0084 | A(V)^G(L)^G(L)^g^a^g^a^5(x)^a^t^g^g^5(x)^G(L)^T(L)^A(V) |
| L-0085 | A(V)^G(L)^A(L)^g^g^a^a^g^a^g^t^t^g^G(L)^A(L)^A(V) |
| L-0086 | T(V)^T(L)^G(L)^a^g^a^a^g^t^g^g^a^a^G(L)^A(L)^G(V) |
| L-0087 | G(V)^G(L)^T(L)^g^t^a^a^5(x)^a^t^g^g^G(L)^T(L)^A(V) |
| L-0088 | A(V)^G(L)^G(L)^g^g^t^g^t^g^a^a^5(x)^a^T(L)^G(L)^G(V) |
| L-0089 | G(V)^G(L)^A(L)^a^a^g^g^g^t^a^g^5(x)^5(x)^G(L)^A(L)^A(V) |
| L -0090 | 5(V)^5(L)^G(L)^t^t^t^t^g^ta^g^5(x)^5(x)^T(L)^G(L)^5(V) |
| L -0091 | T(V)^G(L)^G(L)^5(x)^t^g^t^5(x)^t^t^g^t^a^G(L)^G(L)^5(V) |
| L -0092 | 5(V)^5(L)^T(L)^g^t^t^t^g^a^t^5(x)^t^g^5(L)^G(L)^T(V) |
| L-0093 | 5(V)^T(L)^5(L)^5(x)^t^g^t^t^t^g^a^t^5(x)^T(L)^G(L)^5(V) |
| L -0094 | G(V)^G(L)^T(L)^5(x)^t^5(x)^a^t^a^5(x)^t^5(x)^5(x)^T(L)^5(L)^A(V) |
| L -0095 | G(V)^T(L)^A(L)^5(x)^a^t^5(x)^t^a^5(x)^5(x)^a^5(x)^5(L)^T(L)^T(V) |
| L-0096 | T(V)^5(L)^5(L)^a^g^g^t^g^t^t^t^g^t^T(L)^G(L)^A(V) |
| L-0097 | 5(V)^A(L)^5(L)^g^a^a^g^t^a^5(x)^a^g^g^T(L)^5(L)^G(V) |
| L -0098 | T(V)^G(L)^A(L)^g^5(x)^5(x)^a^a^5(x)^t^t^5(x)^a^5(L)^G(L)^5(V) |
| L-0099 | A(V)^T(L)^T(L)^5(x)^a^5(x)^t^g^a^g^a^g^t^G(L)^5(L)^G(V) |
| L-0100 | 5(V)^A(L)^T(L)^a^t^t^5(x)^a^5(x)^t^g^a^g^A(L)^G(L)^T(V) |

**[Table 8]**

| | |
|---|---|
| L-0101 | G(V)^G(L)^5(L)^a^t^a^t^t^5(x)^a^5(x)^t^g^A(L)^G(L)^A(V) |
| L-0102 | 5(V)^G(L)^G(L)^g^5(x)^a^t^a^t^t^5(x)^a^5(x)^T(L)^G(L)^A(V) |
| L-0103 | 5(V)^5(L)^G(L)^g^g^5(x)^a^t^a^t^t^5(x)^a^5(L)^T(L)^G(V) |
| L-0104 | A(V)^G(L)^A(L)^5(x)^a^t^g^a^5(x)^a^t^g^a^G(L)^G(L)^5(V) |
| L-0105 | G(V)^G(L)^A(L)^g^a^5(x)^a^t^g^a^5(x)^a^t^G(L)^A(L)^G(V) |
| L-0106 | 5(V)^5(L)^A(L)^g^g^a^g^a^5(x)^a^t^g^a^5(L)^A(L)^T(V) |
| L-0107 | 5(V)^G(L)^T(L)^a^g^a^a^t^g^g^a^t^g^G(L)^T(L)^T(V) |
| L-0108 | 5(V)^A(L)^T(L)^t^g^t^a^a^5(x)^5(x)^5(x)^a^g^G(L)^A(L)^G(V) |
| L-0109 | 5(V)^5(L)^G(L)^g^a^5(x)^a^t^t^g^t^a^A(L)^5(L)^5(V) |
| L-0110 | T(V)^5(L)^A(L)^5(x)^t^g^5(x)^t^g^t^a^(x)^a^G(L)^G(L)^G(V) |
| L-0111 | 5(V)^T(L)^G(L)^g^a^t^5(x)^a^5(x)^t^g^5(x)^t^G(L)^T(L)^A(V) |
| L-0112 | G(V)^5(L)^T(L)^g^g^a^t5(x)^a^5(x)^t^g^5(x)^T(L)^G(L)^T(V) |
| L-0113 | T(V)^5(L)^A(L)^5(x)^5(x)^t^5(x)^a^a^a^g^5(x)^5(x)^A(L)^G(L)^G(V) |
| L-0114 | G(V)^5(L)^T(L)^5(x)^a^5(x)^t^a^g^g^5(x)^t^t^5(L)^A(L)^5(V) |
| L-0115 | 5(V)^G(L)^G(L)^a^t^g^a^a^a^g^g^g^g^A(L)^A(L)^A(V) |
| L-0116 | 5(V)^A(L)^T(L)^a^g^g^a^5(x)^a^t^g^t^5(x)^A(L)^G(L)^G(V) |
| L-0117 | T(V)^5(L)^A(L)^g^5(x)^a^t^a^g^g^a^5(x)^a^T(L)^G(L)^T(V) |
| L-0118 | 5(V)^T(L)^5(L)^a^g^5(x)^a^t^a^g^g^a^5(x)^A(L)^T(L)^G(V) |
| L-0119 | 5(V)^G(L)^5(L)^t^5(x)^a^g^5(x)^a^t^a^g^g^A(L)^5(L)^A(V) |
| L-0120 | 5(V)^A(L)^5(L)^a^t^g^a^g^5(x)^a^t^5(x)^T(L)^G(L)^5(V) |
| L-0121 | A(V)^G(L)^T(L)^5(x)^a^5(x)^a^t^t^g^a^g^5(x)^A(L)^T(L)^5(V) |
| L-0122 | G(V)^G(L)^G(L)^a^g^t^5(x)^a^5(x)^a^t^t^g^A(L)^G(L)^5(V) |
| L-0123 | A(V)^T(L)^A(L)^g^5(x)^a^t^5(x)^t^t^g^5(x)^t^G(L)^G(L)^T(V) |
| L-0124 | A(V)^T(L)^G(L)^g^a^t^a^g^5(x)^a^t^5(x)^t^T(L)^G(L)^5(V) |
| L-0125 | 5(V)^T(L)^G(L)^(x)^a^t^g^g^a^t^a^g^5(x)^A(L)^T(L)^5(V) |
| L-0126 | T(V)^5(L)^5(L)^g^g^g^t^a^a^g^g^t^g^A(L)^G(L)^A(V) |
| L-0127 | 5(V)^T(L)^G(L)^t^a^g^t^a^g^t^5(x)^5(x)^t^5(L)^5(L)^T(V) |
| L-0128 | T(V)^5(L)^T(L)^t^5(x)^a^g^g^t^g^a^5(x)^t^G(L)^T(L)^A(V) |
| L-0129 | T(V)^5(L)^G(L)^5(x)^a^g^g^t^t^5(x)^t^a^5(x)^A(L)^G(L)^T(V) |
| L-0130 | A(V)^T(L)^5(L)^g^5(x)^a^g^g^t^t^5(x)^t^a^5(L)^A(L)^G(V) |
| L-0131 | A(V)^G(L)^5(L)^5(x)^a^t^g^g^t^g^5(x)^t^5(x)^T(L)^5(L)^T(V) |
| L-0132 | 5(V)^5(L)^A(L)^a^t^g^t^a^g^g^a^g^5(x)^5(L)^A(L)^T(V) |
| L-0133 | G(V)^G(L)^T(L)^5(x)^5(x)^a^a^t^g^t^a^g^g^A(L)^G(L)^5(V) |
| L-0134 | 5(V)^T(L)^5(L)^5(x)^a^a^g^g^t^5(x)^5(x)^a^a^T(L)^G(L)^T(V) |
| L-0135 | T(V)^G(L)^5(L)^t^5(x)^5(x)^a^a^g^g^t^5(x)^5(x)^A(L)^A(L)^T(V) |
| L-0136 | 5(V)^5(L)^A(L)^a^g^a^t^t^5(x)^t^g^t^g^5(L)^G(L)^5(V) |
| L-0137 | A(V)^5(L)^5(L)^t^g^g^t^5(x)^5(x)^a^a^g^a^T(L)^T(L)^5(V) |
| L-0138 | G(V)^A(L)^5(L)^a^a^g^g^a^a^g^a^g^a^G(L)^A(L)^5(V) |
| L-0139 | T(V)^A(L)^A(L)^t^5(x)^a^5(x)^a^5(x)^5(x)^a^a^5(x)^G(L)^G(L)^G(V) |
| L-0140 | A(V)^A(L)^T(L)^a^a^t^5(x)^a^5(x)^a^5(x)^5(x)^a^5A(L)^5(L)^G(V) |
| L-0141 | A(V)^A(L)^A(L)^t^a^a^t^5(x)^a^5(x)^a^5(x)^5(x)^A(L)^A(L)^5(V) |
| L-0142 | G(V)^A(L)^A(L)^a^t^a^a^t5(x)^a^5(x)^a^5(x)^5(L)^A(L)^A(V) |
| L-0143 | A(V)^T(L)^G(L)^a^a^a^t^a^a^t^5(x)^a^5(x)^A(L)^5(L)^5(V) |
| L-0144 | 5(V)^T(L)^A(L)^a^5(x)^a^g^a^t^g^a^a^a^T(L)^A(L)^A(V) |
| L-0145 | 5(V)^5(L)^A(L)^5(x)^a^t^5(x)^t^a^a^5(x)^a^g^A(L)^T(L)^G(V) |
| L-0146 | 5(V)^A(L)^G(L)^5(x)^5(x)^a^5(x)^a^t^5(x)^t^a^a^5(L)^A(L)^G(V) |
| L-0147 | A(V)^5(L)^A(L)^g^5(x)^5(x)^a^5(x)^a^t^5(x)^t^a^A(L)^5(L)^A(V) |
| L-0148 | A(V)^5(L)^G(L)^5(x)^a^a^a^a^5(x)^a^g^5(x)^5(x)^A(L)^5(L)^A(V) |
| L-0149 | A(V)^A(L)^G(L)^g^g^g^5(x)^a^t^a^5(x)^a^t^T(L)^G(L)^5(V) |
| L-0150 | G(V)^5(L)^A(L)^a^g^g^g^g^5(x)^a^t^a^5(x)^A(L)^T(L)^T(V) |

**[Table 9]**

| | |
|---|---|
| L-0151 | 5(V)^G(L)^T(L)^5(x)^a^t^a^t^a^g^a^t^a^T(L)^T(L)^T(V) |
| L-0152 | A(V)^G(L)^5(L)^t^a^t^5(x)^5(x)^a^g^a^t^5(x)^G(L)^G(L)^A(V) |
| L -0153 | T(V)^5(L)^5(L)^a^a^g^5(x)^t^a^t^5(x)^5(x)^a^G(L)^A(L)^T(V) |
| L-0154 | A(V)^T(L)^A(L)^t^5(x)^5(x)^5(x)^t^a^g^g^g^t^5(L)^G(L)^5(V) |
| L-0155 | 65(V)^G(L)^A(L)^t^a^t^5(x)^5(x)^5(x)^t^g^g^G(L)^T(L)^5(V) |
| L-0156 | 5(V)^5(L)^A(L)^g^a^a^g^a^t^g^a^g^t^5(L)^A(L)^G(V) |
| L-0157 | G(V)^5(L)^5(L)^a^g^a^a^g^a^t^g^a^gT(L)^5(L)^A(V) |
| L-0158 | A(V)^G(L)^G(L)^5(x)^5(x)^a^g^a^a^g^a^t^g^A(L)^G(L)^T(V) |
| L-0159 | 5(V)^A(L)^G(L)^g^5(x)^5(x)^a^g^a^a^g^a^t^G(L)^A(L)^G(V) |
| L-0160 | G(V)^G(L)^T(L)^g^t^5(x)^a^5(x)^a^g^a^a^g^G(L)^A(L)^T(V) |
| L-0161 | G(V)^G(L)^G(L)^t^g^t^5(x)^a^5(x)^a^g^a^a^G(L)^G(L)^A(V) |
| L-0162 | G(V)^T(L)^G(L)^g^g^t^g^t^5(x)^a^5(x)^a^g^A(L)^A(L)^G(V) |
| L-0163 | A(V)^G(L)^T(L)^g^g^g^t^g^t^5(x)^a^5(x)^a^G(L)^A(L)^A(V) |
| L-0164 | G(V)^A(L)^G(L)^5(x)^5(x)^t^g^g^a^a^5(x)^a^t^T(L)^5(L)^G(V) |
| L-0165 | A(V)^T(L)^G(L)^g^g^a^g^t^a^g^t^g^t^g^g^G(L)^T(L)^G(V) |
| L-0166 | A(V)^A(L)^A(L)^t^g^g^g^a^g^t^a^g^t^G(L)^G(L)^G(V) |
| L-0167 | G(V)^A(L)^A(L)^a^t^g^g^g^a^g^t^a^g^T(L)^G(L)^G(V) |
| L-0168 | G(V)^5(L)^T(L)^t^g^a^t^a^5(x)^t^g^a^a^A(L)^T(L)^G(V) |
| L-0169 | A(V)^G(L)^5(L)^t^t^g^a^t^a^5(x)^t^g^a^A(L)^A(L)^T(V) |
| L-0170 | A(V)^G(L)^A(L)^g^5(x)^t^t^g^a^t^a^5(x)^t^G(L)^A(L)^A(V) |
| L-0171 | 5(V)^A(L)^G(L)^a^g^5(x)^t^t^g^a^t^a^5(x)^T(L)^G(L)^A(V) |
| L-0172 | G(V)^5(L)^A(L)^g^a^g^5(x)^t^a^5(x)^t^a^5(x)^T(L)^A(L)^G(V) |
| L-0173 | T(V)^G(L)^5(L)a^g^a^g^5(x)^t^a^5(x)^t^a^5(L)^T(L)^A(V) |
| L-0174 | G(V)^T(L)^G(L)^t^t^g^a^g^a^5(x)^t^g^g^T(L)^G(L)^G(V) |
| L-0175 | T(V)^G(L)^T(L)^g^t^t^g^a^g^a^5(x)^t^g^G(L)^T(L)^G(V) |
| L-0176 | G(V)^A(L)^T(L)^g^t^g^t^t^g^a^g^a^5(x)^T(L)^G(L)^G(V) |
| L-0177 | T(V)^G(L)^A(L)^t^g^t^g^t^t^g^a^g^a^5(L)^T(L)^G(V) |
| L-0178 | T(V)^G(L)^T(L)^g^a^a^5(x)^a^t^g^g^g^t^A(L)^G(L)^G(V) |
| L-0179 | G(V)^T(L)^G(L)^t^g^a^a^5(x)^a^t^g^g^g^T(L)^A(L)^G(V) |
| L-0180 | G(V)^G(L)^G(L)^t^g^t^g^a^a^5(x)^a^t^g^G(L)^G(L)^T(V) |
| L-0181 | T(V)^G(L)^G(L)^a^a^a^g^g^g^t^a^g^5(x)^5(L)^G(L)^A(V) |
| L-0182 | T(V)^A(L)^T(L)^t^5(x)^a^5(x)^t^g^a^g^a^g^T(L)^G(L)^5(V) |
| L-0183 | A(V)^T(L)^A(L)^t^t^5(x)^a^5(x)^t^g^a^g^a^G(L)^T(L)^G(V) |
| L-0184 | G(V)^5(L)^A(L)^t^a^t^t^5(x)^a^5(x)^t^g^a^G(L)^A(L)^G(V) |
| L-0185 | G(V)^G(L)^G(L)^5(x)^a^t^a^t^t^5(x)^a^5(x)^t^G(L)^A(L)^G(V) |
| L-0186 | G(V)^5(L)^5(L)^g^g^g^5(x)^a^t^a^t^t^5(x)^A(L)^5(L)^T(V) |
| L-0187 | A(V)^5(L)^A(L)^t^g^a^5(x)^a^t^g^a^g^g^5(L)^5(L)^G(V) |
| L-0188 | G(V)^A(L)^5(L)^a^t^g^a^5(x)^a^t^g^a^g^G(L)^5(L)^5(V) |
| L-0189 | G(V)^A(L)^G(L)^a^5(x)^a^t^g^a^5(x)^a^t^g^A(L)^G(L)^G(V) |
| L-0190 | G(V)^5(L)^5(L)^g^g^g^a^5(x)^a^t^t^g^t^A(L)^A(L)^5(V) |
| L-0191 | G(V)^A(L)^T(L)^g^a^a^a^g^g^g^g^a^a^A(L)^G(L)^G(V) |
| L-0192 | G(V)^G(L)^A(L)^t^g^a^a^a^g^g^g^g^a^A(L)^A(L)^G(V) |
| L-0193 | T(V)^5(L)^G(L)^g^a^t^g^a^a^a^g^g^g^G(L)^A(L)^A(V) |
| L-0194 | 5(V)^T(L)^5(L)^g^g^a^t^g^a^a^a^g^g^G(L)^G(L)^A(V) |
| L-0195 | 5(V)^A(L)^G(L)^5(x)^a^t^a^g^g^a^5(x)^a^t^G(L)^T(L)^5(V) |
| L-0196 | G(V)^5(L)^T(L)^5(x)^a^g^5(x)^a^t^a^g^g^a^5(L)^A(L)^T(V) |
| L-0197 | G(V)^A(L)^G(L)^t^5(x)^a^5(x)^a^t^t^g^a^g^5(L)^A(L)^T(V) |
| L-0198 | G(V)^G(L)^A(L)^g^t^5(x)^a^5(x)a^t^t^g^a^G(L)^(L)^A(V) |
| L-0199 | G(V)^G(L)^G(L)^g^a^g^t^5(x)^a^5(x)^a^t^t^G(L)^A(L)^G(V) |
| L -0200 | T(V)^G(L)^G(L)^g^g^a^g^t^5(x)^a^5(x)^a^t^T(L)^G(L)^A(V) |

**[Table 10]**

| | |
|---|---|
| L-0201 | T(V)^A(L)^A(L)^g^g^t^g^a^g^a^5(x)^5(x)^5(x)^T(L)^G(L)^A(V) |
| L-0202 | G(V)^T(L)^A(L)^a^g^g^t^g^a^g^a^5(x)^5(x)^5(L)^T(L)^G(V) |
| L-0203 | G(V)^G(L)^T(L)^a^a^g^g^t^g^a^g^a^5(x)^5(L)^5(L)^T(V) |
| L-0204 | G(V)^G(L)^G(L)^t^a^a^g^g^t^g^a^g^a^5(L)^5(L)^5(V) |
| L-0205 | 5(v)^G(L)^G(L)^g^t^a^a^g^g^t^g^a^g^A(L)^5(L)^5(V) |
| L-0206 | 5(V)^5(L)^G(L)^g^g^t^a^a^g^g^t^g^a^G(L)^A(L)^5(V) |
| L-0207 | A(V)^T(L)^5(L)^5(x)^g^g^g^t^a^a^g^g^t^G(L)^A(L)^G(V) |
| L-0208 | 5(V)^A(L)^A(L)^g^a^t^t^5(x)^t^g^t^g^5(x)^G(L)^5(L)^T(V) |
| L-0209 | T(V)^5(L)^5(L)^a^a^g^a^t^t^5(x)^t^g^t^G(L)^5(L)^G(V) |
| L-0210 | G(V)^T(L)^5(L)^5(x)^a^a^g^a^t^t^5(x)^t^g^T(L)^G(L)^5(V) |
| L-0211 | 5(V)^A(L)^A(L)^g^g^g^g^5(x)^a^t^a5(x)^a^T(L)^T(L)^G(V) |
| L-0212 | G(V)^G(L)^5(L)^a^a^g^g^g^g^5(x)^a^t^a^5(L)^A(L)^T(V) |
| L-0213 | G(V)^A(L)^G(L)^a^g^g^g^t^g^5(x)^a^5(x)^5(x)^G(L)^A(L)^G(V) |
| L-0214 | T(V)^G(L)^A(L)^g^a^g^g^g^t^g^5(x)^a^5(x)^5(L)^G(L)^A(V) |
| L-0215 | 5(V)^A(L)^A(L)^t^g^a^g^a^g^g^g^t^g^5(L)^A(Ln(V) |
| L-0216 | T(V)^5(L)^A(L)^a^t^g^a^g^a^g^g^g^t^G(L)^5(L)^A(V) |
| L-0217 | G(V)^T(L)^5(L)^a^a^t^g^a^g^a^g^g^g^T(L)^G(L)^5(V) |
| L-0218 | G(V)^G(L)^T(L)^5(x)^a^a^t^g^a^g^a^g^g^G(L)^T(L)^G(V) |
| L-0219 | G(V)^G(L)^G(L)^t^5(x)^a^a^t^a^g^a^g^a^g^G(L)^G(L)^T(V) |
| L-0220 | G(V)^G(L)^G(L)^g^t-5(x)^a^a^t^g^ag^a^G(L)^G(L)^G(V) |
| L-0221 | G(V)^G(L)^G(L)^g^g^t^5(x)^a^at^g^a^g^A(L)"G(L)"G(V) |
| L-0222 | A(V)^G(L)^G(L)^g^g^g^t^5(x)^a^a^t^g^a^G(L)^A(L)^G(V) |
| L-0223 | 5(V)^A(L)^G(L)^g^g^g^t^5(x)^a^a^t^g^A(L)^G(L)^A(V) |
| L-0224 | 5(V)^5(L)^A(L)^g^g^g^g^g^t^5(x)^a^a^t^G(L)-A(L)^G(V) |
| L-0225 | G(V)^5(L)^5(L)^a^g^g^g^g^g^t^5(x)^a^a^T(L)^G(L)^A(V) |
| L-0226 | A(V)^G(L)^G(L)^5(x)^5(x)^a^g^g^g^g^g^t^5(x)^A(L)^A(L)^T(V) |
| L-0227 | T(V)^G(L)^A(L)^g^a^5(x)^5(x)^5(x)^t^g^a^g^g^5(L)^5(L)^A(V) |
| L-0228 | A(V)^G(L)^G^t^a^g^a^5(x)^5(x)^5(x)^t^g^A(L)^G(L)^G(V) |
| L-0229 | A(V)^A(L)^G(L)^g^t^g^a^g^a^5(x)^5(x)^5(x)^t^G(L)^A(L)^G(V) |
| L-0230 | G(V)^A(L)^T(L)^5(x)^5(x)^g^g^g^t^a^a^g^g^T(L)^G(L)^A(V) |
| L-0231 | T(V)^A(L)^G(L)^g^g^g^a^t^5(x)^5(x)^g^g^g^T(L)^A(L)^A(V) |
| L-0232 | A(V)^G(L)^5(L)^t^g^g^g^t^a^g^g^g^g^A(L)^T(L)^5(V) |
| L-0233 | A(V)^G(L)^G(L)^g^a^g^a^g^t^t^5(x)^5(x)^a^G(L)^5(L)^T(V) |
| L -0234 | T(V)^A(L)^G(L)^g^g^a^g^a^g^t^t^5(x)^5(x)^A(L)^G(L)^5(V) |
| L-0235 | T(V)^T(L)^A(L)^g^g^g^a^g^a^g^t^t^5(x)^5(L)^A(L)^G(V) |
| L-0236 | G(V)^T(L)^T(L)^a^g^g^g^a^g^a^g^t^t^5(L)^5(L)^A(V) |
| L-0237 | G(V)^G(L)^T(L)^t^a^g^g^g^a^g^a^g^t^T(L)^5(L)^5(V) |
| L-0238 | G(V)^G(L)^G(L)^t^t^a^g^g^g^a^g^a^g^T(L)^T(L)^5(V) |
| L-0239 | G(V)^G(L)^G(L)^g^t^t^a^g^g^g^a^g^a^G(L)^T(V) |

Positions in a sequence of human ATN1 mRNA or pre-mRNA targeted by the antisense oligonucleotides presented in Tables 1 to 10, and sequence numbers and nucleobase sequences corresponding to the antisense oligonucleotides are presented in Tables 11 to 20.

In the notation of a sequence in Tables 11 to 20, "SEQ1 START" means "SEQ ID NO: 1 start site" and represents the position number of a nucleoside on a side closest to a 5' side targeted by an antisense oligonucleotide in the mRNA sequence of human ATN1 (SEQ ID NO: 1). "SEQ1 END" means "SEQ ID NO: 1 termination site" and represents the position number of a nucleoside on a side closest to a 3' side targeted by an antisense oligonucleotide in the mRNA sequence of human ATN1 (SEQ ID NO: 1).

"SEQ2 START" means "SEQ ID NO: 2 start site" and represents the position number of a nucleoside on a side closest to a 5' side targeted by an antisense oligonucleotide in the pre-mRNA sequence of human ATN1 (SEQ ID NO: 2). "SEQ2 END" means "SEQ ID NO: 2 termination site" and represents the position number of a nucleoside on a side closest to a 3' side targeted by an antisense oligonucleotide in the pre-mRNA sequence of human ATN1 (SEQ ID NO: 2).

Each antisense oligonucleotide is targeted by human ATN1 mRNA designated herein as SEQ ID NO: 1 and/or human ATN1 pre-mRNA designated herein as SEQ ID NO: 2 or SEQ ID NO: 717.

"-" (Hyphen) indicates that an antisense oligonucleotide does not target a sequence of mRNA or pre-mRNA with 100% complementarity.

"SEQ No" represents a sequence number, and "BASE SEQUENCE" represents a nucleobase sequence of an antisense oligonucleotide.

Note that the title rows in Tables 12 to 20 are the same as that in Table 11, and thus are omitted.

**[Table 11]**

| Cmpd No | SEQ1 START | SEQ1 END | SEQ2 START | SEQ2 END | SEQ No | BASE SEQUENCE |
|---|---|---|---|---|---|---|
| M-0001 | 59 | 78 | - | - | 242 | TCAATGAAACCTGCTGGTAG |
| M-0002 | 65 | 84 | - | - | 243 | CTGTTTTCAATGAAACCTGC |
| M-0003 | 68 | 87 | 5527 | 5546 | 244 | GATCTGTTTTCAATGAAACC |
| M-0004 | 70 | 89 | 5529 | 5548 | 245 | AGGATCTGTTTTCAATGAAA |
| M-0005 | 77 | 96 | 5536 | 5555 | 246 | CTTTTGCAGGATCTGTTTTC |
| M-0006 | 109 | 128 | 5568 | 5587 | 247 | GATCTCCAAGTTTCCAGTGT |
| M-0007 | 114 | 133 | 5573 | 5592 | 248 | AGCAGGATCTCCAAGTTTCC |
| M-0008 | 118 | 137 | 5577 | 5596 | 249 | GGGAAGCAGGATCTCCAAGT |
| M-0009 | 164 | 183 | 5623 | 5642 | 250 | ACAGAAACTTCTCTGCTCCA |
| M-0010 | 166 | 185 | 5625 | 5644 | 251 | ATACAGAAACTTCTCTGCTC |
| M-0011 | 169 | 188 | 5628 | 5647 | 252 | TGAATACAGAAACTTCTCTG |
| M-0012 | 173 | 192 | 5632 | 5651 | 253 | CAGCTGAATACAGAAACTTC |
| M-0013 | 207 | 226 | 5666 | 5685 | 254 | CAGGCTGTGGAGACCCCATT |
| M-0014 | 219 | 238 | 5678 | 5697 | 255 | GTCTTCATTCTTCAGGCTGT |
| M-0015 | 222 | 241 | 5681 | 5700 | 256 | CGTGTCTTCATTCTTCAGGC |
| M-0016 | 226 | 245 | 5685 | 5704 | 257 | CTGTCGTGTCTTCATTCTTC |
| M-0017 | 241 | 260 | - | - | 258 | CATCGAGTCTTTATTCTGTC |
| M-0018 | 253 | 272 | - | - | 259 | ACTCCTCATTGACATCGAGT |
| M-0019 | 254 | 273 | - | - | 260 | CACTCCTCATTGACATCGAG |
| M-0020 | 256 | 275 | - | - | 261 | TCCACTCCTCATTGACATCG |
| M-0021 | 262 | 281 | 5868 | 5887 | 262 | CTTCCGTCCACTCCTCATTG |
| M-0022 | 300 | 319 | 5906 | 5925 | 263 | CTCGATCTCAGTTCTTCCCG |
| M-0023 | 353 | 372 | 5959 | 5978 | 264 | CAGCTTTGCCATCACTGCTG |
| M-0024 | 356 | 375 | 5962 | 5981 | 265 | TCTCAGCTTTGCCATCACTG |
| M-0025 | 357 | 376 | 5963 | 5982 | 266 | TTCTCAGCTTTGCCATCACT |
| M-0026 | 366 | 385 | 5972 | 5991 | 267 | TGCCTGGACTTCTCAGCTTT |
| M-0027 | 367 | 386 | 5973 | 5992 | 268 | CTGCCTGGACTTCTCAGCTT |
| M-0028 | 417 | 436 | 6174 | 6193 | 269 | TTGTTGACCTTTGGGGTGGA |
| M-0029 | 425 | 444 | 6182 | 6201 | 270 | GACCCTGCTTGTTGACCTTT |
| M-0030 | 446 | 465 | 6203 | 6222 | 271 | TCTCTGAGATCTCCTCACTC |
| M-0031 | 450 | 469 | 6207 | 6226 | 272 | TCACTCTCTGAGATCTCCTC |
| M-0032 | 454 | 473 | 6211 | 6230 | 273 | ACTTTCACTCTCTGAGATCT |
| M-0033 | 458 | 477 | 6215 | 6234 | 274 | CCTCACTTTCACTCTCTGAG |
| M-0034 | 460 | 479 | 6217 | 6236 | 275 | CTCCTCACTTTCACTCTCTG |
| M-0035 | 463 | 482 | 6220 | 6239 | 276 | GGTCTCCTCACTTTCACTCT |
| M-0036 | 483 | 502 | 6240 | 6259 | 277 | TTGGTCTTTTTTGGTGCATT |
| M-0037 | 490 | 509 | 6247 | 6266 | 278 | CTCAGTTTTGGTCTTTTTTG |
| M-0038 | 495 | 514 | - | - | 279 | TCCTGCTCAGTTTTGGTCTT |
| M-0039 | 523 | 542 | 7248 | 7267 | 280 | ATCGGAGGGAGACTGTGGCC |
| M-0040 | 536 | 555 | 7261 | 7280 | 281 | CCAAGCTATCCAGATCGGAG |
| M-0041 | 559 | 578 | 7284 | 7303 | 282 | ATCATCATTAAGGCTCCGCC |
| M-0042 | 561 | 580 | 7286 | 7305 | 283 | CCATCATCATTAAGGCTCCG |
| M-0043 | 563 | 582 | 7288 | 7307 | 284 | TGCCATCATCATTAAGGCTC |
| M-0044 | 564 | 583 | 7289 | 7308 | 285 | CTGCCATCATCATTAAGGCT |
| M-0045 | 566 | 585 | 7291 | 7310 | 286 | TGCTGCCATCATCATTAAGG |
| M-0046 | 568 | 587 | 7293 | 7312 | 287 | GCTGCTGCCATCATCATTAA |
| M-0047 | 627 | 646 | 7352 | 7371 | 288 | CCAGGGCTGTAGATACTGGG |
| M-0048 | 642 | 661 | 7367 | 7386 | 289 | TCATTCTCCACACTTCCAGG |
| M-0049 | 649 | 668 | 7374 | 7393 | 290 | GTCAGAGTCATTCTCCACAC |
| M-0050 | 651 | 670 | 7376 | 7395 | 291 | GAGTCAGAGTCATTCTCCAC |

**[Table 12]**

| | | | | | | |
|---|---|---|---|---|---|---|
| M-0051 | 653 | 672 | 7378 | 7397 | 292 | ATGAGTCAGAGTCATTCTCC |
| M-0052 | 656 | 675 | 7381 | 7400 | 293 | AAGATGAGTCAGAGTCATTC |
| M-0053 | 666 | 685 | 7391 | 7410 | 294 | GACAGGCCAGAAGATGAGTC |
| M-0054 | 763 | 782 | 7488 | 7507 | 295 | TTCAAAGCTAGCCTCTGGCT |
| M-0055 | 766 | 785 | 7491 | 7510 | 296 | GGGTTCAAAGCTAGCCTCTG |
| M-0056 | 787 | 806 | 7512 | 7531 | 297 | AGTGGGTGTCACAGAAGGAT |
| M-0057 | 800 | 819 | 7525 | 7544 | 298 | GAGCATGATATCCAGTGGGT |
| M-0058 | 880 | 899 | 7605 | 7624 | 299 | GCCCCCAGGATAGAGCTGTG |
| M-0059 | 899 | 918 | 7624 | 7643 | 300 | CAGACAAAACTCCACCAGTG |
| M-0060 | 901 | 920 | 7626 | 7645 | 301 | TCCAGACAAAACTCCACCAG |
| M-0061 | 924 | 943 | 7649 | 7668 | 302 | CCCCCCTTGGGACCCATTGG |
| M-0062 | 970 | 989 | 7695 | 7714 | 303 | GTGCTGCTTACCCCCATTAG |
| M-0063 | 995 | 1014 | 7720 | 7739 | 304 | CTGAAATGGGAGTAGTGGGT |
| M-0064 | 998 | 1017 | 7723 | 7742 | 305 | ATACTGAAATGGGAGTAGTG |
| M-0065 | 1007 | 1026 | 7732 | 7751 | 306 | CAGAGCTTGATACTGAAATG |
| M-0066 | 1010 | 1029 | 7735 | 7754 | 307 | CCCCAGAGCTTGATACTGAA |
| M-0067 | 1013 | 1032 | 7738 | 7757 | 308 | TAGCCCCAGAGCTTGATACT |
| M-0068 | 1020 | 1039 | 7745 | 7764 | 309 | GCACCACTAGCCCCAGAGCT |
| M-0069 | 1055 | 1074 | 7780 | 7799 | 310 | CACCCACTGGAGTGGTAGGC |
| M-0070 | 1079 | 1098 | 7804 | 7823 | 311 | GTGGAGCAGAAGGTAGGTTC |
| M-0071 | 1101 | 1120 | 7826 | 7845 | 312 | ACATGGGGGAAGTTGGCTGG |
| M-0072 | 1157 | 1176 | 7882 | 7901 | 313 | AGGCTGATGCATTGTTGAGG |
| M-0073 | 1196 | 1215 | 7921 | 7940 | 314 | GATGACCAGGTAGTGGTTGG |
| M-0074 | 1204 | 1223 | 7929 | 7948 | 315 | AGAGGGCAGATGACCAGGTA |
| M-0075 | 1310 | 1329 | 8035 | 8054 | 316 | GAGCAGAAGAGGAAGCAGGA |
| M-0076 | 1339 | 1358 | 8064 | 8083 | 317 | TGAATAAGGAAACCTCATGG |
| M-0077 | 1351 | 1370 | 8076 | 8095 | 318 | ACTACTAGAGGATGAATAAG |
| M-0078 | 1354 | 1373 | 8079 | 8098 | 319 | GCTACTACTAGAGGATGAAT |
| M-0079 | 1364 | 1383 | 8089 | 8108 | 320 | CTGCTGCAGAGCTACTACTA |
| M-0080 | 1380 | 1399 | 8105 | 8124 | 321 | GAACTGGAAGAGGAGGCTGC |
| M-0081 | 1392 | 1411 | 8117 | 8136 | 322 | GAAGAGGAGGAAGAACTGGA |
| M-0082 | 1423 | 1442 | 8148 | 8167 | 323 | TGCCTGGGAAGCTGGGAAGG |
| M-0083 | 1614 | 1633 | 8339 | 8358 | 324 | GCCCCAGTAGAGGGAGGGAA |
| M-0084 | 1651 | 1670 | 8376 | 8395 | 325 | GTGATGTGTTGAGACTGGTG |
| M-0085 | 1806 | 1825 | 8531 | 8550 | 326 | ATGGCGTAAGGGTGTGCGTG |
| M-0086 | 1846 | 1865 | 8571 | 8590 | 327 | CCCTGGTGGGTAGGGCCTCA |
| M-0087 | 1896 | 1915 | 8621 | 8640 | 328 | CCTGCTTGGCTGTAGGACAC |
| M-0088 | 1924 | 1943 | 8649 | 8668 | 329 | AGAGGAAGAGACTGGAGGGC |
| M-0089 | 1956 | 1975 | 8681 | 8700 | 330 | GACCCTTGAGAAGTGGAAGA |
| M-0090 | 2023 | 2042 | 8748 | 8767 | 331 | CACCGGTGGGAAAGGGTAGG |
| M-0091 | 2060 | 2079 | 8785 | 8804 | 332 | CCGTGGAAAGGGTAGCCGAA |
| M-0092 | 2105 | 2124 | 8830 | 8849 | 333 | AGGCCGTTTTGTAGCCTGCT |
| M-0093 | 2170 | 2189 | 8895 | 8914 | 334 | GGTGGCTGTCTTGTAGGCCC |
| M-0094 | 2398 | 2417 | 9123 | 9142 | 335 | CTCCTGTTTGATCTGCGTGG |
| M-0095 | 2427 | 2446 | 9152 | 9171 | 336 | CTCTCGGGGGTCTCATACTC |
| M-0096 | 2480 | 2499 | 9205 | 9224 | 337 | GTACATCTACCACCTTGGGA |
| M-0097 | 2484 | 2503 | 9209 | 9228 | 338 | CTGGGTACATCTACCACCTT |
| M-0098 | 2501 | 2520 | 9226 | 9245 | 339 | CAGACTGACTGGCATGGCTG |
| M-0099 | 2526 | 2545 | 9534 | 9553 | 340 | CGATCCAGGTGTTTGTTGAA |
| M-0100 | 2543 | 2562 | 9551 | 9570 | 341 | CGCACGAGTTGAAGCCGCGA |

**[Table 13]**

| | | | | | | |
|---|---|---|---|---|---|---|
| M-0101 | 2568 | 2587 | 9576 | 9595 | 342 | GGCACGAAGTACAGGTCGCT |
| M-0102 | 2742 | 2761 | - | - | 343 | TCCTGAGCCAACTTCACGCT |
| M-0103 | 2853 | 2872 | 10274 | 10293 | 344 | GGAGTGTCAGGACCCAGGTA |
| M-0104 | 2877 | 2896 | 10298 | 10317 | 345 | TATTCACTGAGAGTGCGCAA |
| M-0105 | 2903 | 2922 | 10324 | 10343 | 346 | CAGGAGACATGACATGAGGC |
| M-0106 | 2904 | 2923 | 10325 | 10344 | 347 | CCAGGAGACATGACATGAGG |
| M-0107 | 2931 | 2950 | 10352 | 10371 | 348 | GGCACGTAGAATGGATGGTT |
| M-0108 | 2972 | 2991 | 10393 | 10412 | 349 | GGACATTGTAACCCAGGAGC |
| M-0109 | 2992 | 3011 | 10413 | 10432 | 350 | ATCACTGCTGTACAGGGCCG |
| M-0110 | 3069 | 3088 | 10490 | 10509 | 351 | TTCACCTCAAAGCCAGGCTT |
| M-0111 | 3078 | 3097 | 10499 | 10518 | 352 | TCACTAGGCTTCACCTCAAA |
| M-0112 | 3085 | 3104 | 10506 | 10525 | 353 | TTCCAGCTCACTAGGCTTCA |
| M-0113 | 3094 | 3113 | 10515 | 10534 | 354 | ATGTAGGGGTTCCAGCTCAC |
| M-0114 | 3109 | 3128 | 10530 | 10549 | 355 | CGGCCCAGGGACCCCATGTA |
| M-0115 | 3193 | 3212 | 10614 | 10633 | 356 | GCTCGGATGAAAGGGGAAAG |
| M-0116 | 3269 | 3288 | 10690 | 10709 | 357 | GCTCAGCATAGGACATGTCA |
| M-0117 | 3333 | 3352 | 10754 | 10773 | 358 | CGGGCCAGTGGGTCATTGCC |
| M-0118 | 3355 | 3374 | 10776 | 10795 | 359 | AGTCACATTGAGCATCTGCA |
| M-0119 | 3358 | 3377 | 10779 | 10798 | 360 | GGGAGTCACATTGAGCATCT |
| M-0120 | 3421 | 3440 | 10842 | 10861 | 361 | ATGGATAGCATCTTGCTGGT |
| M-0121 | 3424 | 3443 | 10845 | 10864 | 362 | TGCATGGATAGCATCTTGCT |
| M-0122 | 3452 | 3471 | 12575 | 12594 | 363 | CAATGAGAGGGTGCACCGAG |
| M-0123 | 3479 | 3498 | 12602 | 12621 | 364 | TAAGGTGAGACCCTGAGGCC |
| M-0124 | 3486 | 3505 | 12609 | 12628 | 365 | ATCCGGGTAAGGTGAGACCC |
| M-0125 | 3516 | 3535 | 12639 | 12658 | 366 | GGGTTAGGGAGAGTTCCAGC |
| M-0126 | 3538 | 3557 | 12661 | 12680 | 367 | GTGCAGAGGGTGAGGAAGCA |
| M-0127 | 3754 | 3773 | - | - | 368 | GTGACTGTAGTAGTCCTCCT |
| M-0128 | 3765 | 3784 | - | - | 369 | TCCTTCTTCAGGTGACTGTA |
| M-0129 | 3768 | 3787 | 13433 | 13452 | 370 | CTTTCCTTCTTCAGGTGACT |
| M-0130 | 3789 | 3808 | 13454 | 13473 | 371 | AGGTTCTACAGTGGCTTGTC |
| M-0131 | 3812 | 3831 | 13477 | 13496 | 372 | AGCCATGGTGCTCTCTTGAT |
| M-0132 | 3826 | 3845 | 13491 | 13510 | 373 | AGGTCCAATGTAGGAGCCAT |
| M-0133 | 3834 | 3853 | 13499 | 13518 | 374 | GTGCTCCAAGGTCCAATGTA |
| M-0134 | 3888 | 3907 | 13553 | 13572 | 375 | GCACCCTCTTGGCTCTGGGT |
| M-0135 | 3906 | 3925 | 13571 | 13590 | 376 | AGGCCCTGCAACTGAGCAGC |
| M-0136 | 4022 | 4041 | 13687 | 13706 | 377 | TCCAAGATTCTGTGCGCTTT |
| M-0137 | 4032 | 4051 | 13697 | 13716 | 378 | AGAGACCTGGTCCAAGATTC |
| M-0138 | 4040 | 4059 | 13705 | 13724 | 379 | CAAGGAAGAGAGACCTGGTC |
| M-0139 | 4043 | 4062 | 13708 | 13727 | 380 | GGACAAGGAAGAGAGACCTG |
| M-0140 | 4117 | 4136 | 13782 | 13801 | 381 | AATAATCACACCAACGGGGC |
| M-0141 | 4118 | 4137 | 13783 | 13802 | 382 | AAATAATCACACCAACGGGG |
| M-0142 | 4122 | 4141 | 13787 | 13806 | 383 | GATGAAATAATCACACCAAC |
| M-0143 | 4125 | 4144 | 13790 | 13809 | 384 | ACAGATGAAATAATCACACC |
| M-0144 | 4128 | 4147 | 13793 | 13812 | 385 | CTAACAGATGAAATAATCAC |
| M-0145 | 4129 | 4148 | 13794 | 13813 | 386 | TCTAACAGATGAAATAATCA |
| M-0146 | 4131 | 4150 | 13796 | 13815 | 387 | CATCTAACAGATGAAATAAT |
| M-0147 | 4133 | 4152 | 13798 | 13817 | 388 | CACATCTAACAGATGAAATA |
| M-0148 | 4139 | 4158 | 13804 | 13823 | 389 | AACAGCCACATCTAACAGAT |
| M-0149 | 4146 | 4165 | 13811 | 13830 | 390 | TACGCAAAACAGCCACATCT |
| M-0150 | 4215 | 4234 | 13880 | 13899 | 391 | CAAGGGGCATACATTGCAGA |

**[Table 14]**

| | | | | | | |
|---|---|---|---|---|---|---|
| M-0151 | 1362 | 1381 | 8087 | 8106 | 392 | GCTGCAGAGCTACTACTAGA |
| M-0152 | 1365 | 1384 | 8090 | 8109 | 393 | GCTGCTGCAGAGCTACTACT |
| M-0153 | 1844 | 1863 | 8569 | 8588 | 394 | CTGGTGGGTAGGGCCTCAGA |
| M-0154 | 1845 | 1864 | 8570 | 8589 | 395 | CCTGGTGGGTAGGGCCTCAG |
| M-0155 | 2058 | 2077 | 8783 | 8802 | 396 | GTGGAAAGGGTAGCCGAAGA |
| M-0156 | 2059 | 2078 | 8784 | 8803 | 397 | CGTGGAAAGGGTAGCCGAAG |
| M-0157 | 2061 | 2080 | 8786 | 8805 | 398 | ACCGTGGAAAGGGTAGCCGA |
| M-0158 | 2062 | 2081 | 8787 | 8806 | 399 | GACCGTGGAAAGGGTAGCCG |
| M-0159 | 2103 | 2122 | 8828 | 8847 | 400 | GCCGTTTTGTAGCCTGCTGG |
| M-0160 | 2104 | 2123 | 8829 | 8848 | 401 | GGCCGTTTTGTAGCCTGCTG |
| M-0161 | 2481 | 2500 | 9206 | 9225 | 402 | GGTACATCTACCACCTTGGG |
| M-0162 | 2482 | 2501 | 9207 | 9226 | 403 | GGGTACATCTACCACCTTGG |
| M-0163 | 2876 | 2895 | 10297 | 10316 | 404 | ATTCACTGAGAGTGCGCAAG |
| M-0164 | 2879 | 2898 | 10300 | 10319 | 405 | CATATTCACTGAGAGTGCGC |
| M-0165 | 2901 | 2920 | 10322 | 10341 | 406 | GGAGACATGACATGAGGCCG |
| M-0166 | 2990 | 3009 | 10411 | 10430 | 407 | CACTGCTGTACAGGGCCGGG |
| M-0167 | 2994 | 3013 | 10415 | 10434 | 408 | GGATCACTGCTGTACAGGGC |
| M-0168 | 3267 | 3286 | 10688 | 10707 | 409 | TCAGCATAGGACATGTCAGG |
| M-0169 | 3271 | 3290 | 10692 | 10711 | 410 | CCGCTCAGCATAGGACATGT |
| M-0170 | 3335 | 3354 | 10756 | 10775 | 411 | GCCGGGCCAGTGGGTCATTG |
| M-0171 | 3419 | 3438 | 10840 | 10859 | 412 | GGATAGCATCTTGCTGGTGC |
| M-0172 | 3420 | 3439 | 10841 | 10860 | 413 | TGGATAGCATCTTGCTGGTG |
| M-0173 | 3422 | 3441 | 10843 | 10862 | 414 | CATGGATAGCATCTTGCTGG |
| M-0174 | 3423 | 3442 | 10844 | 10863 | 415 | GCATGGATAGCATCTTGCTG |
| M-0175 | 3426 | 3445 | - | - | 416 | GCTGCATGGATAGCATCTTG |
| M-0176 | 3449 | 3468 | 12572 | 12591 | 417 | TGAGAGGGTGCACCGAGGCA |
| M-0177 | 3450 | 3469 | 12573 | 12592 | 418 | ATGAGAGGGTGCACCGAGGC |
| M-0178 | 3451 | 3470 | 12574 | 12593 | 419 | AATGAGAGGGTGCACCGAGG |
| M-0179 | 3453 | 3472 | 12576 | 12595 | 420 | TCAATGAGAGGGTGCACCGA |
| M-0180 | 3454 | 3473 | 12577 | 12596 | 421 | GTCAATGAGAGGGTGCACCG |
| M-0181 | 3455 | 3474 | 12578 | 12597 | 422 | GGTCAATGAGAGGGTGCACC |
| M-0182 | 3456 | 3475 | 12579 | 12598 | 423 | GGGTCAATGAGAGGGTGCAC |
| M-0183 | 3457 | 3476 | 12580 | 12599 | 424 | GGGGTCAATGAGAGGGTGCA |
| M-0184 | 3458 | 3477 | 12581 | 12600 | 425 | GGGGGTCAATGAGAGGGTGC |
| M-0185 | 3459 | 3478 | 12582 | 12601 | 426 | AGGGGGTCAATGAGAGGGTG |
| M-0186 | 3460 | 3479 | 12583 | 12602 | 427 | CAGGGGGTCAATGAGAGGGT |
| M-0187 | 3461 | 3480 | 12584 | 12603 | 428 | CCAGGGGGTCAATGAGAGGG |
| M-0188 | 3462 | 3481 | 12585 | 12604 | 429 | GCCAGGGGGTCAATGAGAGG |
| M-0189 | 3463 | 3482 | 12586 | 12605 | 430 | GGCCAGGGGGTCAATGAGAG |
| M-0190 | 3464 | 3483 | 12587 | 12606 | 431 | AGGCCAGGGGGTCAATGAGA |
| M-0191 | 3465 | 3484 | 12588 | 12607 | 432 | GAGGCCAGGGGGTCAATGAG |
| M-0192 | 3466 | 3485 | 12589 | 12608 | 433 | TGAGGCCAGGGGGTCAATGA |
| M-0193 | 3467 | 3486 | 12590 | 12609 | 434 | CTGAGGCCAGGGGGTCAATG |
| M-0194 | 3468 | 3487 | 12591 | 12610 | 435 | CCTGAGGCCAGGGGGTCAAT |
| M-0195 | 3469 | 3488 | 12592 | 12611 | 436 | CCCTGAGGCCAGGGGGTCAA |
| M-0196 | 3470 | 3489 | 12593 | 12612 | 437 | ACCCTGAGGCCAGGGGGTCA |
| M-0197 | 3472 | 3491 | 12595 | 12614 | 438 | AGACCCTGAGGCCAGGGGGT |
| M-0198 | 3474 | 3493 | 12597 | 12616 | 439 | TGAGACCCTGAGGCCAGGGG |
| M-0199 | 3475 | 3494 | 12598 | 12617 | 440 | GTGAGACCCTGAGGCCAGGG |
| M-0200 | 3476 | 3495 | 12599 | 12618 | 441 | GGTGAGACCCTGAGGCCAGG |

**[Table 15]**

| | | | | | | |
|---|---|---|---|---|---|---|
| M-0201 | 3477 | 3496 | 12600 | 12619 | 442 | AGGTGAGACCCTGAGGCCAG |
| M-0202 | 3478 | 3497 | 12601 | 12620 | 443 | AAGGTGAGACCCTGAGGCCA |
| M-0203 | 3480 | 3499 | 12603 | 12622 | 444 | GTAAGGTGAGACCCTGAGGC |
| M-0204 | 3481 | 3500 | 12604 | 12623 | 445 | GGTAAGGTGAGACCCTGAGG |
| M-0205 | 3482 | 3501 | 12605 | 12624 | 446 | GGGTAAGGTGAGACCCTGAG |
| M-0206 | 3483 | 3502 | 12606 | 12625 | 447 | CGGGTAAGGTGAGACCCTGA |
| M-0207 | 3484 | 3503 | 12607 | 12626 | 448 | CCGGGTAAGGTGAGACCCTG |
| M-0208 | 3485 | 3504 | 12608 | 12627 | 449 | TCCGGGTAAGGTGAGACCCT |
| M-0209 | 3487 | 3506 | 12610 | 12629 | 450 | GATCCGGGTAAGGTGAGACC |
| M-0210 | 3488 | 3507 | 12611 | 12630 | 451 | GGATCCGGGTAAGGTGAGAC |
| M-0211 | 3489 | 3508 | 12612 | 12631 | 452 | GGGATCCGGGTAAGGTGAGA |
| M-0212 | 3490 | 3509 | 12613 | 12632 | 453 | GGGGATCCGGGTAAGGTGAG |
| M-0213 | 3491 | 3510 | 12614 | 12633 | 454 | AGGGGATCCGGGTAAGGTGA |
| M-0214 | 3492 | 3511 | 12615 | 12634 | 455 | TAGGGGATCCGGGTAAGGTG |
| M-0215 | 3493 | 3512 | 12616 | 12635 | 456 | GTAGGGGATCCGGGTAAGGT |
| M-0216 | 3494 | 3513 | 12617 | 12636 | 457 | GGTAGGGGATCCGGGTAAGG |
| M-0217 | 3495 | 3514 | 12618 | 12637 | 458 | GGGTAGGGGATCCGGGTAAG |
| M-0218 | 3496 | 3515 | 12619 | 12638 | 459 | TGGGTAGGGGATCCGGGTAA |
| M-0219 | 3497 | 3516 | 12620 | 12639 | 460 | CTGGGTAGGGGATCCGGGTA |
| M-0220 | 3498 | 3517 | 12621 | 12640 | 461 | GCTGGGTAGGGGATCCGGGT |
| M-0221 | 3499 | 3518 | 12622 | 12641 | 462 | AGCTGGGTAGGGGATCCGGG |
| M-0222 | 3500 | 3519 | 12623 | 12642 | 463 | CAGCTGGGTAGGGGATCCGG |
| M-0223 | 3501 | 3520 | 12624 | 12643 | 464 | CCAGCTGGGTAGGGGATCCG |
| M-0224 | 3502 | 3521 | 12625 | 12644 | 465 | TCCAGCTGGGTAGGGGATCC |
| M-0225 | 3503 | 3522 | 12626 | 12645 | 466 | TTCCAGCTGGGTAGGGGATC |
| M-0226 | 3504 | 3523 | 12627 | 12646 | 467 | GTTCCAGCTGGGTAGGGGAT |
| M-0227 | 3505 | 3524 | 12628 | 12647 | 468 | AGTTCCAGCTGGGTAGGGGA |
| M-0228 | 3512 | 3531 | 12635 | 12654 | 469 | TAGGGAGAGTTCCAGCTGGG |
| M-0229 | 3513 | 3532 | 12636 | 12655 | 470 | TTAGGGAGAGTTCCAGCTGG |
| M-0230 | 3514 | 3533 | 12637 | 12656 | 471 | GTTAGGGAGAGTTCCAGCTG |
| M-0231 | 3515 | 3534 | 12638 | 12657 | 472 | GGTTAGGGAGAGTTCCAGCT |
| M-0232 | 3517 | 3536 | 12640 | 12659 | 473 | GGGGTTAGGGAGAGTTCCAG |
| M-0233 | 3518 | 3537 | 12641 | 12660 | 474 | GGGGGTTAGGGAGAGTTCCA |
| M-0234 | 3519 | 3538 | 12642 | 12661 | 475 | AGGGGGTTAGGGAGAGTTCC |
| M-0235 | 3520 | 3539 | 12643 | 12662 | 476 | CAGGGGGTTAGGGAGAGTTC |
| M-0236 | 3522 | 3541 | 12645 | 12664 | 477 | AGCAGGGGGTTAGGGAGAGT |
| M-0237 | 3524 | 3543 | 12647 | 12666 | 478 | GAAGCAGGGGGTTAGGGAGA |
| M-0238 | 3814 | 3833 | 13479 | 13498 | 479 | GGAGCCATGGTGCTCTCTTG |
| M-0239 | 3889 | 3908 | 13554 | 13573 | 480 | AGCACCCTCTTGGCTCTGGG |
| M-0240 | 3904 | 3923 | 13569 | 13588 | 481 | GCCCTGCAACTGAGCAGCAC |
| M-0241 | 3907 | 3926 | 13572 | 13591 | 482 | GAGGCCCTGCAACTGAGCAG |
| M-0242 | 4038 | 4057 | 13703 | 13722 | 483 | AGGAAGAGAGACCTGGTCCA |
| M-0243 | 4039 | 4058 | 13704 | 13723 | 484 | AAGGAAGAGAGACCTGGTCC |
| M-0244 | 4041 | 4060 | 13706 | 13725 | 485 | ACAAGGAAGAGAGACCTGGT |
| M-0245 | 4042 | 4061 | 13707 | 13726 | 486 | GACAAGGAAGAGAGACCTGG |
| M-0246 | 4120 | 4139 | 13785 | 13804 | 487 | TGAAATAATCACACCAACGG |

**[Table 16]**

| | | | | | | |
|---|---|---|---|---|---|---|
| L-0001 | 63 | 78 | - | - | 3 | TCAATGAAACCTGCTG |
| L-0002 | 64 | 79 | - | - | 4 | TTCAATGAAACCTGCT |
| L-0003 | 67 | 82 | 5526 | 5541 | 5 | GTTTTCAATGAAACCT |
| L-0004 | 69 | 84 | 5528 | 5543 | 6 | CTGTTTTCAATGAAAC |
| L-0005 | 71 | 86 | 5530 | 5545 | 7 | ATCTGTTTTCAATGAA |
| L-0006 | 75 | 90 | 5534 | 5549 | 8 | CAGGATCTGTTTTCAA |
| L-0007 | 77 | 92 | 5536 | 5551 | 9 | TGCAGGATCTGTTTTC |
| L-0008 | 89 | 104 | 5548 | 5563 | 10 | ACCTGGAACTTTTGCA |
| L-0009 | 91 | 106 | 5550 | 5565 | 11 | GCACCTGGAACTTTTG |
| L-0010 | 110 | 125 | 5569 | 5584 | 12 | CTCCAAGTTTCCAGTG |
| L-0011 | 118 | 133 | 5577 | 5592 | 13 | AGCAGGATCTCCAAGT |
| L-0012 | 120 | 135 | 5579 | 5594 | 14 | GAAGCAGGATCTCCAA |
| L-0013 | 170 | 185 | 5629 | 5644 | 15 | ATACAGAAACTTCTCT |
| L-0014 | 221 | 236 | 5680 | 5695 | 16 | CTTCATTCTTCAGGCT |
| L-0015 | 226 | 241 | 5685 | 5700 | 17 | CGTGTCTTCATTCTTC |
| L-0016 | 238 | 253 | 5697 | 5712 | 18 | TCTTTATTCTGTCGTG |
| L-0017 | 239 | 254 | 5698 | 5713 | 19 | GTCTTTATTCTGTCGT |
| L-0018 | 241 | 256 | 5700 | 5715 | 20 | GAGTCTTTATTCTGTC |
| L-0019 | 244 | 259 | - | - | 21 | ATCGAGTCTTTATTCT |
| L-0020 | 257 | 272 | 5863 | 5878 | 22 | ACTCCTCATTGACATC |
| L-0021 | 300 | 315 | 5906 | 5921 | 23 | ATCTCAGTTCTTCCCG |
| L-0022 | 302 | 317 | 5908 | 5923 | 24 | CGATCTCAGTTCTTCC |
| L-0023 | 304 | 319 | 5910 | 5925 | 25 | CTCGATCTCAGTTCTT |
| L-0024 | 353 | 368 | 5959 | 5974 | 26 | TTTGCCATCACTGCTG |
| L-0025 | 354 | 369 | 5960 | 5975 | 27 | CTTTGCCATCACTGCT |
| L-0026 | 371 | 386 | 5977 | 5992 | 28 | CTGCCTGGACTTCTCA |
| L-0027 | 419 | 434 | 6176 | 6191 | 29 | GTTGACCTTTGGGGTG |
| L-0028 | 428 | 443 | 6185 | 6200 | 30 | ACCCTGCTTGTTGACC |
| L-0029 | 458 | 473 | 6215 | 6230 | 31 | ACTTTCACTCTCTGAG |
| L-0030 | 464 | 479 | 6221 | 6236 | 32 | CTCCTCACTTTCACTC |
| L-0031 | 465 | 480 | 6222 | 6237 | 33 | TCTCCTCACTTTCACT |
| L-0032 | 473 | 488 | 6230 | 6245 | 34 | TGCATTGGTCTCCTCA |
| L-0033 | 483 | 498 | 6240 | 6255 | 35 | TCTTTTTTGGTGCATT |
| L-0034 | 497 | 512 | - | - | 36 | CTGCTCAGTTTTGGTC |
| L-0035 | 538 | 553 | 7263 | 7278 | 37 | AAGCTATCCAGATCGG |
| L-0036 | 539 | 554 | 7264 | 7279 | 38 | CAAGCTATCCAGATCG |
| L-0037 | 540 | 555 | 7265 | 7280 | 39 | CCAAGCTATCCAGATC |
| L-0038 | 544 | 559 | 7269 | 7284 | 40 | CCGTCCAAGCTATCCA |
| L-0039 | 559 | 574 | 7284 | 7299 | 41 | TCATTAAGGCTCCGCC |
| L-0040 | 562 | 577 | 7287 | 7302 | 42 | TCATCATTAAGGCTCC |
| L-0041 | 568 | 583 | 7293 | 7308 | 43 | CTGCCATCATCATTAA |
| L-0042 | 569 | 584 | 7294 | 7309 | 44 | GCTGCCATCATCATTA |
| L-0043 | 570 | 585 | 7295 | 7310 | 45 | TGCTGCCATCATCATT |
| L-0044 | 585 | 600 | 7310 | 7325 | 46 | TATCCCTAGGGTCGCT |
| L-0045 | 587 | 602 | 7312 | 7327 | 47 | GATATCCCTAGGGTCG |
| L-0046 | 603 | 618 | 7328 | 7343 | 48 | TTCGGTTGTCCTGGTC |
| L-0047 | 626 | 641 | 7351 | 7366 | 49 | GCTGTAGATACTGGGG |
| L-0048 | 630 | 645 | 7355 | 7370 | 50 | CAGGGCTGTAGATACT |
| L-0049 | 642 | 657 | 7367 | 7382 | 51 | TCTCCACACTTCCAGG |
| L-0050 | 643 | 658 | 7368 | 7383 | 52 | TTCTCCACACTTCCAG |

**[Table 17]**

| | | | | | | |
|---|---|---|---|---|---|---|
| L-0051 | 651 | 666 | 7376 | 7391 | 53 | CAGAGTCATTCTCCAC |
| L-0052 | 654 | 669 | 7379 | 7394 | 54 | AGTCAGAGTCATTCTC |
| L-0053 | 666 | 681 | 7391 | 7406 | 55 | GGCCAGAAGATGAGTC |
| L-0054 | 767 | 782 | 7492 | 7507 | 56 | TTCAAAGCTAGCCTCT |
| L-0055 | 768 | 783 | 7493 | 7508 | 57 | GTTCAAAGCTAGCCTC |
| L-0056 | 771 | 786 | 7496 | 7511 | 58 | GGGGTTCAAAGCTAGC |
| L-0057 | 789 | 804 | 7514 | 7529 | 59 | TGGGTGTCACAGAAGG |
| L-0058 | 804 | 819 | 7529 | 7544 | 60 | GAGCATGATATCCAGT |
| L-0059 | 838 | 853 | 7563 | 7578 | 61 | GCCTGGAACATTCGAG |
| L-0060 | 839 | 854 | 7564 | 7579 | 62 | AGCCTGGAACATTCGA |
| L-0061 | 880 | 895 | 7605 | 7620 | 63 | CCAGGATAGAGCTGTG |
| L-0062 | 901 | 916 | 7626 | 7641 | 64 | GACAAAACTCCACCAG |
| L-0063 | 904 | 919 | 7629 | 7644 | 65 | CCAGACAAAACTCCAC |
| L-0064 | 907 | 922 | 7632 | 7647 | 66 | GGTCCAGACAAAACTC |
| L-0065 | 972 | 987 | 7697 | 7712 | 67 | GCTGCTTACCCCCATT |
| L-0066 | 995 | 1010 | 7720 | 7735 | 68 | AATGGGAGTAGTGGGT |
| L-0067 | 999 | 1014 | 7724 | 7739 | 69 | CTGAAATGGGAGTAGT |
| L-0068 | 1009 | 1024 | 7734 | 7749 | 70 | GAGCTTGATACTGAAA |
| L-0069 | 1014 | 1029 | 7739 | 7754 | 71 | CCCCAGAGCTTGATAC |
| L-0070 | 1080 | 1095 | 7805 | 7820 | 72 | GAGCAGAAGGTAGGTT |
| L-0071 | 1103 | 1118 | 7828 | 7843 | 73 | ATGGGGGAAGTTGGCT |
| L-0072 | 1158 | 1173 | 7883 | 7898 | 74 | CTGATGCATTGTTGAG |
| L-0073 | 1204 | 1219 | 7929 | 7944 | 75 | GGCAGATGACCAGGTA |
| L-0074 | 1238 | 1253 | 7963 | 7978 | 76 | TCCACCCATACCCTGT |
| L-0075 | 1245 | 1260 | 7970 | 7985 | 77 | GAGGAAGTCCACCCAT |
| L-0076 | 1313 | 1328 | 8038 | 8053 | 78 | AGCAGAAGAGGAAGCA |
| L-0077 | 1340 | 1355 | 8065 | 8080 | 79 | ATAAGGAAACCTCATG |
| L-0078 | 1342 | 1357 | 8067 | 8082 | 80 | GAATAAGGAAACCTCA |
| L-0079 | 1354 | 1369 | 8079 | 8094 | 81 | CTACTAGAGGATGAAT |
| L-0080 | 1362 | 1377 | 8087 | 8102 | 82 | CAGAGCTACTACTAGA |
| L-0081 | 1365 | 1380 | 8090 | 8105 | 83 | CTGCAGAGCTACTACT |
| L-0082 | 1382 | 1397 | 8107 | 8122 | 84 | ACTGGAAGAGGAGGCT |
| L-0083 | 1652 | 1667 | 8377 | 8392 | 85 | ATGTGTTGAGACTGGT |
| L-0084 | 1818 | 1833 | 8543 | 8558 | 86 | AGGGAGACATGGCGTA |
| L-0085 | 1943 | 1958 | 8668 | 8683 | 87 | AGAGGAAGAGTTGGAA |
| L-0086 | 1955 | 1970 | 8680 | 8695 | 88 | TTGAGAAGTGGAAGAG |
| L-0087 | 1977 | 1992 | 8702 | 8717 | 89 | GGTGTGAACATGGGTA |
| L-0088 | 1980 | 1995 | 8705 | 8720 | 90 | AGGGGTGTGAACATGG |
| L-0089 | 2060 | 2075 | 8785 | 8800 | 91 | GGAAAGGGTAGCCGAA |
| L-0090 | 2106 | 2121 | 8831 | 8846 | 92 | CCGTTTTGTAGCCTGC |
| L-0091 | 2172 | 2187 | 8897 | 8912 | 93 | TGGCTGTCTTGTAGGC |
| L-0092 | 2400 | 2415 | 9125 | 9140 | 94 | CCTGTTTGATCTGCGT |
| L-0093 | 2402 | 2417 | 9127 | 9142 | 95 | CTCCTGTTTGATCTGC |
| L-0094 | 2423 | 2438 | 9148 | 9163 | 96 | GGTCTCATACTCCTCA |
| L-0095 | 2484 | 2499 | 9209 | 9224 | 97 | GTACATCTACCACCTT |
| L-0096 | 2527 | 2542 | 9535 | 9550 | 98 | TCCAGGTGTTTGTTGA |
| L-0097 | 2570 | 2585 | 9578 | 9593 | 99 | CACGAAGTACAGGTCG |
| L-0098 | 2743 | 2758 | - | - | 100 | TGAGCCAACTTCACGC |
| L-0099 | 2880 | 2895 | 10301 | 10316 | 101 | ATTCACTGAGAGTGCG |
| L-0100 | 2883 | 2898 | 10304 | 10319 | 102 | CATATTCACTGAGAGT |

**[Table 18]**

| | | | | | | |
|---|---|---|---|---|---|---|
| L-0101 | 2885 | 2900 | 10306 | 10321 | 103 | GGCATATTCACTGAGA |
| L-0102 | 2887 | 2902 | 10308 | 10323 | 104 | CGGGCATATTCACTGA |
| L-0103 | 2888 | 2903 | 10309 | 10324 | 105 | CCGGGCATATTCACTG |
| L-0104 | 2903 | 2918 | 10324 | 10339 | 106 | AGACATGACATGAGGC |
| L-0105 | 2905 | 2920 | 10326 | 10341 | 107 | GGAGACATGACATGAG |
| L-0106 | 2908 | 2923 | 10329 | 10344 | 108 | CCAGGAGACATGACAT |
| L-0107 | 2931 | 2946 | 10352 | 10367 | 109 | CGTAGAATGGATGGTT |
| L-0108 | 2973 | 2988 | 10394 | 10409 | 110 | CATTGTAACCCAGGAG |
| L-0109 | 2979 | 2994 | 10400 | 10415 | 111 | CCGGGACATTGTAACC |
| L-0110 | 2995 | 3010 | 10416 | 10431 | 112 | TCACTGCTGTACAGGG |
| L-0111 | 3000 | 3015 | 10421 | 10436 | 113 | CTGGATCACTGCTGTA |
| L-0112 | 3001 | 3016 | 10422 | 10437 | 114 | GCTGGATCACTGCTGT |
| L-0113 | 3072 | 3087 | 10493 | 10508 | 115 | TCACCTCAAAGCCAGG |
| L-0114 | 3084 | 3099 | 10505 | 10520 | 116 | GCTCACTAGGCTTCAC |
| L-0115 | 3194 | 3209 | 10615 | 10630 | 117 | CGGATGAAAGGGGAAA |
| L-0116 | 3267 | 3282 | 10688 | 10703 | 118 | CATAGGACATGTCAGG |
| L-0117 | 3271 | 3286 | 10692 | 10707 | 119 | TCAGCATAGGACATGT |
| L-0118 | 3272 | 3287 | 10693 | 10708 | 120 | CTCAGCATAGGACATG |
| L-0119 | 3274 | 3289 | 10695 | 10710 | 121 | CGCTCAGCATAGGACA |
| L-0120 | 3356 | 3371 | 10777 | 10792 | 122 | CACATTGAGCATCTGC |
| L-0121 | 3359 | 3374 | 10780 | 10795 | 123 | AGTCACATTGAGCATC |
| L-0122 | 3362 | 3377 | 10783 | 10798 | 124 | GGGAGTCACATTGAGC |
| L-0123 | 3421 | 3436 | 10842 | 10857 | 125 | ATAGCATCTTGCTGGT |
| L-0124 | 3425 | 3440 | 10846 | 10861 | 126 | ATGGATAGCATCTTGC |
| L-0125 | 3429 | 3444 | 10850 | 10865 | 127 | CTGCATGGATAGCATC |
| L-0126 | 3489 | 3504 | 12612 | 12627 | 128 | TCCGGGTAAGGTGAGA |
| L-0127 | 3754 | 3769 | 13227 | 13242 | 129 | CTGTAGTAGTCCTCCT |
| L-0128 | 3765 | 3780 | - | - | 130 | TCTTCAGGTGACTGTA |
| L-0129 | 3797 | 3812 | 13462 | 13477 | 131 | TCGCAGGTTCTACAGT |
| L-0130 | 3798 | 3813 | 13463 | 13478 | 132 | ATCGCAGGTTCTACAG |
| L-0131 | 3816 | 3831 | 13481 | 13496 | 133 | AGCCATGGTGCTCTCT |
| L-0132 | 3826 | 3841 | 13491 | 13506 | 134 | CCAATGTAGGAGCCAT |
| L-0133 | 3829 | 3844 | 13494 | 13509 | 135 | GGTCCAATGTAGGAGC |
| L-0134 | 3835 | 3850 | 13500 | 13515 | 136 | CTCCAAGGTCCAATGT |
| L-0135 | 3837 | 3852 | 13502 | 13517 | 137 | TGCTCCAAGGTCCAAT |
| L-0136 | 4025 | 4040 | 13690 | 13705 | 138 | CCAAGATTCTGTGCGC |
| L-0137 | 4032 | 4047 | 13697 | 13712 | 139 | ACCTGGTCCAAGATTC |
| L-0138 | 4046 | 4061 | 13711 | 13726 | 140 | GACAAGGAAGAGAGAC |
| L-0139 | 4119 | 4134 | 13784 | 13799 | 141 | TAATCACACCAACGGG |
| L-0140 | 4121 | 4136 | 13786 | 13801 | 142 | AATAATCACACCAACG |
| L-0141 | 4122 | 4137 | 13787 | 13802 | 143 | AAATAATCACACCAAC |
| L-0142 | 4123 | 4138 | 13788 | 13803 | 144 | GAAATAATCACACCAA |
| L-0143 | 4125 | 4140 | 13790 | 13805 | 145 | ATGAAATAATCACACC |
| L-0144 | 4132 | 4147 | 13797 | 13812 | 146 | CTAACAGATGAAATAA |
| L-0145 | 4138 | 4153 | 13803 | 13818 | 147 | CCACATCTAACAGATG |
| L-0146 | 4141 | 4156 | 13806 | 13821 | 148 | CAGCCACATCTAACAG |
| L-0147 | 4142 | 4157 | 13807 | 13822 | 149 | ACAGCCACATCTAACA |
| L-0148 | 4149 | 4164 | 13814 | 13829 | 150 | ACGCAAAACAGCCACA |
| L-0149 | 4218 | 4233 | 13883 | 13898 | 151 | AAGGGGCATACATTGC |
| L-0150 | 4220 | 4235 | 13885 | 13900 | 152 | GCAAGGGGCATACATT |

**[Table 19]**

| | | | | | | |
|---|---|---|---|---|---|---|
| L-0151 | 4266 | 4281 | 13931 | 13946 | 153 | CGTCATATAGATATTT |
| L-0152 | 537 | 552 | 7262 | 7277 | 154 | AGCTATCCAGATCGGA |
| L-0153 | 541 | 556 | 7266 | 7281 | 155 | TCCAAGCTATCCAGAT |
| L-0154 | 586 | 601 | 7311 | 7326 | 156 | ATATCCCTAGGGTCGC |
| L-0155 | 588 | 603 | 7313 | 7328 | 157 | CGATATCCCTAGGGTC |
| L-0156 | 664 | 679 | 7389 | 7404 | 158 | CCAGAAGATGAGTCAG |
| L-0157 | 665 | 680 | 7390 | 7405 | 159 | GCCAGAAGATGAGTCA |
| L-0158 | 667 | 682 | 7392 | 7407 | 160 | AGGCCAGAAGATGAGT |
| L-0159 | 668 | 683 | 7393 | 7408 | 161 | CAGGCCAGAAGATGAG |
| L-0160 | 787 | 802 | 7512 | 7527 | 162 | GGTGTCACAGAAGGAT |
| L-0161 | 788 | 803 | 7513 | 7528 | 163 | GGGTGTCACAGAAGGA |
| L-0162 | 790 | 805 | 7515 | 7530 | 164 | GTGGGTGTCACAGAAG |
| L-0163 | 791 | 806 | 7516 | 7531 | 165 | AGTGGGTGTCACAGAA |
| L-0164 | 840 | 855 | 7565 | 7580 | 166 | GAGCCTGGAACATTCG |
| L-0165 | 994 | 1009 | 7719 | 7734 | 167 | ATGGGAGTAGTGGGTG |
| L-0166 | 996 | 1011 | 7721 | 7736 | 168 | AAATGGGAGTAGTGGG |
| L-0167 | 997 | 1012 | 7722 | 7737 | 169 | GAAATGGGAGTAGTGG |
| L-0168 | 1007 | 1022 | 7732 | 7747 | 170 | GCTTGATACTGAAATG |
| L-0169 | 1008 | 1023 | 7733 | 7748 | 171 | AGCTTGATACTGAAAT |
| L-0170 | 1010 | 1025 | 7735 | 7750 | 172 | AGAGCTTGATACTGAA |
| L-0171 | 1011 | 1026 | 7736 | 7751 | 173 | CAGAGCTTGATACTGA |
| L-0172 | 1363 | 1378 | 8088 | 8103 | 174 | GCAGAGCTACTACTAG |
| L-0173 | 1364 | 1379 | 8089 | 8104 | 175 | TGCAGAGCTACTACTA |
| L-0174 | 1650 | 1665 | 8375 | 8390 | 176 | GTGTTGAGACTGGTGG |
| L-0175 | 1651 | 1666 | 8376 | 8391 | 177 | TGTGTTGAGACTGGTG |
| L-0176 | 1653 | 1668 | 8378 | 8393 | 178 | GATGTGTTGAGACTGG |
| L-0177 | 1654 | 1669 | 8379 | 8394 | 179 | TGATGTGTTGAGACTG |
| L-0178 | 1975 | 1990 | 8700 | 8715 | 180 | TGTGAACATGGGTAGG |
| L-0179 | 1976 | 1991 | 8701 | 8716 | 181 | GTGTGAACATGGGTAG |
| L-0180 | 1978 | 1993 | 8703 | 8718 | 182 | GGGTGTGAACATGGGT |
| L-0181 | 2061 | 2076 | 8786 | 8801 | 183 | TGGAAAGGGTAGCCGA |
| L-0182 | 2881 | 2896 | 10302 | 10317 | 184 | TATTCACTGAGAGTGC |
| L-0183 | 2882 | 2897 | 10303 | 10318 | 185 | ATATTCACTGAGAGTG |
| L-0184 | 2884 | 2899 | 10305 | 10320 | 186 | GCATATTCACTGAGAG |
| L-0185 | 2886 | 2901 | 10307 | 10322 | 187 | GGGCATATTCACTGAG |
| L-0186 | 2889 | 2904 | 10310 | 10325 | 188 | GCCGGGCATATTCACT |
| L-0187 | 2901 | 2916 | 10322 | 10337 | 189 | ACATGACATGAGGCCG |
| L-0188 | 2902 | 2917 | 10323 | 10338 | 190 | GACATGACATGAGGCC |
| L-0189 | 2904 | 2919 | 10325 | 10340 | 191 | GAGACATGACATGAGG |
| L-0190 | 2980 | 2995 | 10401 | 10416 | 192 | GCCGGGACATTGTAAC |
| L-0191 | 3192 | 3207 | 10613 | 10628 | 193 | GATGAAAGGGGAAAGG |
| L-0192 | 3193 | 3208 | 10614 | 10629 | 194 | GGATGAAAGGGGAAAG |
| L-0193 | 3195 | 3210 | 10616 | 10631 | 195 | TCGGATGAAAGGGGAA |
| L-0194 | 3196 | 3211 | 10617 | 10632 | 196 | CTCGGATGAAAGGGGA |
| L-0195 | 3270 | 3285 | 10691 | 10706 | 197 | CAGCATAGGACATGTC |
| L-0196 | 3273 | 3288 | 10694 | 10709 | 198 | GCTCAGCATAGGACAT |
| L-0197 | 3360 | 3375 | 10781 | 10796 | 199 | GAGTCACATTGAGCAT |
| L-0198 | 3361 | 3376 | 10782 | 10797 | 200 | GGAGTCACATTGAGCA |
| L-0199 | 3363 | 3378 | 10784 | 10799 | 201 | GGGGAGTCACATTGAG |
| L-0200 | 3364 | 3379 | 10785 | 10800 | 202 | TGGGGAGTCACATTGA |

**[Table 20]**

| | | | | | | |
|---|---|---|---|---|---|---|
| L-0201 | 3483 | 3498 | 12606 | 12621 | 203 | TAAGGTGAGACCCTGA |
| L-0202 | 3484 | 3499 | 12607 | 12622 | 204 | GTAAGGTGAGACCCTG |
| L-0203 | 3485 | 3500 | 12608 | 12623 | 205 | GGTAAGGTGAGACCCT |
| L-0204 | 3486 | 3501 | 12609 | 12624 | 206 | GGGTAAGGTGAGACCC |
| L-0205 | 3487 | 3502 | 12610 | 12625 | 207 | CGGGTAAGGTGAGACC |
| L-0206 | 3488 | 3503 | 12611 | 12626 | 208 | CCGGGTAAGGTGAGAC |
| L-0207 | 3490 | 3505 | 12613 | 12628 | 209 | ATCCGGGTAAGGTGAG |
| L-0208 | 4024 | 4039 | 13689 | 13704 | 210 | CAAGATTCTGTGCGCT |
| L-0209 | 4026 | 4041 | 13691 | 13706 | 211 | TCCAAGATTCTGTGCG |
| L-0210 | 4027 | 4042 | 13692 | 13707 | 212 | GTCCAAGATTCTGTGC |
| L-0211 | 4219 | 4234 | 13884 | 13899 | 213 | CAAGGGGCATACATTG |
| L-0212 | 4221 | 4236 | 13886 | 13901 | 214 | GGCAAGGGGCATACAT |
| L-0213 | 3452 | 3467 | 12575 | 12590 | 215 | GAGAGGGTGCACCGAG |
| L-0214 | 3453 | 3468 | 12576 | 12591 | 216 | TGAGAGGGTGCACCGA |
| L-0215 | 3456 | 3471 | 12579 | 12594 | 217 | CAATGAGAGGGTGCAC |
| L-0216 | 3457 | 3472 | 12580 | 12595 | 218 | TCAATGAGAGGGTGCA |
| L-0217 | 3458 | 3473 | 12581 | 12596 | 219 | GTCAATGAGAGGGTGC |
| L-0218 | 3459 | 3474 | 12582 | 12597 | 220 | GGTCAATGAGAGGGTG |
| L-0219 | 3460 | 3475 | 12583 | 12598 | 221 | GGGTCAATGAGAGGGT |
| L-0220 | 3461 | 3476 | 12584 | 12599 | 222 | GGGGTCAATGAGAGGG |
| L-0221 | 3462 | 3477 | 12585 | 12600 | 223 | GGGGGTCAATGAGAGG |
| L-0222 | 3463 | 3478 | 12586 | 12601 | 224 | AGGGGGTCAATGAGAG |
| L-0223 | 3464 | 3479 | 12587 | 12602 | 225 | CAGGGGGTCAATGAGA |
| L-0224 | 3465 | 3480 | 12588 | 12603 | 226 | CCAGGGGGTCAATGAG |
| L-0225 | 3466 | 3481 | 12589 | 12604 | 227 | GCCAGGGGGTCAATGA |
| L-0226 | 3468 | 3483 | 12591 | 12606 | 228 | AGGCCAGGGGGTCAAT |
| L-0227 | 3478 | 3493 | 12601 | 12616 | 229 | TGAGACCCTGAGGCCA |
| L-0228 | 3481 | 3496 | 12604 | 12619 | 230 | AGGTGAGACCCTGAGG |
| L-0229 | 3482 | 3497 | 12605 | 12620 | 231 | AAGGTGAGACCCTGAG |
| L-0230 | 3491 | 3506 | 12614 | 12629 | 232 | GATCCGGGTAAGGTGA |
| L-0231 | 3496 | 3511 | 12619 | 12634 | 233 | TAGGGGATCCGGGTAA |
| L-0232 | 3503 | 3518 | 12626 | 12641 | 234 | AGCTGGGTAGGGGATC |
| L-0233 | 3515 | 3530 | 12638 | 12653 | 235 | AGGGAGAGTTCCAGCT |
| L-0234 | 3516 | 3531 | 12639 | 12654 | 236 | TAGGGAGAGTTCCAGC |
| L-0235 | 3517 | 3532 | 12640 | 12655 | 237 | TTAGGGAGAGTTCCAG |
| L-0236 | 3518 | 3533 | 12641 | 12656 | 238 | GTTAGGGAGAGTTCCA |
| L-0237 | 3519 | 3534 | 12642 | 12657 | 239 | GGTTAGGGAGAGTTCC |
| L-0238 | 3520 | 3535 | 12643 | 12658 | 240 | GGGTTAGGGAGAGTTC |
| L-0239 | 3521 | 3536 | 12644 | 12659 | 241 | GGGGTTAGGGAGAGTT |

### [Evaluation Example 1] Antisense inhibition of human ATN1 in SH-SY5Y cells

An effect of an antisense oligonucleotide targeting ATN1 nucleic acid, which is a causative gene of DRPLA, on an ATN1 RNA transcript was tested in vitro. After approximately 24 hours, a 300 nM antisense oligonucleotide (manufactured in Manufacturing Example 1) was transfected into SH-SY5Y cells seeded in a 96 well plate at a density of 12,000 cells/well (ASO was added such that a final concentration thereof was 300 nM) using Lipofectamin (registered trademark) RNAiMAX Transfection Reagent (Thermo Fisher Scientific). After approximately 48 hours, RNA was isolated from the cells using RNeasy Micro Kit (QIAGEN), and then reverse-transcribed with High-Capacity cDNA Reverse Transcription Kit (Thermo Fisher Scientific) to create cDNA. Using the created cDNA, an ATN1 gene expression level was measured by quantitative real-time PCR with TaqMan (registered trademark) Gene Expression Assays (Thermo Fisher Scientific). Results thereof are presented in Tables 21 to 24 as percent expression of ATN1 relative to untreated control cells. Note that the title rows in Tables 22 to 24 are the same as that in Table 21, and thus are omitted.

**[Table 21]**

| Cmpd No | ATN1 mRNA remain % |
|---|---|
| M-0001 | 78.6 |
| M-0006 | 73.5 |
| M-0007 | 79.0 |
| M-0012 | 57.8 |
| M-0013 | 66.4 |
| M-0014 | 75.0 |
| M-0015 | 75.8 |
| M-0017 | 65.8 |
| M-0018 | 57.0 |
| M-0022 | 70.3 |
| M-0023 | 64.1 |
| M-0033 | 75.3 |
| M-0035 | 78.8 |
| M-0036 | 73.5 |
| M-0037 | 77.2 |
| M-0039 | 79.0 |
| M-0040 | 65.8 |
| M-0042 | 79.7 |
| M-0045 | 66.4 |
| M-0046 | 74.0 |
| M-0047 | 55.6 |
| M-0050 | 72.5 |
| M-0051 | 69.8 |
| M-0052 | 77.4 |
| M-0053 | 68.0 |
| M-0055 | 78.8 |
| M-0056 | 69.2 |
| M-0057 | 60.4 |
| M-0058 | 59.1 |
| M-0059 | 75.8 |
| M-0061 | 58.8 |
| M-0062 | 64.2 |
| M-0063 | 70.1 |
| M-0064 | 73-9 |
| M-0065 | 64.1 |
| M-0066 | 709 |
| M-0068 | 73.4 |
| M-0069 | 62.9 |
| M-0070 | 76.1 |
| M-0072 | 67.2 |
| M-0073 | 72.7 |
| M-0074 | 66.0 |
| M-0075 | 58.6 |
| M-0076 | 74.9 |
| M-0078 | 75.7 |
| M-0079 | 55.4 |
| M-0080 | 68.6 |
| M-0082 | 64.0 |
| M-0083 | 71.5 |
| M-0084 | 75.0 |
| M-0085 | 62-0 |
| M-0086 | 45.1 |
| M-0087 | 62.8 |
| M-00B8 | 77.4 |
| M-0089 | 63.6 |
| M-0090 | 74.5 |
| M-0091 | 50.1 |
| M-0092 | 49.9 |
| M-0094 | 68.8 |
| M-0096 | 44.9 |
| M-0097 | 74.4 |
| M-0098 | 66.1 |
| M-0099 | 70.3 |
| M-0100 | 66.8 |
| M-0101 | 71.4 |
| M-0103 | 67.2 |
| M-0104 | 54.0 |
| M-0105 | 58.5 |
| M-0106 | 68.6 |
| M-0107 | 73.9 |
| M-0108 | 75.1 |
| M-0109 | 53.6 |
| M-0114 | 56.9 |
| M-0116 | 58.3 |
| M-0117 | 53.2 |
| M-0118 | 76.6 |
| M-0119 | 713 |
| M-0120 | 52.8 |
| M-0121 | 556 |
| M-0122 | 23.8 |
| M-0123 | 10.9 |
| M-0124 | 28.5 |
| M-0125 | 47.0 |
| M-0126 | 65.6 |
| M-0128 | 74.9 |
| M-0130 | 77.2 |
| M-0131 | 61.8 |
| M-0132 | 75.7 |
| M-0133 | 69.6 |
| M-0134 | 62.1 |
| M-0135 | 56.5 |
| M-0136 | 69.6 |
| M-0137 | 68.9 |
| M-0138 | 52.2 |
| M-0139 | 73.4 |
| M-0141 | 62.9 |
| M-0151 | 68.9 |
| M-0152 | 70-7 |
| M-0153 | 60.2 |

**[Table 22]**

| | |
|---|---|
| M-0154 | 50.1 |
| M-0155 | 70.5 |
| M-0156 | 55.3 |
| M-0157 | 61.4 |
| M-0158 | 73.0 |
| M-0159 | 58.4 |
| M-0160 | 40.2 |
| M-0161 | 70.1 |
| M-0162 | 63.3 |
| M-0163 | 57.8 |
| M-0164 | 66.7 |
| M-0165 | 65.7 |
| M-0166 | 58.8 |
| M-0168 | 66.3 |
| M-0169 | 48.4 |
| M-0170 | 52.8 |
| M-0171 | 33.2 |
| M-0172 | 45.8 |
| M-0173 | 38.7 |
| M-0174 | 44.1 |
| M-0175 | 39.7 |
| M-0176 | 7.0 |
| M-0177 | 13.6 |
| M-0178 | 21.5 |
| M-0179 | 49.3 |
| M-0180 | 33.1 |
| M-0181 | 26.2 |
| M-0182 | 21.2 |
| M-0183 | 16.9 |
| M-0184 | 13.7 |
| M-0185 | 21.8 |
| M-0186 | 33.7 |
| M-0187 | 25.4 |
| M-0188 | 28.7 |
| M-0189 | 28.6 |
| M-0190 | 27.6 |
| M-0191 | 13.5 |
| M-0192 | 23.6 |
| M-0193 | 23.9 |
| M-0194 | 15.3 |
| M-0195 | 22.5 |
| M-0196 | 46.4 |
| M-0197 | 54.8 |
| M-0198 | 28.7 |
| M-0199 | 20.1 |
| M-0200 | 13.7 |
| M-0201 | 20.1 |
| M-0202 | 11.5 |
| M-0203 | 27.1 |
| M-0204 | 21.3 |
| M-0205 | 35.2 |
| M-0206 | 59.2 |
| M-0207 | 60.9 |
| M-0208 | 57.9 |
| M-0209 | 21.7 |
| M-0210 | 15.0 |
| M-0211 | 14.0 |
| M-0212 | 17.2 |
| M-0213 | 13.2 |
| M-0214 | 21.2 |
| M-0215 | 29.1 |
| M-0216 | 447 |
| M-0217 | 46.0 |
| M-0218 | 34.0 |
| M-0219 | 19.4 |
| M-0220 | 12.6 |
| M-0221 | 19.3 |
| M-0222 | 15.1 |
| M-0223 | 11.4 |
| M-0224 | 10.5 |
| M-0225 | 11.3 |
| M-0226 | 22.4 |
| M-0227 | 27.7 |
| M-0228 | 51-7 |
| M-0229 | 65.1 |
| M-0230 | 54.9 |
| M-0231 | 49.9 |
| M-0232 | 49.8 |
| M-0233 | 68.4 |
| M-0234 | 70.4 |
| M-0235 | 73.2 |
| M-0236 | 62.5 |
| M-0237 | 67.9 |
| M-0238 | 62.3 |
| M0239 | 57.1 |
| M-0240 | 77.4 |
| M-0241 | 52.8 |
| M-0242 | 59.4 |
| M-0243 | 585 |
| M-0244 | 56.0 |
| M-0245 | 65.0 |
| M-0246 | 78.6 |

**[Table 23]**

| | |
|---|---|
| L-0001 | 74.6 |
| L-0002 | 75.8 |
| L-0007 | 69.4 |
| L-0008 | 70.6 |
| L-0010 | 78.6 |
| L-0011 | 75.1 |
| L-0016 | 74.5 |
| L-0017 | 63.0 |
| L-0018 | 63.7 |
| L-0019 | 67.3 |
| L-0024 | 76.8 |
| L-0027 | 66.5 |
| L-0032 | 66.2 |
| L-0035 | 42.4 |
| L-0036 | 56.7 |
| L-0037 | 73.9 |
| L-0038 | 74.4 |
| L-0042 | 67.6 |
| L-0043 | 77.3 |
| L-0044 | 71.9 |
| L-0045 | 48.0 |
| L-0046 | 69.8 |
| L-0047 | 79.6 |
| L-0048 | 68.8 |
| L-0050 | 78.9 |
| L-0053 | 45.2 |
| L-0057 | 60.2 |
| L-0058 | 75.7 |
| L-0059 | 56.7 |
| L-0060 | 67.4 |
| L-0061 | 70.7 |
| L-0064 | 71.6 |
| L-0065 | 68.8 |
| L-0066 | 58.5 |
| L-0067 | 73.9 |
| L-0068 | 59.1 |
| L-0070 | 67.4 |
| L-0072 | 71.6 |
| L-0073 | 74.6 |
| L-0076 | 65.8 |
| L-0077 | 78.4 |
| L-0080 | 74.1 |
| L-0081 | 58.6 |
| L-0082 | 69.5 |
| L-0083 | 65.0 |
| L-0084 | 69.9 |
| L-0085 | 79.8 |
| L-0086 | 69.2 |
| L-0087 | 64.5 |
| L-0089 | 64.2 |
| L-0090 | 76.0 |
| L-0092 | 79.3 |
| L-0093 | 76.9 |
| L-0095 | 77.6 |
| L-0097 | 75.0 |
| L-0099 | 64.9 |
| L-0100 | 54.4 |
| L-0101 | 66.6 |
| L-0102 | 62.7 |
| L-0103 | 50.4 |
| L-0104 | 58.3 |
| L-0105 | 67.2 |
| L-0106 | 75.1 |
| L-0107 | 68.4 |
| L-0108 | 78.3 |
| L-0109 | 43.7 |
| L-0110 | 69.5 |
| L-0111 | 60.6 |
| L-0112 | 64.5 |
| L-011 3 | 74.8 |
| L-0114 | 76.2 |
| L-0115 | 59.2 |
| L-0116 | 73.9 |
| L-01 17 | 72.5 |
| L-0118 | 58.2 |
| L-0119 | 64.4 |
| L-0120 | 68.8 |
| L-0121 | 78.2 |
| L-0122 | 61.4 |
| L-0124 | 74.2 |
| L-0125 | 66-6 |
| L-0126 | 6.0 |
| L-0129 | 77.6 |
| L-0130 | 77.9 |
| L-0131 | 77.4 |
| L-0135 | 79.5 |
| L-0136 | 59.4 |
| L-0137 | 66.6 |
| L-0146 | 74.7 |
| L-0149 | 70.5 |
| L-0150 | 56.4 |
| L-0151 | 69.6 |
| L-0152 | 48.4 |
| L-0154 | 53.6 |
| L-0155 | 50.2 |
| L-0156 | 62.1 |
| L-0157 | 54.3 |
| L-0158 | 48.5 |
| L-0159 | 48.5 |
| L-0160 | 61.4 |

**[Table 24]**

| | |
|---|---|
| L-0161 | 49.3 |
| L-0162 | 70.9 |
| L-0163 | 74.4 |
| L-0164 | 67.3 |
| L-0165 | 61.9 |
| L-0166 | 45.9 |
| L-0167 | 49.5 |
| L-0168 | 58.9 |
| L-0169 | 54.7 |
| L-0170 | 57.7 |
| L-0171 | 61.2 |
| L-0172 | 49.5 |
| L-0173 | 59.0 |
| L-0174 | 56.4 |
| L-0175 | 71-3 |
| L-0176 | 69.9 |
| L-0177 | 72.9 |
| L-0178 | 49.3 |
| L-0179 | 61.2 |
| L-0181 | 75.7 |
| L-0182 | 65.9 |
| L-0183 | 68.0 |
| L-0184 | 60.7 |
| L-0185 | 66.3 |
| L-0186 | 58.9 |
| L-0187 | 69.3 |
| L-0188 | 62.5 |
| L-0189 | 77.2 |
| L-0190 | 65.1 |
| L-0191 | 76.6 |
| L-0192 | 65.8 |
| L-0193 | 71.3 |
| L-0194 | 61.7 |
| L-0195 | 67.9 |
| L-0196 | 68.2 |
| L-0198 | 62.4 |
| L-0199 | 65.8 |
| L-0200 | 68.2 |
| L-0201 | 63.5 |
| L-0202 | 30.4 |
| L-0203 | 52.4 |
| L-0204 | 66.6 |
| L-0205 | 15.1 |
| L-0206 | 5.4 |
| L-0207 | 19.1 |
| L-0208 | 65.8 |
| L-0209 | 69.4 |
| L-0210 | 71.8 |
| L-0211 | 65.6 |
| L-0212 | 54.9 |
| L-0213 | 3.5 |
| L-0214 | 13.3 |
| L-0215 | 60.7 |
| L-0216 | 44.3 |
| L-0217 | 26.8 |
| L-0218 | 11.5 |
| L-0219 | 7.9 |
| L-0220 | 8.7 |
| L-0221 | 7.0 |
| L-0222 | 29.9 |
| L-0223 | 37.1 |
| L-0224 | 8.2 |
| L-0225 | 4.9 |
| L-0226 | 27.8 |
| L-0227 | 19.9 |
| L-0228 | 13.7 |
| L-0229 | 42.3 |
| L-0230 | 11.5 |
| L-0231 | 32.8 |
| L-0232 | 20.6 |
| L-0233 | 56.5 |
| L-0234 | 57.8 |
| L-0236 | 70.5 |
| L-0237 | 56.9 |
| L-0238 | 47.6 |
| L-0239 | 55.4 |

### [Evaluation Example 2] Dose-dependent antisense inhibition of human ATN1 in SH-SY5Y cells

The antisense oligonucleotide that exhibited inhibition of ATN1 in SH-SY5Y cells in Evaluation Example 1 was tested at various doses. After approximately 24 hours, antisense oligonucleotides at concentrations of 0.003 nM, 0.03 nM, 0.3 nM, 3 nM, 30 nM, and 300 nM were each transfected into SH-SY5Y cells seeded in a 96 well plate at a density of 12,000 cells/well (ASO was added such that final concentrations thereof were the above concentrations, respectively) using Lipofectamin (registered trademark) RNAiMAX Transfection Reagent (Thermo Fisher Scientific). After approximately 48 hours, RNA was isolated from the cells using RNeasy Micro Kit (QIAGEN), and then reverse-transcribed with High-Capacity cDNA Reverse Transcription Kit (Thermo Fisher Scientific) to create cDNA. Using the created cDNA, an ATN1 gene expression level was measured by quantitative real-time PCR with TaqMan (registered trademark) Gene Expression Assays (Thermo Fisher Scientific). Results thereof were calculated as a concentration (IC₅₀ value) at which an RNA transcript level was 50% relative to untreated control cells and are presented in Table 25.

**[Table 25]**

| Cmpd No | IC₅₀ (nM) |
|---|---|
| M-0176 | 10.3 |
| M-0177 | 13.7 |
| M-0178 | 25.8 |
| M-0182 | 6.5 |
| M-0183 | 1.7 |
| M-0184 | 0.5 |
| M-0191 | 9.3 |
| M-0194 | 13.3 |
| M-0199 | 37.7 |
| M-0200 | 8.3 |
| M-0201 | 36.8 |
| M-0202 | 11.6 |
| M-0204 | 46.0 |
| M-0209 | 15.3 |
| M-0210 | 6.7 |
| M-0211 | 3.7 |
| M-0212 | 1.3 |
| M-0213 | 1.7 |
| M-0214 | 3.7 |
| M-0219 | 7.0 |
| M-0220 | 4.9 |
| M-0221 | 5.8 |
| M-0222 | 3.1 |
| M-0223 | 2.6 |
| M-0224 | 4.2 |
| M-0225 | 9.6 |
| M-0226 | 7.3 |
| L-0126 | 8.7 |
| L-0202 | 50.5 |
| L-0205 | 3.4 |
| L-0206 | 1.4 |
| L-0207 | 26.6 |
| L-0213 | 0.7 |
| L-0214 | 5.2 |
| L-0217 | 7.9 |
| L-0218 | 1.2 |
| L-0219 | 0.7 |
| L-0220 | 0.7 |
| L-0221 | 3.1 |
| L-0224 | 19.4 |
| L-0225 | 4.6 |
| L-0226 | 28.5 |
| L-0227 | 12.8 |
| L-0228 | 9.1 |
| L-0230 | 3.9 |
| L-0231 | 4.1 |
| L-0232 | 2.3 |

### (Manufacturing Example 2)

Antisense oligonucleotides (compounds represented by chemical structures corresponding to compound numbers) described in Tables 26 to 33 were prepared using an automatic nucleic acid synthesizer nS-8II (manufactured by GeneDesign Inc.). Note that the title rows in Tables 28 to 33 are the same as that in Table 27, and thus are omitted.

**[Table 26]**

| Cmpd No | Chemical structure |
|---|---|
| M-0248 | A(V)^A(V)^T(V)^G(V)^A(V)^g^a^g^g^g^t^g^5(x)^a^5(x)^5(V)^G(V)^A(V)^G(V)^G(V) |
| M-0249 | G(V)^G(V)^G(V)^T(V)^5(V)^a^a^t^g^a^g^a^g^g^g^T(V)^G(V)^5(V)^A(V)^5(V) |
| M-0250 | 5(V)^5(V)^T(V)^G(V)^A(V)^g^g^5(x)^5(x)^a^g^g^g^g^g^T(V)^5(V)^A(V)^A(V)^T(V) |
| M-0251 | G(V)^G(V)^T(V)^G(V)^A(V)^g^a^5(x)^5(x)^5(x)^t^g^a^g^g^5(V)^5(V)^A(V)^G(V)^G(V) |
| M-0252 | A(V)^G(V)^G(V)^T(V)^G(V)^a^g^a^5(x)^5(x)^5(x)^t^g^a^g^G(V)^5(V)^5(V)^A(V)^G(V) |
| M-0253 | A(V)^A(V)^G(V)^G(V)^T(V)^g^a^g^a^5(x)^5(x)^5(x)^t^g^a^G(V)^G(V)^5(V)^5(V)^A(V) |
| M-0254 | G(V)^A(V)^T(V)^5(V)^5(V)^g^g^g^t^a^a^g^g^t^g^A(V)^G(V)^A(V)^5(V)^5(V) |
| M-0255 | G(V)^G(V)^A(V)^T(V)^5(V)^5(x)^g^g^g^t^a^a^g^g^t^G(V)^A(V)^G(V)^A(V)^5(V) |
| M-0256 | G(V)^G(V)^G(V)^A(V)^T(V)^5(x)^5(x)^g^g^g^t^a^a^g^g^T(V)^G(V)^A(V)^G(V)^A(V) |
| M-0257 | G(V)^G(V)^G(V)^G(V)^A(V)^t^5(x)^5(x)^g^g^g^t^a^a^g^G(V)^T(V)^G(V)^A(V)^G(V) |
| M-0258 | A(V)^G(V)^G(V)^G(V)^G(V)^a^t^5(x)^5(x)^g^g^g^t^a^a^G(V)^G(V)^T(V)^G(V)^A(V) |
| M-0259 | 5(V)^T(V)^G(V)^G(V)^G(V)^t^a^g^g^g^g^a^t^5(x)^5(x)^G(V)^G(V)^G(V)^T(V)^A(V) |
| M-0260 | A(V)^G(V)^5(V)^T(V)^G(V)^g^g^t^a^g^g^g^g^a^t^5(V)^5(V)^G(V)^G(V)^G(V) |
| M-0261 | G(V)^T(V)^T(V)^5(V)^5(V)^a^g^5(x)^t^g^g^g^t^a^g^G(V)^G(V)^G(V)^A(V)^T(V) |

**[Table 27]**

| Cmpd No | Chemical structure |
|---|---|
| L -0240 | G(V)^T(V)^5(L)^a^a^t^g^a^g^a^g^g^g^T(L)^G(L)^5(V) |
| L-0241 | G(V)^T(L)^5(L)^a^a^t^g^a^g^a^g^g^g^T(L)^G(V)^5(V) |
| L -0242 | G(L)^T(V)^5(L)^a^a^t^g^a^g^a^g^g^g^T(L)^G(V)^5(L) |
| L-0243 | G(V)^G(L)^T(V)^5(L)^a^a⁻t^g^a^g^a^g^g^g^T(L)^G(V)^5(L)^A(V) |
| L-0244 | G(V)^G(V)^T(L)^5(x)^a^a^t^g^a^g^a^g^g^G(L)^T(L)^G(V) |
| L-0245 | G(V)^G(L)^T(L)^5(x)^a^a^t^g'a^g^a^g^g^G(L)^T(V)^G(V) |
| L-0246 | G(L)^G(V)^T(L)^5(x)^a^a^t^g^a^g^a^g^g^G(L)^T(V)^G(L) |
| L-0247 | G(V)^G(L)^G(V)^T(L)^5(x)^a^a^t^g^a^g^a^g^g^G(L)^T(V)^G(L)^5(V) |
| L-0248 | G(V)^G(V)^G(L)^g^t^5(x)^a^a^t^g^a^g^a^G(L)^G(L)^G(V) |
| L-0249 | G(V)^G(L)^G(L)^a^t^5(x)^a^a^t^g^a^g^a^G(L)^G(V)^G(V) |
| L -0250 | G(L)^G(V)^G(L)^g^t^5(x)^a^a^t^g^a^g^a^G(L)^G(V)^G(L) |
| L-0251 | G(V)^G(L)^G(V)^G(L)^g^t^5(x)^a^a^t^g^a^g^a^G(L)^G(V)^G(L)^T(V) |
| L-0252 | T(V)^A(V)^G(L)^g^g^g^a^t^5(x)^5(x)^g^g^g^T(L)^A(L)^A(V) |
| L -0253 | T(V)^A(L)^G(L)^g^g^k^a^t^5(x)^5(x)^g^g^g^T(L)^A(V)^A(V) |
| L -0254 | T(L)^A(V)^G(L)^g^g^g^a^t^5(x)^5(x)^g^g^g^T(L)^A(V)^A(L) |
| L-0255 | G(V)^T(L)^A(V)^G(L)^g^g^g^a^t^5(x)^5(x)^g^g^g^T(L)^A(V)^A(L)^G(V) |
| L-0256 | A(V)^G(V)^5(L)^t^g^g^g^t^a^g^g^g^g^A(L)^T(L)^5(V) |
| L-0257 | A(V)^G(L)^5(L)^t^g^g^g^t^a^g^g^g^g^g^A(L)^T(V)^5(V) |
| L-0258 | A(L)^G(V)^5(L)^t^g^g^g^t^a^g^g^g^g^A(L)^T(V)^5(L) |
| L-0259 | 5(V)^A(L)^G(V)^5(L)^t^g^g^g^t^a^g^g^g^g^A(L)^T(V)^5(L)^5(V) |
| L-0260 | 5(V)^T(L)^5(L)^5(x)^t^5(x)^t^t^t^t^g^a^g^T(L)^5(L)^T(V) |
| L-0261 | 5(V)^T(L)^5(L)^g^a^t^g^t^t^5(x)^5(x)^t^5(x)^5(L)^T(L)^5(V) |
| L-0262 | A(V)^5(L)^5(L)^5(x)^5(x)^a^5(x)^t^5(x)^a^5(x)^a^g^5(L)^A(L)^T(V) |
| L-0263 | 5(V)^T(L)^A(L)^t^5(x)^5(x)^a^t^t^5(x)^t^g^5(x)^5(L)^T(L)^T(V) |
| L-0264 | T(V)^5(L)^T(L)^5(x)^t^5(x)^5(x)^t^a^t^5(x)^5(x)^a^T(L)^T(L)^5(V) |
| L-0265 | T(V)^T(L)^5(L)^5(x)^t^a^a^a^5(x)^5(x)^5(x)^5(x)^5(L)^A(L)^A(V) |
| L-0266 | 5(V)^G(L)^T(L)^5(x)^t^a^t^t^5(x)^5(x)^t^a^a^A(L)^G(L)^5(V) |
| L-0267 | T(V)^T(L)^5(L)^5(x)^a^5(x)^5(x)^t^a^5(x)^5(x)^a^5(x)^A(L)^5(L)^5(V) |
| L -0268 | G(V)^5(L)^5(L)^a^5(x)^a^a^t^5(x)^5(x)^5(x)^t^5(x)^T(L)^T(L)^T(V) |
| L-0269 | A(V)^5(L)^5(L)^t^5(x)^t^5(x)^t^t^t^g^5(x)^5(x)^5(L)^A(L)^A(V) |
| L-0270 | 5(V)^A(L)^A(L)^a^a^a^t^g^g^a^5(x)^5(x)^t^5(L)^T(L)^5(V) |
| L-0271 | A(V)^5(L)^G(L)^5(x)^t^g^g^a^a^a^a^5(x)^5(x)^T(L)^5(L)^5(V) |
| L-0272 | A(V)^5(L)^5(L)^g^t^5(x)^a^5(x)^a^a^a^g^a^5(L)^A(L)^T(V) |
| L -0273 | 5(V)^T(L)^5(L)^t^5(x)^5(x)^a^a^g^a^a^a^t^A(L)^A(L)^5(V) |
| L -0274 | T(V)^5(L)^5(L)^t^5(x)^5(x)^t^5(x)^a^5(x)^t^a^g^T(L)^T(L)^T(V) |
| L-0275 | G(V)^T(L)^T(L)^5(x)^5(x)^t^g^t^t^t^t^5(x)^5(x)^T(L)^5(L)^5(V) |
| L -0276 | 5(V)'5(L)^5(L)^t^t^5(x)^t^g^t^g^t^t^5(x)^5(L)T(L)^G(V) |
| L-0277 | T(V)^5(L)^5(L)^5(x)^a^g^a^a^5(x)^5(x)^5(x)^5(x)^t^T(L)^T(L)^5(V) |
| L-0278 | T(V)^5(L)^T(L)^5(x)^5(x)^a^t^t^t^5(x)^5(x)^a^t^5(L)^5(L)^T(V) |
| L-0279 | G(V)^T(L)^T(L)^g^5(x)^t^5(x)^a^a^t^t^5(x)^t^5(L)^5(L)^A(V) |
| L-0280 | T(V)^A(L)^T(L)^a^g^a^t^5(x)^a^5(x)^a^g^5(x)^T(L)^5(L)^5(V) |
| L-0281 | 5(V)^T(L)^5(L)^5(x)^5(x)^t^a^t^a^t^a^g^a^T(L)^5(L)^A(V) |
| L-0282 | G(V)^T(L)^A(L)^a^5(x)^t^a^a^g^g^5(x)^5(x)^t^5(L)^5(L)^5(V) |
| L -0283 | T(V)^A(L)^A(L)^g^5(x)^5(x)^5(x)^a^t^g^5(x)^a^5(x)^A(L)^A(L)^A(V) |
| L-0284 | 5(V)^A(L)^A(L)^a^a^t^a^a^g^5(x)^5(x)^5(x)^a^T(L)^G(L)^5(V) |
| L-0285 | 5(V)^5(L)^T(L)^a^5(x)^5(x)^5(x)^t^a^g^t^5(x)^a^A(L)^A(L)^A(V) |
| L-0286 | A(V)^5(L)^5(L)^5(x)^t^5(x)^5(x)^t^a^t^a^g^5(x)^A(L)^5(L)^5(V) |
| L-0287 | A(V)^G(L)^5(L)^a^a^5(x)^t^5(x)^5(x)^t^a^5(x)^5(x)^5(L)^T(L)^5(V) |
| L-0288 | 5(V)^5(L)^5(L)^a^g^t^t^g^5(x)^5(x)^a^a^a^G(L)^5(L)^A(V) |
| L-0289 | T(V)^5(L)^A(L)^a^g^a^g^a^5(x)^5(x)^a^t^5(x)^5(L)^5(L)^A(V) |

**[Table 28]**

| | |
|---|---|
| L-0290 | A(V)^5(L)^5(L)^a^t^a^g^5(x)^a^a^5(x)^a^5(x)^A(L)^G(L)^T(V) |
| L-0291 | A(V)^T(L)^5(L)^5(x)^5(x)^a^5(x)^t^g^g^a^t^5(x)^A(L)^5(L)^T(V) |
| L-0292 | A(V)^T(L)^5(L)^t^g^a^5(x)^a^t^5(x)^5(x)^5(x)^a^5(L)^T(L)^G(V) |
| L-0293 | 5(V)^5(L)^5(L)^a^a^5(x)^5(x)^a^a^t^5(x)^t^g^A(L)^5(L)^A(V) |
| L-0294 | 5(V)^5(L)^A(L)^a^t^a^a^a^5(x)^a^t^t^5(x)^G(L)^A(L)^A(V) |
| L-0295 | A(V)^5(L)^5(L)^t^5(x)^5^(x)^a^a^a^a^5(x)^5(x)^5(x)^A(L)^G(L)^5(V) |
| L-0296 | T(V)^5(L)^5(L)^a^g^a^a^a^5(x)^a^a^5(x)^5(x)^T(L)^5(L)^5(V) |
| L-0297 | 5(V)^5(L)^A(L)^5(x)^5(x)^a^a^5(x)^a^g^a^t^5(x)^5(L)^A(L)^G(V) |
| L-0298 | A(V)^A(L)^5(L)^t^5(x)^5(x)^5(x)^a^a^5(x)^5(x)^a^5(x)^5(L)^A(L)^A(V) |
| L-0299 | 5(V)^5(L)^A(L)^a^5(x)^a^g^a^5(x)^t^t^5(x)^a^A(L)^5(L)^A(V) |
| L-0300 | A(V)^5(L)^G(L)^t^a^t^5(x)^t^a^5(x)^5(x)^t^g^T(L)^T(L)^5(V) |
| L -0301 | A(V)^5(L)^A(L)^5(x)^5(x)^t^a^t^g^a^t^5(x)^t^G(L)^5(L)^A(V) |
| L-0302 | 5(V)^G(L)^T(L)^t^t^t^5(x)^a^t^5(x)^5(x)^t^5(x)^5(L)^5(L)^A(V) |
| L-0303 | 5(V)^A(L)^G(L)^a^a^5(x)^5(x)^a^a^5(x)^t^g^5(x)^5(L)^A(L)^A(V) |
| L-0304 | 5(V)^A(L)^A(L)^a^a^a^g^a^5(x)^a^a^5(x)^t^5(L)^T(L)^A(V) |
| L-0305 | 5(V)^5(L)^5(L)^a^g^5(x)^5(x)^a^t^t^t^5(x)^t^A(L)^5(L)^A(V) |
| L-0306 | A(V)^G(L)^5(L)^t^g^t^t^t^5(x)^a^5(x)^a^g^5(L)^5(L)^5(V) |
| L-0307 | 5(V)^T(L)^A(L)^t^a^g^t^a^t^t^t^5(x)^5(x)^5(L)^5(L)^T(V) |
| L-0308 | 5(V)^T(L)^A(L)^g^a^5(x)^a^5(x)^a^g^g^t^5(x)^T(L)^5(L)^5(V) |
| L-0309 | 5(V)^T(L)^T(L)^a^a^5(x)^a^a^t^g^a^a^g^5(L)^5(L)^5(V) |
| L-0310 | 5(V)^T(L)^5(L)^a^t^5(x)^t^a^t^a^g^5(x)^5(x)^5(L)^5(L)^T(V) |
| L-0311 | G(V)^A(L)^T(L)^5(x)^a^t^5(x)^t^5(x)^a^t^5(x)^t^A(L)^T(L)^A(V) |
| L -0312 | 5(V)^A(L)^5(L)^t^g^g^5(x)^5(x)^t^a^a^g^5(x)^5(L)^T(L)^T(V) |
| L -0313 | A(V)^5(L)^T(L)^5(x)^5(x)^t^a^g^a^t^a^a^5(x)^A(L)^A(L)^T(V) |
| L-0314 | T(V)^5(L)^5(L)^a^a^a^g^t^g^a^5(x)^t^5(x)^5(L)^T(L)^A(V) |
| L-0315 | 5(V)^T(L)^A(L)^a^5(x)^a^a^a^5(x)^5(x)^a^g^t^5(L)^5(L)^A(V) |
| L -0316 | 5(V)^5(L)^5(L)^5(x)^5(x)^a^a^5(x)^a^t^5(x)^a^t^T(L)^5(L)^T(V) |
| L-0317 | 5(V)^5(L)^5(L)^5(x)^t^a^a^g^t^a^a^g^a^A(L)^5(L)^A(V) |
| L-0318 | G(V)^5(L)^5(L)^a^t^t^a^5(x)^5(x)^t^t^a^5(x)^T(L)^T(L)^T(V) |
| L-0319 | 5(V)^T(L)^T(L)^5(x)^a^t^t^a^5(x)^5(x)^t^t^g^5(L)^T(L)^T(V) |
| L-0320 | G(V)^5(L)^A(L)^5(x)^a^t^t^5(x)^a^5(x)^5(x)^t^t^5(L)^A(L)^T(V) |
| L-0321 | G(V)^5(L)^A(L)^5(x)^5(x)^t^a^5(x)^t^5(x)^t^g^t^A(L)^T(L)^5(V) |
| L-0322 | 5(V)^A(L)^A(L)^t^5(x)^a^5(x)^t^g^t^a^a^5(x)^T(L)^A(L)^A(V) |
| L-0323 | 5(V)^A(L)^5(L)^t^5(x)^5(x)^t^a^g^5(x)^a^g^g^5(L)^A(L)^A(V) |
| L-0324 | 5(V)^A(L)^A(L)^t^5(x)^a^t^a^g^t^a^t^g^5(L)^5(L)^5(V) |
| L-0325 | 5(V)^5(L)^T(L)^g^t^t^5(x)^a^5(x)^5(x)^a^a^g^T(L)^5(L)^A(V) |
| L-0326 | T(V)^T(L)^5(L)^5(x)^5(x)^5(x)^a^t^5(x)^a^5(x)^a^g^A(L)^A(L)^5(V) |
| L-0327 | A(V)^5(L)^5(L)^5(x)^a^5(x)^5(x)^a^t^a^g^5(x)^a^A(L)^A(L)^A(V) |
| L-0328 | A(V)^G(L)^A(L)^a^5(x)^a^a^5(x)^a^5(x)^5(x)^5(x)^a^5(L)^5(L)^A(V) |
| L-0329 | T(V)^5(L)^5(L)^g^a^a^g^a^a^5(x)^a^a^5(x)^A(L)^5(L)^5(V) |
| L-0330 | 5(V)^5(L)^5(L)^a^5(x)^a^t^t^t^t^t^t^a^G(L)^T(L)^T(V) |
| L-0331 | 5(V)^A(L)^A(L)^5(x)^t^a^5(x)^a^g^5(x)^t^a^t^T(L)^T(L)^T(V) |
| L-0332 | T(V)^5(L)^T(L)^t^t^g^5(x)^t^5(x)^t^t^g^t^T(L)^5(L)^5(V) |
| L-0333 | T(V)^G(L)^A(L)^t^5(x)^t^5(x)^a^5(x)^t^5(x)^a^5(x)^T(L)^G(L)^5(V) |
| L-0334 | A(V)^A(L)^5(L)^t^g^a^t^g^5(x)^t^5(x)^t^g^5(L)^5(L)^5(V) |
| L-0335 | T(V)^T(L)^5(L)^t^g^a^a^5(x)^t^t^t^5(x)^a^A(L)^5(L)^T(V) |
| L-0336 | 5(V)^T(L)^T(L)^5(x)^a^5(x)^a^a^t^t^t^a^5(x)^T(L)^T(L)^A(V) |
| L-0337 | 5(V)^5(L)^A(L)^g^g^a^a^a^5(x)^t^t^5(x)^a^5(L)^A(L)^A(V) |
| L-0338 | A(V)^G(L)^T(L)^5(x)^t^a^a^5(x)^5(x)^t^t^t^5(x)^5(L)^A(L)^G(V) |
| L-0339 | T(V)^T(L)^5(L)^5(x)^5(x)^a^a^g^a^a^a^5(x)^a^5(L)^A(L)^G(V) |

**[Table 29]**

| | |
|---|---|
| L-0340 | A(V)^5(L)^5(L)^5(x)^a^t^g^t^a^t^t^5(x)^t^A(L)^T(L)^T(V) |
| L-0341 | A(V)^5(L)^T(L)^a^a^g^5(x)^t^t^a^5(x)^5(x)^5(x)^A(L)^T(L)^G(V) |
| L-0342 | A(V)^T(L)^T(L)^g^a^5(x)^5(x)^t^t^t^a^a^a^5(L)^5(L)^5(V) |
| L -0343 | 5(V)^T(L)^T(L)^a^5(x)^t^g^t^t^t^t^5(x)^t^T(L)^5(L)^5(V) |
| L-0344 | 5(V)^5(L)^T(L)^5(x)^5(x)^a^a^a^t^t^a^5(x)^t^G(L)^A(L)^T(V) |
| L-0345 | A(V)^G(L)^T(L)^g^t^5(x)^a^a^g^t^5(x)^5(x)^5(x)^T(L)^5(L)^5(V) |
| L -0346 | 5(V)^T(L)^T(L)^a^t^5(x)^5(x)^a^a^a^t^5(x)^5(L)^5(L)^T(V) |
| L-0347 | G(V)^T(L)^A(L)^t^5(x)^t^a^t^5(x)^t^t^t^a^T(L)^5(L)^5(V) |
| L -0348 | 5(V)^5(L)^G(L)^t^a^t^5(x)^t^g^t^5(x)^t^a^T(L)^5(L)^A(V) |
| L-0349 | A(V)^G(L)^5(L)^5(x)^a^a^t^5(x)^5(x)^a^t^5(x)^5(x)^G(L)^T(L)^A(V) |
| L-0350 | A(V)^5(L)^A(L)^5(x)^a^g^t^t^t^5(x)^t^5(x)^a^5(L)^A(L)^G(V) |
| L-0351 | T(V)^A(L)^A(L)^g^5(x)^5(x)^t^a^t^5(x)^5(x)^t^5(x)^5(L)^T(L)^A(V) |
| L-0352 | 5(V)^A(L)^A(L)^5(x)^a^5(x)^a^g^a^t^g^t^a^5(L)^5(L)^A(V) |
| L-0353 | A(V)^A(L)^G(L)^a^5(x)^t^5(x)^a^a^a^a^t^a^T(L)^5(L)^5(V) |
| L-0354 | 5(V)^5(L)^A(L)^t^t^5(x)^t^5(x)^t^a^g^t^a^5(L)^T(L)^T(V) |
| L-0355 | 5(V)^5(L)^5(L)^5(x)^a^a^a^t^t^g^t^a^g^A(L)^A(L)^5(V) |
| L-0356 | 5(V)^5(L)^T(L)^a^g^g^t^a^g^5(x)^5(x)^5(x)^5(x)^A(L)^A(L)^A(V) |
| L-0357 | 5(V)^A(L)^5(L)^a^g^5(x)^5(x)^5(x)^t^a^5(x)^5(x)^a^A(L)^T(L)^5(V) |
| L-0358 | 5(V)^5(L)^A(L)^t^t^5(x)^t^a^t^a^a^t^a^5(L)^A(L)^A(V) |
| L-0359 | T(V)^5(L)^T(L)^t^t^t^5(x)^t^5(x)^a^5(x)^a^t^5(L)^A(L)^G(V) |
| L-0360 | T(V)^G(L)^5(L)^t^g^a^t^t^5(x)^t^5(x)^5(x)^T(L)^5(L)^A(V) |
| L-0361 | T(V)^T(L)^5(L)^t^t^5(x)^t^a^t^a^5(x)^t^t^T(L)^G(L)^5(V) |
| L-0362 | A(V)^A(L)^T(L)^5(x)^a^a^a^5(x)^5(x)^5(x)^a^t^g^T(L)^5(L)^G(V) |
| L-0363 | T(V)^G(L)^T(L)^g^a^5(x)^t^a^g^5(x)^5(x)^5(x)^a^A(L)^5(L)^5(V) |
| L-0364 | T(V)^A(L)^5(L)^5(x)^5(x)^t^5(x)^a^t^t^a^t^a^T(L)^A(L)^G(V) |
| L-0365 | T(V)^A(L)^5(L)^a^a^5(x)^5(x)^a^a^5(x)^5(x)^5(x)^t^G(L)^A(L)^A(V) |
| L-0366 | T(V)^5(L)^A(L)^5(x)^a^t^a^5(x)^a^t^g^a^t^T(L)^T(L)^5(V) |
| L-0367 | T(V)^A(L)^5(L)^5(x)^t^t^5(x)^t^t^t^a^t^a^T(L)^5(L)^A(V) |
| L-0368 | A(V)^5(L)^A(L)^5(x)^a^5(x)^a^t^t^5(x)^5(x)^a^5(x)^G(L)^G(L)^A(V) |
| L-0369 | 5(V)^T(L)^T(L)^5(x)^a^t^g^a^t^5(x)^5(x)^5(x)^t^A(L)^A(L)^A(V) |
| L-0370 | 5(V)^A(L)^5(L)^a^t^t^t^a^5(x)^5(x)^5(x)^t^T(L)^5(L)^A(V) |
| L-0371 | A(V)^T(L)^G(L)^t^a^5(x)^5(x)^t^a^5(x)^a^a^g^5^(L)^5(L)^5(V) |
| L-0372 | T(V)^A(L)^T(L)^a^a^t^5(x)^t^5(x)^t^t^t^5(x)^A(L)^T(L)^5(V) |
| L-0373 | 5(V)^5(L)^T(L)^t^a^g^5(x)^t^a^t^5(x)^t^a^A(L)^A(L)^A(V) |
| L-0374 | 5(V)^A(L)^G(L)^a^t^a^5(x)^5(x)^a^5(x)^a^a^g^A(L)^5(L)^A(V) |
| L-0375 | 5(V)^A(L)^A(L)^5(x)^5(x)^5(x)^t^a^a^g^5(x)^5(x)^t^T(L)^T(L)^G(V) |
| L-0376 | 5(V)^A(L)^T(L)^5(x)^a^g^a^g^t^5(x)^t^5(x)^5(x)^A(L)^A(L)^5(V) |
| L-0377 | 5(V)^T(L)^5(L)^a^t^t^t^t^a^a^5(x)^t^5(x)^A(L)^5(L)^5(V) |
| L-0378 | T(V)^5(L)^T(L)^t^t^5(x)^a^t^g^t^5(x)^t^5(x)^T(L)^5(L)^A(V) |
| L-0379 | 5(V)^5(L)^T(L)^5(x)^a^t^5(x)^t^t^t^5(x)^a^t^G(L)^T(L)^5(V) |
| L-0380 | T(V)^T(L)^5(L)^5(x)^t^5(x)^5(x)^t^a^g^5(x)^t^t^G(L)^5(L)^5(V) |
| L-0381 | T(V)^T(L)^G(L)^t^t^5(x)^t^t^5(x)^t^t^5(x)^t^5(L)^T(L)^A(V) |
| L-0382 | T(V)^T(L)^G(L)^5(x)^a^5(x)^5(x)^t^a^a^5(x)^t^t^T(L)^A(L)^T(V) |
| L-0383 | T(V)^A(L)^A(L)^5(x)^a^5(x)^t^g^t^t^5(x)^t^t^T(L)^A(L)^5(V) |
| L-0384 | A(V)^A(L)^5(L)^a^g^g^a^a^g^t^t^5(x)^5(x)^5(L)^5(L)^5(V) |
| L-0385 | T(V)^5(L)^5(L)^t^g^t^5(x)^t^t^t^t^5(x)^5(x)^5(L)^T(L)^A(V) |
| L-0386 | T(V)^T(L)^5(L)^t^5(x)^t^t^a^t^g^t^5(x)^5(x)^5(L)^A(L)^G(V) |
| L-0387 | 5(V)^T(L)^5(L)^t^5(x)^a^t^a^a^g^5(x)^a^5(x)^T(L)^A(L)^T(V) |
| L-0388 | G(V)^5(L)^T(L)^5(x)^t^a^a^g^a^a^t^5(x)^t^5(L)^T(L)^5(V) |
| L-0389 | A(V)^T(L)^5(L)^5(x)^5(x)^5(x)^5(x)^a^a^5(x)^t^5(x)^t^T(L)^5(L)^T(V) |

**[Table 30]**

| | |
|---|---|
| L-0390 | G(V)^A(L)^5(L)^t^a^a^g^a^5(x)^t^t^5(x)^t^5(L)^A(L)^5(V) |
| L-0391 | T(V)^T(L)^5(L)^t^g^5(x)^a^t^t^t^t^5(x)^5(x)^T(L)^T(L)^A(V) |
| L-0392 | A(V)^A(L)^G(L)^a^5(x)^5^(x)^a^5(x)^t^t^5(x)^t^g^5(L)^A(L)^T(V) |
| L-0393 | T(V)^T(L)^T(L)^t^a^t^a^5(x)^5(x)^a^5(x)^t^t^5(L)^T(L)^G(V) |
| L-0394 | T(V)^T(L)^T(L)^a^a^5(x)^5(x)^a^t^5(x)^5(x)^t^5(x)^5(L)^A(L)^T(V) |
| L -0395 | 5(V)^A(L)^T(L)^5(x)^t^5(x)^a^t^a^a^5(x)^5(x)^a^5(L)^5(L)^A(V) |
| L-0396 | G(V)^A(L)^5(L)^t^5(x)^5(x)^a^a^5(x)^t^5(x)^5(x)^5(x)^T(L)^G(L)^T(V) |
| L-0397 | 5(V)^A(L)^5(L)^5(x)^t^t^g^t^5(x)^t^5(x)^t^a^5(L)^5(L)^5(V) |
| L -0398 | 5(V)^5(L)^T(L)^5(x)^a^a^g^g^a^t^5(x)^5(x)^a^T(L)^5(L)^T(V) |
| L-0399 | A(V)^A(L)^5(L)^5(x)^5(x)^t^a^t^t^5(x)^5(x)^t^t^5(L)^5(L)^T(V) |
| L -0400 | A(V)^G(L)^T(L)^5(x)^t^t^g^t^t^t^5(x)^5(x)^5(x)^G(L)^5(L)^5(V) |
| L-0401 | T(V)^T(L)^5(L)^5(x)^t^5(x)^a^5(x)^a^t^t^5(x)^t^T(L)^G(L)^G(V) |
| L -0402 | G(V)^A(L)^T(L)^t^t^5(x)^t^5(x)^a^5(x)^t^t^t^5(L)^5(L)^T(V) |
| L-0403 | A(V)^5^(L)^5(L)^t^5(x)^t^t^a^5(x)^5(x)^a^t^5(x)^T(L)^G(L)^G(V) |
| L-0404 | A(V)^A(L)^A(L)^a^t^5(x)^a^t^t^t^5(x)^5(x)^5(x)^5(L)^T(L)^5(V) |
| L-0405 | A(V)^5(L)^A(L)^5(x)^t^a^5(x)^a^5(x)^5(x)^a^g^5(x)^G(L)^5(L)^A(V) |
| L-0406 | 5(V)^T(L)^5(L)^g^5(x)^t^g^a^a^a^a^5(x)^5(x)^T(L)^T(L)^T(V) |
| L-0407 | T(V)^T(L)^T(L)^t^5(x)^5(x)^5(x)^a^a^5(x)^5(x)^5(x)^a^G(L)^G(L)^A(V) |
| L-0408 | 5(V)^5(L)^A(L)^t^5(x)^t^g^a^g^5(x)^a^t^g^5(L)^5(L)^T(V) |
| L-0409 | 5(V)^G(L)^A(L)^a^t^a^t^t^t^g^5(x)^5(x)^5(x)^T(L)^T(L)^5(V) |
| L-0410 | 5(V)^A(L)^5(L)^5(x)^t^g^g^a^a^5(x)^t^t^a^A(L)^5(L)^T(V) |
| L-0411 | T(V)^G(L)^5(L)^5(x)^t^5(x)^a^a^5(x)^t^5(x)^a^t^T(L)^G(L)^A(V) |
| L-0412 | 5(V)^T(L)^T(L)^5(x)^a^a^a^a^g^5(x)^t^5(x)^5(x)^5(L)^G(L)^T(V) |
| L-0413 | A(V)^5(L)^A(L)^5(x)^5(x)^5(x)^a^a^a^5(x)^5(x)^5(x)^a^A(L)^A(L)^G(V) |
| L-0414 | A(V)^T(L)^5(L)^g^5(x)^5(x)^t^a^t^g^a^5(x)^a^5(L)^5(L)^5(V) |
| L-0415 | A(V)^A(L)^A(L)^g^5(x)^t^5(x)^a^t^t^a^t^5(x)^G(L)^5(L)^5(V) |
| L-0416 | 5(V)^A(L)^T(L)^5(x)^5(x)^t^t^a^5(x)^5(x)^a^a^a^G(L)^A(L)^G(V) |
| L-0417 | 5(V)^5(L)^5(L)^a^a^a^t^a^5(x)^5(x)^t^5(x)^5(x)^T(L)^T(L)^T(V) |
| L-0418 | A(V)^G(L)^5(L)^t^5(x)^t^5(x)^5(x)^t^a^t^t^5(x)^5(L)^5(L)^A(V) |
| L-0419 | 5(V)^T(L)^G(L)^a^5(x)^a^5(x)^t^a^a^a^a^5(x)^T(L)^5(L)^G(V) |
| L-0420 | A(V)^5(L)^A(L)^5(x)^5(x)^5(x)^a^g^t^t^5(x)^t^g^T(L)^A(L)^A(V) |
| L-0421 | G(V)^5(L)^T(L)^5(x)^5(x)^t^t^t^a^5(x)^a^t^a^5(L)^A(L)^A(V) |
| L-0422 | T(V)^A(L)^A(L)^5(x)^a^a^g^t^a^5(x)^5(x)^t^g^5(L)^T(L)^5(V) |
| L-0423 | T(V)^5(L)^5(L)^a^a^a^g^t^g^g^a^a^5(x)^A(L)^A(L)^A(V) |
| L-0424 | 5(V)^A(L)^A(L)^a^5(x)^a^5(x)^a^a^g^g^a^g^G(L)^T(L)^5(V) |
| L-0425 | A(V)^G(L)^T(L)^5(x)^a^g^a^5(x)^a^a^a^5(x)^a^5(L)^A(L)^A(V) |
| L-0426 | A(V)^5(L)^T(L)^g^a^g^t^5(x)^a^g^a^5(x)^a^A(L)^A(L)^5(V) |
| L-0427 | G(V)^5(L)^A(L)^a^a^5(x)^5(x)^a^t^g^5(x)^a^t^T(L)^T(L)^G(V) |
| L-0428 | G(V)^G(L)^G(L)^5(x)^a^a^a^5(x)^5(x)^a^t^g^5(x)^A(L)^T(L)^T(V) |
| L-0429 | G(V)^5(V)^A(L)^g^a^g^5(x)^t^a^5(x)^t^a^5(x)^T(L)^A(L)^G(V) |
| L-0430 | G(V)^5(L)^A(L)^g^a^g^5(x)^t^a^5(x)^t^a^5(x)^T(L)^A(V)^G(V) |
| L-0431 | G(L)^5(V)^A(L)^g^a^g^5(x)^t^a^5(x)^t^a^5(x)^T(L)^A(V)^G(L) |
| L -0432 | T(V)^G(L)^5(V)^A(L)^g^a^g^5(x)^t^a^5(x)^t^a^5(x)^T(L)^A(V)^G(L)^A(V) |
| L -0433 | 5(V)^5(V)^G(L)^g^g^5(x)^a^t^a^t^t^5(x)^a^5(L)^T(L)^G(V) |
| L-0434 | 5(V)^5(L)^G(L)^g^g^5(x)^a^t^a^t^t^5(x)^a^5(L)^T(V)^G(V) |
| L-0435 | 5(L)^5(V)^G(L)^g^g^5(x)^a^t^a^t^t^5(x)^a^5(L)^T(V)^G(L) |
| L-0436 | G(V)^5(L)^5(V)^G(L)^g^g^5(x)^a^t^a^t^t^5(x)^a^5(L)^T(V)^G(L)^A(V) |
| L-0437 | G(V)^G(V)^G(L)^t^t^a^g^g^g^a^g^a^g^T(L)^T(L)^5(V) |
| L-0438 | G(V)^G(L)^G(L)^t^t^a^g^g^g^a^g^a^g^T(L)^T(V)^5(V) |
| L-0439 | G(L)^G(V)^G(L)^t^t^a^g^g^g^a^g^a^g^T(L)^T(V)^5(L) |

**[Table 31]**

| | |
|---|---|
| L-0440 | G(V)^G(L)^G(V)^G(L)^t^t^a^g^g^g^a^g^a^g^T(L)^T(V)^5(L)^5(V) |
| L-0441 | T(V)^G(V)^A(L)^g^a^g^g^g^t^g^5(x)^a^5(x)^5(L)^G(L)^A(V) |
| L-0442 | T(V)^G(L)^A(L)^g^a^g^g^g^t^g^5(x)^a^5(x)^5(L)^G(V)^A(V) |
| L-0443 | T(L)^G(V)^A(L)^g^a^g^g^g^t^g^5(x)^a^5(x)^5(L)^G(V)^A(L) |
| L-0444 | A(V)^T(L)^G(V)^A(L)^g^a^g^g^g^t^g^5(x)^a^5(x)^5(L)^G(V)^A(L)^G(V) |
| L-0445 | T(V)^5(V)^A(L)^a^t^g^a^g^a^g^g^g^t^G(L)^5(L)^A(V) |
| L-0446 | T(V)^5(L)^A(L)^a^t^g^a^g^a^g^g^g^t^G(L)^5(V)^A(V) |
| L-0447 | T(L)^5(V)^A(L)^a^t^g^a^g^a^g^g^g^t^G(L)^5(V)^A(L) |
| L-0448 | G(V)^T(L)^5(V)^A(L)^a^t^g^a^g^a^g^g^g^t^G(L)^5(V)^A(L)^5(V) |
| L -0449 | G(V)^G(V)^G(L)^g^g^t^5(x)^a^a^t^g^a^g^A(L)^G(L)^G(V) |
| L-0450 | G(V)^G(L)^G(L)^g^g^t^5(x)^a^a^t^g^a^g^A(L)^G(V)^G(V) |
| L-0451 | G(L)^G(V)^G(L)^g^g^t^5(x)^a^a^t^g^a^g^A(L)^G(V)^G(L) |
| L-0452 | A(V)^G(L)^G(V)^(L)^g^g^t^5(x)^a^a^t^g^a^g^A(L)^G(V)^G(L)^G(V) |
| L-0453 | A(V)^G(V)^G(L)^g^g^g^t^5(x)^a^a^t^g^a^G(L)^A(L)^G(V) |
| L-0454 | A(V)^G(L)^G(L)^g^g^g^t^5(x)^a^a^t^g^a^G(L)^A(V)^G(V) |
| L-0455 | A(L)^G(V)^G(L)^g^g^g^t^5(x)^a^a^t^g^a^G(L)^A(V)^G(L) |
| L-0456 | 5(V)^A(L)^G(V)^G(L)^g^g^g^t^5(x)^a^a^t^g^a^G(L)^A(V)^G(L)^G(V) |
| L-0457 | 5(V)^A(V)^G(L)^g^g^g^g^t^5(x)^a^a^t^g^A(L)^G(L)^A(V) |
| L-0458 | 5(V)^A(L)^G(L)^g^g^g^g^t^5(x)^a^a^t^g^A(L)^G(V)^A(V) |
| L-0459 | 5(L)^A(V)^G(L)^g^g^g^g^t^5(x)^a^a^t^g^A(L)^G(V)^A(L) |
| L-0460 | 5(V)^5(L)^A(V)^(L)^g^g^g^g^t^5(x)^a^a^t^g^A(L)^G(V)^A(L)^G(V) |
| L -0461 | 5(V)^5(V)^A(L)^g^g^g^g^g^t^5(x)^a^a^t^G(L)^A(L)^G(V) |
| L-0462 | 5(V)^5(L)^A(L)^g^g^g^g^g^t^5(x)^a^a^t^G(L)^A(V)^G(V) |
| L-0463 | 5(L)^5(V)^A(L)^g^g^g^g^g^t^5(x)^a^a^t^G(L)^A(V)^G(L) |
| L-0464 | G(V)^5(L)^5(V)^A(L)^g^g^g^g^g^t^5(x)^a^a^t^G(L)^A(V)^G(L)^A(V) |
| L-0465 | G(V)^5(V)^5(L)^a^g^g^g^g^g^t^5(x)^a^a^T(L)^G(L)^A(V) |
| L-0466 | G(V)^5(L)^5(L)^a^g^g^g^g^g^t^5(x)^a^a^T(L)^G(V)^A(V) |
| L-0467 | G(L)^5(V)^5(L)^a^g^g^g^g^g^t^5(x)^a^a^T(L)^G(V)^A(L) |
| L-0468 | G(V)^G(L)^5(V)^5L)^a^g^g^g^g^g^t^5(x)^a^a^T(L)^G(V)^A(L)^G(V) |
| L-0469 | A(V)^G(V)^G(L)^5(x)^5(x)^a^g^g^g^g^g^t^5(x)^A(L)^A(L)^T(V) |
| L-0470 | A(V)^G(L)^G(L)^5(x)^5(x)^a^g^g^g^g^g^t^5(x)^A(L)^A(V)^T(V) |
| L-0471 | A(L)^G(V)^G(L)^5(x)^5(x)^a^g^g^g^g^g^t^5(x)^A(L)^A(V)^T(L) |
| L -0472 | G(V)^A(L)^G(V)^G(L)^5(x)^5x^a^g^g^g^g^g^t^5(x)^A(L)^A(V)^T(L)^G(V) |
| L-0473 | 5(V)^5(V)^G(L)^g^g^t^a^a^g^g^t^g^a^G(L)^A(L)^5(V) |
| L-0474 | 5(V)^5(L)^G(L)^g^g^t^a^a^g^g^t^g^a^G(L)^A(V)^5(V) |
| L-0475 | 5(L)^5(V)^G(L)^g^g^t^a^a^g^g^t^g^a^G(L)^A(V)^5(L) |
| L-0476 | T(V)^5(L)^5(V)^G(L)^g^g^t^a^a^g^g^t^g^a^G(L)^A(V)^5(L)^5(V) |
| L-0477 | G(V)^A(V)^T(L)^5(x)^5(x)^g^g^g^t^a^a^g^g^T(L)^G(L)^A(V) |
| L-0478 | G(V)^A(L)^T(L)^5(x)^5(x)^g^g^g^t^a^a^g^g^T(L)^G(V)^A(V) |
| L-0479 | G(L)^A(V)^T(L)^5(x)^5(x)^g^g^g^t^a^a^g^g^T(L)^G(V)^A(L) |
| L-0480 | G(V)^G(L)^A(V)^T(L)^5(x)^5(x)^g^g^g^t^a^a^g^g^T(L)^G(V)^A(L)^G(V) |
| L-0481 | A(V)^G(V)^G(L)^t^g^a^g^a^5(x)^5(x)^5(x)^t^g^A(L)^G(L)^G(V) |
| L-0482 | A(V)^G(L)^G(L)^t^g^a^g^a^5(x)^5(x)^5(x)^t^g^A(L)^G(V)^G(V) |
| L-0483 | A(L)^G(V)^G(L)^t^g^a^g^a^5(x)^5(x)^5(x)^t^g^A(L)^G(V)^G(L) |
| L-0484 | A(V)^A(L)^G(V)^G(L)^t^g^a^g^a^5(x)^5(x)^5(x)^t^g^A(L)^G(V)^G(L)^5(V) |
| L-0485 | G(V)^T(V)^A(L)^a^g^g^t^g^a^g^a^5(x)^5(x)^5(L)^(L)^G(V) |
| L-0486 | G(V)^T(L)^A(L)^a^g^g^t^g^a^g^a^5(x)^5(x)^5(L)^T(V)^G(V) |
| L-0487 | G(L)^T(V)^A(L)^a^g^g^t^g^a^g^a^5(x)^5(x)^5(L)^T(V)^G(L) |
| L-0488 | G(V)^G(L)^T(V)^A(L)^a^g^g^t^g^a^g^a^5(x)^5(x)^5(L)^T(V)^G(L)^A(V) |
| L-0489 | A(V)^A(V)^G(L)^g^t^g^a^g^a^5(x)^5(x)^5(x)^t^G(L)^A(L)^G(V) |

**[Table 32]**

| | |
|---|---|
| L-0490 | A(V)^A(L)^G(L)^g^t^g^a^g^a^5(x)^5(x)^5(x)^t^G(L)^A(V)^G(V) |
| L-0491 | A(L)^A(V)^G(L)^g^t^g^a^g^a^5(x)^5(x)^5(x)^t^G(L)^A(V)^G(L) |
| L-0492 | T(V)^A(L)^A(V)^(L)^g^t^g^a^g^a^5(x)^5(x)^5(x)^t^G(L)^A(V)^G(L)^G(V) |
| L-0498 | G(V)^5(L)^5(L)^5(x)^t^a^5(x)^5(x)^5(x)^a^t^a^5(x)^A(L)^G(L)^A(V) |
| L-0499 | G(V)^5(L)^5(L)^5(x)^a^5(x)^a^5(x)^t^g^a^a^a^5(L)^T(L)^A(V) |
| L-0500 | 5(V)^T(L)^A(L)^g^5(x)^t^g^t^a^a^5(x)^5(x)^5(x)^A(L)^A(L)^T(V) |
| L-0501 | T(V)^T(L)^5(L)^5(x)^a^a^a^t^5(x)^5(x)^5(x)^t^t^5(L)^5(L)^A(V) |
| L-0502 | 5(V)^A(L)^G(L)^t^t^5(x)^a^g^t^5(x)^t^5(x)^5(x)^5(L)^A(L)^5(V) |
| L-0503 | 5(V)^T(L)^5(L)^a^g^t^t^5(x)^a^g^t^5(x)^t^5(L)^5(L)^5(V) |
| L-0504 | 5(V)^T(L)^G(L)^t^a^5(x)^5(x)^a^5(x)^t^5(x)^a^g^T(L)^T(L)^5(V) |
| L-0505 | 5(V)^5(L)^5(L)^5(x)^a^t^g^g^5(x)^a^t^t^t^5(L)^5(L)^T(V) |
| L-0506 | 5(V)^5(L)^5(L)^5(x)^t^g^a^5(x)^t^t^t^g^t^T(L)^5(L)^5(V) |
| L-0507 | 5(V)^A(L)^5(L)^5(x)^5(x)^5(x)^t^g^a^5(x)^t^t^t^G(L)^T(L)^T(V) |
| L-0508 | T(V)^5(L)^5(L)^a^a^g^5(x)^a^5(x)^a^5(x)^5(x)^t^T(L)^5(L)^5(V) |
| L-0509 | A(V)^5(L)^5(L)^a^5(x)^t^5(x)^5(x)^a^a^g^5(x)^a^5(L)^A(L)^5(V) |
| L-0510 | 5(V)^A(L)^T(L)^5(x)^a^5(x)^t^5(x)^t^g^a^a^T(L)^5(L)^5(V) |
| L-0511 | G(V)^T(L)^5(L)^t^5(x)^a^5(x)^a^a^t^t^5(x)^5(x)^T(L)^T(L)^G(V) |
| L-0512 | 5(V)^A(L)^5(L)^5(x)^a^5(x)^5(x)^a^g^t^t^5(x)^5(x)^T(L)^T(L)^5(V) |
| L-0513 | G(V)^T(L)^T(L)^g^5(x)^t^5(x)^a^a^t^5(x)^5(x)^5(x)^A(L)^5(L)^T(V) |
| L-0514 | 5(V)^A(L)^5(L)^t^a^a^a^g^a^t^5(x)^5(x)^5(x)^5(L)^T(L)^G(V) |
| L-0515 | 5(V)^5^(L)^T(L)^t^5(x)^t^5(x)^a^a^a^t^5(x)^t^G(L)^T(L)^T(V) |
| L-0516 | G(V)^5(L)^5(L)^a^g^a^5(x)^t^a^a^5(x)^a^a^A(L)^5(L)^5(V) |
| L-0517 | A(V)^T(L)^5(L)^5(x)^5(x)^t^a^t^a^g^5(x)^5(x)^a^G(L)^5(L)^T(V) |
| L-0518 | A(V)^G(L)^5(L)^5(x)^5(x)^t^a^5(x)^5(x)^a^a^t^5(x)^5(L)^5(L)^A(V) |
| L-0519 | G(V)^5(L)^5(L)^5(x)^t^t^5(x)^a^5(x)^a^t^a^5(x)^A(L)^T(L)^G(V) |
| L -0520 | G(V)^5(L)^5(L)^5(x)^a^a^5(x)^5(x)^5(x)^t^a^a^g^5(L)^5(L)^T(V) |
| L -0521 | 5(V)^5(L)^A(L)^5(x)^5(x)^5(x)^5(x)^a^a^g^t^t^5(x)^5(L)^5(L)^5(V) |
| L -0522 | T(V)^5(L)^T(L)^5(x)^t^a^g^a^5(x)^5(x)^5(x)^t^t^5(L)^5(L)^T(V) |
| L -0523 | T(V)^T(L)^T(L)^5(x)^5(x)^5(x)^t^a^5(x)^5(x)^t^5(x)^5(x)^5(L)^G(L)^A(V) |
| L-0524 | A(V)^5(L)^T(L)^5(x)^t^5(x)^t^g^a^g^a^t^5(x)^T(L)^5(L)^5(V) |
| L-0525 | A(V)^G(L)^A(L)^5(x)^t^a^g^a^a^t^5(x)^5(x)^5(x)^5(L)^5(L)^A(V) |
| L-0526 | G(V)^T(L)^5(L)^t^5(x)^t^a^5(x)^5(x)^5(x)^a^5(x)^a^G(L)^5(L)^T(V) |
| L-0527 | A(V)^T(L)^5(L)^5(x)^a^t^5(x)^t^5(x)^a^a^g^5(x)^A(L)^5(L)^5(V) |
| L-0528 | 5(V)^5(L)^G(L)^5(x)^5(x)^t^g^t^a^a^a^a^5(x)^5(L)^5(L)^T(V) |
| L-0529 | T(V)^5(L)^T(L)^t^g^5(x)^t^5(x)^a^a^g^5(x)^5(x)^5(L)^A(L)^5(V) |
| L-0530 | 5(V)^5(L)^5(L)^t^g^5(x)^t^a^5(x)^t^5(x)^5(x)^5(x)^T(L)^5(L)^T(V) |
| L-0531 | G(V)^5(L)^5^(L)^t^5(x)^a^a^a^a^5(x)^5(x)^a^5(x)^5(L)^5^(L)^5(V) |
| L-0532 | A(V)^5(L)^5(L)^5(x)^5(x)^a^a^5(x)^t^t^(x)^5(x)^a^T(L)^5(L)^5(V) |
| L-0533 | G(V)^5(L)^T(L)^5(x)^5(x)^t^a^5(x)^5(x)^5(x)^5(x)^a^A(L)^5(L)^T(V) |
| L-0534 | 5(V)^5(L)^5(L)^5(x)^a^5(x)^5(x)^5(x)^a^a^a^t^a^5(L)^5(L)^T(V) |
| L-0535 | G(V)^A(V)^G(L)^a^g^g^g^t^g^5(x)^a^5(x)^5(x)^G(L)^A(L)^G(V) |
| L-0536 | G(V)^A(L)^G(L)^a^g^g^g^t^g^5(x)^a^5(x)^5(x)^G(L)^A(V)^G(V) |
| L-0537 | G(L)^A(V)^G(L)^a^g^g^g^t^g^5(x)^a^5(x)^5(x)^G(L)^A(V)^G(L) |
| L-0538 | T(V)^G(L)^A(V)^G)^G(L)^a^g^g^g^t^g^5(x)^a^5(x)^5(x)^G(L)^A(V)^G(L)^G(V) |
| L-0539 | G(L)^A(L)^G(V)^a^g^g^g^t^g^5(x)^a^5(x)^5(x)^G(V)^A(L)^G(L) |
| L-0540 | G(V)^A(L)G(L)^a^g^g^g^t^g^5(x)^a^5(x)^5(x)^G(L)A(L)^G(V) |
| L-0541 | G(V)^A(L)G(L)a^g^g^g^t^g^5(x)^a^5(x)^5(x)^G(L)A(L)^G(V) |
| L-0542 | G(V)^A(L)G(L)A(V)^g^g^g^t^g^5(x)^a^5(x)^5(x)^G(L)A(L)^G(V) |
| L-0543 | G(V)^A(L)G(L)a^g^g^g^t^g^5(x)^a^5(x)^5(V)G(L)A(L)^G(V) |

**[Table 33]**

| | |
|---|---|
| L-0544 | G(V)^A(L)G(L)^a^gg^g^tg^5(x)^a5(x)^5(x)^G(L)A(L)^G(V) |
| L-0545 | G(V)^G(V)^G(L)^t^5(x)^a^a^t^g^a^g^a^g^G(L)^G(L)^T(V) |
| L-0546 | G(V)^G(L)^G(L)^t^5(x)^a^a^t^g^a^g^a^g^G(L)^G(V)^T(V) |
| L-0547 | G(L)^G(V)^G(L)^t^5(x)^a^a^t^g^a^g^a^g^G(L)^G(V)^T(L) |
| L -0548 | G(V)^G(L)^G(V)^G(L)^t^5(x)^a^a^t^g^a^g^a^g^G(L)^G(V)^T(L)^G(V) |
| L-0549 | G(L)^G(L)^G(V)^t^5(x)^a^a^t^g^a^g^a^g^G(V)^G(L)^T(L) |

### Positions in a sequence of human ATN1 mRNA or pre-mRNA targeted by the antisense oligonucleotides presented in Tables 26 to 33, and sequence numbers and nucleobase sequences corresponding to the antisense oligonucleotides are presented in Tables 34 to 41. The notations in Tables 34 to 41 are the same as those in Tables 11 to 20.

Note that the title rows in Tables 36 to 41 are the same as that in Table 35, and thus are omitted.

**[Table 34]**

| Cmpd No | SEQ1 START | SEQ1 END | SEQ2 START | SEQ2 END | SEQ No | BASE SEQUENCE |
|---|---|---|---|---|---|---|
| M-0248 | 3451 | 3470 | 12574 | 12593 | 419 | AATGAGAGGGTGCACCGAGG |
| M-0249 | 3456 | 3475 | 12579 | 12598 | 423 | GGGTCAATGAGAGGGTGCAC |
| M-0250 | 3468 | 3487 | 12591 | 12610 | 435 | CCTGAGGCCAGGGGGTCAAT |
| M-0251 | 3476 | 3495 | 12599 | 12618 | 441 | GGTGAGACCCTGAGGCCAGG |
| M-0252 | 3477 | 3496 | 12600 | 12619 | 442 | AGGTGAGACCCTGAGGCCAG |
| M-0253 | 3478 | 3497 | 12601 | 12620 | 443 | AAGGTGAGACCCTGAGGCCA |
| M-0254 | 3487 | 3506 | 12610 | 12629 | 450 | GATCCGGGTAAGGTGAGACC |
| M-0255 | 3488 | 3507 | 12611 | 12630 | 451 | GGATCCGGGTAAGGTGAGAC |
| M-0256 | 3489 | 3508 | 12612 | 12631 | 452 | GGGATCCGGGTAAGGTGAGA |
| M-0257 | 3490 | 3509 | 12613 | 12632 | 453 | GGGGATCCGGGTAAGGTGAG |
| M-0258 | 3491 | 3510 | 12614 | 12633 | 454 | AGGGGATCCGGGTAAGGTGA |
| M-0259 | 3497 | 3516 | 12620 | 12639 | 460 | CTGGGTAGGGGATCCGGGTA |
| M-0260 | 3499 | 3518 | 12622 | 12641 | 462 | AGCTGGGTAGGGGATCCGGG |
| M-0261 | 3504 | 3523 | 12627 | 12646 | 467 | GTTCCAGCTGGGTAGGGGAT |

**[Table 35]**

| Cmpd No | SEQ1 START | SEQ1 END | SEQ2 START | SEQ2 END | SEQ No | BASE SEQUENCE |
|---|---|---|---|---|---|---|
| L-0240 | 3458 | 3473 | 12581 | 12596 | 219 | GTCAATGAGAGGGTGC |
| L-0241 | 3458 | 3473 | 12581 | 12596 | 219 | GTCAATGAGAGGGTGC |
| L-0242 | 3458 | 3473 | 12581 | 12596 | 219 | GTCAATGAGAGGGTGC |
| L-0243 | 3457 | 3474 | 12580 | 12597 | 488 | GGTCAATGAGAGGGTGCA |
| L-0244 | 3459 | 3474 | 12582 | 12597 | 220 | GGTCAATGAGAGGGTG |
| L-0245 | 3459 | 3474 | 12582 | 12597 | 220 | GGTCAATGAGAGGGTG |
| L-0246 | 3459 | 3474 | 12582 | 12597 | 220 | GGTCAATGAGAGGGTG |
| L-0247 | 3458 | 3475 | 12581 | 12598 | 489 | GGGTCAATGAGAGGGTGC |
| L-0248 | 3461 | 3476 | 12584 | 12599 | 222 | GGGGTCAATGAGAGGG |
| L-0249 | 3461 | 3476 | 12584 | 12599 | 222 | GGGGTCAATGAGAGGG |
| L-0250 | 3461 | 3476 | 12584 | 12599 | 222 | GGGGTCAATGAGAGGG |
| L-0251 | 3460 | 3477 | 12583 | 12600 | 490 | GGGGGTCAATGAGAGGGT |
| L-0252 | 3496 | 3511 | 12619 | 12634 | 233 | TAGGGGATCCGGGTAA |
| L-0253 | 3496 | 3511 | 12619 | 12634 | 233 | TAGGGGATCCGGGTAA |
| L-0254 | 3496 | 3511 | 12619 | 12634 | 233 | TAGGGGATCCGGGTAA |
| L-0255 | 3495 | 3512 | 12618 | 12635 | 491 | GTAGGGGATCCGGGTAAG |
| L-0256 | 3503 | 3518 | 12626 | 12641 | 234 | AGCTGGGTAGGGGATC |
| L-0257 | 3503 | 3518 | 12626 | 12641 | 234 | AGCTGGGTAGGGGATC |
| L-0258 | 3503 | 3518 | 12626 | 12641 | 234 | AGCTGGGTAGGGGATC |
| L-0259 | 3502 | 3519 | 12625 | 12642 | 492 | CAGCTGGGTAGGGGATCC |
| L-0260 | - | - | 373 | 388 | 493 | CTCCTCTTTTGAGTCT |
| L-0261 | - | - | 383 | 398 | 494 | CTCGATGTTCCTCCTC |
| L-0262 | - | - | 426 | 441 | 495 | ACCCCACTCACAGCAT |
| L-0263 | - | - | 450 | 465 | 496 | CTATCCATTCTGCCTT |
| L-0264 | - | - | 456 | 471 | 497 | TCTCTCCTATCCATTC |
| L-0265 | - | - | 546 | 561 | 498 | TTCCTAAAGCCCCCAA |
| L-0266 | - | - | 552 | 567 | 499 | CGTCTATTCCTAAAGC |
| L-0267 | - | - | 589 | 604 | 500 | TTCCACCTACCACACC |
| L-0268 | - | - | 603 | 618 | 501 | GCCACAATCCCTCTTT |
| L-0269 | - | - | 644 | 659 | 502 | ACCTCTCTTTGCCCAA |
| L-0270 | - | - | 653 | 668 | 503 | CAAAAATGGACCTCTC |
| L-0271 | - | - | 678 | 693 | 504 | ACGCTGGAAAACCTCC |
| L-0272 | - | - | 709 | 724 | 505 | ACCGTCACAAAGACAT |
| L-0273 | - | - | 780 | 795 | 506 | CTCTCCAAGAAATAAC |
| L-0274 | - | - | 828 | 843 | 507 | TCCTCCTCACTAGTTT |
| L-0275 | - | - | 838 | 853 | 508 | GTTCCTGTTTTCCTCC |
| L-0276 | - | - | 847 | 862 | 509 | CCCTTCTGTGTTCCTG |
| L-0277 | - | - | 874 | 889 | 510 | TCCCAGAACCCCTTTC |
| L-0278 | - | - | 889 | 904 | 511 | TCTCCATTTCCATCCT |
| L-0279 | - | - | 899 | 914 | 512 | GTTGCTCAATTCTCCA |
| L-0280 | - | - | 986 | 1001 | 513 | TATAGATCACAGCTCC |
| L-0281 | - | - | 993 | 1008 | 514 | CTCCCTATATAGATCA |
| L-0282 | - | - | 1004 | 1019 | 515 | GTAACTAAGGCCTCCC |
| L-0283 | - | - | 1043 | 1058 | 516 | TAAGCCCATGCACAAA |
| L-0284 | - | - | 1048 | 1063 | 517 | CAAAATAAGCCCATGC |
| L-0285 | - | - | 1059 | 1074 | 518 | CCTACCCTAGTCAAAA |
| L-0286 | - | - | 1098 | 1113 | 519 | ACCCTCCTATAGCACC |
| L-0287 | - | - | 1108 | 1123 | 520 | AGCAACTCCTACCCTC |
| L-0288 | - | - | 1120 | 1135 | 521 | CCCAGTTGCCAAAGCA |
| L-0289 | - | - | 1163 | 1178 | 522 | TCAAGAGACCATCCCA |

**[Table 36]**

| | | | | | | |
|---|---|---|---|---|---|---|
| L-0290 | - | - | 1194 | 1209 | 523 | ACCATAGCAACACAGT |
| L-0291 | - | - | 1210 | 1225 | 524 | ATCCCACTGGATCACT |
| L-0292 | - | - | 1217 | 1232 | 525 | ATCTGACATCCCACTG |
| L-0293 | - | - | 1225 | 1240 | 526 | CCCAACCAATCTGACA |
| L-0294 | - | - | 1246 | 1261 | 527 | CCAATAAACATTCGAA |
| L-0295 | - | - | 1294 | 1309 | 528 | ACCTCCAAAACCCAGC |
| L-0296 | - | - | 1304 | 1319 | 529 | TCCAGAAACAACCTCC |
| L-0297 | - | - | 1315 | 1330 | 530 | CCACCAACAGATCCAG |
| L-0298 | - | - | 1324 | 1339 | 531 | AACTCCCAACCACCAA |
| L-0299 | - | - | 1363 | 1378 | 532 | CCAACAGACTTCAACA |
| L-0300 | - | - | 1378 | 1393 | 533 | ACGTATCTACCTGTTC |
| L-0301 | - | - | 1398 | 1413 | 534 | ACACCTATGATCTGCA |
| L-0302 | - | - | 1442 | 1457 | 535 | CGTTTTCATCCTCCCA |
| L-0303 | - | - | 1482 | 1497 | 536 | CAGAACCAACTGCCAA |
| L-0304 | - | - | 1529 | 1544 | 537 | CAAAAAGACAACTCTA |
| L-0305 | - | - | 1658 | 1673 | 538 | CCCAGCCATTTCTACA |
| L-0306 | - | - | 1713 | 1728 | 539 | AGCTGTTTCACAGCCC |
| L-0307 | - | - | 1735 | 1750 | 540 | CTATAGTATTTCCCCT |
| L-0308 | - | - | 1762 | 1777 | 541 | CTAGACACAGGTCTCC |
| L-0309 | - | - | 1827 | 1842 | 542 | CTTAACAATGAAGCCC |
| L-0310 | - | - | 1847 | 1862 | 543 | CTCATCTATAGCCCCT |
| L-0311 | - | - | 1853 | 1868 | 544 | GATCATCTCATCTATA |
| L-0312 | - | - | 1902 | 1917 | 545 | CACTGGCCTAAGCCTT |
| L-0313 | - | - | 1944 | 1959 | 546 | ACTCCTAGATAACAAT |
| L-0314 | - | - | 1953 | 1968 | 547 | TCCAAAGTGACTCCTA |
| L-0315 | - | - | 1965 | 1980 | 548 | CTAACAAACCAGTCCA |
| L-0316 | - | - | 1993 | 2008 | 549 | CCCCCAACATCATTCT |
| L-0317 | - | - | 2114 | 2129 | 550 | CCCCTAAGTAAGAACA |
| L-0318 | - | - | 2189 | 2204 | 551 | GCCATTACCTTACTTT |
| L-0319 | - | - | 2522 | 2537 | 552 | CTTCATTACCTTGCTT |
| L-0320 | - | - | 2532 | 2547 | 553 | GCACATTCACCTTCAT |
| L-0321 | - | - | 2562 | 2577 | 554 | GCACCTACTCTGTATC |
| L-0322 | - | - | 2587 | 2602 | 555 | CAATCACTGTAACTAA |
| L-0323 | - | - | 2600 | 2615 | 556 | CACTCCTAGCAGGCAA |
| L-0324 | - | - | 2789 | 2804 | 557 | CAATCATAGTATGCCC |
| L-0325 | - | - | 2863 | 2878 | 558 | CCTGTTCACCAAGTCA |
| L-0326 | - | - | 2878 | 2893 | 559 | TTCCCCATCACAGAAC |
| L-0327 | - | - | 2989 | 3004 | 560 | ACCCACCATAGCAAAA |
| L-0328 | - | - | 2997 | 3012 | 561 | AGAACAACACCCACCA |
| L-0329 | - | - | 3002 | 3017 | 562 | TCCGAAGAACAACACC |
| L-0330 | - | - | 3066 | 3081 | 563 | CCCACATTTTTTAGTT |
| L-0331 | - | - | 3082 | 3097 | 564 | CAACTACAGCTATTTT |
| L-0332 | - | - | 3380 | 3395 | 565 | TCTTTGCTCTTGTTCC |
| L-0333 | - | - | 3662 | 3677 | 566 | TGATCTCACTCACTGC |
| L-0334 | - | - | 3762 | 3777 | 567 | AACTGATGCTCTGCCC |
| L-0335 | - | - | 3774 | 3789 | 568 | TTCTGAACTTTCAACT |
| L-0336 | - | - | 3821 | 3836 | 569 | CTTCACAATTTACTTA |
| L-0337 | - | - | 3829 | 3844 | 570 | CCAGGAAACTTCACAA |
| L-0338 | - | - | 3841 | 3856 | 571 | AGTCTAACCTTTCCAG |
| L-0339 | - | - | 3908 | 3923 | 572 | TTCCCAAGAAACACAG |

**[Table 37]**

| | | | | | | |
|---|---|---|---|---|---|---|
| L-0340 | - | - | 3978 | 3993 | 573 | ACCCATGTATTCTATT |
| L-0341 | - | - | 3987 | 4002 | 574 | ACTAAGCTTACCCATG |
| L-0342 | - | - | 4061 | 4076 | 575 | ATTGACCTTTAAACCC |
| L-0343 | - | - | 4108 | 4123 | 576 | CTTACTGTTTTCTTCC |
| L-0344 | - | - | 4157 | 4172 | 577 | CCTCCAAATTACTGAT |
| L-0345 | - | - | 4168 | 4183 | 578 | AGTGTCAAGTCCCTCC |
| L-0346 | - | - | 4227 | 4242 | 579 | CTTTATCCAAATCCCT |
| L-0347 | - | - | 4235 | 4250 | 580 | GTATCTATCTTTATCC |
| L-0348 | - | - | 4255 | 4270 | 581 | CCGTATCTGTCTATCA |
| L-0349 | - | - | 4266 | 4281 | 582 | AGCCAATCCATCCGTA |
| L-0350 | - | - | 4280 | 4295 | 583 | ACACAGTTTCTCACAG |
| L-0351 | - | - | 4314 | 4329 | 584 | TAAGCCTATCCTCCTA |
| L-0352 | - | - | 4335 | 4350 | 585 | CAACACAGATGTACCA |
| L-0353 | - | - | 4360 | 4375 | 586 | AAGACTCAAAATATCC |
| L-0354 | - | - | 4425 | 4440 | 587 | CCATTCTCTAGTACTT |
| L-0355 | - | - | 4468 | 4483 | 588 | CCCCAAATTGTAGAAC |
| L-0356 | - | - | 4477 | 4492 | 589 | CCTAGGTAGCCCCAAA |
| L-0357 | - | - | 4504 | 4519 | 590 | CACAGCCCTACCAATC |
| L-0358 | - | - | 4525 | 4540 | 591 | CCATTCTATAATACAA |
| L-0359 | - | - | 4562 | 4577 | 592 | TCTTTTCTCACATCAG |
| L-0360 | - | - | 4636 | 4651 | 593 | TGCTGTATTCTCCTCA |
| L-0361 | - | - | 4649 | 4664 | 594 | TTCTTCTATACTTTGC |
| L-0362 | - | - | 4687 | 4702 | 595 | AATCAAACCCATGTCG |
| L-0363 | - | - | 4734 | 4749 | 596 | TGTGACTAGCCCAACC |
| L-0364 | - | - | 4780 | 4795 | 597 | TACCCTCATTATATAG |
| L-0365 | - | - | 4809 | 4824 | 598 | TACAACCAACCCTGAA |
| L-0366 | - | - | 4835 | 4850 | 599 | TCACATACATGATTTC |
| L-0367 | - | - | 4857 | 4872 | 600 | TACCTTCTTTATATCA |
| L-0368 | - | - | 5205 | 5220 | 601 | ACACACATTCCACGGA |
| L-0369 | - | - | 5235 | 5250 | 602 | CTTCATGATCCCTAAA |
| L-0370 | - | - | 5246 | 5261 | 603 | CACAGTTTACCCTTCA |
| L-0371 | - | - | 5277 | 5292 | 604 | ATGTACCTACAAGCCC |
| L-0372 | - | - | 5316 | 5331 | 605 | TATAATCTCTTTCATC |
| L-0373 | - | - | 5354 | 5369 | 606 | CCTTAGCTATCTAAAA |
| L-0374 | - | - | 5393 | 5408 | 607 | CAGATACCACAAGACA |
| L-0375 | - | - | 5421 | 5436 | 608 | CAACCCTAAGCCTTTG |
| L-0376 | - | - | 5497 | 5512 | 609 | CATCAGAGTCTCCAAC |
| L-0377 | - | - | 5716 | 5731 | 610 | CTCATTTTAACTCACC |
| L-0378 | - | - | 5728 | 5743 | 611 | TCTTTCATGTCTCTCA |
| L-0379 | - | - | 5733 | 5748 | 612 | CCTCATCTTTCATGTC |
| L-0380 | - | - | 5758 | 5773 | 613 | TTCCTCCTAGCTTGCC |
| L-0381 | - | - | 5779 | 5794 | 614 | TTGTTCTTCTTCTCTA |
| L-0382 | - | - | 5807 | 5822 | 615 | TTGCACCTAACTTTAT |
| L-0383 | - | - | 5824 | 5839 | 616 | TAACACTGTTCTTTAC |
| L-0384 | - | - | 6069 | 6084 | 617 | AACAGGAAGTTCCCCC |
| L-0385 | - | - | 6115 | 6130 | 618 | TCCTGTCTTTTCCCTA |
| L-0386 | - | - | 6356 | 6371 | 619 | TTCTCTTATGTCCCAG |
| L-0387 | - | - | 6382 | 6397 | 620 | CTCTCATAAGCACTAT |
| L-0388 | - | - | 6420 | 6435 | 621 | GCTCTAAGAATCTCTC |
| L-0389 | - | - | 6478 | 6493 | 622 | ATCCCCCAACTCTTCT |

**[Table 38]**

| | | | | | | |
|---|---|---|---|---|---|---|
| L-0390 | - | - | 6506 | 6521 | 623 | GACTAAGACTTCTCAC |
| L-0391 | - | - | 6540 | 6555 | 624 | TTCTGCATTTTCCTTA |
| L-0392 | - | - | 6548 | 6563 | 625 | AAGACCACTTCTGCAT |
| L-0393 | - | - | 6875 | 6890 | 626 | TTTTATACCACTTCTG |
| L-0394 | - | - | 6907 | 6922 | 627 | TTTAACCATCCTCCAT |
| L-0395 | - | - | 6924 | 6939 | 628 | CATCTCATAACCACCA |
| L-0396 | - | - | 6947 | 6962 | 629 | GACTCCAACTCCCTGT |
| L-0397 | - | - | 6969 | 6984 | 630 | CACCTTGTCTCTACCC |
| L-0398 | - | - | 6989 | 7004 | 631 | CCTCAAGGATCCATCT |
| L-0399 | - | - | 7002 | 7017 | 632 | AACCCTATTCCTTCCT |
| L-0400 | - | - | 7023 | 7038 | 633 | AGTCTTGTTTCCCGCC |
| L-0401 | - | - | 7055 | 7070 | 634 | TTCCTCACATTCTTGG |
| L-0402 | - | - | 7066 | 7081 | 635 | GATTTCTCACTTTCCT |
| L-0403 | - | - | 7083 | 7098 | 636 | ACCTCTTACCATCTGG |
| L-0404 | - | - | 7147 | 7162 | 637 | AAAATCATTTCCCCTC |
| L-0405 | - | - | 9336 | 9351 | 638 | ACACTACACCAGCGCA |
| L-0406 | - | - | 9923 | 9938 | 639 | CTCGCTGAAAACCTTT |
| L-0407 | - | - | 9951 | 9966 | 640 | TTTTCCCAACCCAGGA |
| L-0408 | - | - | 9966 | 9981 | 641 | CCATCTGAGCATGCCT |
| L-0409 | - | - | 10001 | 10016 | 642 | CGAATATTTGCCCTTC |
| L-0410 | - | - | 10031 | 10046 | 643 | CACCTGGAACTTAACT |
| L-0411 | - | - | 10056 | 10071 | 644 | TGCCTCAACTCATTGA |
| L-0412 | - | - | 11122 | 11137 | 645 | CTTCAAAAGCTCCCGT |
| L-0413 | - | - | 11621 | 11636 | 646 | ACACCCAAACCCAAAG |
| L-0414 | - | - | 11631 | 11646 | 647 | ATCGCCTATGACACCC |
| L-0415 | - | - | 11641 | 11656 | 648 | AAAGCTCATTATCGCC |
| L-0416 | - | - | 12705 | 12720 | 649 | CATCCTTACCAAAGAG |
| L-0417 | - | - | 12758 | 12773 | 650 | CCCAAATACCTCCTTT |
| L-0418 | - | - | 12835 | 12850 | 651 | AGCTCTCCTATTCCCA |
| L-0419 | - | - | 12873 | 12888 | 652 | CTGACACTAAAACTCG |
| L-0420 | - | - | 12931 | 12946 | 653 | ACACCCAGTTCTGTAA |
| L-0421 | - | - | 12974 | 12989 | 654 | GCTCCTTTACATACAA |
| L-0422 | - | - | 13008 | 13023 | 655 | TAACAAGTACCTGCTC |
| L-0423 | - | - | 12442 | 12457 | 656 | TCCAAAGTGGAACAAA |
| L-0424 | - | - | 12456 | 12471 | 657 | CAAACACAAGGAGGTC |
| L-0425 | - | - | 12463 | 12478 | 658 | AGTCAGACAAACACAA |
| L-0426 | - | - | 12467 | 12482 | 659 | ACTGAGTCAGACAAAC |
| L-0427 | - | - | 12506 | 12521 | 660 | GCAAACCATGCATTTG |
| L-0428 | - | - | 12508 | 12523 | 661 | GGGCAAACCATGCATT |
| L-0429 | 1363 | 1378 | 8088 | 8103 | 174 | GCAGAGCTACTACTAG |
| L-0430 | 1363 | 1378 | 8088 | 8103 | 174 | GCAGAGCTACTACTAG |
| L-0431 | 1363 | 1378 | 8088 | 8103 | 174 | GCAGAGCTACTACTAG |
| L-0432 | 1362 | 1379 | 8087 | 8104 | 662 | TGCAGAGCTACTACTAGA |
| L-0433 | 2888 | 2903 | 10309 | 10324 | 105 | CCGGGCATATTCACTG |
| L-0434 | 2888 | 2903 | 10309 | 10324 | 105 | CCGGGCATATTCACTG |
| L-0435 | 2888 | 2903 | 10309 | 10324 | 105 | CCGGGCATATTCACTG |
| L-0436 | 2887 | 2904 | 10308 | 10325 | 663 | GCCGGGCATATTCACTGA |
| L-0437 | 3520 | 3535 | 12643 | 12658 | 240 | GGGTTAGGGAGAGTTC |
| L-0438 | 3520 | 3535 | 12643 | 12658 | 240 | GGGTTAGGGAGAGTTC |
| L-0439 | 3520 | 3535 | 12643 | 12658 | 240 | GGGTTAGGGAGAGTTC |

**[Table 39]**

| | | | | | | |
|---|---|---|---|---|---|---|
| L-0440 | 3519 | 3536 | 12642 | 12659 | 664 | GGGGTTAGGGAGAGTTCC |
| L-0441 | 3453 | 3468 | 12576 | 12591 | 216 | TGAGAGGGTGCACCGA |
| L-0442 | 3453 | 3468 | 12576 | 12591 | 216 | TGAGAGGGTGCACCGA |
| L-0443 | 3453 | 3468 | 12576 | 12591 | 216 | TGAGAGGGTGCACCGA |
| L-0444 | 3452 | 3469 | 12575 | 12592 | 665 | ATGAGAGGGTGCACCGAG |
| L-0445 | 3457 | 3472 | 12580 | 12595 | 218 | TCAATGAGAGGGTGCA |
| L-0446 | 3457 | 3472 | 12580 | 12595 | 218 | TCAATGAGAGGGTGCA |
| L-0447 | 3457 | 3472 | 12580 | 12595 | 218 | TCAATGAGAGGGTGCA |
| L-0448 | 3456 | 3473 | 12579 | 12596 | 666 | GTCAATGAGAGGGTGCAC |
| L-0449 | 3462 | 3477 | 12585 | 12600 | 223 | GGGGGTCAATGAGAGG |
| L-0450 | 3462 | 3477 | 12585 | 12600 | 223 | GGGGGTCAATGAGAGG |
| L-0451 | 3462 | 3477 | 12585 | 12600 | 223 | GGGGGTCAATGAGAGG |
| L-0452 | 3461 | 3478 | 12584 | 12601 | 667 | AGGGGGTCAATGAGAGGG |
| L-0453 | 3463 | 3478 | 12586 | 12601 | 224 | AGGGGGTCAATGAGAG |
| L-0454 | 3463 | 3478 | 12586 | 12601 | 224 | AGGGGGTCAATGAGAG |
| L-0455 | 3463 | 3478 | 12586 | 12601 | 224 | AGGGGGTCAATGAGAG |
| L-0456 | 3462 | 3479 | 12585 | 12602 | 668 | CAGGGGGTCAATGAGAGG |
| L-0457 | 3464 | 3479 | 12587 | 12602 | 225 | CAGGGGGTCAATGAGA |
| L-0458 | 3464 | 3479 | 12587 | 12602 | 225 | CAGGGGGTCAATGAGA |
| L-0459 | 3464 | 3479 | 12587 | 12602 | 225 | CAGGGGGTCAATGAGA |
| L-0460 | 3463 | 3480 | 12586 | 12603 | 669 | CCAGGGGGTCAATGAGAG |
| L-0461 | 3465 | 3480 | 12588 | 12603 | 226 | CCAGGGGGTCAATGAG |
| L-0462 | 3465 | 3480 | 12588 | 12603 | 226 | CCAGGGGGTCAATGAG |
| L-0463 | 3465 | 3480 | 12588 | 12603 | 226 | CCAGGGGGTCAATGAG |
| L-0464 | 3464 | 3481 | 12587 | 12604 | 670 | GCCAGGGGGTCAATGAGA |
| L-0465 | 3466 | 3481 | 12589 | 12604 | 227 | GCCAGGGGGTCAATGA |
| L-0466 | 3466 | 3481 | 12589 | 12604 | 227 | GCCAGGGGGTCAATGA |
| L-0467 | 3466 | 3481 | 12589 | 12604 | 227 | GCCAGGGGGTCAATGA |
| L-0468 | 3465 | 3482 | 12588 | 12605 | 671 | GGCCAGGGGGTCAATGAG |
| L-0469 | 3468 | 3483 | 12591 | 12606 | 228 | AGGCCAGGGGGTCAAT |
| L-0470 | 3468 | 3483 | 12591 | 12606 | 228 | AGGCCAGGGGGTCAAT |
| L-0471 | 3468 | 3483 | 12591 | 12606 | 228 | AGGCCAGGGGGTCAAT |
| L-0472 | 3467 | 3484 | 12590 | 12607 | 672 | GAGGCCAGGGGGTCAATG |
| L-0473 | 3488 | 3503 | 12611 | 12626 | 208 | CCGGGTAAGGTGAGAC |
| L-0474 | 3488 | 3503 | 12611 | 12626 | 208 | CCGGGTAAGGTGAGAC |
| L-0475 | 3488 | 3503 | 12611 | 12626 | 208 | CCGGGTAAGGTGAGAC |
| L-0476 | 3487 | 3504 | 12610 | 12627 | 673 | TCCGGGTAAGGTGAGACC |
| L-0477 | 3491 | 3506 | 12614 | 12629 | 232 | GATCCGGGTAAGGTGA |
| L-0478 | 3491 | 3506 | 12614 | 12629 | 232 | GATCCGGGTAAGGTGA |
| L-0479 | 3491 | 3506 | 12614 | 12629 | 232 | GATCCGGGTAAGGTGA |
| L-0480 | 3490 | 3507 | 12613 | 12630 | 674 | GGATCCGGGTAAGGTGAG |
| L-0481 | 3481 | 3496 | 12604 | 12619 | 230 | AGGTGAGACCCTGAGG |
| L-0482 | 3481 | 3496 | 12604 | 12619 | 230 | AGGTGAGACCCTGAGG |
| L-0483 | 3481 | 3496 | 12604 | 12619 | 230 | AGGTGAGACCCTGAGG |
| L-0484 | 3480 | 3497 | 12603 | 12620 | 675 | AAGGTGAGACCCTGAGGC |
| L-0485 | 3484 | 3499 | 12607 | 12622 | 204 | GTAAGGTGAGACCCTG |
| L-0486 | 3484 | 3499 | 12607 | 12622 | 204 | GTAAGGTGAGACCCTG |
| L-0487 | 3484 | 3499 | 12607 | 12622 | 204 | GTAAGGTGAGACCCTG |
| L-0488 | 3483 | 3500 | 12606 | 12623 | 676 | GGTAAGGTGAGACCCTGA |
| L-0489 | 3482 | 3497 | 12605 | 12620 | 231 | AAGGTGAGACCCTGAG |

**[Table 40]**

| | | | | | | |
|---|---|---|---|---|---|---|
| L-0490 | 3482 | 3497 | 12605 | 12620 | 231 | AAGGTGAGACCCTGAG |
| L-0491 | 3482 | 3497 | 12605 | 12620 | 231 | AAGGTGAGACCCTGAG |
| L-0492 | 3481 | 3498 | 12604 | 12621 | 677 | TAAGGTGAGACCCTGAGG |
| L-0498 | - | - | - | - | 678 | GCCCTACCCATACAGA |
| L-0499 | - | - | - | - | 679 | GCCCACACTGAAACTA |
| L-0500 | - | - | - | - | 680 | CTAGCTGTAACCCAAT |
| L-0501 | - | - | - | - | 681 | TTCCAAATCCCTTCCA |
| L-0502 | - | - | - | - | 682 | CAGTTCAGTCTCCCAC |
| L-0503 | - | - | - | - | 683 | CTCAGTTCAGTCTCCC |
| L-0504 | - | - | - | - | 684 | CTGTACCACTCAGTTC |
| L-0505 | - | - | - | - | 685 | CCCCATGGCATTTCCT |
| L-0506 | - | - | - | - | 686 | CCCCTGACTTTGTTCC |
| L-0507 | - | - | - | - | 687 | CACCCCTGACTTTGTT |
| L-0508 | - | - | - | - | 688 | TCCAAGCACACCTTCC |
| L-0509 | - | - | - | - | 689 | ACCACTCCAAGCACAC |
| L-0510 | - | - | - | - | 690 | CATCACTCTGAAGTCC |
| L-0511 | - | - | - | - | 691 | GTCTCACAATTCCTTG |
| L-0512 | - | - | - | - | 692 | CACCACCAGTTCCTTC |
| L-0513 | - | - | - | - | 693 | GTTGCTCAATCCCACT |
| L-0514 | - | - | - | - | 694 | CACTAAAGATCCCCTG |
| L-0515 | - | - | - | - | 695 | CCTTCTCAAATCTGTT |
| L-0516 | - | - | 1971 | 1986 | 696 | GCCAGACTAACAAACC |
| L-0517 | - | - | 4217 | 4232 | 697 | ATCCCTATAGCCAGCT |
| L-0518 | - | - | 4501 | 4516 | 698 | AGCCCTACCAATCCCA |
| L-0519 | - | - | 4840 | 4855 | 699 | GCCCTTCACATACATG |
| L-0520 | - | - | 5424 | 5439 | 700 | GCCCAACCCTAAGCCT |
| L-0521 | 151 | 166 | 5610 | 5625 | 701 | CCACCCCAAGTTCCCC |
| L-0522 | - | - | 5769 | 5784 | 702 | TCTCTAGACCCTTCCT |
| L-0523 | - | - | 6107 | 6122 | 703 | TTTCCCTACCTCCCGA |
| L-0524 | 452 | 467 | 6209 | 6224 | 704 | ACTCTCTGAGATCTCC |
| L-0525 | - | - | 6486 | 6501 | 705 | AGACTAGAATCCCCCA |
| L-0526 | - | - | 6963 | 6978 | 706 | GTCTCTACCCACAGCT |
| L-0527 | - | - | 6981 | 6996 | 707 | ATCCATCTCAAGCACC |
| L-0528 | - | - | 7012 | 7027 | 708 | CCGCCTGTAAAACCCT |
| L-0529 | - | - | 7097 | 7112 | 709 | TCTTGCTCAAGCCCAC |
| L-0530 | - | - | 9312 | 9327 | 710 | CCCTGCTACTCCCTCT |
| L-0531 | - | - | 9425 | 9440 | 711 | GCCTCAAAACCACCCC |
| L-0532 | - | - | 12716 | 12731 | 712 | ACCCCAACTTCCATCC |
| L-0533 | - | - | 12723 | 12738 | 713 | GCTGCCTACCCCAACT |
| L-0534 | - | - | 12763 | 12778 | 714 | CCCCACCCAAATACCT |
| L-0535 | 3452 | 3467 | 12575 | 12590 | 215 | GAGAGGGTGCACCGAG |
| L-0536 | 3452 | 3467 | 12575 | 12590 | 215 | GAGAGGGTGCACCGAG |
| L-0537 | 3452 | 3467 | 12575 | 12590 | 215 | GAGAGGGTGCACCGAG |
| L-0538 | 3451 | 3468 | 12574 | 12591 | 715 | TGAGAGGGTGCACCGAGG |
| L-0539 | 3452 | 3467 | 12575 | 12590 | 215 | GAGAGGGTGCACCGAG |
| L-0540 | 3452 | 3467 | 12575 | 12590 | 215 | GAGAGGGTGCACCGAG |
| L-0541 | 3452 | 3467 | 12575 | 12590 | 215 | GAGAGGGTGCACCGAG |
| L-0542 | 3452 | 3467 | 12575 | 12590 | 215 | GAGAGGGTGCACCGAG |
| L-0543 | 3452 | 3467 | 12575 | 12590 | 215 | GAGAGGGTGCACCGAG |
| L-0544 | 3452 | 3467 | 12575 | 12590 | 215 | GAGAGGGTGCACCGAG |

**[Table 41]**

| | | | | | | |
|---|---|---|---|---|---|---|
| L-0545 | 3460 | 3475 | 12583 | 12598 | 221 | GGGTCAATGAGAGGGT |
| L-0546 | 3460 | 3475 | 12583 | 12598 | 221 | GGGTCAATGAGAGGGT |
| L-0547 | 3460 | 3475 | 12583 | 12598 | 221 | GGGTCAATGAGAGGGT |
| L-0548 | 3459 | 3476 | 12582 | 12599 | 716 | GGGGTCAATGAGAGGGTG |
| L-0549 | 3460 | 3475 | 12583 | 12598 | 221 | GGGTCAATGAGAGGGT |

### Positions in a sequence of human ATN1 pre-mRNA targeted by L-0498 to 0515 presented in Table 40, and sequence numbers and nucleobase sequences corresponding to the antisense oligonucleotides are presented in Table 42.

"SEQ717 START" means "SEQ ID NO: 717 start site" and represents the position number of a nucleoside on a side closest to a 5' side targeted by an antisense oligonucleotide in the pre-mRNA sequence of human ATN1 (SEQ ID NO: 717). "SEQ717 END" means "SEQ ID NO: 717 termination site" and represents the position number of a nucleoside on a side closest to a 3' side targeted by an antisense oligonucleotide in the pre-mRNA sequence of human ATN1 (SEQ ID NO: 717). The notations in Table 42 are similar to those in Tables 11 to 20.

Note that SEQ ID NO: 2 corresponds to position number 4451 to 18401 of nucleobases of SEQ ID NO: 717.

**[Table 42]**

| Cmpd No | SEQ717 START | SEQ717 END | SEQ No | BASE SEQUENCE |
|---|---|---|---|---|
| L-0498 | 735 | 750 | 678 | GCCCTACCCATACAGA |
| L-0499 | 845 | 860 | 679 | GCCCACACTGAAACTA |
| L-0500 | 1106 | 1121 | 680 | CTAGCTGTAACCCAAT |
| L-0501 | 1479 | 1494 | 681 | TTCCAAATCCCTTCCA |
| L-0502 | 1515 | 1530 | 682 | CAGTTCAGTCTCCCAC |
| L-0503 | 1517 | 1532 | 683 | CTCAGTTCAGTCTCCC |
| L-0504 | 1525 | 1540 | 684 | CTGTACCACTCAGTTC |
| L-0505 | 1547 | 1562 | 685 | CCCCATGGCATTTCCT |
| L-0506 | 1593 | 1608 | 686 | CCCCTGACTTTGTTCC |
| L-0507 | 1595 | 1610 | 687 | CACCCCTGACTTTGTT |
| L-0508 | 1703 | 1718 | 688 | TCCAAGCACACCTTCC |
| L-0509 | 1708 | 1723 | 689 | ACCACTCCAAGCACAC |
| L-0510 | 1748 | 1763 | 690 | CATCACTCTGAAGTCC |
| L-0511 | 1873 | 1888 | 691 | GTCTCACAATTCCTTG |
| L-0512 | 2193 | 2208 | 692 | CACCACCAGTTCCTTC |
| L-0513 | 2341 | 2356 | 693 | GTTGCTCAATCCCACT |
| L-0514 | 2421 | 2436 | 694 | CACTAAAGATCCCCTG |
| L-0515 | 2446 | 2461 | 695 | CCTTCTCAAATCTGTT |

### [Evaluation Example 3] Antisense inhibition of human ATN1 in SH-SY5Y cells

An effect of an antisense oligonucleotide on an ATN1 RNA transcript was tested in vitro using the same evaluation method as in Evaluation Example 1. Results thereof are presented in Tables 43 and 44 as percent expression of ATN1 relative to untreated control cells.

**[Table 43]**

| Cmpd No | ATN1 mRNA remain % |
|---|---|
| M-0248 | 39.3 |
| M-0249 | 9.6 |
| M-0250 | 26.5 |
| M-0251 | 7.6 |
| M-0252 | 17.2 |
| M-0253 | 17.8 |
| M-0254 | 39.5 |
| M-0255 | 16.7 |
| M-0256 | 5.9 |
| M-0257 | 5.5 |
| M-0258 | 5.5 |
| M-0259 | 15.5 |
| M-0260 | 18.8 |
| M-0261 | 16.9 |
| L-0240 | 45.2 |
| L-0241 | 21.1 |
| L-0242 | 21.1 |
| L-0243 | 9.4 |
| L-0244 | 18.8 |
| L-0245 | 13.9 |
| L-0246 | 7.1 |
| L-C247 | 3.9 |
| L-0248 | 31.2 |
| L-0249 | 8.7 |
| L-0250 | 6.9 |
| L-0251 | 7.8 |
| L-0252 | 59.7 |
| L-0253 | 23.0 |
| L-0254 | 13.7 |
| L-0255 | 9.4 |
| L-0256 | 18.9 |
| L-0257 | 20.9 |
| L-0258 | 23.7 |
| L-0259 | 22.0 |
| L-0261 | 66.0 |
| L-0280 | 602 |
| L-0282 | 61.9 |
| L-0300 | 69.2 |
| L-0301 | 77.0 |
| L-0311 | 61.3 |
| L-0318 | 70.3 |
| L-0319 | 79.9 |
| L-0321 | 74.1 |
| L-0333 | 69.5 |
| L-0341 | 72.0 |
| L-0349 | 73.2 |
| L-0352 | 73.0 |
| L-0353 | 66.0 |
| L-0356 | 61.4 |
| L-0359 | 75.6 |
| L-0360 | 72.4 |
| L-0362 | 76.1 |
| L-0363 | 63.0 |
| L-0405 | 65.0 |
| L-0406 | 77.6 |
| L-0409 | 777 |
| L-0411 | 79.0 |
| L-0428 | 73.7 |
| L-0429 | 51.1 |
| L-0430 | 48.9 |
| L-0431 | 58.8 |
| L-0432 | 56.3 |
| L-0433 | 45.4 |

**[Table 44]**

| | |
|---|---|
| L-0434 | 48.2 |
| L-0435 | 50.3 |
| L-0436 | 43.0 |
| L-0437 | 40.7 |
| L-0438 | 49.4 |
| L-0439 | 48.5 |
| L-0440 | 48.6 |
| L-0441 | 19.1 |
| L-0442 | 8.7 |
| L-0443 | 2.8 |
| L-0444 | 1.5 |
| L-0445 | 45.3 |
| L-0446 | 53.8 |
| L-0447 | 31.2 |
| L-0448 | 12.4 |
| L-0449 | 16.8 |
| L-0450 | 16.4 |
| L-0451 | 6.1 |
| L-0452 | 6.9 |
| L-0453 | 42 |
| L-0454 | 19.6 |
| L-0455 | 118 |
| L-0456 | 5.7 |
| L-0457 | 63.7 |
| L-0458 | 45.8 |
| L-0459 | 31.4 |
| L-0460 | 2.5 |
| L-0461 | 24.3 |
| L-0462 | 12.5 |
| L-0463 | 5.8 |
| L-0464 | 3.6 |
| L-0465 | 14.8 |
| L-0466 | 5.1 |
| L-0467 | 49 |
| L-0468 | 3.7 |
| L-0469 | 38.4 |
| L-0470 | 43.7 |
| L-0471 | 34.7 |
| L-047 2 | 3 1 |
| L-0473 | 7.8 |
| L-0474 | 11.0 |
| L-0475 | 7.2 |
| L-0476 | 5.3 |
| L-0477 | 36.5 |
| L-0478 | 10.6 |
| L-0479 | 87 |
| L-0480 | 3.2 |
| L-0481 | 26.8 |
| L-0482 | 45.3 |
| L-0483 | 12.2 |
| L-0484 | 9.5 |
| L-0485 | 64.3 |
| L-0486 | 46.6 |
| L-0487 | 43.4 |
| L-0488 | 23.0 |
| L-0489 | 76.8 |
| L-0490 | 56.2 |
| L-0491 | 43.2 |
| L-0492 | 5.3 |
| L-0516 | 79.3 |
| L-0517 | 62.4 |
| L-0519 | 64.5 |
| L-0526 | 74.9 |
| L-0533 | 74.2 |
| L-0535 | 13.8 |
| L-0536 | 5.4 |
| L-0537 | 1.6 |
| L-0538 | 1.0 |
| L-0539 | 2.5 |
| L-0540 | 2.6 |
| L-0541 | 3.1 |
| L-0542 | 16 |
| L-0543 | 2.6 |
| L-0544 | 8.2 |
| L-0545 | 25.3 |
| L-0546 | 9.5 |
| L-0547 | 65 |
| L-0543 | 15.1 |
| L-0549 | 6.1 |

### [Evaluation Example 4] Dose-dependent antisense inhibition of human ATN1 in SH-SY5Y cells

The antisense oligonucleotide that exhibited inhibition of ATN1 in SH-SY5Y cells in Evaluation Examples 1 and 3 was tested at various doses using the same evaluation method as in Evaluation Example 2. Results thereof were calculated as a concentration (IC₅₀ value) at which an RNA transcript level was 50% relative to untreated control cells and are presented in Table 45.

**[Table 45]**

| Cmpd No | IC₅₀ (nM) |
|---|---|
| L-0238 | 61.8 |
| L-0441 | 95.3 |
| L-0443 | 36.0 |
| L-0450 | 64.6 |

### [Evaluation Example 5] Antisense inhibition of human ATN1 in GM13716 cells [DRPLA patient-derived cells]

An effect of an antisense oligonucleotide targeting ATN1 nucleic acid, which is a causative gene of DRPLA, on an ATN1 RNA transcript was tested in vitro. An antisense oligonucleotide (manufactured in Manufacturing Example 1) was added to GM13716 cells cultured at a density of 7,500 cells/well such that a final concentration thereof was 100 nM using Lipofectamin (registered trademark) RNAiMAX Transfection Reagent (Thermo Fisher Scientific). After approximately 48 hours, RNA was isolated from the cells using RNeasy Micro Kit (QIAGEN), and then reverse-transcribed with High-Capacity cDNA Reverse Transcription Kit (Thermo Fisher Scientific) to create cDNA. An ATN1 RNA transcript level was measured by quantitative real-time PCR with TaqMan (registered trademark) Gene Expression Assays (Thermo Fisher Scientific). Results thereof are presented in Table 46 as percent expression of ATN1 relative to untreated control cells.

**[Table 46]**

| Cmpd No | ATN1 mRNA remain % |
|---|---|
| L-0044 | 19.3 |
| L-0057 | 14.7 |
| L-0103 | 10.4 |
| L-0126 | 2.3 |
| L-0155 | 28.8 |
| L-0172 | 20.1 |
| L-0205 | 4.3 |
| L-0207 | 31.1 |
| L-0219 | 2.2 |
| L-0238 | 12.1 |
| M-0053 | 70.1 |
| M-0092 | 71.2 |
| M-0121 | 70.4 |
| M-0124 | 50.5 |
| M-0174 | 66.0 |
| M-0175 | 50.2 |
| M-0184 | 10.5 |
| M-0187 | 28.3 |
| M-0204 | 29.8 |
| M-0220 | 10.7 |

### [Evaluation Example 6] Antisense inhibition of human ATN1 in GM13716 cells [DRPLA patient-derived cells] (Free-Uptake)

An effect of an antisense oligonucleotide targeting ATN1 nucleic acid, which is a causative gene of DRPLA, on an ATN1 RNA transcript was tested in vitro. An antisense oligonucleotide (manufactured in Manufacturing Example 1) was added to GM13716 cells cultured at a density of 7,500 cells/well such that a final concentration thereof was 100 nM (Free-Uptake). After approximately 48 hours, RNA was isolated from the cells using RNeasy Micro Kit (QIAGEN), and then reverse-transcribed with High-Capacity cDNA Reverse Transcription Kit (Thermo Fisher Scientific) to create cDNA. An ATN1 RNA transcript level was measured by quantitative real-time PCR with TaqMan (registered trademark) Gene Expression Assays (Thermo Fisher Scientific). Results thereof are presented in Table 47 as percent expression of ATN1 relative to untreated control cells.

**[Table 47]**

| Cmpd No | ATN1 mRNA remain % |
|---|---|
| L-0126 | 26.2 |
| L-0205 | 43.4 |
| L-0207 | 54.0 |
| L-0219 | 12.7 |
| L-0238 | 69.9 |
| M-0092 | 88.7 |
| M-0175 | 73.7 |
| M-0184 | 12.5 |
| M-0187 | 29.8 |
| M-0204 | 50.4 |
| M-0220 | 25.2 |

### (Manufacturing Example 3)

Antisense oligonucleotides (compounds represented by chemical structures corresponding to compound numbers) described in Table 48 were prepared using an automatic nucleic acid synthesizer nS-8II (manufactured by GeneDesign Inc.).

**[Table 48]**

| Cmpd **No** | Chemical structure |
|---|---|
| L-0565 | A(V)^5(L)^A(L)^a^5(x)^a^5(x)^a^g^a^t^g^t^A(L)^5(L)^5(V) |
| L-0566 | A(V)^A(L)^A(L)^g^a^5(x)^t^S(x)^a^a^a^a^t^A(L)^T(L)^5(V) |
| L-0567 | A(V)^G(L)^A(L)^5(x)^t^5(x)^a^a^a^a^t^a^T(L)^5(L)^5(V) |
| L-0568 | A(V)^A(L)^G(L)^a^5(x)^t^5(x)^a^a^a^a^t^A(L)^T(L)^5(V) |
| L-0569 | A(V)^A(L)^G(L)^A(L)^5(x)^t^5(x)^a^a^a^a^t^a^T(L)^5(L)^5(V) |
| L-0570 | A(V)^A(L)^G(L)^a^5(x)^t^5(x)^a^a^a^a^t^A(L)^T(L)^5(L)^5(V) |
| L-0571 | A(L)^A(L)^G(L)^a^5(x)^t^5(x)^a^a^a^a^t^a^T(L)^5(L)^5(V) |
| L-0572 | A(V)^A(L)^G(L)^a^5(x)^t^5(x)^a^a^a^a^t^a^T(L)^5(L)^5(L) |
| L-0573 | A(L)^A(L)^G(L)^a^5(x)^t^5(x)^a^a^a^a^t^a^T(L)^5(L)^5(L) |
| L-0574 | G(L)^G(L)^5(L)^a^g^g^t^t^t^g^g^g^g^5(L)^A(L)^A(L) |
| L-0575 | G(L)^T(L)^5(L)^a^5(x)^5(x)^a^g^a^a^g^g^5(x)^A(L)^G(L)^G(L) |
| L-0576 | A(L)^G(L)^A(L)^g^g^a^a^g^g^t^g^t^5(x)^A(L)^5(L)^5(L) |
| L-0577 | A(L)^G(L)^A(L)^g^a^a^g^a^g^g^a^a^g^G(L)^T(L)^G(L) |
| L-0578 | A(L)^G(L)^G(L)^g^5(x)^a^g^a^g^a^a^g^a^G(L)^G(L)^A(L) |
| L-0579 | G(L)^5(L)^T(L)^g^g^a^g^g^g^5(x)^a^g^a^G(L)^A(L)^A(L) |
| L-0580 | 5(L)^A(L)G(L)^a^g^g^5(x)^t^g^g^a^g^g^G(L)^5(L)^A(L) |
| L-0581 | 5(L)^5(L)^G(L)^a^g^g^5(x)^a^g^a^g^g^5(x)^T(L)^G(L)^G(L) |
| L-0582 | A(L)^5(L)^5(L)^g^a^g^g^5(x)^a^g^a^g^g^5(L)^T(L)^G(L) |
| L-0583 | 5(L)^A(L)^5(L)^5(x)^g^a^g^g^5(x)^a^g^a^g^G(L)^5(L)^T(L) |
| L-0584 | G(L)^5(L)^A(L)^5(x)^5(x)^g^a^g^g^5(x)^a^g^a^G(L)^(G(L)^5(L) |
| L-0585 | T(L)^G(L)^5(L)^a^5(x)^5(x)^g^a^g^g^5(x)^a^g^A(L)^G(L)^G(L) |
| L-0586 | G(L)^T(L)^G(L)^5(x)^a^5(x)^5(x)^g^a^g^g^5(x)^a^G(L)^A(L)^G(L) |
| L-0587 | G(L)^G(L)^T(L)^g^5(x)^a^5(x)^5(x)^g^a^g^g^5(x)^A(L)^G(L)^A(L) |
| L-0588 | G(L)^G(L)^G(L)^t^g^5(x)^a^5(x)^5(x)^g^a^g^g^5(L)^A(L)^G(L) |
| L-0589 | A(L)^G(L)^G(L)^g^t^g^5(x)^a^5(x)^5(x)^g^a^g^G(L)^5(L)^A(L) |
| L-0590 | G(L)^A(L)^G(L)^g^g^t^g^5(x)^a^5(x)^5(x)^g^a^G(L)^G(L)^5(L) |
| L-0591 | A(L)^G(L)^A(L)^g^g^g^t^g^5(x)^a^5(x)^5(x)^5(x)^g^A(L)^G(L)^G(L) |
| L-0592 | A(L)^T(L)^G(L)^a^g^a^g^g^g^t^g^5(x)^a^5(L)^5(L)^G(L) |
| L-0593 | A(L)^A(L)^T(L)^g^a^g^a^g^g^g^t^g^5(x)^A(L)^5(L)^5(L) |
| L-0594 | G(L)^G(L)^5(L)^5(x)^a^g^g^g^g^g^t^5(x)^a^A(L)^T(L)^G(L) |
| L-0595 | G(L)^A(L)^G(L)^g^5(x)^5(x)^a^g^g^g^g^g^t^5(L)^5(L)^A(L)^A(L) |
| L-0596 | T(L)^G(L)^A(L)^g^g^5(x)^5(x)^a^g^g^g^g^g^T(L)^5(L)^A(L) |
| L-0597 | 5(L)^T(L)^G(L)^a^g^g^5(x)^a^g^g^g^g^G(L)^T(L)^5(L) |
| L-0598 | 5(L)^5(L)^T(L)^g^a^g^g^5(x)^5(x)^a^g^g^g^G(L)^G(L)^T(L) |
| L-0599 | 5(L)^5(L)^5(L)^t^g^a^g^g^5(x)^5(x)^a^g^g^G(L)^G(L)^G(L) |
| L-0600 | A(L)^5(L)^5(L)^5(x)^t^g^a^g^g^5(x)^5(x)^a^g^G(L)^G(L)^G(L) |
| L-0601 | G(L)^A(L)^5(L)^5(x)^5(x)^t^g^a^g^g^5(x)^5(x)^a^G(L)^G(L) |
| L-0602 | A(L)^G(L)^A(L)^5(x)^5(x)^5(x)^t^g^a^g^g^5(x)^5(x)^A(L)^G(L)^G(L) |
| L-0603 | G(L)^A(L)^G(L)^a^5(x)^5(x)^5(x)^t^g^a^g^g |
| L-0604 | G(L)^T(L)^G(L)^a^g^a^5(x)^5(x)^5(x)^t^g^a^g^G(L)^5(L)^5(L) |
| L-0605 | G(L)^G(L)^T(L)^g^a^g^a^5(x)^5(x)^5(x)^t^g^a^G(L)^G(L)^5(L) |
| L-0606 | G(L)^G(L)^A(L)^t^5(x)^5(x)^g^g^g^t^a^a^g^G(L)^T(L)^G(L) |
| L-0607 | G(L)^G(L)^G(L)^a^t^5(x)^5(x)^g^g^g^t^a^a^G(L)^G(L)^T(L) |
| L-0608 | G(L)^G(L)^G(L)^g^a^t^5(x)^5(x)^g^g^g^t^a^A(L)^G(L)^G(L) |
| L-0609 | A(L)^G(L)^G(L)^g^g^a^t^5(x)^5(x)^g^g^g^t^A(L)^A(L)^G(L) |
| L-0610 | G(L)^T(L)^A(L)^g^g^g^g^a^t^5(x)^5(x)^g^g^G(L)^T(L)^A(L) |
| L-0611 | G(L)^G(L)^T(L)^a^g^g^g^g^g^a^t^5(x)^5(x)^g^G(L)^G(L)^T(L) |
| L-0612 | G(L)^G(L)^G(L)^t^a^g^g^g^g^a^t^5(x)^5(x)^G(L)^G(L)^G(L) |
| L-0613 | T(L)^G(L)^G(L)^g^t^a^g^g^g^g^a^t^5(x)^5(L)5G(L)^G(L) |
| L-0614 | 5(L)^T(L)^G(L)^g^g^t^a^g^g^g^g^a^t^5(L)^G(L) |
| L-0615 | G(L)^5(L)^T(L)^g^g^g^t^a^g^g^g^g^aT(L)^5(L)^5(L) |
| L-0616 | 5(L)^5(L)6A(L)^g^5(x)^t^g^g^g^t^a^g^g^G(L)^G(L)^A(L) |
| L-0617 | T(L)^T(L)^5(L)^5(x)^a^g^5(x)^t^g^g^g^t^a^G(L)^G(L)^G(L) |
| L-0618 | A(L)^G(L)^T(L)^t^5(x)^a^g^5(x)^t^g^g^g^T(L)^A(L)^G(L) |
| L-0619 | A(L)^G(L)^A(L)^g^t^t^5(x)^5(x)^a^g^5(x)^t^g^G(L)^G(L)^T(L) |
| L-0620 | G(L)^(L)^A(L)^g^a^g^t^t^5(x)^5(x)^a^g^5(x)^T(L)^G(L)^G(L) |
| L-0621 | G(L)^A(L)^G(L)^g^a^a^g^5(x)^a^g^g^g^g^G(L)^T(L)^T(L) |
| L-0622 | G(L)^T(L)^G(L)^5(x)^a^g'a^g^g^g^t^g^a^G(L)^G(L)^A(L) |

Positions in a sequence of human ATN1 mRNA or pre-mRNA targeted by the antisense oligonucleotides presented in Table 48, and sequence numbers and nucleobase sequences corresponding to the antisense oligonucleotides are presented in Table 49. The notations in Table 49 are the same as those in Tables 11 to 20.

**[Table 49]**

| Cmpd No | SEQI START | SEQI END | SEQ 2 START | SEQ2 END | SEQ No | BASE SEQUENCE |
|---|---|---|---|---|---|---|
| L-0565 | - | - | 4336 | 4351 | 718 | ACAACACAGATGTACC |
| L-0566 | - | - | 4361 | 4376 | 719 | AAAGACTCAAAATATC |
| L-0567 | - | - | 4360 | 4374 | 720 | AGACTCAAAATATCG |
| L-0568 | - | - | 4361 | 4375 | 721 | AAGAGTCAAAATATC |
| L-0569 | - | - | 4360 | 4375 | 586 | AAGACTCAAAATATCC |
| L-0570 | - | - | 4360 | 4375 | 586 | AAGACTCAAAATATCC |
| L-0571 | - | - | 4360 | 4375 | 586 | AAGACTCAAAATATCC |
| L-0572 | - | - | 4360 | 4375 | 586 | AAGACTCAAAATATCC |
| L-0573 | - | - | 4360 | 4375 | 586 | AAGACTCAAAATATCG |
| L-0574 | - | - | 12518 | 12533 | 722 | TTGCCCCAAACCTGCC |
| L-0575 | - | - | 12528 | 12543 | 723 | CCTGCCTTCTGGTGAC |
| L-0576 | - | - | 12538 | 12553 | 724 | GGTGACACCTTCCTCT |
| L-0577 | - | - | 12543 | 12558 | 725 | CACCTTCCTCTTCTCT |
| L-0578 | - | - | 12548 | 12563 | 726 | TCCTCTTCTCTGCCCT |
| L-0579 | - | - | 12553 | 12568 | 727 | TTCTCTGCCCTCCAGC |
| L-0580 | - | - | 12558 | 12573 | 728 | TGCCCTCCAGCCTCTG |
| L-0581 | - | - | 12564 | 12579 | 729 | CCAGCCTCTGCCTCGG |
| L-0582 | 3442 | 3457 | 12565 | 12580 | 730 | CAGGCTCTGCCTCGGT |
| L-0583 | 3443 | 3458 | 12566 | 12581 | 731 | AGCCTCTGCCTGGGTG |
| L-0584 | 3444 | 3459 | 12567 | 12582 | 732 | GCCTCTGCCTCGGTGC |
| L-0585 | 3445 | 3460 | 12568 | 12583 | 733 | CCTCTGCCTCGGTGCA |
| L-0586 | 3446 | 3461 | 12569 | 12584 | 734 | CTCTGCCTCGGTGCAC |
| L-0587 | 3447 | 3462 | 12570 | 12585 | 735 | TCTGCCTCGGTGCACC |
| L-0588 | 3448 | 3463 | 12571 | 12586 | 736 | CTGCCTCGGTGCACCC |
| L-0589 | 3449 | 3464 | 12572 | 12587 | 737 | TGCCTCGGTGGACCCT |
| L-0590 | 3450 | 3465 | 12573 | 12588 | 738 | GCCTCGGTGCACCCΣC |
| L-0591 | 3451 | 3466 | 12574 | 12589 | 739 | CCTCGGTGGACCCTCT |
| L-0592 | 3454 | 3469 | 12577 | 12592 | 740 | CGGTGCACCCTCTCA T |
| L-0593 | 3455 | 3470 | 12578 | 12593 | 741 | GGTGCAGCCTCTCATT |
| L-0594 | 3467 | 3482 | 12590 | 12605 | 742 | CATTGACCCCCTGGGC |
| L-0595 | 3469 | 3484 | 12592 | 12607 | 743 | TTGAGCGCCTGGGCTG |
| L-0596 | 3470 | 3485 | 12593 | 12608 | 744 | TGACCCCCTGGCCTCA |
| L-0597 | 3471 | 3486 | 12594 | 12609 | 745 | GACCCCCTGGCCTCAG |
| L-0598 | 3472 | 3487 | 12595 | 12610 | 746 | ACCCCCTGGCCTCAGG |
| L-0599 | 3473 | 3488 | 12596 | 12611 | 747 | CCCCCTGGCCTCAGGG |
| L-0600 | 3474 | 3489 | 12597 | 12612 | 748 | GCCCTGGGGTCAGGGT |
| L-0601 | 3475 | 3490 | 12598 | 12613 | 749 | CCCTGGCCTCAGGGTC |
| L-0602 | 3476 | 3491 | 12599 | 12614 | 750 | CCTGGCCTCAGGGTCT |
| L-0603 | 3477 | 3492 | 12600 | 12615 | 751 | CTGGCCTCAGGGTCTG |
| L 0604 | 3479 | 3494 | 12602 | 12617 | 752 | GGCCTCAGGGTGTCAC |
| L-0605 | 3480 | 3495 | 12603 | 12618 | 753 | GCCTCAGGGTCTCACC |
| L-0606 | 3492 | 3507 | 12615 | 12630 | 754 | CACCTTACCCGGATCC |
| L-0607 | 3493 | 3508 | 12616 | 12631 | 755 | ACCTTACCCGGATCCC |
| L-0608 | 3494 | 3509 | 12617 | 12632 | 756 | GCTTAGGCGGATGCCG |
| L-0609 | 3495 | 3510 | 12618 | 12633 | 757 | CTTACGCGGATCCCCT |
| L-0610 | 3497 | 3512 | 12620 | 12635 | 758 | TACCCGGATCCCCTAC |
| L-0611 | 3498 | 3513 | 12621 | 12636 | 759 | ACCCGGATCCCCTACC |
| L-0612 | 3499 | 3514 | 12622 | 12637 | 760 | GCCGGATGGCGTACGG |
| L-0613 | 3500 | 3515 | 12623 | 12638 | 761 | CCGGATCCCCTACCCA |
| L-0614 | 3501 | 3516 | 12624 | 12639 | 762 | CGGATCCCCTACCCAG |
| L-0615 | 3502 | 3517 | 12625 | 12640 | 763 | GGATCCCCTACGCAGC |
| L-0616 | 3505 | 3520 | 12628 | 12643 | 764 | TCCCCTACCCAGCTGG |
| L-0617 | 3507 | 3522 | 12630 | 12645 | 765 | CCCTACCCAGCTGGAA |
| L-0618 | 3509 | 3524 | 12632 | 12647 | 766 | CTACCCAGCTGGAACT |
| L-0619 | 3511 | 3526 | 12634 | 12649 | 767 | AGCCAGCTGGAACTCT |
| L-0620 | 3513 | 3528 | 12636 | 12651 | 768 | CCAGCTGGAACTCTCC |
| L-0621 | 3531 | 3546 | 12654 | 12669 | 769 | AACCCCCTGCTTCCTC |
| L-0622 | 3542 | 3557 | 12665 | 12680 | 770 | TCCTCACCCTCTGCAC |

### [Evaluation Example 7] Antisense inhibition of human ATN1 in SH-SY5Y cells

An effect of an antisense oligonucleotide on an ATN1 RNA transcript was tested in vitro using the same evaluation method as in Evaluation Example 1. Results thereof are presented in Table 50 as percent expression of ATN1 relative to untreated control cells.

**[Table 50]**

| Cmpd No | ATN1 mRNA remain % |
|---|---|
| L-0574 | 77.2 |
| L-0575 | 40.5 |
| L-0576 | 68.6 |
| L-0579 | 56.0 |
| L-0580 | 71.0 |
| L-0581 | 27.5 |
| L-0582 | 11.2 |
| L-0583 | 33.6 |
| L-0584 | 79.5 |
| L-0585 | 16.5 |
| L-0586 | 14.6 |
| L-0587 | 22.0 |
| L-0588 | 1.8 |
| L-0589 | 2.7 |
| L-0590 | 0.7 |
| L-0591 | 1.2 |
| L-0592 | 11.7 |
| L-0593 | 29.0 |
| L-0594 | 26.6 |
| L-0595 | 4.5 |
| L-0596 | 3.2 |
| L-0597 | 2.1 |
| L-0598 | 15.8 |
| L-0600 | 74.6 |
| L-0602 | 24.8 |
| L-0603 | 7.4 |
| L-0604 | 2.7 |
| L-0605 | 5.0 |
| L-0606 | 11.2 |
| L-0607 | 17.4 |
| L-0608 | 27.6 |
| L-0609 | 30.6 |
| L-0610 | 49.2 |
| L-0611 | 33.7 |
| L-0612 | 42.1 |
| L-0613 | 31.7 |
| L-0614 | 36.1 |
| L-0615 | 24.0 |
| L-0616 | 46.6 |
| L-0617 | 47.5 |
| L-0618 | 45.0 |
| L-0619 | 34.9 |
| L-0620 | 56.9 |
| L-0621 | 30.2 |
| L-0622 | 46.4 |

### (Manufacturing Example 4)

Antisense oligonucleotides (compounds represented by chemical structures corresponding to compound numbers) described in Table 51 were prepared using an automatic nucleic acid synthesizer nS-8II (manufactured by GeneDesign Inc.).

**[Table 51]**

| Cmpd No | Chemical structure |
|---|---|
| L-0623 | G(L)^A(L)G(L)a^g^g^g^t^g^5(x)^a^5(x)^5(x)^G(L)A(L)^G(L) |
| L-0624 | G(L)^A(V)G(L)^a^g^g^g^t^g^5(x)^a^5(x)^5(x)^G(L)A(V)^G(L) |
| L-0625 | G(L)^A(V)G(L)a^g^g^g^t^g^5(x)^a^5(x)^5(x)^G(L)A(V)^G(L) |
| L-0626 | G(L)^A(V)G(L)A(V)g^g^g^t^g^5(x)^a^5(x)^5(x)^G(L)A(V)^G(L) |
| L-0627 | G(L)^A(L)G(V)^a^g^g^t^g^5(x)^a^5(x)^5(x)^G(V)A(L)^G(L) |
| L-0628 | G(L)^A(L)G(V)a^g^g^g^t^g^5(x)^a^5(x)^5(x)^G(V)A(L)^G(L) |
| L-0629 | G(L)^A(L)G(V)A(V)g^g^g^t^g^5(x)^a^5(x)^5(x)^G(V)A(L)^G(L) |
| L-0630 | G(V)^A(L)G(L)A(V)g^g^g^t^g^5(x)^a^5(x)^5(x)^G(L)A(L)^G(V) |
| L-0631 | G(L)^G(L)G(L)t^5(x)^a^a^t^g^a^g^a^g^G(L)G(L)^T(L) |
| L-0632 | G(L)^G(V)G(L)^t^5(x)^a^a^t^g^a^g^a^g^G(L)G(V)^T(L) |
| L-0633 | G(L)^G(V)G(L)t^5(x)^a^a^t^g^a^g^a^g^G(L)G(V)^T(L) |
| L-0634 | G(L)^G(V)G(L)T(V)5(x)^a^a^t^g^a^g^a^g^G(L)G(V)^T(L) |
| L-0635 | G(L)^G(L)G(V)^t^5(x)^a^a^t^g^a^g^a^g^G(V)G(L)^T(L |
| L-0636 | G(L)^G(L)G(V)t^5(x)^a^a^t^g^a^g^a^g^G(V)G(L)^T(L) |
| L-0637 | G(L)^G(L)G(V)T(V)5(x)^a^a^t^g^a^g^a^g^G(V)G(L)^T(L) |
| L-0638 | G(V)^G(L)G(L)^t^5(x)^a^a^t^g^a^g^a^g^G(L)G(L)^T(V) |
| L-0639 | G(V)^G(L)G(L)t^5(x)^a^a^t^g^a^g^a^g^G(L)G(L)^T(V) |
| L-0640 | G(V)^G(L)G(L)T(V)5(x)^a^a^t^g^a^g^a^g^G(L)G(L)^T(V) |
| L-0641 | G(V)^G(L)G(L)t^5(x)^a^a^t^g^a^g^a^G(V)G(L)G(L)^T(V) |
| L -0642 | G(V)^G(L)T(L)^5(x)^a^a^t^g^a^g^a^g^g^G(L)T(L)^G(V) |
| L-0643 | G(V)^G(L)T(L)5(x)^a^a^t^g^a^g^a^g^g^G(L)T(L)^G(V) |
| L-0644 | G(V)^G(L)T(L)5(V)a^a^t^g^a^g^a^g^g^G(L)T(L)^G(V) |
| L-0645 | G(L)^G(L)T(L)5(x)^a^a^t^g^a^a^g^g^G(L)T(L)^G(L) |
| L-0646 | G(V)^G(L)G(L)^g^t^5(x)^a^a^t^g^a^g^aG(L)G(L)^G(V) |
| L-0647 | G(V)^G(L)G(L)g^t^5(x)^a^t^g^a^g^a^G(L)G(L)^G(V |
| L-0648 | G(V)^G(L)G(L)G(V)t^5(x)^a^a^t^g^a^g^a^G(L)G(L)^G(V) |
| L-0649 | G(L)^G(L)G(L)g^t^5(x)^a^a^t^g^a^g^a^G(L)G(L)^G(L) |
| L-0650 | 5(V)^5(L)G(L)^g^g^t^a^a^g^g^t^g^a^G(L)A(L)^5(V) |
| L-0651 | 5(V)^5(L)G(L)g^g^t^a^a^g^g^t^g^a^G(L)A(L)^5(V) |
| L-0652 | 5(V)^5(L)G(L)G(V)g^t^a^a^g^g^t^g^a^G(L)A(L)^5(V) |
| L-0653 | 5(L)^5(L)G(L)g^g^t^a^a^g^g^t^g^aG(L)A(L)^5(L) |

Positions in a sequence of human ATN1 mRNA or pre-mRNA targeted by the antisense oligonucleotides presented in Table 51, and sequence numbers and nucleobase sequences corresponding to the antisense oligonucleotides are presented in Table 52. The notations in Table 52 are the same as those in Tables 11 to 20.

**[Table 52]**

| Cmpd No | SEQ1 START | SEQ1 END | SEQ2 START | SEQ2 END | SEQ No | BASE SEQUENCE |
|---|---|---|---|---|---|---|
| L-0623 | 3452 | 3467 | 12575 | 12590 | 215 | GAGAGGGTGCACCGAG |
| L-0624 | 3452 | 3467 | 12575 | 12590 | 215 | GAGAGGGTGCACCGAG |
| L-0625 | 3452 | 3467 | 12575 | 12590 | 215 | GAGAGGGTGCACCGAG |
| L-0626 | 3452 | 3467 | 12575 | 12590 | 215 | GAGAGGGTGCACCGAG |
| L-0627 | 3452 | 3467 | 12575 | 12590 | 215 | GAGAGGGTGCACCGAG |
| L-0628 | 3452 | 3467 | 12575 | 12590 | 215 | GAGAGGGTGCACCGAG |
| L-0629 | 3452 | 3467 | 12575 | 12590 | 215 | GAGAGGGTGCACCGAG |
| L-0630 | 3452 | 3467 | 12575 | 12590 | 215 | GAGAGGGTGCACCGAG |
| L-0631 | 3460 | 3475 | 12583 | 12598 | 221 | GGGTCAATGAGAGGGT |
| L-0632 | 3460 | 3475 | 12583 | 12598 | 221 | GGGTCAATGAGAGGGT |
| L-0633 | 3460 | 3475 | 12583 | 12598 | 221 | GGGTCAATGAGAGGGT |
| L-0634 | 3460 | 3475 | 12583 | 12598 | 221 | GGGTCAATGAGAGGGT |
| L-0635 | 3460 | 3475 | 12583 | 12598 | 221 | GGGTCAATGAGAGGGT |
| L-0636 | 3460 | 3475 | 12583 | 12598 | 221 | GGGTCAATGAGAGGGT |
| L-0637 | 3460 | 3475 | 12583 | 12598 | 221 | GGGTCAATGAGAGGGT |
| L-0638 | 3460 | 3475 | 12583 | 12598 | 221 | GGGTCAATGAGAGGGT |
| L-0639 | 3460 | 3475 | 12583 | 12598 | 221 | GGGTCAATGAGAGGGT |
| L-0640 | 3460 | 3475 | 12583 | 12598 | 221 | GGGTCAATGAGAGGGT |
| L-0641 | 3460 | 3475 | 12583 | 12598 | 221 | GGGTCAATGAGAGGGT |
| L-0642 | 3459 | 3474 | 12582 | 12597 | 220 | GGTCAATGAGAGGGTG |
| L-0643 | 3459 | 3474 | 12582 | 12597 | 220 | GGTCAATGAGAGGGTG |
| L-0644 | 3459 | 3474 | 12582 | 12597 | 220 | GGTCAATGAGAGGGTG |
| L-0645 | 3459 | 3474 | 12582 | 12597 | 220 | GGTCAATGAGAGGGTG |
| L-0646 | 3461 | 3476 | 12584 | 12599 | 222 | GGGGTCAATGAGAGGG |
| L-0647 | 3461 | 3476 | 12584 | 12599 | 222 | GGGGTCAATGAGAGGG |
| L-0648 | 3461 | 3476 | 12584 | 12599 | 222 | GGGGTCAATGAGAGGG |
| L-0649 | 3461 | 3476 | 12584 | 12599 | 222 | GGGGTCAATGAGAGGG |
| L-0650 | 3488 | 3503 | 12611 | 12626 | 208 | CCGGGTAAGGTGAGAC |
| L-0651 | 3488 | 3503 | 12611 | 12626 | 208 | CCGGGTAAGGTGAGAC |
| L-0652 | 3488 | 3503 | 12611 | 12626 | 208 | CCGGGTAAGGTGAGAC |
| L-0653 | 3488 | 3503 | 12611 | 12626 | 208 | CCGGGTAAGGTGAGAC |

### [Evaluation Example 8] Antisense inhibition of human ATN1 in SH-SY5Y cells

An effect of an antisense oligonucleotide on an ATN1 RNA transcript was tested in vitro using the same evaluation method as in Evaluation Example 1. Results thereof are presented in Table 53 as percent expression of ATN1 relative to untreated control cells.

**[Table 53]**

| Cmpd No | ATN1 mRNA remain % |
|---|---|
| L-0623 | 7.2 |
| L-0624 | 6.7 |
| L-0625 | 13.0 |
| L-0626 | 23.9 |
| L-0627 | 8.6 |
| L-0628 | 25.1 |
| L-0629 | 14.2 |
| L-0630 | 19.8 |
| L-0631 | 1.0 |
| L-0632 | 4.6 |
| L-0633 | 3.8 |
| L-0634 | 3.3 |
| L-0635 | 4.4 |
| L-0636 | 3.9 |
| L-0637 | 2.3 |
| L-0638 | 4.9 |
| L-0639 | 4.0 |
| L-0640 | 2.6 |
| L-0641 | 2.4 |
| L-0642 | 7.9 |
| L-0643 | 7.0 |
| L-0644 | 6.7 |
| L-0645 | 1.7 |
| L-0646 | 7.2 |
| L-0647 | 5.1 |
| L-0648 | 5.7 |
| L-0649 | 4.5 |
| L-0650 | 17.9 |
| L-0651 | 31.5 |
| L-0652 | 42.4 |
| L-0653 | 16.9 |

All publications or parts thereof, patents, and patent applications cited herein are hereby incorporated by reference in their entirety.

### Industrial Applicability

The antisense oligonucleotide of the present invention inhibits expression of an ATN1 gene, and therefore is useful for treating, preventing, and/or ameliorating a disease to which inhibition of expression of an ATN1 gene is effective, particularly dentatorubral-pallidoluysian atrophy.

## Claims

1. A compound comprising a modified oligonucleotide which consists of 8 to 80 nucleosides and has a nucleobase sequence including at least 8 contiguous nucleobases of any one of nucleobase sequences of SEQ ID NOs: 3 to 716 and 718 to 770, or a pharmacologically acceptable salt thereof.

2. The compound or pharmacologically acceptable salt thereof according to claim 1, comprising a modified oligonucleotide which has a nucleobase sequence including any one of nucleobase sequences of SEQ ID NOs: 3 to 716 and 718 to 770.

3. The compound or pharmacologically acceptable salt thereof according to claim 1 or 2, comprising a modified oligonucleotide which has any one of nucleobase sequences of SEQ ID NOs: 3 to 716 and 718 to 770.

4. A compound comprising a modified oligonucleotide which consists of 8 to 80 nucleosides complementary to a part in a nucleobase sequence selected from the group consisting of nucleobase sequences represented by position numbers 59 to 104, 109 to 133, 173 to 192, 207 to 272, 300 to 319, 353 to 372, 419 to 434, 458 to 509, 523 to 559, 561 to 618, 626 to 685, 766 to 785, 787 to 819, 838 to 855, 880 to 922, 924 to 943, 970 to 989, 994 to 1039, 1055 to 1074, 1079 to 1098, 1157 to 1176, 1196 to 1223, 1310 to 1329, 1339 to 1399, 1423 to 1442, 1614 to 1633, 1650 to 1670, 1806 to 1833, 1844 to 1865, 1896 to 1915, 1924 to 1992, 2023 to 2042, 2058 to 2081, 2103 to 2124, 2398 to 2417, 2480 to 2520, 2526 to 2562, 2568 to 2587, 2853 to 2872, 2876 to 2923, 2931 to 2950, 2972 to 3016, 3072 to 3099, 3109 to 3128, 3192 to 3211, 3267 to 3290, 3333 to 3379, 3419 to 3557, 3765 to 3784, 3789 to 3853, 3888 to 3926, 4022 to 4062, 4118 to 4139, 4141 to 4156, 4218 to 4236, and 4266 to 4281 of nucleobases of SEQ ID NO: 1, wherein the modified oligonucleotide is at least 80% complementary to the part in the selected nucleobase sequence of SEQ ID NO: 1, or a pharmacologically acceptable salt thereof.

5. The compound or pharmacologically acceptable salt thereof according to claim 4, comprising a modified oligonucleotide which consists of 8 to 80 nucleosides complementary to a part in a nucleobase sequence represented by position numbers 3419 to 3557 of nucleobases of SEQ ID NO: 1, wherein the modified oligonucleotide is at least 80% complementary to the part in the nucleobase sequence represented by position numbers 3419 to 3557 of the nucleobases of SEQ ID NO: 1.

6. A compound comprising a modified oligonucleotide which consists of 8 to 80 nucleosides complementary to a part in a nucleobase sequence selected from the group consisting of nucleobase sequences represented by position numbers 383 to 398, 986 to 1001, 1004 to 1019, 1378 to 1393, 1398 to 1413, 1853 to 1868, 1971 to 1986, 2189 to 2204, 2522 to 2537, 2562 to 2577, 3662 to 3677, 3987 to 4002, 4217 to 4232, 4266 to 4281, 4335 to 4350, 4360 to 4375, 4477 to 4492, 4562 to 4577, 4636 to 4651, 4687 to 4702, 4734 to 4749, 4840 to 4855, 5536 to 5563, 5568 to 5592, 5632 to 5651, 5666 to 5715, 5906 to 5925, 5959 to 5978, 6176 to 6191, 6215 to 6266, 6963 to 6978, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9336 to 9351, 9534 to 9570, 9576 to 9595, 9923 to 9938, 10001 to 10016, 10056 to 10071, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12508 to 12680, 12723 to 12738, 13454 to 13518, 13553 to 13591, 13687 to 13727, 13783 to 13804, 13806 to 13821, 13883 to 13901, and 13931 to 13946 of nucleobases of SEQ ID NO: 2, wherein the modified oligonucleotide is at least 80% complementary to the part in the selected nucleobase sequence of SEQ ID NO: 2, or a pharmacologically acceptable salt thereof.

7. The compound or pharmacologically acceptable salt thereof according to claim 6, comprising a modified oligonucleotide which consists of 8 to 80 nucleosides complementary to a part in a nucleobase sequence selected from the group consisting of nucleobase sequences represented by position numbers 5536 to 5563, 5568 to 5592, 5632 to 5651, 5666 to 5715, 5906 to 5925, 5959 to 5978, 6176 to 6191, 6215 to 6266, 7248 to 7284, 7286 to 7343, 7351 to 7410, 7491 to 7510, 7512 to 7544, 7563 to 7580, 7605 to 7647, 7649 to 7668, 7695 to 7714, 7719 to 7764, 7780 to 7799, 7804 to 7823, 7882 to 7901, 7921 to 7948, 8035 to 8054, 8064 to 8124, 8148 to 8167, 8339 to 8358, 8375 to 8395, 8531 to 8558, 8569 to 8590, 8621 to 8640, 8649 to 8717, 8748 to 8767, 8783 to 8806, 8828 to 8849, 9123 to 9142, 9205 to 9245, 9534 to 9570, 9576 to 9595, 10274 to 10293, 10297 to 10344, 10352 to 10371, 10393 to 10437, 10493 to 10520, 10530 to 10549, 10613 to 10632, 10688 to 10711, 10754 to 10800, 10840 to 10865, 12572 to 12680, 13454 to 13518, 13553 to 13591, 13687 to 13727, 13783 to 13804, 13806 to 13821, 13883 to 13901, and 13931 to 13946 of nucleobases of SEQ ID NO: 2, wherein the modified oligonucleotide is at least 80% complementary to the part in the selected nucleobase sequence of SEQ ID NO: 2.

8. A compound comprising a modified oligonucleotide which consists of 8 to 80 nucleosides complementary to a part in a nucleobase sequence selected from the group consisting of nucleobase sequences represented by position numbers 4320 to 4390 and 12508 to 12680 of nucleobases of SEQ ID NO: 2, wherein the modified oligonucleotide is at least 80% complementary to the part in the selected nucleobase sequence of SEQ ID NO: 2, or a pharmacologically acceptable salt thereof.

9. The compound or pharmacologically acceptable salt thereof according to claim 6 or 7, comprising a modified oligonucleotide which consists of 8 to 80 nucleosides complementary to a part in a nucleobase sequence selected from the group consisting of nucleobase sequences represented by position numbers 10840 to 10865 and 12572 to 12680 of nucleobases of SEQ ID NO: 2, wherein the modified oligonucleotide is at least 80% complementary to the part in the selected nucleobase sequence of SEQ ID NO: 2.

10. The compound or pharmacologically acceptable salt thereof according to any one of claims 1 to 9, comprising a modified oligonucleotide which consists of 8 to 80 nucleosides and has a nucleobase sequence including at least 8 contiguous nucleobases of any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 494, 513, 515, 533, 534, 544, 551, 552, 554, 566, 574, 582, 585, 586, 589, 592, 593, 595, 596, 638, 639, 642, 644, 661, 662, 663, 664, 665, 666, 667, 668, 669,670, 671, 672, 673, 674, 675, 676, 677, 696, 697, 699, 706, 713, 715, 716, 722, 723, 724, 727, 728, 729, 730, 731, 732, 733, 734, 735, 736, 737, 738, 739, 740, 741, 742, 743, 744, 745, 746, 748, 750, 751, 752, 753, 754, 755, 756, 757, 758, 759, 760, 761, 762, 763, 764, 765, 766, 767, 768, 769, and 770.

11. The compound or pharmacologically acceptable salt thereof according to any one of claims 1 to 10, comprising a modified oligonucleotide which consists of 8 to 80 nucleosides and has a nucleobase sequence including at least 8 contiguous nucleobases of any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.

12. The compound or pharmacologically acceptable salt thereof according to any one of claims 1 to 11, comprising a modified oligonucleotide which consists of 12 to 80 nucleosides and has a nucleobase sequence including any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, 241, 488, 489, 490, 491, 492, 494, 513, 515, 533, 534, 544, 551, 552, 554, 566, 574, 582, 585, 586, 589, 592, 593, 595, 596, 638, 639, 642, 644, 661, 662, 663, 664, 665, 666, 667, 668, 669,670, 671, 672, 673, 674, 675, 676, 677, 696, 697, 699, 706, 713, 715, 716, 722, 723, 724, 727, 728, 729, 730, 731, 732, 733, 734, 735, 736, 737, 738, 739, 740, 741, 742, 743, 744, 745, 746, 748, 750, 751, 752, 753, 754, 755, 756, 757, 758, 759, 760, 761, 762, 763, 764, 765, 766, 767, 768, 769, and 770.

13. The compound or pharmacologically acceptable salt thereof according to any one of claims 1 to 12, comprising a modified oligonucleotide which consists of 12 to 80 nucleosides and has a nucleobase sequence including any one of nucleobase sequences selected from the group consisting of SEQ ID NOs: 3, 4, 9, 10, 12, 13, 18, 19, 20, 21, 26, 29, 34, 37, 38, 39, 40, 44, 45, 46, 47, 48, 49, 50, 52, 55, 59, 60, 61, 62, 63, 66, 67, 68, 69, 70, 72, 74, 75, 78, 79, 82, 83, 84, 85, 86, 87, 88, 89, 91, 92, 94, 95, 97, 99, 101, 102, 103, 104, 105, 106, 107, 108, 109, 110, 111, 112, 113, 114, 115, 116, 117, 118, 119, 120, 121, 122, 123, 124, 126, 127, 128, 131, 132, 133, 137, 138, 139, 148, 151, 152, 153, 154, 156, 157, 158, 159, 160, 161, 162, 163, 164, 165, 166, 167, 168, 169, 170, 171, 172, 173, 174, 175, 176, 177, 178, 179, 180, 181, 183, 184, 185, 186, 187, 188, 189, 190, 191, 192, 193, 194, 195, 196, 197, 198, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 238, 239, 240, and 241.

14. The compound or pharmacologically acceptable salt thereof according to any one of claims 1 to 11, comprising a modified oligonucleotide which consists of 16 to 80 nucleosides and has a nucleobase sequence including any one of nucleobase sequences selected from the group consisting of SEQ ID NOs: 242, 247, 248, 253, 254, 255, 256, 258, 259, 263, 264, 274, 276, 277, 278, 280, 281, 283, 286, 287, 288, 291, 292, 293, 294, 296, 297, 298, 299, 300, 302, 303, 304, 305, 306, 307, 309, 310, 311, 313, 314, 315, 316, 317, 319, 320, 321, 323, 324, 325, 326, 327, 328, 329, 330, 331, 332, 333, 335, 337, 338, 339, 340, 341, 342, 344, 345, 346, 347, 348, 349, 350, 355, 357, 358, 359, 360, 361, 362, 363, 364, 365, 366, 367, 369, 371, 372, 373, 374, 375, 376, 377, 378, 379, 380, 382, 392, 393, 394, 395, 396, 397, 398, 399, 400, 401, 402, 403, 404, 405, 406, 407, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, and 487.

15. The compound or pharmacologically acceptable salt thereof according to any one of claims 1 to 14, wherein the modified oligonucleotide includes a phosphorothioate bond.

16. The compound or pharmacologically acceptable salt thereof according to any one of claims 1 to 15, wherein the modified oligonucleotide includes at least one nucleoside selected from the group consisting of a 2'-modified nucleoside and a 2'-4'-bridged nucleoside.

17. The compound or pharmacologically acceptable salt thereof according to claim 16, wherein the 2'-4'-bridged nucleoside is at least one selected from the group consisting of LNA, ENA, cEt, AmNA, scpBNA, and GuNA.

18. The compound or pharmacologically acceptable salt thereof according to claim 17, wherein the 2'-4'-bridged nucleoside is LNA.

19. The compound or pharmacologically acceptable salt thereof according to any one of claims 16 to 18, wherein the 2'-modified nucleoside is at least one selected from the group consisting of a 2'-O-MCE nucleoside, a 2'-O-MOE nucleoside, a 2'-O-NMA nucleoside, and a 2'-O-Me nucleoside.

20. The compound or pharmacologically acceptable salt thereof according to claim 19, wherein the 2'-modified nucleoside is at least one selected from the group consisting of a 2'-O-MCE nucleoside and a 2'-O-MOE nucleoside.

21. The compound or pharmacologically acceptable salt thereof according to any one of claims 1 to 20, wherein the modified oligonucleotide includes 5-methylcytosine.

22. The compound or pharmacologically acceptable salt thereof according to any one of claims 1 to 21, wherein
the modified oligonucleotide includes a gap segment, a 5' wing segment, and a 3' wing segment,
the gap segment includes at least two deoxyribonucleosides, and a 5'-terminal and a 3'-terminal of the gap segment are deoxyribonucleosides,
the nucleoside at the 3'-terminal of the 5' wing segment is a sugar-modified nucleoside and is linked to the 5'-terminal of the gap segment, and
the nucleoside at the 5'-terminal of the 3' wing segment is a sugar-modified nucleoside and is linked to the 3'-terminal of the gap segment.

23. The compound or pharmacologically acceptable salt thereof according to claim 22, wherein
the gap segment consists of 5 to 30 deoxyribonucleosides,
the 5' wing segment and the 3' wing segment each independently consist of 1 to 10 sugar-modified nucleosides independently selected from the group consisting of LNA, a 2'-O-MCE nucleoside, and a 2'-O-MOE nucleoside,
each of the wing segments includes at least one phosphorothioate bond, and
each of cytosines of the gap segment and each wing segment is replaced with 5-methylcytosine.

24. The compound or pharmacologically acceptable salt thereof according to claim 23, wherein
the gap segment consists of 8 to 12 deoxyribonucleosides,
the 5' wing segment and the 3' wing segment each independently consist of 2 to 5 sugar-modified nucleosides independently selected from the group consisting of LNA and a 2'-O-MCE nucleoside, and include at least one 2'-O-MCE nucleoside, and
the gap segment includes at least one phosphorothioate bond.

25. The compound or pharmacologically acceptable salt thereof according to claim 23, wherein
the gap segment consists of 8 to 12 deoxyribonucleosides,
the 5' wing segment and the 3' wing segment each independently consist of 3 to 6 sugar-modified nucleosides selected from the group consisting of a 2'-O-MOE nucleoside and a 2'-O-MCE nucleoside, and
the gap segment includes at least one phosphorothioate bond.

26. The compound or pharmacologically acceptable salt thereof according to claim 25, wherein the 5' wing segment and the 3' wing segment each independently consist of five 2'-O-MOE nucleosides.

27. The compound or pharmacologically acceptable salt thereof according to claim 25, wherein the 5' wing segment and the 3' wing segment each independently consist of five 2'-O-MCE nucleosides.

28. The compound or pharmacologically acceptable salt thereof according to claim 24, wherein the 5' wing segment and the 3' wing segment are each independently selected from the group consisting of VLL, LVL, LLV, LVV, VLV, VVL, LLL, VVLL, VLVL, VLLV, LVLV, LLVV, LVVL, VLLL, LVLL, LLVL, LLLV, LVVV, VLVV, VVLV, and VVVL, in which L represents LNA, and V represents a 2'-O-MCE nucleoside.

29. The compound or pharmacologically acceptable salt thereof according to claim 24, wherein the 5' wing segment and the 3' wing segment are each independently selected from the group consisting of VLL, LVL, LLV, LVV, VLV, VVL, VVLL, VLVL, LVLV, LLVV, VLLL, LVLL, LLVL, LLLV, LVVV, VLVV, VVLV, and VVVL, in which L represents LNA, and V represents a 2'-O-MCE nucleoside.

30. The compound or pharmacologically acceptable salt thereof according to any one of claims 1 to 29, wherein the modified oligonucleotide consists of 15 to 25 nucleosides.

31. The compound or pharmacologically acceptable salt thereof according to any one of claims 1 to 30, wherein the modified oligonucleotide is an antisense oligonucleotide.

32. The compound or pharmacologically acceptable salt thereof according to any one of claims 1 to 31, wherein the compound including the modified oligonucleotide includes a prodrug moiety.

33. The compound or pharmacologically acceptable salt thereof according to any one of claims 1 to 32, wherein the compound including the modified oligonucleotide includes a functional molecule.

34. The compound or pharmacologically acceptable salt thereof according to any one of claims 1 to 33, consisting of the modified oligonucleotide.

35. The salt according to any one of claims 1 to 34, wherein the pharmacologically acceptable salt is a sodium salt.

36. A medicinal drug comprising, as an active ingredient, the compound or pharmacologically acceptable salt according to any one of claims 1 to 35.

37. A drug for treating, preventing, and/or ameliorating a disease or a condition to which an ATN1 gene expression inhibitory action is effective, the drug comprising, as an active ingredient, the compound or pharmacologically acceptable salt according to any one of claims 1 to 35.

38. An ATN1 gene expression inhibitor comprising, as an active ingredient, the compound or pharmacologically acceptable salt according to any one of claims 1 to 35.

39. A drug for treating, preventing, and/or ameliorating dentatorubral-pallidoluysian atrophy, the drug comprising, as an active ingredient, the compound or pharmacologically acceptable salt according to any one of claims 1 to 35.
